# EUROPEAN PATENT SPECIFICATION

(11) **EP 3 038 595 B1**
(45) Date of publication and mention of the grant of the patent: **14.08.2019**
(21) Application number: 14755659.1
(22) Date of filing: 25.08.2014
(51) Int. Cl.: A61K 8/44, C12N 9/00, C12N 9/10, C12P 13/04

(54) **A METHOD FOR PRODUCING ACYL AMINO ACIDS**
VERFAHREN ZUR HERSTELLUNG VON ACYLAMINOSÄUREN
PROCÉDÉ DE PRODUCTION D'ACIDES ACYL AMINÉS

(30) Priority: 27.08.2013 EP 13181841; 23.05.2014 EP 14169650
(43) Date of publication of application: 06.07.2016
(73) Proprietor: Evonik Degussa GmbH, 45128 Essen (DE)
(72) Inventor: GRAMMANN, Katrin, 45739 Oer-Erkenschwick (DE); WOLTER, Jan, 40489 Düsseldorf (DE); SCHAFFER, Steffen, 45699 Herten (DE); CORTHALS, Jasmin, 44879 Bochum (DE); HAAS, Thomas, 48161 Münster (DE); POTGRAVE, Nicole, 46286 Dorsten (DE); REINECKE, Liv, 45134 Essen (DE); OLFERT, Maik, 45711 Datteln (DE)
(74) Representative: Evonik Patent Association
(86) International application number: PCT/EP2014/067990
(87) International publication number: WO 2015/028423

(56) References cited:
- EP-A1- 0 415 598
- EP-A2- 0 500 332
- WO-A1-2014/144649
- WO-A2-2008/131002
- WO-A2-2012/016944
- WO-A2-2012/025895
- S. F. BRADY ET AL: "Long-Chain N-Acyltyrosine Synthases from Environmental DNA", APPLIED AND ENVIRONMENTAL MICROBIOLOGY, vol. 70, no. 11, 1 November 2004 (2004-11-01), pages 6865-6870, XP55083151, ISSN: 0099-2240, DOI: 10.1128/AEM.70.11.6865-6870.2004
- HUCKLE K R ET AL: "Species variations in the renal and hepatic conjugation of 3-phenoxybenzoic acid with glycine", XENOBIOTICA, TAYLOR AND FRANCIS, LONDON, GB, vol. 11, no. 9, 1 January 1981 (1981-01-01), pages 635-644, XP009181038, ISSN: 0049-8254
- MHASKAR S Y ET AL: "'Synthesis of N-Acyl Amino Acids and Correlation of Structure with Surfactant Properties of Their Sodium Salt'", JOURNAL OF THE AMERICAN OIL CHEMISTS' SOCIETY. (JAOCS) ED.2, SPRINGER, DE, vol. 67, no. 12, 1 December 1990 (1990-12-01), pages 1015-1019, XP008165315, ISSN: 0003-021X, DOI: 10.1007/BF02541868
- EIKO WADA ET AL: "Enzymatic synthesis of N-acyl-l-amino acids in a glycerol-water system using acylase I from pig kidney", JOURNAL OF THE AMERICAN OIL CHEMISTS' SOCIETY, vol. 79, no. 1, 1 January 2002 (2002-01-01), pages 41-46, XP55097537, ISSN: 0003-021X, DOI: 10.1007/s11746-002-0432-7

## Description

The present invention relates to a bacterial cell expressing an amino acid-N-acyl-transferase, which is preferably recombinant, and an acyl-CoA synthetase, which is preferably recombinant, wherein the cell has a deleted, or inactivated gene encoding an enzyme involved in the β-oxidation pathway that results in a reduced fatty acid degradation capacity of the cell, wherein the enzyme involved in the β-oxidation pathway is selected from the group comprising acyl-CoA dehydrogenase, 2,4-dienoyl-CoA reductase, enoyl-CoA hydratase and 3-ketoacyl-CoA thiolase, and the amino acid-N-acyl-transferase is capable of catalysing the conversion of acyl-CoA and an amino acid to an acyl amino acid, a method for producing acyl amino acids, comprising the steps of (a) converting a fatty acid to an acyl CoA using an acyl-CoA synthetase, (b) contacting an amino acid and the acyl-CoA in the presence of an amino acid-N-acyl-transferase, which is preferably isolated and/or recombinant, and/or
(c) hydrogenating acyl residues of the acyl amino acid from (b) wherein the acyl-CoA synthetase and the amino acid-N-acyl-transferase are provided in the form of a bacterial cell and wherein the cell has a deleted, or inactivated gene encoding an enzyme involved in the β-oxidation pathway that results in a reduced fatty acid degradation capacity of the cell, wherein the enzyme involved in the β-oxidation pathway is selected from the group comprising acyl-CoA dehydrogenase, 2,4-dienoyl-CoA reductase, enoyl-CoA hydratase and 3-ketoacyl-CoA thiolase, and the amino acid-N-acyl-transferase is capable of catalysing the conversion of acyl-CoA and an amino acid to an acyl amino acid; wherein the amino acid-N-acyl-transferase is preferably a human amino acid-N-acyl-transferase, or culturing the cell, and a reaction mixture comprising an amino acid-N-acyl-transferase, which is preferably isolated and/or recombinant, an acyl-CoA synthetase, which is preferably isolated and/or recombinant, an amino acid and a fatty acid
wherein the acyl-CoA synthetase and the amino acid-N-acyl-transferase are provided in the form of a bacterial cell and wherein the cell has a deleted, or inactivated gene encoding an enzyme involved in the β-oxidation pathway that results in a reduced fatty acid degradation capacity of the cell, wherein the enzyme involved in the β-oxidation pathway is selected from the group comprising acyl-CoA dehydrogenase, 2,4-dienoyl-CoA reductase, enoyl-CoA hydratase and 3-ketoacyl-CoA thiolase, and the amino acid-N-acyl-transferase is capable of catalysing the conversion of acyl-CoA and an amino acid to an acyl amino acid..

Acyl amino acids are a class of surface-active agents with a variety of uses, for example as detergents for washing purposes, emulsifiers in food products and as essential ingredients in various personal care products such as shampoos, soaps, moisturizing agents and the like. In addition to having both hydrophobic and hydrophilic regions, a prerequisite for use as a surfactant, the compounds (surfactants) are made of naturally occurring molecules, more specifically amino acids and fatty acids, which are not only non-hazardous and environmentally acceptable but may be readily produced at a large scale using inexpensive biological raw materials. In pharmacological research, acyl amino acids are used as neuromodulators and probes for new drug targets.

Acyl amino acids have been isolated from a multitude of biological sources and are believed to have a range of functions, for example as signalling molecules in mammalian tissues (Tan, B., O'Dell, D. K., Yu, Y. W., Monn, M. F., Hughes, H. V., Burstein, S., Walker, J.M. (2010), Identification of endogenous acyl amino acids based on a targeted lipidomics approach, J. Lipid Res. 51(1), 112-119)), as building blocks for antibiotics in bacterial cultures (Clardy, J., and Brady, S. F. (2007), Cyclic AMP directly activates NasP, an N-acyl amino acid antibiotic biosynthetic enzyme cloned from an uncultured beta-proteobacterium, J. Bacteriol. 189(17), 6487-6489) or as compounds involved in bacterial protein sorting (Craig, J. W., Cherry, M. A., Brady, S. F.(2011), Long-chain N-acyl amino acid synthases are linked to the putative PEP-CTERM/exosortase protein-sorting system in Gram-negative bacteria, J. Bacteriol. 193(20), 5707-5715).

Traditionally acyl amino acids have been produced at an industrial scale starting with materials derived from petrochemicals. More specifically, activated fatty acids provided in the form of acid chlorides may be used to acylate amino acids in an aqueous alkaline medium as described in GB 1 483 500. Shortcomings of such approaches include the need to add hazardous chemicals such as sulphuric acid or anhydrides thereof. Other synthetic approaches are associated with the accumulation of by-products such as chloride salts which have undesirable effects on surfactancy.

A range of biotechnological routes towards production of acyl amino acids has been described. However, none of them is adequate for the commercial large-scale production of acyl amino acids owing to low yields, insufficient purities and the need for multi-step purification procedures. In particular, only a small proportion of the carbon substrates fed to biotechnologically useful organisms is actually converted to the sought-after product, whilst much of it is consumed by reactions of the primary metabolism.

Another problem associated with biotechnological routes is the fact that a mixture of products is obtained and thus the composition is difficult to control. More specifically, a range of fatty acids may be converted to acyl amino acids, even though production of a single adduct may be desirable. Since the mixture comprises compounds highly related in terms of chemical structure, purifying or at least enriching a single component in an efficient and straightforward manner is usually beyond technical feasibility.

These problems may be solved by the subject matter of the attached claims. In particular, the present invention may provide an efficient biotechnological route towards acyl amino acids. In particular, the yield and purity of the product of the present invention, in terms of catalysts or unwanted by-products, may be improved compared to the processes in the state of the art.

The present invention also provides a method for making acyl amino acids, wherein the spectrum of fatty acids converted to acyl amino acids is broader than the processes in the state of the art. In particular, the method of the present invention may be suitable for converting short and unsaturated fatty acids to acyl amino acids.

The present invention may also provide a biotechnological method for making acyl amino acids, wherein the length of the acyl residue in the acyl amino acid product may be controlled, preferably such that lauryl is enriched or prevalent.

In a first aspect, the present invention provides a cell expressing an amino acid-N-acyl-transferase, which may be preferably recombinant, and an acyl-CoA synthetase, which may be preferably recombinant, wherein the cell has a deleted, or inactivated gene encoding an enzyme involved in the β-oxidation pathway that results in a reduced fatty acid degradation capacity of the cell, wherein the enzyme involved in the β-oxidation pathway is selected from the group comprising acyl-CoA dehydrogenase, 2,4-dienoyl-CoA reductase, enoyl-CoA hydratase and 3-ketoacyl-CoA thiolase, and the amino acid-N-acyl-transferase is capable of catalysing the conversion of acyl-CoA and an amino acid to an acyl amino acid,. In particular, the cell may compared to the wild type cell, have increased expression of amino acid-N-acyl-transferase and/or acyl-CoA synthetase. In a first embodiment of the first aspect, wherein the amino acid-N-acyl-transferase is SEQ ID NO: 4, SEQ ID NO: 5 or a variant thereof and the variant comprises a sequence at least 70% identical to SEQ ID NO: 4 or SEQ ID NO 5. In particular, the amino acid-N-acyl-transferase may have a polypeptide sequence comprising SEQ ID NO: 4, SEQ ID NO: 5 or a variant thereof.

In a second embodiment, which is also an embodiment of the first embodiment, the cell may be an enterobacterial cell, more preferably *E. coli.*

In a third embodiment, which is also an embodiment of the first to second embodiments, the cell may be capable of making proteinogenic amino acids and/or fatty acids. In particular, the cell comprises at least one enzyme selected from the group consisting of β-ketoacyl-ACP synthase I, 3-oxoacyl-ACP-synthase I, Malonyl-CoA-ACP transacylase, enoyl ACP reductase, and β-ketoacyl-ACP synthase III capable of making proteinogenic amino acids and/or fatty acids. In a fourth embodiment, which is also an embodiment of the first to third embodiments, the cell expresses an acyl-CoA thioesterase, which is preferably recombinant and is more preferably SEQ ID NO: 1 or a variant thereof.

In a fifth embodiment, which is also an embodiment of the first to fourth embodiments, the acyl-CoA synthetase may be SEQ ID NO: 6 or a variant thereof and the variant comprises a sequence at least 70% identical to SEQ ID NO: 6.

In a second aspect the present invention provides a method for producing acyl amino acids, comprising the steps of
a) converting a fatty acid to an acyl CoA using an acyl-CoA synthetase, and
b) contacting an amino acid and the acyl CoA in the presence of an amino acid-N-acyl-transferase, which is capable of catalysing the conversion of acyl-CoA and an amino acid to the acyl amino acid, and/or which may be preferably isolated and/or recombinant,
c) hydrogenating acyl residues of the acyl amino acid from b)
   wherein the acyl-CoA synthetase and the amino acid-N-acyl-transferase are provided in the form of a bacterial cell and wherein the cell has a deleted, or inactivated gene encoding an enzyme involved in the β-oxidation pathway that results in a reduced fatty acid degradation capacity of the cell, wherein the enzyme involved in the β-oxidation pathway is selected from the group comprising acyl-CoA dehydrogenase, 2,4-dienoyl-CoA reductase, enoyl-CoA hydratase and 3-ketoacyl-CoA thiolase, and the amino acid-N-acyl-transferase is capable of catalysing the conversion of acyl-CoA and an amino acid to an acyl amino acid; or culturing the cell according to the first aspect or any embodiment thereof. In a first embodiment of the second aspect, the amino acid-N-acyl-transferase is SEQ ID NO: 4, SEQ ID NO: 5 or a variant thereof and the variant comprises a sequence at least 70% identical to SEQ ID NO: 4 or SEQ ID NO 5, and the acyl-CoA synthetase is SEQ ID NO:6 or a variant thereof and the variant comprises a sequence at least 70% identical to SEQ ID NO: 6.. In a third aspect the present invention provides a reaction mixture comprising
   an amino acid-N-acyl-transferase, which may be preferably isolated and/or recombinant, an acyl-CoA synthetase, which may be preferably isolated and/or recombinant,
   an amino acid and
   a fatty acid,wherein the acyl-CoA synthetase and the amino acid-N-acyl-transferase are provided in the form of a bacterial cell and wherein the cell has a deleted, or inactivated gene encoding an enzyme involved in the β-oxidation pathway that results in a reduced fatty acid degradation capacity of the cell,
   wherein the enzyme involved in the β-oxidation pathway is selected from the group comprising acyl-CoA dehydrogenase, 2,4-dienoyl-CoA reductase, enoyl-CoA hydratase and 3-ketoacyl-CoA thiolase, and
   the amino acid-N-acyl-transferase is capable of catalysing the conversion of acyl-CoA and an amino acid to an acyl amino acid

In a first embodiment of the third aspect, the amino acid-N-acyl-transferase may preferably be a human amino acid-N-acyl-transferase, more preferably SEQ ID NO: 4, SEQ ID NO: 5 or a variant thereof,
and wherein the acyl-CoA synthetase is preferably SEQ ID NO: 6 or a variant thereof.

In a second embodiment, which is also an embodiment of the first embodiment, the amino acid may be a proteinogenic amino acid, preferably selected from the group consisting of glycine, glutamine, glutamate, asparagine and alanine and may be more preferably glycine.

In a third embodiment, which is also an embodiment of the first to second embodiments, the fatty acid may be an unsaturated fatty acid and may be preferably selected from the group consisting of myristoleic acid, lauroleic acid, palmitoleic acid and cis-vaccenic acid.

In another embodiment of the second or third aspect, the fatty acid may be a saturated fatty acid and may be preferably selected from the group consisting of laurate, myristate and palmitate.

In another embodiment of the second or third aspect, the fatty acid may be provided in the form of an organic phase comprising a liquid organic solvent and the fatty acid, wherein the organic solvent may be preferably an ester of the fatty acid.

According to any aspect of the present invention, the acyl amino acids formed may be a mixture of amino acids. The mixture of amino acids may comprise at least two proteinogenic amino acids as defined below. In particular, the mixture of amino acids may have 3, 4, 5, 6, 7, 8, 9, 10, 11, 12, 13, 14, 15, 16, 17, 18, 19, 20, or 21 proteinogenic amino acids. In one example, the mixture of amino acids may be used to form cocoyl glycine and salts thereof.

The present invention is based on the surprising finding that the combination of amino acid-N-acyl transferase and an acyl-CoA synthetase, preferably expressed by a cell having reduced fatty acid degradation capacity, may be used to convert a variety of fatty acids, more preferably a mixture comprising unsaturated and saturated fatty acids, to acyl amino acids.

Moreover, the present invention is based on the surprising finding that there are amino acid-N-acyl transferases that may be used to convert short unsaturated fatty acids such as lauroleic acid to an acyl amino acid.

Moreover, the present invention is based on the surprising finding that employing an amino acid-N-acyl-transferase capable of converting a variety of fatty acids including short unsaturated fatty acids such as lauroleic acid to an acyl amino acid may increase the yields of acyl amino acids produced.

Moreover, the present invention is based on the surprising finding that the composition of acyl amino acids produced in a cell, more specifically the length of fatty acids incorporated into such acyl amino acids, may be controlled by introducing into the cell one or more specific acyl-CoA thioesterases or altering the expression of one or more acyl-CoA thioesterases endogenously expressed by the cell.

The present invention centres around the use of an amino acid-N-acyl transferase and an acyl-CoA synthetase for making acyl amino acids. In a preferred embodiment, the term "amino acid-N-acyl transferase", as used herein, refers to an enzyme capable of catalysing the conversion of acyl-CoA, preferably the CoA ester of lauroleic acid, and an amino acid, preferably a proteinogenic amino acid, more preferably glycine, to an acyl amino acid. Suitable amino acid-N-acyl transferases have been described in the prior art, for example in Waluk, D. P., Schultz, N., and Hunt, M. C. (2010), Identification of glycine N-acyltransferase-like 2 (GLYATL2) as a transferase that produces N-acyl glycines in humans, FASEB J. 24, 2795-2803. In a preferred embodiment, the amino acid-N-acyl transferase comprises a nucleotide sequence of SEQ ID NO:4. In particular, the amino acid sequence of amino acid-N-acyl transferase may be selected from the group consisting of NP_001010904.1,NP_659453.3, XP_001147054.1, AAH16789.1, AAO73139.1, XP_003275392.1, XP_002755356.1, XP_003920208.1, XP_004051278.1, XP_006147456.1, XP_006214970.1, XP_003801413.1, XP_006189704.1, XP_003993512.1, XP_005862181.1, XP_007092708.1, XP_006772167.1, XP_006091892.1, XP_005660936.1, XP_005911029.1, NP_001178259.1, XP_004016547.1, XP_005954684.1, ELR45061.1, XP_005690354.1, XP_004409352.1, XP_007519553.1, XP_004777729.1, XP_005660935.1, XP_004824058.1, XP_006068141.1, XP_006900486.1, XP_007497585.1, XP_002821801.2, XP_007497583.1, XP_003774260.1, XP_001377648.2, XP_003909843.1, XP_003801448.1, XP_001091958.1, XP_002821798.1, XP_005577840.1, XP_001092197.1, NP_001207423.1, NP_001207425.1, XP_003954287.1, NP_001271595.1, XP_003909848.1, XP_004087850.1, XP_004051279.1, XP_003920209.1, XP_005577835.1, XP_003774402.1, XP_003909846.1, XP_004389401.1, XP_002821802.1, XP_003774401.1, XP_007497581.1, EHH21814.1, XP_003909845.1, XP_005577839.1, XP_003774403.1, XP_001092427.1, XP_003275395.2, NP_542392.2, XP_001147271.1, XP_005577837.1, XP_003826420.1, XP_004051281.1, XP_001147649.2, XP_003826678.1, XP_003909847.1, XP_004682812.1, XP_004682811.1, XP_003734315.1, XP_004715052.1, BAG62195.1, XP_003777804.1, XP_003909849.1, XP_001092316.2, XP_006167891.1, XP_540580.2, XP_001512426.1, EAW73833.1, XP_003464217.1, XP_007519551.1, XP_003774037.1, XP_005954680.1, XP_003801411.1, NP_803479.1, XP_004437460.1, XP_006875830.1, XP_004328969.1, XP_004264206.1, XP_004683490.1, XP_004777683.1, XP_005954681.1, XP_003480745.1, XP_004777682.1, XP_004878093.1, XP_007519550.1, XP_003421399.1, EHH53167.1, XP_006172214.1 ,XP_003993453.1 ,AAI12537.1, XP_006189705.1 ,Q2KIR7.2, XP_003421465.1, NP_001009648.1, XP_003464328.1, XP_001504745.1, ELV11036.1, XP_005690351.1, XP_005216632.1, EPY77465.1, XP_005690352.1, XP_004016544.1, XP_001498276.2, XP_004264205.1, XP_005690353.1, XP_005954683.1, XP_004667759.1, XP_004479306.1, XP_004645843.1, XP_004016543.1, XP_002928268.1, XP_006091904.1, XP_005331614.1, XP_007196549.1, XP_007092705.1, XP_004620532.1, XP_004869789.1, EHA98800.1, XP_004016545.1, XP_004479307.1, XP_004093105.1, NP_001095518.1, XP_005408101.1, XP₋004409350. 1, XP_001498290.1, XP_006056693.1, XP_005216639.1, XP_007455745.1, XP_005352049.1, XP_004328970.1, XP_002709220.1, XP_004878092.1, XP_007196553.1, XP_006996816.1, XP_005331615.1, XP_006772157.1, XP_007196552.1, XP_004016546.1, XP_007628721.1, NP_803452.1, XP_004479304.1, DAA21601.1, XP_003920207.1, XP_006091906.1, XP_003464227.1, XP_006091903.1, XP_006189706.1, XP_007455744.1, XP_004585544.1, XP_003801410.1, XP_007124812.1, XP_006900488.1, XP_004777680.1, XP_005907436.1, XP_004389356.1, XP_007124811.1, XP_005660937.1, XP_007628724.1, XP_003513512.1, XP_004437813.1, XP_007628723.1, ERE78858.1, EPQ15380.1, XP_005862178.1, XP_005878672.1, XP_540581.1, XP_002928267.1, XP_004645845.1, EPQ05184.1, XP_003513511.1, XP_006214972.1, XP_007196545.1, XP_007196547.1, XP_006772160.1, XP_003801409.1, NP_001119750.1, XP_003801412.1, XP_006772159.1, EAW73832.1, XP_006091897.1, XP_006772163.1, XP_006091898.1, XP_005408105.1, XP_006900487.1, XP_003993454.1, XP_003122754.3, XP_007455746.1, XP_005331618.1, XP_004585337.1, XP_005063305.1, XP_006091895.1, XP_006772156.1, XP_004051276.1, XP_004683488.1, NP_666047.1, NP_001013784.2, XP_006996815.1, XP_006996821.1, XP_006091893.1, XP_006173036.1, XP_006214971.1, EPY89845.1, XP_003826423.1, NP_964011.2, XP_007092707.1, XP_005063858.1, BAL43174.1, XP_001161154.2, XP_007124813.1, NP_083826.1XP_003464239.1, XP_003275394.1, ELK23978.1, XP_004878097.1, XP_004878098.1, XP_004437459.1, XP_004264204.1, XP_004409351.1, XP_005352047.1, Q5RFP0.1, XP_005408107.1, XP_007659164.1, XP_003909852.1, XP_002755355.1, NP_001126806.1, AAP92593.1, NP_001244199.1, BAA34427.1, XP_005063859.1, NP_599157.2, XP_004667761.1, XP_006900489.1, XP_006215013.1, XP_005408100.1, XP_007628718.1, XP_003514769.1, XP_006160935.1, XP_004683489.1, XP_003464329.1, XP_004921258.1, XP_003801447.1, XP_006167892.1, XP_004921305.1, AAH89619.1, XP_004706162.1, XP_003583243.1, EFB16804.1, XP_006728603.1, EPQ05185.1, XP_002709040.1, XP_006875861.1, XP_005408103.1, XP_004391425.1, EDL41477.1, XP_006772158.1, EGW06527.1, AAH15294.1, XP_006772162.1, XP_005660939.1, XP_005352050.1, XP_006091901.1, XP_005878675.1, XP_004051323.1, EHA98803.1, XP_003779925.1, EDM12924.1, XP_003421400.1, XP_006160939.1, XP_006160938.1, XP_006160937.1, XP_006160936.1, XP_005702185.1, XP_005313023.1, XP_003769190.1, XP_002714424.1, XP_004715051.1, XP_007661593.1, XP_004590594.1, ELK23975.1, XP_004674085.1, XP_004780477.1, XP_006231186.1, XP_003803573.1, XP_004803176.1, EFB16803.1, XP_006056694.1, XP_005441626.1, XP_005318647.1XP_004605904.1, XP_005862182.1, XP_003430682.1, XP_004780478.1, XP_005239278.1, XP_003897760.1, XP_007484121.1, XP_004892683.1, XP_004414286.1, XP_006927013.1, XP_003923145.1, XP_852587.2, AAP97178.1, EHH53105.1, XP_005408113.1, XP_002915474.1, XP_005377590.1, XP_527404.2, XP_005552830.1, XP_004044211.1, NP_001180996.1, XP_003513513.2, XP_001498599.2, XP_002746654.1, XP_005072349.1, XP_006149181.1, EAX04334.1, XP_003833230.1, XP_005216635.1, XP_003404197.1, XP_007523363.1, XP_007433902.1, XP_003254235.1, XP_004471242.1, XP_005216634.1, XP_006860675.1, XP_004771956.1, XP_006038833.1, NP_001138534.1, XP_007068532.1, XP_003510714.1, ERE87950.1, XP_003986313.1, XP_006728644.1, XP_004878099.1, XP_003468014.1, XP_007095614.1, XP_004648849.1, XP_004869795.1, XP_004018927.1, XP_005696454.1, XP_006201985.1, XP_005960697.1, XP_004813725.1, XP_005496926.1, ELR45088.1, XP_004696625.1, XP_005860982.1, XP_005911003.1, XP_006260162.1, EPQ04414.1, XP_006099775.1, NP_001138532.1, XP_006190795.1, XP_004649775.1, XP_004424497.1, XP_004390885.1, XP_005911004.1, XP_003777803.1, XP_004312259.1, XP_005529140.1, XP_005314582.1, XP_006926523.1, XP_006926522.1, XP_004683491.1, XP_003826680.1, XP_003215018.1, XP_003215087.1, EGW12611.1, XP_006113023.1, XP_006882182.1, XP_007425200.1, XP_006041342.1, NP_001138533.1, EMP27694.1, XP_007497753.1, XP_006034252.1, or a variant thereof. Throughout this application, any data base code, unless specified to the contrary, refers to a sequence available from the NCBI data bases, more specifically the version online on 5^{th} August 2013, and comprises, if such sequence is a nucleotide sequence, the polypeptide sequence obtained by translating the former.

In a preferred embodiment, the term "acyl amino acid", as used herein, refers to the product of the reaction catalysed by an amino acid-N-acyl transferase, more preferably a compound represented by the formula acyl-CO-NH-CHR-COOH, wherein R is the side chain of a proteinogenic amino acid, and wherein the term "acyl" refers to the acyl residue of a fatty acid. In a preferred embodiment of the present invention, the term "fatty acid", as used herein, means a carboxylic acid, preferably alkanoic acid, with at least 6, preferably 8, more preferably 10, most preferably 12 carbon atoms. In a preferred embodiment it is a linear fatty acid, in another embodiment it is branched. In a preferred embodiment it is a saturated fatty acid. In an especially preferred embodiment it is unsaturated. In another preferred embodiment it is a linear fatty acid with at least 12 carbon atoms comprising a double bond, preferably at position 9. In another preferred embodiment it is a simple unsaturated fatty acid having one double bond, which double bond is located at position 9 or 11. In the most preferred embodiment it is lauroleic acid (9-dodecenoic acid). In an especially preferred embodiment it is a fatty acid with 6, 7, 8, 9, 10, 11, 12, 13, 14, 15, 16, 17, 18, 19, 20, 21, 22, 23, 24, 25, 26, 27, 28, 29, 30 carbon atoms, preferably 12 carbon atoms.

Throughout this application, a formula referring to a chemical group that represents the dissociated or undissociated state of a compound capable of dissociating in an aqueous solution, comprises both the dissociated and the undissociated state and the various salt forms of the group. For example, the residue -COOH comprises both the protonated (-COOH) as well as the unprotonated (-COO⁻) carboxylic acid.

The acyl-CoA substrate consumed in the inventive reaction may be purified from a cell, chemically synthesised or produced using an acyl-CoA synthetase, the latter being the preferred option. In a preferred embodiment, the term "acyl-CoA synthetase", as used herein, refers to an enzyme capable of catalysing the ATP-dependent conversion of a fatty acid and CoA to acyl CoA.

According to any aspect of the present invention, the term 'acyl-CoA synthetase' may refer to an acyl-CoA/ACP synthetase that may be capable of producing acyl glycinates and/or catalysing the following reaction:

fatty acid + CoA/ACP + ATP → acyl-CoA/ACP + ADP + Pi

Examples of acyl-CoA/ACP synthetases may include EC 6.2.1.3, EC 6.2.1.10, EC 6.2.1.15, EC 6.2.1.20 and the like. The state of the art describes various methods to detect acyl-CoA synthetase activity. For example, the activity of an acyl-CoA synthetase may be assayed by incubating the sample of interest in 100 mM Tris-HCI at pH 8 in the presence of 250 µM lauroyl-CoA, 500 µM glycine and DTNB (5,5'-dithiobis-2-nitrobenzoic acid, also referred to as Ellman's reagent) and spectrophotometrically monitoring the absorbance at 410 nm following release of free thiol groups in the form of CoASH as the reaction progresses and reaction with Ellman's reagent.

Various acyl-CoA synthetases have been described in the state of the art, for example YP_001724804.1, WP_001563489.1 and NP_707317.1. In a preferred embodiment, the acyl-CoA synthetase comprises SEQ ID NO 6 or YP_001724804.1 or a variant thereof. The activity of an acyl-CoA synthetase may be assayed as described in the state of the art, for example Kang, Y., Zarzycki-Siek, J., Walton, C. B., Norris, M. H., and Hoang, T. T. (2010), Multiple FadD Acyl-CoA Synthetases Contribute to Differential Fatty Acid Degradation and Virulence in Pseudomonas aeruginosa, PLOS ONE 5 (10), e13557. Briefly, the amount of free thiol in the form of unreacted CoASH is determined by adding Ellmann's reagent and spectrophotometrically monitoring the absorbance at 410 nm, preferably in a reaction buffer comprising 150 mM Tris-HCI (pH 7.2), 10 mM MgCl₂, 2 mM EDTA, 0.1 % Triton X-100, 5 mM ATP, 0.5 mM coenzyme A (CoASH) and a fatty acid (30 to 300 mM).

In one example, the cell according to any aspect of the present invention may be genetically modified to overexpress at least the enzymes amino acid-N-acyl transferase and acyl-CoA synthetase. In particular, the cell may over express enzymes glycine N-acyl transferase and acyl-CoA/ACP synthetase.

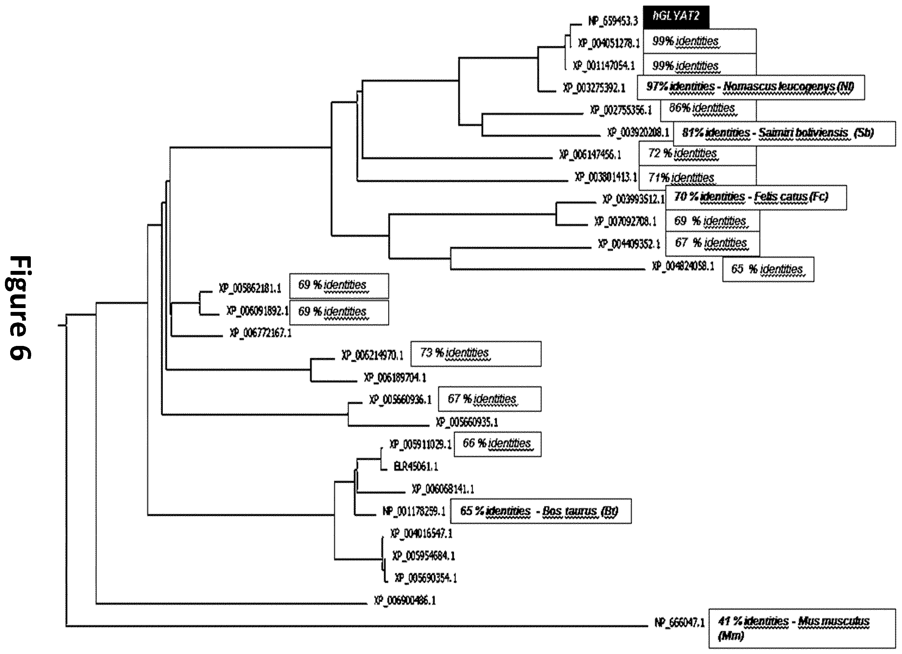

The teachings of the present invention may not only be carried out using biological macromolecules having the exact amino acid or nucleic acid sequences referred to in this application explicitly, for example by name or accession number, or implicitly, but also using variants of such sequences. In a preferred embodiment, the term "variant", as used herein, comprises amino acid or nucleic acid sequences, respectively, that are at least 70, 75, 80, 85, 90, 92, 94, 95, 96, 97, 98 or 99 % identical to the reference amino acid or nucleic acid sequence, wherein preferably amino acids other than those essential for the function, for example the catalytic activity of a protein, or the fold or structure of a molecule are deleted, substituted or replaced by insertions or essential amino acids are replaced in a conservative manner to the effect that the biological activity of the reference sequence or a molecule derived therefrom is preserved. The state of the art comprises algorithms that may be used to align two given nucleic acid or amino acid sequences and to calculate the degree of identity, see Arthur Lesk (2008), Introduction to bioinformatics, 3rd edition, Thompson et al., Nucleic Acids Research 22, 4637-4680, 1994, and Katoh et al., Genome Information, 16(1), 22-33, 2005. The term "variant" is used synonymously and interchangeably with the term "homologue". Such variants may be prepared by introducing deletions, insertions or substitutions in amino acid or nucleic acid sequences as well as fusions comprising such macromolecules or variants thereof. In a preferred embodiment, the term "variant", with regard to amino acid sequence, comprises, preferably in addition to the above sequence identity, amino acid sequences that comprise one or more conservative amino acid changes with respect to the respective reference or wild type sequence or comprises nucleic acid sequences encoding amino acid sequences that comprise one or more conservative amino acid changes. In a preferred embodiment, the term "variant" of an amino acid sequence or nucleic acid sequence comprises, preferably in addition to the above degree of sequence identity, any active portion and/or fragment of the amino acid sequence or nucleic acid sequence, respectively, or any nucleic acid sequence encoding an active portion and/or fragment of an amino acid sequence. In a preferred embodiment, the term "active portion", as used herein, refers to an amino acid sequence or a nucleic acid sequence, which is less than the full length amino acid sequence or codes for less than the full length amino acid sequence, respectively, wherein the amino acid sequence or the amino acid sequence encoded, respectively retains at least some of its essential biological activity. For example an active portion and/or fragment of a protease is capable of hydrolysing peptide bonds in polypeptides. In a preferred embodiment, the term "retains at least some of its essential biological activity", as used herein, means that the amino acid sequence in question has a biological activity exceeding and distinct from the background activity and the kinetic parameters characterising said activity, more specifically k_{cat} and K_{M}, are preferably within 3, more preferably 2, most preferably one order of magnitude of the values displayed by the reference molecule with respect to a specific substrate. In a preferred embodiment, the term "variant" of a nucleic acid comprises nucleic acids the complementary strand of which hybridises, preferably under stringent conditions, to the reference or wild type nucleic acid. Examples of variants of amino acid-N-acyl-transferases may at least be provided in Figure 6. A skilled person would be able to easily determine the amino acid-N-acyl-transferases that will be capable of making proteinogenic amino acids and/or fatty acids. In particular, the variants may include but are not limited to an amino acid-N-acyl-transferase selected from the group of organisms consisting of *Nomascus leucogenys* (NI, XP_003275392.1, SEQ ID No: 53), *Saimiri boliviensis* (Sb, XP_003920208.1, SEQ ID No: 54), *Felis catus* (Fc, XP_003993512.1, SEQ ID No 55), *Bos taurus* (Bt, NP_001178259.1, SEQ ID No: 56), *Mus musculus* (Mm, NP_666047.1, SEQ ID No: 57).

Stringency of hybridisation reactions is readily determinable by one of ordinary skilled in the art, and generally is an empirical calculation dependent on probe length, washing temperature and salt concentration. In general, longer probes require higher temperatures for proper annealing, while shorter probes need lower temperatures. Hybridisation generally depends on the ability of denatured DNA to reanneal to complementary strands when present in an environment below their melting temperature. The higher the degree of desired homology between the probe and hybridisable sequence, the higher the relative temperature which may be used. As a result it follows that higher relative temperatures would tend to make the reaction conditions more stringent, while lower temperature less so. For additional details and explanation of stringency of hybridisation reactions, see F. M. Ausubel (1995), Current Protocols in Molecular Biology. John Wiley & Sons, Inc. Moreover, the person skilled take in the art may follow the instructions given in the manual "The DIG System Users Guide for Filter Hybridization", Boehringer Mannheim GmbH, Mannheim, Germany, 1993 and in Liebl et al. (International Journal of Systematic Bacteriology 41: 255-260 (1991) on how to identify DNA sequences by means of hybridisation. In a preferred embodiment, stringent conditions are applied for any hybridisation, i.e. hybridisation occurs only if the probe is 70 % or more identical to the target sequence. Probes having a lower degree of identity with respect to the target sequence may hybridise, but such hybrids are unstable and will be removed in a washing step under stringent conditions, for example lowering the concentration of salt to 2 x SSC or, optionally and subsequently, to 0,5 x SSC, while the temperature is, in order of increasing preference, approximately 50 °C - 68 °C, approximately 52 °C - 68 °C, approximately 54 °C - 68 °C, approximately 56 °C - 68 °C, approximately 58 °C - 68 °C, approximately 60 °C - 68 °C, approximately 62 °C - 68 °C, approximately 64 °C - 68 °C, approximately 66 °C - 68 °C. In a particularly preferred embodiment, the temperature is approximately 64 °C - 68 °C or approximately 66 °C - 68 °C. It is possible to adjust the concentration of salt to 0.2 x SSC or even 0.1 x SSC. Polynucleotide fragments having a degree of identity with respect to the reference or wild type sequence of at least 70, 80, 90, 91, 92, 93, 94, 95, 96, 97, 98, 99 % may be isolated. In a preferred embodiment, the term "homologue" of a nucleic acid sequence, as used herein, refers to any nucleic acid sequence that encodes the same amino acid sequence as the reference nucleic acid sequence, in line with the degeneracy of the genetic code.

The enzymes used to practise the present invention are provided in the form of a cell, and the cell has a deleted, or inactivated gene encoding an enzyme involved in the β-oxidation pathway that results in a reduced fatty acid degradation capacity. In a preferred embodiment, the term "having a reduced fatty acid degradation capacity", as used herein, means that the respective cell degrades fatty acids, preferably those taken up from the environment, at a lower rate than a comparable cell or wild type cell having normal fatty acid degradation capacity would under identical conditions. In a preferred embodiment, the fatty acid degradation of such a cell is lower on account of deletion, or inactivation of at least one gene encoding an enzyme involved in the β-oxidation pathway. In a preferred embodiment of the present invention, at least one enzyme involved in the β-oxidation pathway has lost, its activity relative to the activity of the same enzyme under comparable conditions in the respective wild type microorganism. The person skilled in the art is familiar with various techniques that may be used to delete a gene encoding an enzyme or reduce the activity of such an enzyme in a cell, for example by exposition of cells to radioactivity followed by accumulation or screening of the resulting mutants, site-directed introduction of point mutations or knock out of a chromosomally integrated gene encoding for an active enzyme, as described in Sambrook/Fritsch/Maniatis (1989). In addition, the transcriptional repressor FadR may be over expressed to the effect that expression of enzymes involved in the β-oxidation pathway is repressed (Fujita, Y., Matsuoka, H., and Hirooka, K. (2007) Mol. Microbiology 66(4), 829-839). In a preferred embodiment, the term "deletion of a gene", as used herein, means that the nucleic acid sequence encoding said gene is modified such that the expression of active polypeptide encoded by said gene is reduced. For example, the gene may be deleted by removing in-frame a part of the sequence comprising the sequence encoding for the catalytic active centre of the polypeptide. Alternatively, the ribosome binding site may be altered such that the ribosomes no longer translate the corresponding RNA. Moreover, the person skilled in the art is able to routinely measure the activity of enzymes expressed by living cells using standard essays as described in enzymology text books, for example Cornish-Bowden (1995), Fundamentals of Enzyme Kinetics, Portland Press Limited, 1995.

Degradation of fatty acids is accomplished by a sequence of enzymatically catalysed reactions. First of all, fatty acids are taken up and translocated across the cell membrane *via* a transport/acyl-activation mechanism involving at least one outer membrane protein and one inner membrane-associated protein which has fatty acid-CoA ligase activity, referred to in the case of *E. coli* as FadL and FadD / FadK, respectively. Inside the cell, the fatty acid to be degraded is subjected to enzymes catalysing other reactions of the β-oxidation pathway. The first intracellular step involves the conversion of acyl-CoA to enoyl-CoA through acyl-CoA dehydrogenase, the latter referred to as FadE in the case of *E. coli.* The activity of an acyl-CoA dehydrogenase may be assayed as described in the state of art, for example by monitoring the concentration of NADH spectrophotometrically at 340 nm in 100 mM MOPS, pH 7.4, 0.2 mM Enoyl-CoA, 0.4 mM NAD⁺. The resulting enoyl-CoA is converted to 3-ketoacyl-CoA *via* 3-hydroxylacyl-CoA through hydration and oxidation, catalysed by enoyl-CoA hydratase/3-hydroxyacyl-CoA dehydrogenase, referred to as FadB and FadJ in *E. coli.* Enoyl-CoA hydratase/3-hydroxyacyl-CoA dehydrogenase activity, more specifically formation of the product NADH may be assayed spectrophotometrically as described in the state of the art, for example as outlined for FadE. Finally, 3-ketoacyl-CoA thiolase, FadA and Fadl in *E. coli,* catalyses the cleavage of 3-ketoacyl-CoA, to give acetyl-CoA and the input acyl-CoA shortened by two carbon atoms. The activity of ketoacyl-CoA thiolase may be assayed as described in the state of the art, for example in Antonenkov, V., D.. Van Veldhoven, P., P., Waelkens, E., and Mannaerts, G.P. (1997) Substrate specificities of 3-oxoacyl-CoA thiolase and sterol carrier protein 2/3-oxoacyl-coa thiolase purified from normal rat liver peroxisomes. Sterol carrier protein 2/3-oxoacyl-CoA thiolase is involved in the metabolism of 2-methyl-branched fatty acids and bile acid intermediates. J. Biol. Chem. 1997, 272:26023-26031*.* In a preferred embodiment, the term "a cell having a reduced fatty acid degradation capacity", as used herein, refers to a cell having a reduced capability of taking up and/or degrading fatty acids, preferably those having at least eight carbon chains. The fatty acid degradation capacity of a cell may be reduced in various ways. In a preferred embodiment, the cell has, compared to its wild type, a reduced activity of an enzyme involved in the β-oxidation pathway. In a preferred embodiment, the term "enzyme involved in the β-oxidation pathway", as used herein, refers to an enzyme that interacts directly with a fatty acid or a derivative thereof formed as part of the degradation of said fatty acid *via* the β-oxidation pathway the sequence of reactions effecting the conversion of a fatty acid to acetyl-CoA and the CoA ester of the shortened fatty acid, preferably by recognizing the fatty acid or derivative thereof as a substrate, and converts it to a metabolite formed as a part of the β-oxidation pathway. For example, the acyl-CoA dehydrogenase is an enzyme involved in the β-oxidation pathway as it interacts with fatty acid-CoA and converts fatty acid-CoA ester to enoyl-CoA, which is a metabolite formed as part of the β-oxidation. In a preferred embodiment, the term "enzyme involved in the β-oxidation pathway", as used herein, comprises any polypeptide from the group comprising acyl-CoA dehydrogenase, enoyl-CoA hydratase, 3-hydroxyacyl-CoA dehydrogenase and 3-keto-acyl-CoA thiolase. Subsequently, the acyl-CoA synthetase may catalyse the conversion of a fatty acid to the CoA ester of a fatty acid, *i.e.* a molecule, wherein the functional group -OH of the carboxy group is replaced with -S-CoA, preferably for introducing said fatty acid into the β-oxidation pathway. For example, the polypeptides FadD and FadK in *E*. *coli* (accession number: BAA15609.1) are acyl-CoA dehydrogenases. In a preferred embodiment, the term "acyl-CoA dehydrogenase", as used herein, is a polypeptide capable of catalysing the conversion of an acyl-CoA to enoyl-CoA, preferably as part of the β-oxidation pathway. For example, the polypeptide FadE in *E*. *coli* (accession number: BAA77891.2) is an acyl-CoA dehydrogenase. In a preferred embodiment, the term "2,4-dienoyl-CoA reductase", as used herein, is a polypeptide capable of catalysing the conversion of the 2,4-dienoyl CoA from an unsaturated fatty acid into enoyl-CoA, preferably as part of the β-oxidation pathway. For example, the polypeptide FadH in *E*. *coli* is a 2,4-dienoyl-CoA reductase. In a preferred embodiment, the term "enoyl-CoA hydratase", as used herein, also referred to as 3-hydroxyacyl-CoA dehydrogenase, refers to a polypeptide capable of catalysing the conversion of enoyl-CoA to 3-ketoacyl-CoA through hydration and oxidation, preferably as part of the β-oxidation pathway. For example, the polypeptides FadB and FadJ in *E*. *coli* (accession number: BAE77457.1) are enoyl-CoA hydratases. In a preferred embodiment, the term "ketoacyl-CoA thiolase", as used herein, refers to a polypeptide capable of catalysing the conversion of cleaving 3-ketoacyl-CoA, resulting in an acyl-CoA shortened by two carbon atoms and acetyl-CoA, preferably as the final step of the β-oxidation pathway. For example, the polypeptides FadA and Fadl in *E. coli* (access code: AP009048.1) are ketoacyl-CoA thiolases.

In one example, the cells according to any aspect of the present invention may be genetically modified to result in an increased activity of at least one amino acid N-acyl transferase in combination with increased activity of at least one acyl-CoA synthetase in combination with an increased activity of at least one transporter protein of the FadL and/or the AlkL. In particular, the cell according to any aspect of the present invention may be genetically modified to overexpress glycine N-acyl transferase, acyl-CoA/ACP synthetase and a transporter protein of the FadL and/or the AlkL compared to the wild type cell. These cells may be capable of producing acyl glycinates. In another example, the cell according to any aspect of the present invention may be genetically modified compared to the wild type cell to:
- increase the expression of amino acid N-acyl transferase, acyl-CoA synthetase; and
- reduce activity of at least one enzyme selected from the group consisting of acyl-CoA dehydrogenase FadE, multifunctional 3-hydroxybutyryl-CoA epimerase, Δ3-*cis*- Δ 2-*trans*-enoyl-CoA isomerase, enoyl-CoA hydratase, 3-hydroxyacyl-CoA dehydrogenase FadB, 3-ketoacyl-CoA thiolase, electron-transfer flavoprotein.

In yet another example, the cells according to any aspect of the present invention may be genetically modified compared to the wild type cell to result in:
- increase in the expression of amino acid N-acyl transferase, acyl-CoA synthetase and at least one transporter protein of the FadL and/or the AlkL; and
- reduce activity of at least one enzyme selected from the group consisting of acyl-CoA dehydrogenase FadE, multifunctional 3-hydroxybutyryl-CoA epimerase, Δ3-cis- Δ 2-*trans*-enoyl-CoA isomerase, enoyl-CoA hydratase, 3-hydroxyacyl-CoA dehydrogenase FadB, 3-ketoacyl-CoA thiolase, electron-transfer flavoprotein.

In particular, the acyl-CoA dehydrogenase FadE (EC 1.3.8.7, EC 1.3.8.8 or EC 1.3.8.9) may catalyse the reaction acyl-CoA + electron-transfer flavoprotein = trans-2,3-dehydroacyl-CoA + reduced electron-transfer flavoprotein; the multifunctional 3-hydroxybutyryl-CoA epimerase (EC 5.1.2.3), Δ3-cis-Δ2-trans-enoyl-CoA isomerase (EC 5.3.3.8), enoyl-CoA hydratase (EC 4.2.1.17) and 3-hydroxyacyl-CoA dehydrogenase (EC 1.1.1.35) FadB, may catalyse the reactions (S)-3-hydroxybutanoyl-CoA → (R)-3-hydroxybutanoyl-CoA, (3Z)-3-enoyl-CoA → (2E)-2-enoyl-CoA, (3S)-3-hydroxyacyl-CoA → trans-2-enoyl-CoA + H2O, (S)-3-hydroxyacyl-CoA + NAD+ = 3-oxoacyl-CoA + NADH + H+; 3-ketoacyl-CoA thiolase (EC 2.3.1.16), may catalyse the reaction acyl-CoA + acetyl-CoA → CoA + 3-oxoacyl-CoA; and electron-transfer flavoprotein (EC 1.5.5.1), may catalyse the following reaction: reduced electron-transferring flavoprotein + ubiquinone → electron-transferring flavoprotein + ubiquinol.

More in particular, the cell according to any aspect of the present invention may be genetically modified compared to the wild-type of the cell to increase the expression of glycine N-acyl transferase, acyl-CoA/ACP synthetase, a transporter protein of the FadL and the AlkL; and reduce activity of acyl-CoA dehydrogenase FadE, multifunctional 3-hydroxybutyryl-CoA epimerase, Δ3-*cis*- Δ 2-*trans*-enoyl-CoA isomerase, enoyl-CoA hydratase, 3-hydroxyacyl-CoA dehydrogenase FadB, 3-ketoacyl-CoA thiolase, electron-transfer flavoprotein.

In one example, the cell according to any aspect of the present invention may be genetically modified compared to the wild-type of the cell to:
- increase the expression of amino acid N-acyl transferase, acyl-CoA synthetase; and
- reduce activity of at least one enzyme selected from the group consisting of glycine cleavage system H protein, glycine cleavage system P protein, glycine cleavage system L protein, glycine cleavage system T protein, threonine aldolase, and serine hydroxylmethyltransferase.

In another example, the cell according to any aspect of the present invention may be genetically modified compared to the wild-type of the cell to:
- increase the expression of amino acid N-acyl transferase, acyl-CoA synthetase and at least one transporter protein of the FadL and/or the AlkL; and
- reduce activity of at least one enzyme selected from the group consisting of acyl-CoA dehydrogenase FadE, multifunctional 3-hydroxybutyryl-CoA epimerase, Δ3-*cis*- Δ 2-*trans*-enoyl-CoA isomerase, enoyl-CoA hydratase, 3-hydroxyacyl-CoA dehydrogenase FadB, 3-ketoacyl-CoA thiolase, and electron-transfer flavoprotein.

In yet another example, the cell according to any aspect of the present invention may be genetically modified compared to the wild-type of the cell to:
- increase the expression of amino acid N-acyl transferase, acyl-CoA synthetase and at least one transporter protein of the FadL and/or the AlkL;
- reduce activity of at least one enzyme selected from the group consisting of acyl-CoA dehydrogenase FadE, multifunctional 3-hydroxybutyryl-CoA epimerase, Δ3-*cis*- Δ 2-*trans*-enoyl-CoA isomerase, enoyl-CoA hydratase, 3-hydroxyacyl-CoA dehydrogenase FadB, 3-ketoacyl-CoA thiolase, and electron-transfer flavoprotein; and
- reduce activity of at least one enzyme selected from the group consisting of glycine cleavage system H protein, glycine cleavage system P protein, glycine cleavage system L protein, glycine cleavage system T protein, threonine aldolase, and serine hydroxylmethyltransferase.

In particular, the cell according to any aspect of the present invention may be genetically modified compared to the wild-type of the cell to increase the expression of glycine N-acyl transferase, acyl-CoA/ACP synthetase, a transporter protein of the FadL and the AlkL; reduce activity of acyl-CoA dehydrogenase FadE, multifunctional 3-hydroxybutyryl-CoA epimerase, Δ3-*cis*- Δ 2-*trans*-enoyl-CoA isomerase, enoyl-CoA hydratase, 3-hydroxyacyl-CoA dehydrogenase FadB, 3-ketoacyl-CoA thiolase, electron-transfer flavoprotein; and reduce activity of glycine cleavage system H protein, glycine cleavage system P protein, glycine cleavage system L protein, glycine cleavage system T protein, threonine aldolase, and serine hydroxylmethyltransferase.

More in particular, the glycine cleavage system H protein, carries lipoic acid and interacts with the glycine cleavage system proteins P, L and T; the glycine cleavage system P protein (EC 1.4.4.2), catalyses the reaction glycine + [glycine-cleavage complex H protein]-N(6)-lipoyl-L-lysine → [glycine-cleavage complex H protein]-S-aminomethyl-N(6)-dihydrolipoyl-L-lysine + CO2; glycine cleavage system L protein (EC 1.8.1.4), catalyses the reaction protein N6-(dihydrolipoyl)lysine + NAD+ → protein N6-(lipoyl)lysine + NADH + H+; glycine cleavage system T protein (EC 2.1.2.10), catalyses the reaction [protein]-S8-aminomethyldihydrolipoyllysine + tetrahydrofolate → [protein]-dihydrolipoyllysine + 5,10-methylenetetrahydrofolate + NH3; threonine aldolase (EC 4.2.1.48), catalyses the reaction L-threonine → glycine + acetaldehyde; serine hydroxylmethyltransferase (EC 2.1.2.1), catalyses the reaction 5,10-methylenetetrahydrofolate + glycine + H₂O + tetrahydrofolate + L-serine.

In one example, the cell according to any aspect of the present invention may be genetically modified compared to the wild-type of the cell to increase the expression of amino acid N-acyl transferase, acyl-CoA synthetase, and a genetic modification in the cell capable of producing at least one fatty acid from at least one carbohydrate. A list of non-limiting genetic modification to enzymes or enzymatic activities is provided below in Table 1. The cells according to any aspect of the present invention may comprise a combination of genetic modification that produce fatty acids and convert the fatty acids to N-acyl amino acids. In particular, the cell according to any aspect of the present invention may be genetically modified to increase the expression of amino acid N-acyl transferase, acyl-CoA synthetase and comprise any of the genetic modifications listed in Table 1. More in particular, the cell may be genetically modified to increase the expression of N-acyl transferase, acyl-CoA synthetase, a transporter protein of the FadL and the AlkL and comprise any of the genetic modifications listed in Table 1. Even more in particular, the cell may be genetically modified to increase the expression of N-acyl transferase, acyl-CoA synthetase, a transporter protein of the FadL and the AlkL, reduce activity of acyl-CoA dehydrogenase FadE, multifunctional 3-hydroxybutyryl-CoA epimerase, Δ3-cis- Δ 2-*trans*-enoyl-CoA isomerase, enoyl-CoA hydratase, 3-hydroxyacyl-CoA dehydrogenase FadB, 3-ketoacyl-CoA thiolase, electron-transfer flavoprotein; reduce activity of glycine cleavage system H protein, glycine cleavage system P protein, glycine cleavage system L protein, glycine cleavage system T protein, threonine aldolase, serine hydroxylmethyltransferase, and comprise any of the genetic modifications listed in Table 1.

In one example, the cell according to any aspect of the present invention may be genetically modified to increase the expression of amino acid N-acyl transferase, acyl-CoA/ACP synthetase and decrease the expression of at least one enzyme selected from the group consisting of β-ketoacyl-ACP synthase I, 3-oxoacyl-ACP-synthase I, Malonyl-CoA-ACP transacylase, enoyl ACP reductase, and β-ketoacyl-ACP synthase III. In particular, the genetic modification in the cell according to any aspect of the present invention may comprise an increase in the expression of amino acid N-acyl transferase, acyl-CoA/ACP synthetase and a decrease in the expression of β-ketoacyl-ACP synthase I, 3-oxoacyl-ACP-synthase I, Malonyl-CoA-ACP transacylase, enoyl ACP reductase, and β-ketoacyl-ACP synthase III.

The inventive teachings may be carried out using a wide range of cells. In a preferred embodiment, the cell is a bacterial cell, more preferably one from the group comprising *Pseudomonas, Corynebacterium, Bacillus* and *Escherichia,* most preferably *Escherichia coli.* The microorganism may be an isolated cell, in other words a pure culture of a single strain, or may comprise a mixture of at least two strains. Biotechnologically relevant cells are commercially available, for example from the American Type Culture Collection (ATCC) or the German Collection of Microorganisms and Cell Cultures (DSMZ). Particles for keeping and modifying cells are available from the prior art, for example Sambrook/Fritsch/Maniatis (1989): Molecular cloning - A Laboratory Manual, Cold Spring Harbour Press, 2nd edition, Fuchs/Schlegel (2007), Allgemeine Mikrobiologie, 2008, Georg Thieme Verlag.

Although the inventive teachings may be practiced using wild type cells, it is preferred that at least one of the enzymes involved, in particular at least one or all from the group comprising amino acid amino acid N-acyl-transferase, acyl-CoA synthetase and acyl-CoA thioesterase, is recombinant. In a preferred embodiment, the term "recombinant" as used herein, refers to a molecule or is encoded by such a molecule, preferably a polypeptide or nucleic acid that, as such, does not occur naturally but is the result of genetic engineering or refers to a cell that comprises a recombinant molecule. For example, a nucleic acid molecule is recombinant if it comprises a promoter functionally linked to a sequence encoding a catalytically active polypeptide and the promoter has been engineered such that the catalytically active polypeptide is overexpressed relative to the level of the polypeptide in the corresponding wild type cell that comprises the original unaltered nucleic acid molecule.

Whether or not a nucleic acid molecule, polypeptide, more specifically an enzyme required to practice the invention, is recombinant or not has not necessarily implications for the level of its expression. However, it is preferred that one or more recombinant nucleic acid molecules, polypeptides or enzymes required to practice the invention are overexpressed. In a preferred embodiment, the term "overexpressed", as used herein, means that the respective polypeptide encoded or expressed is expressed at a level higher or at higher activity than would normally be found in the cell under identical conditions in the absence of genetic modifications carried out to increase the expression, for example in the respective wild type cell. The person skilled in the art is familiar with numerous ways to bring about overexpression. For example, the nucleic acid molecule to be overexpressed or encoding the polypeptide or enzyme to be overexpressed may be placed under the control of a strong inducible promoter such as the lac promoter. The state of the art describes standard plasmids that may be used for this purpose, for example the pET system of vectors exemplified by pET-3a (commercially available from Novagen). Whether or not a nucleic acid or polypeptide is overexpressed may be determined by way of quantitative PCR reaction in the case of a nucleic acid molecule, SDS polyacrylamide electrophoreses, Western blotting or comparative activity assays in the case of a polypeptide. Genetic modifications may be directed to transcriptional, translational, and/or post-translational modifications that result in a change of enzyme activity and/or selectivity under selected and/or identified culture conditions. Thus, in various examples of the present invention, to function more efficiently, a microorganism may comprise one or more gene deletions. Gene deletions may be accomplished by mutational gene deletion approaches, and/or starting with a mutant strain having reduced or no expression of one or more of these enzymes, and/or other methods known to those skilled in the art.

Besides, any of the enzymes required to practice the inventive teachings, in particular at least one or all from the group comprising N-acyl-transferase, acyl-CoA synthetase and acyl-CoA thioesterase, may be an isolated enzyme. In any event, any enzyme required to practice the present invention is preferably used in an active state and in the presence of all cofactors, substrates, auxiliary and/or activating polypeptides or factors essential for its activity. In a preferred embodiment, the term "isolated", as used herein, means that the enzyme of interest is enriched compared to the cell in which it occurs naturally. Whether or not an enzyme is enriched may be determined by SDS polyacrylamide electrophoresis and/or activity assays. For example, the enzyme of interest may constitute more than 5, 10, 20, 50, 75, 80, 85, 90, 95 or 99 percent of all the polypeptides present in the preparation as judged by visual inspection of a polyacrylamide gel following staining with Coomassie blue dye.

If a cell expressing an amino acid-N-acyl-transferase and an acyl-CoA synthetase and having a reduced fatty acid degradation capacity is used, it is preferred that the cell be capable of making proteinogenic amino acids and/or fatty acids. In a preferred embodiment, the term "proteinogenic amino acid", as used herein, refers to an amino acid selected from the group comprising alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine and valine. Proteinogenic amino acids and fatty acids are synthesized as part of the primary metabolism of many wild type cells in reactions and using enzymes that have been described in detail in biochemistry textbooks, for example Jeremy M Berg, John L Tymoczko, and Lubert Stryer, Biochemistry, 5th edition, W. H. Freeman, 2002.

In a preferred embodiment, the cell used to practice the inventive teachings expresses an acyl-CoA thioesterase. In a more preferred embodiment, the term "acyl-CoA thioesterase", as used herein, refers to an enzyme capable of hydrolysing acyl-CoA. In a preferred embodiment the acyl-CoA thioesterase comprises a sequence from the group comprising SEQ ID NO 1, AEM72521.1 and AAC49180.1 or a variant thereof, more preferably SEQ ID NO 1 or a variant thereof. The activity of acyl-CoA thioesterase may be assayed using various assays described in the state of the art. Briefly, the reaction of Ellman's reagent, which reacts with free thiol groups associated with CoASH formed upon hydrolysis of acyl-CoA may be detected by spectophotometrically monitoring absorbance at 412 nm.

In a preferred embodiment, the term "contacting", as used herein, means bringing about direct contact between the amino acid, the acyl CoA and the amino acid-N-acyl transferase or the inventive cell and/or any other reagents required to carry out the inventive teachings, preferably in an aqueous solution. For example, the cell, the amino acid and the acyl CoA may not be in different compartments separated by a barrier such as an inorganic membrane. If the amino acid or fatty acid is soluble and may be taken up by the cell or can diffuse across biological membranes, it may simply be added to the inventive cell in an aqueous solution. In case it is insufficiently soluble, it may be solved in a suitable organic solvent prior to addition to the aqueous solution. The person skilled in the art is able to prepare aqueous solutions of amino acids or fatty acids having insufficient solubility by adding suitable organic and/or polar solvents. Such solvents are preferably provided in the form of an organic phase comprising liquid organic solvent. In a preferred embodiment, the organic solvent or phase is considered liquid when liquid at 25 °C and standard atmospheric pressure. In another preferred embodiment, a fatty acid is provided in the form of a fatty acid ester such as the respective methyl or ethyl ester. For example, the fatty acid laurate may be solved in lauric acid methyl ester as described in EP11191520.3. According to the present invention, the compounds and catalysts may be contacted *in vitro,* i.e. in a more or less enriched or even purified state, or may be contacted *in situ,* i.e. they are made as part of the metabolism of the cell and subsequently react inside the cell.

The term "an aqueous solution" comprises any solution comprising water, preferably mainly water as solvent that may be used to keep the inventive cell, at least temporarily, in a metabolically active and/or viable state and comprises, if such is necessary, any additional substrates. The person skilled in the art is familiar with the preparation of numerous aqueous solutions, usually referred to as media that may be used to keep inventive cells, for example LB medium in the case of *E. coli.* It is advantageous to use as an aqueous solution a minimal medium, *i.e.* a medium of reasonably simple composition that comprises only the minimal set of salts and nutrients indispensable for keeping the cell in a metabolically active and/or viable state, by contrast to complex mediums, to avoid dispensable contamination of the products with unwanted side products. For example, M9 medium may be used as a minimal medium.

It is a particular strength of the present invention that not only saturated fatty acids, but also unsaturated fatty acids may be converted to acyl amino acids. In case the end product sought-after is to comprise a higher yield of saturated acyl residues than is present after step b), it may be possible to complement the process by hydrogenating the acyl residues of the acyl amino acids following step b). In this case, the inventive composition according to the fifth aspect of the present invention, which composition comprises a mixture of acyl amino acids having unsaturated acyl residues, constitutes an obligatory intermediate which may be converted to the final product, i.e. a mixture of acyl amino acids having saturated acyl residues. The hydrogenation may be carried out according to various state of the art processes, for example those described in US5734070. Briefly, the compound to be hydrogenated may be incubated at 100 °C in the presence of hydrogen and a suitable catalyst, for example a nickel catalyst on silicon oxide as a support.

The fatty acids that are to be converted to acyl amino acids may be produced by the cell according to the present invention. In one example, the very cell that produces the acyl amino acids is capable of producing the fatty acids from which the acyl amino acids are produced. In particular, the cells may be genetically modified to be able to produce fatty acids. In one example, the genetic modification may be to decrease a specific enzymatic activity and this may be done by a gene disruption or a genetic modification. The genetic modification may also increase a specific enzymatic activity. In particular, the genetic modification may increase microbial synthesis of a selected fatty acid or fatty acid derived chemical product above a rate of a control or wild type cell. This control or wild type cell may lack this genetic modification to produce a selected chemical product.

In particular, the cell comprises at least one genetic mutation that enables the cell to produce at least one fatty acid. In particular, the genetic mutation may enable the cell to produce at least one fatty acid by means of a malonyl-CoA dependent and malonyl-ACP independent fatty acyl-CoA metabolic pathway. More in particular, there is an increase in enzymatic activity in the malonyl-CoA dependent and malonyl-ACP independent fatty acyl-CoA metabolic pathway in the cell relative to the wild type cell. The cell may be genetically modified for increased enzymatic activity in the microorganism's malonyl-CoA dependent, malonyl-ACP independent, fatty acyl-CoA metabolic pathway ("MDMIFAA") This pathway is also referred to herein as malonyl-CoA dependent, but malonyl-ACP independent, fatty acyl-CoA metabolic pathway. Such increase in the cell's malonyl-CoA dependent, malonyl-ACP independent fatty acyl-CoA metabolic pathway can be achieved by an increased activity or expression of a gene or a pathway comprising an acetoacetyl-CoA synthase, a ketoacyl-CoA synthase (or elongase), an enoyl-CoA reductase, a ketoacyl-CoA reductase and/or a 3-hydroxyacyl-CoA dehydratase in combination with a decrease in expression or activity of acetoacetyl-CoA thiolase. Alternatively, increased activity in the microorganism's malonyl-CoA dependent, malonyl-ACP independent fatty acyl-CoA metabolic pathway can be achieved by an increased expression of a gene or a pathway comprising an acetoacetyl-CoA synthase, a ketoacyl-CoA thiolase, a enoyl-CoA reductase, a ketoacyl-CoA reductase and/or a 3-hydroxyacyl-CoA dehydratase in combination with a decrease in expression or activity of acetoacetyl-CoA thiolase.

A list of non-limiting genetic modifications to enzymes or enzymatic activities that may lead a cell to produce a fatty acid and/or acyl coenzyme A thereof and that may be considered as at least one genetic mutation according to any aspect of the present invention is provided below in Table 1 and explained in US20140051136.

In one example, nucleic acid sequences that encode temperature-sensitive forms of these polypeptides may be introduced in place of the native enzymes, and when such genetically modified microorganisms are cultured at elevated temperatures (at which these thermolabile polypeptides become inactivated, partially or completely, due to alterations in protein structure or complete denaturation), there is observed an increase in a chemical product. For example, in *E. coli,* these temperature-sensitive mutant genes could include fabl^{ts}(S241F), fabB^{ts}(A329V) or fabD^{ts}(W257Q) amongst others. In most of these examples, the genetic modifications may increase malonyl-CoA utilization so that there is a reduced conversion of malonyl-CoA to fatty acids via the native pathway, overall biomass, and proportionally greater conversion of carbon source to a chemical product including a fatty acid or fatty acid derived product via a malonyl-CoA dependent and malonyl-ACP independent route. Also, additional genetic modifications, such as to increase malonyl-CoA production, may be made for some examples.

In another example, the enzyme, enoyl-acyl carrier protein reductase (EC No. 1.3.1.9, also referred to as enoyl-ACP reductase) is a key enzyme for fatty acid biosynthesis from malonyl-CoA. In *Escherichia coli* this enzyme, Fabl, is encoded by the gene fabl (Richard J. Heath et al., 1995). In one example, the expression levels of a pyruvate oxidase gene (Chang et al., 1983, Abdel-Ahmid et al., 2001) can be reduced or functionally deleted in the cell according to any aspect of the present invention. The pyruvate oxidase gene may encode an enzyme of, for example, EC 1.2.3.3. In particular, the pyruvate oxidase gene may be a poxB gene. In one example, the expression levels of a lactate dehydrogenase gene (Mat-Jan et al., Bunch et al., 1997) can be reduced or functionally deleted. In some examples, the lactate dehydrogenase gene encodes an enzyme of, for example, EC 1.1.1.27. The lactate dehydrogenase gene may be an NAD-linked fermentative D-lactate dehydrogenase gene. In particular, the lactate dehydrogenase gene is a ldhA gene.

In one example, the genetic mutation that may increase the expression of fatty acids in the cell may be in at least one feedback resistant enzyme of the cell that results in increased expression of the feedback resistant enzyme. In particular, the enzyme may be pantothenate kinase, pyruvate dehydrogenase or the like. In *E. coli,* these feedback resistant mutant genes could include coaA(R106A) and/or Ipd(E354K).

In a further example, the increase in the cell's malonyl-CoA dependent, but malonyl-ACP independent fatty acyl-CoA metabolic pathway may occur through reduction in the acetoacetyl-CoA thiolase activity and/or trigger factor activity and/or in the activity of a molecular chaperone involved in cell division. In one example, the cell may comprise a genetic mutation in tig gene.

In one example, the genetic mutation in the cell may result in increased enzymatic activity in the NADPH-dependent transhydrogenase pathway relative to the wild type cell. This result may occur by introduction of a heterologous nucleic acid sequence coding for a polypeptide encoding nucleotide transhydrogenase activity. In another example, the genetic mutation in the cell may result in decreased expression of fatty acyl-CoA synthetase and/or ligase activity via any method known in the art. In yet another example, the genetic mutation in the cell may result in overexpression of an enzyme having acetyl-CoA carboxylase activity.

In one example, the cell may have increased intracellular bicarbonate levels brought about by introduction of a heterologous nucleic acid sequence coding for a polypeptide having cyanase and/or carbonic anhydrase activity. More in particular, the genetic mutation according to any aspect of the cell may result in increased and/ or decreased levels of fatty acyl-CoA thioesterase activity. This result may increase chain length specificity of a desired fatty acid product by increasing levels of chain length specific fatty acyl-CoA thioesterase activity and decreasing the activity of fatty acyl-CoA thioesterase activity on undesired fatty acid chain lengths. In one example, the increased chain length specificity of fatty acid or fatty acid derived product may occur by increasing levels of chain length specific ketoacyl-CoA thiolase, enoyl-CoA reductase, ketoacyl-CoA reductase or 3-hydroxyacyl-CoA dehydratase activities either individually or in combination.

The genetic mutation in the cell according to any aspect of the present invention may result in an increase or decrease in expression of only one enzyme selected from the list of enzymes mentioned above or an increase or decrease in expression of a combination of enzymes mentioned above.

In another example, the genetic mutation in the cell may be in at least one enzyme selected from the group consisting of acetoacetyl-CoA synthase, ketoacyl-CoA synthase (or elongase), enoyl-CoA reductase, ketoacyl-CoA reductase, 3-hydroxyacyl-CoA dehydratase and acetoacetyl-CoA thiolase. More in particular, the genetic mutation in the cell may result in an increase in expression of acetoacetyl-CoA synthase, ketoacyl-CoA synthase (or elongase), enoyl-CoA reductase, ketoacyl-CoA reductase and 3-hydroxyacyl-CoA dehydratase in combination optionally with a decrease in expression or activity of acetoacetyl-CoA thiolase. In particular, the enoyl-CoA reductase and/or ketoacyl-CoA reductase may either utilize the cofactor NADH and/or NADPH.

In particular, the genetic modification in the cell according to any aspect of the present invention may comprise any of the enzymes listed in Table 1 in combination with the following enzymes acetoacetyl-CoA synthase, ketoacyl-CoA synthase (or elongase), enoyl-CoA reductase, ketoacyl-CoA reductase and/or 3-hydroxyacyl-CoA dehydratase and acetoacetyl-CoA thiolase wherein the expression or activity of enzymes acetoacetyl-CoA synthase, ketoacyl-CoA synthase (or elongase), enoyl-CoA reductase, ketoacyl-CoA reductase and 3-hydroxyacyl-CoA dehydratase is increased and the activity of acetoacetyl-CoA thiolase is decreased.

In yet another example, malonyl-CoA dependent, malonyl-ACP independent fatty acyl-CoA metabolic pathway in the cell according to any aspect of the present invention can be achieved by an increased expression of a gene or a pathway comprising acetoacetyl-CoA synthase, ketoacyl-CoA thiolase, enoyl-CoA reductase, ketoacyl-CoA reductase and/or 3-hydroxyacyl-CoA dehydratase in combination with a decrease in expression or activity of acetoacetyl-CoA thiolase.

In particular, the genetic modification in the cell according to any aspect of the present invention may comprise any of the enzymes listed in Table 1 in combination with the following enzymes acetoacetyl-CoA synthase, ketoacyl-CoA thiolase, enoyl-CoA reductase, ketoacyl-CoA reductase and/or 3-hydroxyacyl-CoA dehydratase in combination with a decrease in expression or activity of acetoacetyl-CoA thiolase.

In one example, the cell according to any aspect of the present invention may comprise a genetic modification in any of the enzymes listed in Table 1 in combination with the following enzymes acetyl-CoA carboxylase, malonyl-CoA:ACP transacylase (FabD), β-ketoacyl-ACP synthase III, β-ketoacyl-ACP synthase I (FabB), β-ketoacyl-ACP synthase II (FabF), 3-oxoacyl-ACP-synthase I and enoyl ACP reductase.

More in particular, the genetic mutation may result in an increase in the expression of at least one enzyme selected from the group consisting of acetyl-CoA carboxylase, malonyl-CoA:ACP transacylase (FabD), β-ketoacyl-ACP synthase III, β-ketoacyl-ACP synthase I (FabB), β-ketoacyl-ACP synthase II (FabF), 3-oxoacyl-ACP-synthase I and enoyl ACP reductase relative to the wild type cell. In particular, the genetic mutation may result in an increase in the expression of more than one enzyme in the cell according to any aspect of the present invention that enables the cell to produce a fatty acid and/or acyl coenzyme A thereof by means of increased enzymatic activity in the cell relative to the wild type cell of malonyl-CoA dependent and malonyl-ACP independent fatty acyl-CoA metabolic pathway.

In one example, there may be an increase in expression of β-ketoacyl-ACP synthase and 3-oxoacyl-ACP-synthase in the cell according to any aspect of the present invention. In another example, there may be an increase in expression of β-ketoacyl-ACP synthase and Malonyl-CoA-ACP transacylase in the cell according to any aspect of the present invention. In yet another example, there may be an increase in expression of β-ketoacyl-ACP synthase and enoyl ACP reductase in the cell according to any aspect of the present invention. In one example, there may be an increase in expression of β-ketoacyl-ACP synthase, Malonyl-CoA-ACP transacylase and enoyl ACP reductase in the cell according to any aspect of the present invention. In all examples, there may be an increase in the expression of enoyl ACP reductase and/or acyl-CoA thioesterase.

The phrase "increased activity of an enzyme", as used herein is to be understood as increased intracellular activity. Basically, an increase in enzymatic activity can be achieved by increasing the copy number of the gene sequence or gene sequences that code for the enzyme, using a strong promoter or employing a gene or allele that code for a corresponding enzyme with increased activity and optionally by combining these measures. Genetically modified cells used according to any aspect of the present invention are for example produced by transformation, transduction, conjugation or a combination of these methods with a vector that contains the desired gene, an allele of this gene or parts thereof and a vector that makes expression of the gene possible. Heterologous expression is in particular achieved by integration of the gene or of the alleles in the chromosome of the cell or an extra-chromosomally replicating vector.

In one example, the genetic modification in a cell of the present invention may include any of the enzymes above in combination with a genetic modification in at least one enzyme selected from the group consisting of β-ketoacyl-ACP synthase I, 3-oxoacyl-ACP-synthase I, Malonyl-CoA-ACP transacylase, enoyl ACP reductase, and β-ketoacyl-ACP synthase III. In particular, the genetic modification in the cell according to any aspect of the present invention may comprise any of the enzymes listed in Table 1 in combination with the following enzymes β-ketoacyl-ACP synthase I, 3-oxoacyl-ACP-synthase I, Malonyl-CoA-ACP transacylase, enoyl ACP reductase, and β-ketoacyl-ACP synthase III.

**Table 1. Examples of genetic modifications in cells of microorganisms for production of fatty acids**

| Genetic Modifications | | | |
|---|---|---|---|
| ENZYME FUNCTION | E.C. CLASSIFICATION No. | GENE NAME IN *E. COLI* | COMMENTS |
| Glucose transporter | N/A | galP | Increase function |
| Pyruvate dehydrogenase Elp | 1.2.4.1 | aceE | Increase function |
| lipoate acetyltransferase/ dihydrolipoamide acetyltransferase | 2.3.1.12 | aceF | Increase function |
| Pyruvate dehydrogenase E3 (lipoamide dehydrogenase) | 1.8.1.4 | Ipd | Increase function or alter such as by mutation to increase resistance to NADH inhibition, |
| Lactate dehydrogenase | 1.1.1.28 | IdhA | Decrease function, including by mutation |
| Pyruvate formate lyase (B "inactive") | 2.3.1. | pflB | Decrease function, including by mutation |
| Pyruvate oxidase | 1.2.2.2 | poxB | Decrease function, including by mutation |
| Phosphate acetyltransferase | 2.3.1.8 | Pta | Decrease function, including by mutation |
| acetate kinase | 2.7.2.15 2.7.2.1 | ackA | Decrease function, including by mutation |
| methylglyoxal synthase | 4.2.3.3 | mgsA | Decrease function, including by mutation |
| Heat, stable, histidyl phosphorylatable protein (of PTS) | N/A | ptsH (HPr) | Decrease function, including by mutation |
| Phosphoryl transfer protein (of PTS) | N/A | ptsI | Decrease function, including by mutation |
| Polypeptide chain (of PTS) | N/A | Crr | Decrease function, including by mutation |
| 3-oxoacyl-ACP synthase I | 2.3.1.179 | fabF | Decrease function, |
| 3-oxoacyl-ACP synthase II monomer | 2.3.1.41 | | including by mutation |
| β-ketoacyl-ACP synthase I, | 2.3.1.41 | fabB | Decrease function, |
| 3-oxoacyl-ACP-synthase I | 2.3.1.- | | including by mutation |
| Malonyl-CoA-ACP | 2.3.1.39 | fabD | Decrease function, |
| transacylase | | | including by mutation |
| enoyl acyl carrier protein | 1.3.1.9, | fabI | Decrease function, |
| reductase | 1.3.1.10 | | including by mutation |
| β-ketoacyl-acyl carrier protein synthase III | 2.3.1.180 | fabH | Decrease function, including by mutation |
| Carboxyl transferase subunit α subunit | 6.4.1.2 | accA | Increase function |
| Biotin carboxyl carrier protein | 6.4.1.2 | accB | Increase function |
| Biotin carboxylase subunit | 6.3.4.14 | accC | Increase function |
| Carboxyl transferase subunit β subunit | 6.4.1.2 | accD | Increase function |
| long chain fatty acyl | 3.1.2.2. | tesA | Increase function as |
| thioesterase I | 3.1.1.5 | | well as alter by mutation to express in cytoplasm or deletion |
| acyl-CoA synthase | 2.3.1.86 | fadD | Decrease via deletion or mutation |
| acetate CoA-transferase | 2.8.3.8 | atoD | Decrease via deletion or mutation |
| acetate CoA-transferase | 2.8.3.8 | atoA | Decrease via deletion or mutation |
| Transporter | N/A | atoE | Decrease via deletion or mutation |
| acetyl-CoA acetyltransferase | 2.3.1.9 | atoB | Decrease via deletion or mutation |
| pantothenate kinase | 2.7.1.33 | coaA | Increase via expression or feedback resistant mutation |
| lactose repressor | N/A | lacI | Decrease via deletion or mutation |
| γ-gfutamyl-γ-aminobutyraldehyde dehydrogenase | 1.2.1.- | puuC | Decrease via deletion or mutation |
| malate synthase A | 2.3.3.9 | aceB | Decrease via deletion or mutation |
| isocitrate lyase | 4.1.3.1 | aceA | Decrease via deletion or mutation |
| isocitrate dehydrogenase | 3.1.3.- | accK | Decrease via deletion or mutation |
| phosphatase/isocitrate dehydrogenase kinase | 2.7.11.5. | | |
| pyruvate formate-lyase deactivase | 1.2.1.10 1.1.1.1 | adhE | Decrease via deletion or mutation |
| aldehyde dehydrogenase A, NAD-linked | 1.2.1.21 1.2.1.22 | aldA | Decrease via deletion or mutation |
| acetaldehyde dehydrogenase | 1.2.1.4 | aldB | Decrease via deletion or mutation |
| Lambda phage DE3 lysogen | N/A | λDE3 | Increase |
| T7 mRNA polymerase | N/A | T7pol | Increase |
| trigger factor | 5.2.1.8 | tig | Decrease via deletion or mutation |
| 3-ketoacyl-CoA thiolase | 2.3.1.16 | fadA | Increase |
| dodecenoyl-CoA δ-isomerase, | 5.3.3.8 1.1.1.35 | fadB | Increase |
| enoyl-CoA hydratase, 3-hydroxybutyryl-CoA epimerase, 3-hydroxyacyl-CoA dehydrogenase | 5.1.2.3 4.2.1.17 | | |
| Sucrose permease | N/A | cscB | Increase |
| Invertase | 3.2.1.26 | cscA | Increase |
| fructokinase | 2.7.1.4 | cscK | Increase |
| carbonic anhydrase | 4.2.1.1 | cynT | Increase |
| carbonic anhydrase | 4.2.1.1 | can | Increase |
| pyridine nucleotide transhydrogenase | 1.6.1.2 | pntAB | Increase |
| pyridine nucleotide transhydrogenase | 1.6.1.1 | udhA | Increase |
| acyl-CoA thioesterase | 3.1.2.20 3.1.2.2 | yciA | Increase and or decrease |
| thioesterase II | 3.1.2.20 3.1.2.2 | tesB | Increase and or decrease |
| thioesterase III | 3.1.2.- | fadM | Increase and or decrease |
| hydroxyphenylacetyl-CoA thioesterase | N/A | paaI | Increase and or decrease |
| esterase/thioesterase | 3.1.2.28 | ybgC | Increase and or decrease |
| proofreading thioesterase in enterobactin biosynthesis | | entH | Increase and or decrease |
| acetoacetyl-CoA synthase | 2.3.1.194 | npth07 | Increase |
| 3-ketoacyl-CoA synthase/elongase | 2.3.1 | EloI | Increase |
| 3-ketoacyl-CoA synthase/elongase | 2.3.1 | Elo2 | Increase |
| 3-Hydroxybutyryl-CoA dehydrogenase | 1.1.1.157 | hbd | Increase |
| 3-oxoacyl-CoA reductase | 1.1.1.100 | fabG | Increase |
| enoyl-CoA hydratase | 4.2.1.17 | crt | Increase |
| enoyl-CoA hydratase | 4.2.1.17 | ech2 | Increase |
| Trans-2-enoyl-reductase | 1.3.1.9 | ter | Increase |
| thioesterase | 3.1.2.20 | paaI | Decrease |

| | | | |
|---|---|---|---|
| E.C. No. - "Enzyme Commission number" | | | |

According to any aspect of the present invention, the cell may be genetically modified to increase the expression of at least one transporter protein compared to the wild type cell. The transporter protein may be FadL and/or AlkL. In one example, the cell may be genetically modified to overexpress both the FadL and the AlkL gene.

The cell may also be genetically modified to increase the expression of at least one enzyme selected from the group consisting of acetoacetyl-CoA synthase, ketoacyl-CoA synthase (or elongase), ketoacyl-CoA thiolase, enoyl-CoA reductase, ketoacyl-CoA reductase and 3-hydroxyacyl-CoA dehydratase and optionally a decrease in in the expression of acetoacetyl-CoA thiolase relative to the wild type cell wherein the cell has a reduced fatty acid degradation capacity.

Accordingly, the cells and methods of the present invention may comprise providing a genetically modified microorganism that comprises both a production pathway to a fatty acid or fatty acid derived product, and a modified polynucleotide that encodes an enzyme of the malonyl-ACP dependent fatty acid synthase system that exhibits reduced activity, so that utilization of malonyl-CoA shifts toward the production pathway compared with a comparable (control) microorganism lacking such modifications. The methods involve producing the chemical product using a population of such genetically modified microorganism in a vessel, provided with a nutrient media. Other genetic modifications described herein, to other enzymes, such as acetyl-CoA carboxylase and/or NADPH-dependent transhydrogenase, may be present in some such examples. Providing additional copies of polynucleotides that encode polypeptides exhibiting these enzymatic activities is shown to increase a fatty acid or fatty acid derived product production. Other ways to increase these respective enzymatic activities is known in the art and may be applied to various examples of the present invention.

Also, without being limiting, a first step in some multi-phase method embodiments of making a fatty acid may be exemplified by providing into a vessel, such as a culture or bioreactor vessel, a nutrient media, such as a minimal media as known to those skilled in the art, and an inoculum of a genetically modified microorganism so as to provide a population of such microorganism, such as a bacterium, and more particularly a member of the family *Enterobacteriaceae,* such as *E. coli,* where the genetically modified microorganism comprises a metabolic pathway that converts malonyl-CoA to a fatty acid. This inoculum is cultured in the vessel so that the cell density increases to a cell density suitable for reaching a production level of a fatty acid or fatty acid derived product that meets overall productivity metrics taking into consideration the next step of the method. In various alternative embodiments, a population of these genetically modified microorganisms may be cultured to a first cell density in a first, preparatory vessel, and then transferred to the noted vessel so as to provide the selected cell density. Numerous multi-vessel culturing strategies are known to those skilled in the art. Any such embodiments provide the selected cell density according to the first noted step of the method.

Also without being limiting, a subsequent step may be exemplified by two approaches, which also may be practiced in combination in various embodiments. A first approach provides a genetic modification to the genetically modified microorganism such that its enoyl-ACP reductase enzymatic activity may be controlled. As one example, a genetic modification may be made to substitute a temperature-sensitive mutant enoyl-ACP reductase (e.g., fabl^{TS} in *E. coli*) for the native enoyl-ACP reductase. The former may exhibit reduced enzymatic activity at temperatures above 30° C. but normal enzymatic activity at 30° C., so that elevating the culture temperature to, for example to 34° C., 35° C., 36° C., 37° C. or even 42° C., reduces enzymatic activity of enoyl-ACP reductase. In such case, more malonyl-CoA is converted to a fatty acid or fatty acid derived product or another chemical product than at 30° C., where conversion of malonyl-CoA to fatty acids is not impeded by a less effective enoyl-ACP reductase.

Other genetic modifications that may be useful in the production of fatty acids and/or amino acids may be included in the cell. For example, the ability to utilise sucrose may be provided, and this would expand the range of feed stocks that can be utilised to produce a fatty acid or fatty acid derived product or other chemical products. Common laboratory and industrial strains of *E. coli,* such as the strains described herein, are not capable of utilizing sucrose as the sole carbon source. Since sucrose, and sucrose-containing feed stocks such as molasses, are abundant and often used as feed stocks for the production by microbial fermentation, adding appropriate genetic modifications to permit uptake and use of sucrose may be practiced in strains having other features as provided herein. Various sucrose uptake and metabolism systems are known in the art (for example, U.S. Pat. No. 6,960,455).

Also, genetic modifications may be provided to add functionality for breakdown of more complex carbon sources, such as cellulosic biomass or products thereof, for uptake, and/or for utilisation of such carbon sources. For example, numerous cellulases and cellulase-based cellulose degradation systems have been studied and characterized (Beguin, P and Aubert, J-P (1994) FEMS Microbial. Rev. 13: 25-58; Ohima, K. et al. (1997) Biotechnol. Genet. Eng. Rev. 14: 365414).

In some examples, genetic modifications increase the pool and availability of the cofactor NADPH, and/or, consequently, the NADPH/NADP⁺ ratio may also be provided. For example, in *E. coli,* this may be done by increasing activity, such as by genetic modification, of one or more of the following genes: *pgi* (in a mutated form), *pntAB,* overexpressed, *gapA:gapN* substitution/replacement, and disrupting or modifying a soluble transhydrogenase such as *sthA,* and/or genetic modifications of one or more of *zwf, gnd,* and *edd.*

Any such genetic modifications may be provided to species not having such functionality, or having a less than desired level of such functionality. More generally, and depending on the particular metabolic pathways of a microorganism selected for genetic modification, any subgroup of genetic modifications may be made to decrease cellular production of fermentation product(s) selected from the group consisting of acetate, acetoin, acetone, acrylic, malate, Benzoyl-CoA, fatty acid ethyl esters, isoprenoids, glycerol, ethylene glycol, ethylene, propylene, butylene, isobutylene, ethyl acetate, vinyl acetate, other acetates, 1,4-butanediol, 2,3-butanediol, butanol, isobutanol, sec-butanol, butyrate, isobutyrate, 2-OH-isobutryate, 3-OH-butyrate, ethanol, isopropanol, D-lactate, L-lactate, pyruvate, itaconate, levulinate, glucarate, glutarate, caprolactam, adipic acid, propanol, isopropanol, fusel alcohols, and 1,2-propanediol, 1,3-propanediol, formate, fumaric acid, propionic acid, succinic acid, valeric acid, and maleic acid. Gene deletions may be made as disclosed generally herein, and other approaches may also be used to achieve a desired decreased cellular production of selected fermentation products.

The inventions is further illustrated by the following figures and non-limiting examples from which further embodiments, aspects and advantages of the present invention may be taken.
Figure. 1 depicts a total ion chromatogram of the 48 h sample from the *E. coli* W3110 ΔfadE pJ294{Ptac}[synUcTE] / pCDF{Ptac}[hGLYAT2(co_Ec)_fadD_Ec] fermentation.
Figure. 2 depicts a total ion chromatogram of the 48 h sample from the *E. coli* W3110 ΔfadE pJ294{Ptac}[synUcTE] / pCDF{Ptac}[hGLYAT3(co_Ec)_fadD_Ec] fermentation.
Figure. 3 depicts a total ion chromatogram of the 48 h sample from the *E. coli* W3110 ΔfadE pJ294{Ptac}[synUcTE] / pCDFDuet-1 fermentation (negative control).
Figure. 4 depicts production of lauroylglycinate by *E.coli* strains W3110 Δ*fadE* pCDF{Ptac}[hGLYAT2(co_Ec)-fadD_Ec]{Placuv5}[alkLmod1].
Figure. 5 depicts production of lauroylglycinate by *E. coli* strain W3110 Δ*fadE* Δ*gcvTHP* pCDF{Ptac}[hGLYAT2(co_Ec)-fadD_Ec]{Placuv5}[alkLmod1].
Figure. 6 A tree showing the percentage sequence identity of GLYAT2 in the various organisms tested in Example 16.

### Examples

### Sequence ID NOs:

Throughout this application a range of SEQ ID NOs are used. These are shown in Table 2 below.

**Table 2. Sequences used in the examples.**

| SEQ ID NO: | Comment |
|---|---|
| 1 | *Umbellularia californica synUcTE* (an acyl-CoA thioesterase) gene (codon-optimized) |
| 2 | tac promoter |
| 3 | Vector pJ294[Ptac-synUcTE], see example 1 |
| 4 | *Homo sapiens* genes hGLYAT2 (an amino acid N-acyl transferase) |
| 5 | *Homo sapiens* genes hGLYAT3 (another amino acid N-acyl transferase) |
| 6 | *Escherichia coli fadD* (an acyl-CoA synthetase) |
| 7 | Vector pCDF[atfA1_Ab(co_Ec)-fadD_Ec], see example 2 |
| 8 | Vector pCDF{Ptac}[hGLYAT2(co_Ec)-fadD_Ec], see example 2 |
| 9 | Vector pCDF{Ptac}[hGLYAT3(co_Ec)-fadD_Ec], see example 2 |
| 10 | alkL (an importer facilitating transport of hydrophobic acyl across cell membranes) gene, see example 3 |
| 11 | lacuv5 promoter, see example 3 |
| 12 | Vector pCDF[alkLmod1], see example 3 |
| 13 | Vector pCDF{Ptac}[hGLYAT2(co_Ec)-fadD_Ec]{Placuv5}[alkLmod1], see example 3 |
| 14 | Vector pET-28b, see example 10 |
| 15 | Vector pET-28b{Ptac}[hGLYAT2(co_Ec)] , see example 10 |
| 16 | pET-28b{Ptac}[hGLYAT3(co_Ec)], see example 10 |

### Example 1

### Generation of an expression vector for the Umbellularia californica gene synUcTE

To generate an expression vector for the *Umbellularia californica synUcTE* gene (SEQ ID NO: 1), which encodes the *Umbellularia californica* acyl CoA-thioesterase, this gene was codon-optimized for expression in *Escherichia coli.* The gene was synthesized together with a *tac* promoter (SEQ ID NO: 2), and, simultaneously, one cleavage site was introduced upstream of the promoter and one cleavage site downstream of the terminator. The synthesized DNA fragment P_{tac}-synUcTE was digested with the restriction endonucleases *BamH*I and *Not*I and ligated into the correspondingly cut vector pJ294 (DNA2.0 Inc., Menlo Park, CA, USA). The finished *E. coli* expression vector was referred to as pJ294[Ptac-synUcTE] (SEQ ID NO: 3).

### Example 2

### Generation of vectors for coexpression of Escherichia coli fadD with either the Homo sapiens genes hGLYAT3 and hGLYAT2

To generate vectors for the coexpression of the *Homo sapiens* genes hGLYAT2 (SEQ ID NO: 4) or hGYLAT3 (SEQ ID NO: 5), which encodes human glycine-N-acyltransferase, with *Escherichia coli fadD* (SEQ ID NO: 6), which encodes the *E. coli* acyl-CoA synthetase, the genes hGLYAT2 and hGLYAT3 were codon-optimized for expression in *Escherichia coli* and synthesized. The synthesized DNA fragments were digested with the restriction endonucleases Sacll and *Eco*47III and ligated into the correspondingly cut pCDF[atfA1_Ab(co_Ec)-fadD_Ec] (SEQ ID NO: 7) with removal of the *aftA*1 gene. The sequence segments which were additionally removed in this process were cosynthesized during gene synthesis. The vector is a pCDF derivative which already comprises a synthetic tac promoter (SEQ ID NO: 2) and the *Escherichia coli fadD* gene. The resulting expression vectors were named pCDF{Ptac}[hGLYAT2(co_Ec)-fadD_Ec] (SEQ ID NO: 8) and pCDF{Ptac}[hGLYAT3(co_Ec)-fadD_Ec] (SEQ ID NO: 9).

### Example 3

### Generation of vectors for the coexpression of the Homo sapiens hGLYAT2, Escherichia coli fadD and Pseudomonas putida alkL genes

To generate vectors for the coexpression of the hGLYAT2 genes with a modified *Pseudomonas putida* alkL gene, which encodes AlkL, an outer membrane protein that facilitates the import of hydrophobic substrates into a cell, the alkL gene (SEQ ID NO: 10) was amplified together with the lacuv5 promoter (SEQ ID NO: 11) from the plasmid pCDF[alkLmod1] (SEQ ID NO: 12) by means of sequence-specific oligonucleotides. The PCR products were cleaved with the restriction endonucleases BamHI and *Nsi*I and ligated into the correspondingly cleaved vector pCDF{Ptac}[hGLYAT2(co_Ec)-fadD_Ec] (SEQ ID NO: 8). The correct insertion of the target genes was checked by restriction analysis and the authenticity of the introduced genes was verified by DNA sequencing. The resulting expression vector was named pCDF{Ptac}[hGLYAT2(co_Ec)-fadD_Ec]{Placuv5}[alkLmod1] (SEQ ID NO: 13).

The following parameters were used for PCR: 1 x: initial denaturation, 98°C, 3:00 min; 35 x denaturation, 98°C, 0:10 min; annealing, 65°C, 0:20 min; elongation, 72°C, 0:17 min; 1 x: final elongation, 72 °C, 10 min. For amplification the Phusion™ High-Fidelity Master Mix from New England Biolabs (Frankfurt) was used according to manufacturer's manual. 50 µL of the PCR reaction were analyzed on a 1 % TAE agarose gel. Procedure of PCR, agarose gel electrophoresis, ethidium bromide staining of DNA and determination of PCR fragment size were carried out known to those skilled in the art

### Example 4

### Generation of an E. coli strain with deletion in the fadE gene, which strain overexpresses the Umbellularia californica synUcTE, Escherichia coli fadD and Homo sapiens hGLYAT2 and hGLYAT3 genes

To generate *E. coli* strains which coexpress the *Umbellularia californica synUcTE* in combination with the *Escherichia coli fadD* and *Homo sapiens* hGLYAT2 or *Homo sapiens* hGLYAT3 genes, the strain *E.coli* W3110 ΔfadE was transformed with the plasmids pJ294{Ptac}[synUcTE] and pCDF{Ptac}[hGLYAT2(co_Ec)_fadD_Ec] or pCDF{Ptac}[hGLYAT3(co_Ec)_fadD_Ec] by means of electroporation and plated onto LB-agar plates supplemented with spectinomycin (100 µg/mL) and ampicillin (100 µg/mL). Transformants were checked for the presence of the correct plasmids by plasmid preparation and analytic restriction analysis. The strains *E.coli* W3110 ΔfadE pJ294{Ptac}[synUcTE] / pCDF{Ptac}[hGLYAT2(co_Ec)_fadD_Ec] and *E.coli* W3110 ΔfadE pJ294{Ptac}[synUcTE] / pCDF{Ptac}[hGLYAT3(co_Ec)_fadD_Ec] were generated thus.

### Example 5

### Generation of an E.coli strain with deletion in the fadE gene, which strain overexpresses the Escherichia coli fadD and either Homo sapiens hGLYAT2 or hGLYAT3 genes

To generate *E. coli* strains which overexpress the *Escherichia coli fadD* gene in combination with the *Homo sapiens* hGLYAT2 or hGLYAT3 genes, electrocompetent cells of *E. coli* strain W3110 ΔfadE were generated. *E.coli* W3110 ΔfadE was transformed with the plasmid pCDF{Ptac}[hGLYAT2(co_Ec)-fadD_Ec]{Placuv5}[alkLmod1] and plated onto LB-agar plates supplemented with spectinomycin (100 µg/mL). Transformants were checked for the presence of the correct plasmids by plasmid preparation and analytic restriction analysis. The strain generated thus was named *E. coli* W3110 ΔfadE pCDF{Ptac}[hGLYAT2(co_Ec)-fadD_Ec]{Placuv5}[alkLmod1].

### Example 6

### Production of fatty acid/amino acid adducts by E. coli strains with deletion in the fadE gene, which strains overexpress the synUcTE and fadD genes in combination with either hGLYAT2 or hGLYAT3

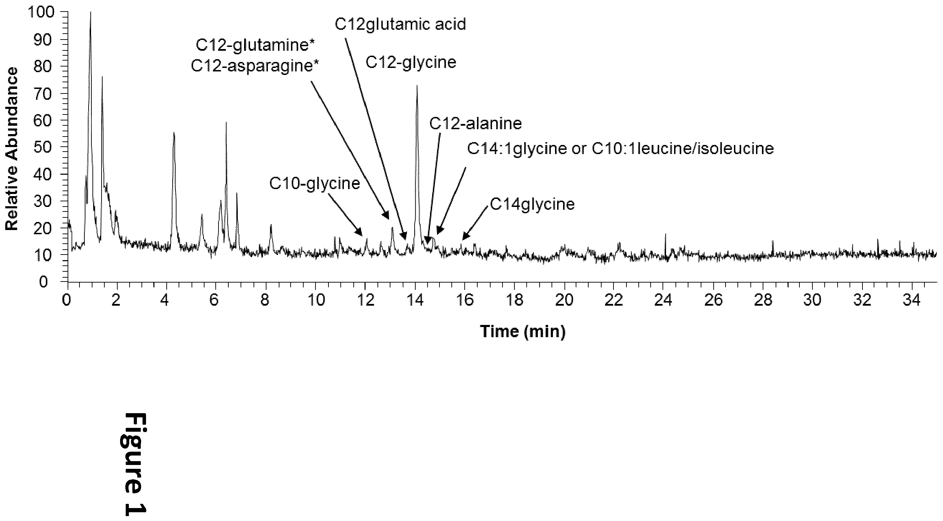

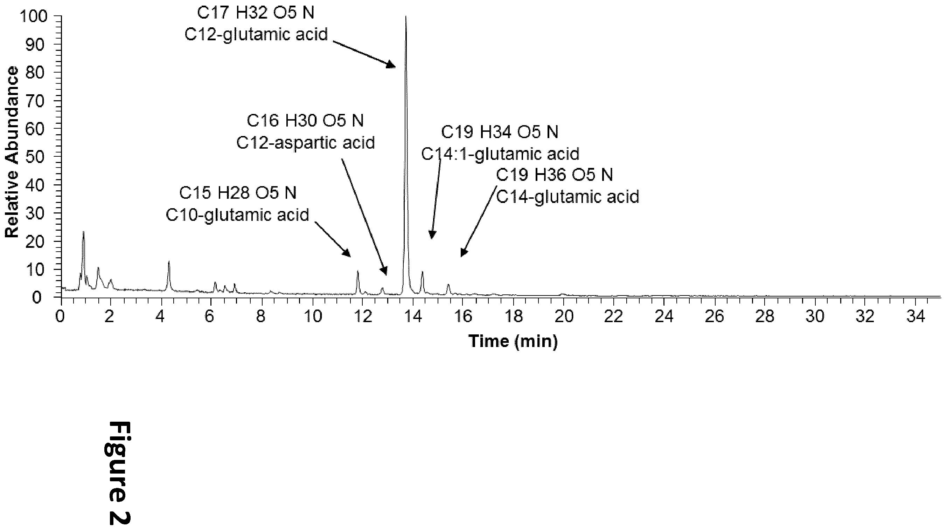

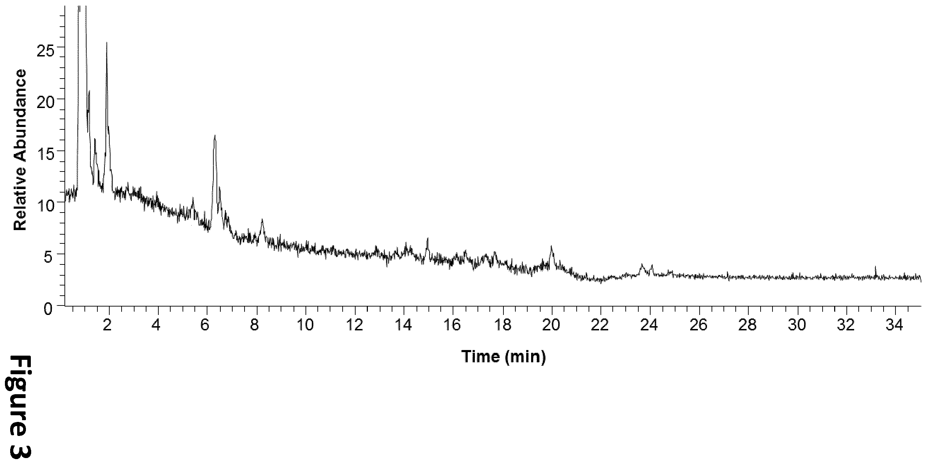

The strains generated in Example 4 were used to study their ability to produce fatty acid/amino acid adducts, proceeding as follows:
Starting from a -80 °C glycerol culture, the strains to be studied were first plated onto an LB-agar plate supplemented with 100 µg/mL ampicillin and 100 µg/mL spectinomycin and incubated overnight at 37 °C. Starting from a single colony in each case, the strains were then grown as a 5-mL preculture in Luria-Bertani broth, Miller (Merck, Darmstadt) supplemented with 100 µg/mL ampicillin and 100 µg/mL spectinomycin. The further culture steps were performed in M9 medium. The medium, composed of 38 mM disodium hydrogenphosphate dihydrate, 22 mM potassium dihydrogenphosphate, 8.6 mM sodium chloride, 37 mM ammonium chloride, 2 % (w/v) glucose, 2 mM magnesium sulphate heptahydrate (all chemicals from Merck, Darmstadt) and 0.1% (v/v) trace element solution, was brought to pH 7.4 with 25 % strength ammonium hydroxide solution. The trace element solution added, composed of 9.7 mM manganese(II) chloride tetrahydrate, 6.5 mM zinc sulphate heptahydrate, 2.5 mM sodium-EDTA (Titriplex III), 4.9 mM boric acid, 1 mM sodium molybdate dihydrate, 32 mM calcium chloride dihydrate, 64 mM iron(II) sulphate heptahydrate and 0.9 mM copper(II) chloride dihydrate, dissolved in 1 M hydrochloric acid (all chemicals from Merck, Darmstadt) was filter-sterilized before being added to the M9 medium. 20 mL of M9 medium supplemented with 100 µg/mL spectinomycin and 100 µg/mL ampicillin were introduced into baffled 100-mL Erlenmeyer flasks and inoculated with 0.5 mL preculture. The flasks were cultured at 37 °C and 200 rpm in a shaker-incubator. After a culture time of 8 hours, 50 mL of M9 medium supplemented with 100 µg/mL spectinomycin and 100 µg/mL ampicillin were introduced into a baffled 250-mL Erlenmeyer flask and inoculated with the 10-mL culture to achieve an optical density (600 nm) of 0.2. The flasks were cultured at 37 °C and 200 rpm in a shaker-incubator. When an optical density (600 nm) of 0.7 to 0.8 was reached, gene expression was induced by addition of 1 mM IPTG. The strains were cultured for a further 48 hours at 30 °C and 200 rpm. Simultaneously with the induction, 1 g/L glycine was added to some of the cultures. During culturing, samples were taken, and fatty acid/amino acid adducts present were analysed. The results are shown in Figures. 1 and 2. It has been possible to demonstrate that both *E.coli* strain W3110 ΔfadE pJ294{Ptac}[synUcTE] / pCDF{Ptac}[hGLYAT2(co_Ec)_fadD_Ec] and *E.coli* strain W3110 ΔfadE pJ294{Ptac}[synUcTE] / pCDF{Ptac}[hGLYAT3(co_Ec)_fadD_Ec] are capable of forming various fatty acid/amino acid adducts, for example lauroyl-glutamic acid, from glucose. By contrast, no such adducts can be found in a cell that, as a negative control, lacks the plasmids (Figure. 3). It appears that slight sequence variations, for example amino acid substitutions that distinguish hGLYAT2 from hGLYAT3, do not compromise the ability of the cell to make the fatty acid/acyl amino acid adducts as shown in Table 3.

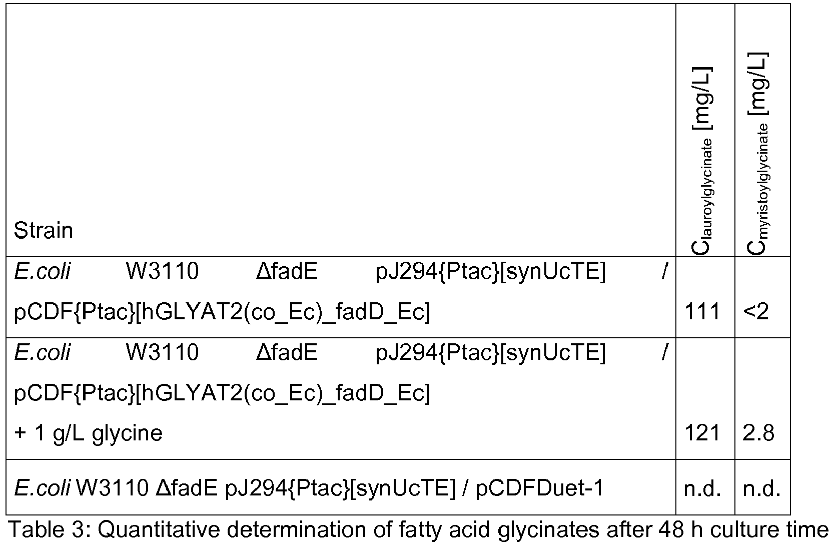

### Example 7

### Chromatographic quantification of products by HPLC/MS

The quantification of N-lauroylglycine, N-myristoylglycine and N-palmitoylglycine and the detection of other acylamino acids in fermentation samples was performed by HPLC-ESI/MS. The quantification was performed with the aid of an external calibration (approx. 0.1 - 50 mg/l) for the three target compounds in the "single ion monitoring" mode (SIM). In parallel, a scan was carried out over a mass range m/z = 100-1000 so as to identify further acylamino acids.

The samples for the determination of the fatty acid glycinates were prepared as follows: 800 µL of solvent (acetone) and 200 µL of sample were pipetted into a 2-mL reaction vessel. The mixture was shaken in a Retsch mill for 1 minute at 30 Hz and then centrifuged for 5 min at approximately 13 000 rpm. The clear supernatant was removed using a pipette and, after suitable dilution with diluent (80 % acetonitrile/20 % water + 0.1 % formic acid), analyzed. The calibration standards used were likewise dissolved and diluted in this diluent.

The following equipment was employed:
- Surveyor HPLC system (Thermo Scientific, Waltham, Massachusetts, USA) composed of MS pump, Autosampler Plus and PDA Detector Plus
- Mass spectrometer TSQ Vantage with HESI II source (Thermo Scientific, Waltham, Massachusetts, USA)
- HPLC column: 100 x 2 mm Pursuit XRS Ultra C8; 2.8 µm (Agilent, Santa Clara, California, USA)

Chemicals:
- Water from a Millipore system
- Acetonitrile for HPLC (Merck AG, Darmstadt, Germany)
- Formic acid, p.a. grade (Merck, Darmstadt, Germany)
- N-propanol Lichrosolv (Merck, Darmstadt, Germany)
- N-lauroylglycine 99 % (Chem-Impex International, Wood Dale, IL, USA)
- N-myristoylglycine > 98 % (Santa Cruz Biotechnology, Texas, USA)
- N-palmitoylglycine > 99 % (provenance unknown)

The HPLC separation was carried out using the abovementioned HPLC column. The injection volume amounted to 2 µL, the column temperature to 40 °C, the flow rate to 0.3 mL/min. The mobile phase consisted of Eluent A (0.1 % strength (v/v) aqueous formic acid) and Eluent B (75 % acetonitrile/25 % n-propanol (v/v) with 0.1 % (v/v) formic acid). The following gradient profile was used:

**Table 4. Gradient profile used in Example 7.**

| **Time [min]** | **Eluent A [%]** | **Eluent B [%]** |
|---|---|---|
| 0 | 90 | 10 |
| 1 | 90 | 10 |
| 20 | 5 | 95 |
| 25 | 5 | 95 |

The HPLC/MS analysis was carried out under positive ionization mode with the following parameters of the ESI source:

| | |
|---|---|
| Spray Voltage: | 3500V |
| Vaporizer Temperature: | 50 °C |
| Sheath Gas Pressure: | 40 |
| Aux. Gas Pressure: | 10 |
| Capillary Temperature: | 250 °C |
| Sprayer Distance: | Ring C |

Detection and quantification of the three analytes were performed by "single ion monitoring" (SIM) with the following parameters shown in Table 5.

**Table 5. Parameters used in SIM of Example 7.**

| **Analyte** | **Ion [M+H] [m/z]** | **Scan range [m/z]** | **Scan time [ms]** | **Resolution Q3** |
|---|---|---|---|---|
| N-lauroylglycine | 258.2 | 0.002 | 50 | 0.7 |
| N-myristoylglycine | 286.2 | 0.002 | 50 | 0.7 |
| N-palmitoylglycine | 314.2 | 0.002 | 50 | 0.7 |

### Example 8

### Production of fatty acid amino acid adducts by E.coli strains with deletion in the fadE gene, which strains overexpress the synUcTE and fadD genes in combination with hGLYAT2 or hGLYAT3 in a parallel fermentation system

The strains generated in Example 4 were used for studying their ability to produce fatty acid amino acid adducts from glucose. For this purpose, the strain was cultured both in a shake flask and in a fed-batch fermentation. The fermentation was carried out in a parallel fermentation system from DASGIP with 8 bioreactors.

The production cells were prepared as described in Example 6.

The fermentation was performed using 1 I reactors equipped with overhead stirrers and impeller blades. pH and pO₂ were measured online for process monitoring. OTR/CTR measurements served for estimating the metabolic activity and cell fitness, inter alia.

The pH electrodes were calibrated by means of a two-point calibration using standard solutions of pH 4.0 and pH 7.0, as specified in DASGIP's technical instructions. The reactors were provided with the necessary sensors and connections as specified in the technical instructions, and the agitator shaft was fitted. The reactors were then charged with 300 mL of water and autoclaved for 20 min at 121 °C to ensure sterility. The pO₂ electrodes were connected to the measuring amplifiers and polarized overnight (for at least 6 h). Thereafter, the water was removed under a clean bench and replaced by M9 medium (pH 7.4) composed of KH₂PO₄ 3.0 g/l, Na₂HPO₄ 6.79 g/l, NaCl 0.5 g/l, NH₄Cl 2.0 g/l, 2 mL of a sterile 1 M MgSO₄^{∗}7H₂O solution and 1 mL/l of a filter-sterilized trace element stock solution (composed of HCI (37 %) 36.50 g/L, MnCl₂^{∗}4H₂O 1.91 g/L, ZnSO₄^{∗}7H₂O 1.87 g/L, ethylenediaminetetraacetic acid dihydrate 0.84 g/L, H₃BO₃ 0.30 g/L, Na₂MoO₄^{∗}2H₂O 0.25 g/L, CaCl₂^{∗}2H₂O 4.70 g/L, FeSO₄^{∗}7H₂O 17.80 g/L, CuCl₂^{∗}2H₂O 0.15 g/L) with 15 g/L glucose as the carbon source (added by metering in 30 mL/L of a sterile feed solution composed of 500 g/L glucose, 1.3 % (w/v) MgSO₄^{∗}7H₂O) supplemented with 100 mg/L spectinomycin and 3 mL/l DOW1500.

Thereafter, the pO₂ electrodes were calibrated to 100 % with a one-point calibration (stirrer: 400 rpm/aeration: 10 sl/h air), and the feed, correction agent and induction agent lines were cleaned by "cleaning in place" as specified in the technical instructions. To this end, the tubes were rinsed first with 70 % ethanol, then with 1 M NaOH, then with sterile fully-demineralized water and, finally, filled with the respective media.

Using the *E. coli* strain of Example 4, a dilution streak was first performed with a cryoculture on an LB agar plate supplemented with 100 mg/l spectinomycin, and the plate was incubated for approximately 16 h at 37 °C. LB medium (10 mL in a 100-mL baffle flask) supplemented with 100 mg/l spectinomycin was then inoculated with a single colony and the culture was grown overnight at 37 °C and 200 rpm for approximately 16 h. Thereafter, this culture was used for a second preculture stage with an initial OD of 0.2 in 50 mL of M9 medium, composed of KH₂PO₄ 3.0 g/l, Na₂HPO₄ 6.79 g/l, NaCl 0.5 g/l, NH₄Cl 2.0 g/l, 2 mL of a sterile 1 M MgSO₄^{∗}7H₂O solution and 1 mL/l of a filter-sterilized trace element stock solution (composed of HCI (37 %) 36.50 g/L, MnCl₂^{∗}4H₂O 1.91 g/L, ZnSO₄^{∗}7H₂O 1.87 g/L, ethylenediaminetetraacetic acid dihydrate 0.84 g/l, H₃BO₃ 0.30 g/l, Na₂MoO₄^{∗}2H₂O 0.25 g/L, CaCl₂^{∗}2H₂O 4.70 g/L, FeSO₄^{∗}7H₂O 17.80 g/L, CuCl₂^{∗}2H₂O 0.15 g/L) supplemented with 20 g/L glucose as carbon source (added by metering in 40 mL/L of a sterile feed solution composed of 500 g/L glucose) together with the above-described antibiotics was transferred into a 500-mL baffle flask and incubated for 8-12 h at 37 °C/200 rpm.

To inoculate the reactors with an optical density of 0.1, the OD₆₀₀ of the second preculture stage was measured and the amount of culture required for the inoculation was calculated. The required amount of culture was placed into the heated and aerated reactor with the aid of a 5-mL syringe through a septum.

The standard program shown in Table 6a-c was used:

**Table 6. Standard program for use of heated and aerated reactor in Example 8**

| a) | | | | | | | | |
|---|---|---|---|---|---|---|---|---|
| | | DO controller | | pH controller | | | | |
| | | Preset | 0 % | Preset | 0 mL/h | | | |
| | | P | 0.1 | P | 5 | | | |
| | | Ti | 300 s | Ti | 200 s | | | |
| | | Min | 0 % | Min | 0 mL/h | | | |
| | | Max | 100 % | Max | 40 mL/h | | | |

| b) | | | | | | | | |
|---|---|---|---|---|---|---|---|---|
| N (Rotation) | from | to | XO2 (gas mixture) | from | to | F (gas flow) | from | to |
| Growth and | 0 % | 30% | Growth and biotransformation | 0% | 100 % | Growth and biotransformation | 15% | 80% |
| biotransformation | 400 rpm | 1500 rpm | | 21 % | 21 % | | 6 sl/h | 72 sl/h |

| c) | | | | | | | | |
|---|---|---|---|---|---|---|---|---|
| | | Script | | | | | | |
| | | Trigger fires | | 31 % DO (1/60h) | | | | |
| | | Induction IPTG | | 2 h after the feed start | | | | |
| | | Feed trigger | | 50 % DO | | | | |
| | | Feed rate | | 3 [mL/h] | | | | |

The pH was adjusted unilaterally to pH 7.0 with 12.5 % strength ammonia solution. During the growth phase and the biotransformation, the dissolved oxygen (pO₂ or DO) in the culture was adjusted to at least 30 % via the stirrer speed and the aeration rate. After the inoculation, the DO dropped from 100 % to these 30 %, where it was maintained stably for the remainder of the fermentation.

The fermentation was carried out as a fed batch, the feed start as the beginning of the feed phase with 5 g/l^{∗}h glucose feed, composed of 500 g/l glucose, 1.3 % (w/v) MgSO₄^{∗}7H₂O, being triggered via the DO peak which indicates the end of the batch phase. From the feed start onwards, the temperature was reduced from 37 °C to 30 °C. 2 h after the feed start, the expression was induced with 1 mM IPTG.

To quantify lauroyl, myristoyl and palmitoyl glycinate, samples were taken 47 h and 64 h after the start of the fermentation. These samples were prepared for analysis, and analyzed as described in Example 7.

It has been possible to demonstrate that strain *E. coli* W3110 ΔfadE pJ294{Ptac}[synUcTE] / pCDF{Ptac}[hGLYAT2(co_Ec)_fadD_Ec] is capable of forming lauroyl glycinate from glucose. The results are shown in Tables 7 and 8.

**Table 7: Quantification of fatty acid glycinates after 47 and 64 h fermentation time.**

| **Analyte** | **Ion [M+H] [m/z]** | **Scan range [m/z]** | **Scan time [ms]** | **Resolution Q3** |
|---|---|---|---|---|
| N-Lauroylglycine | 258.2 | 0.002 | 50 | 0.7 |
| N-Myristoylglycine | 286.2 | 0.002 | 50 | 0.7 |
| N-Palmitoylglycine | 314.2 | 0.002 | 50 | 0.7 |

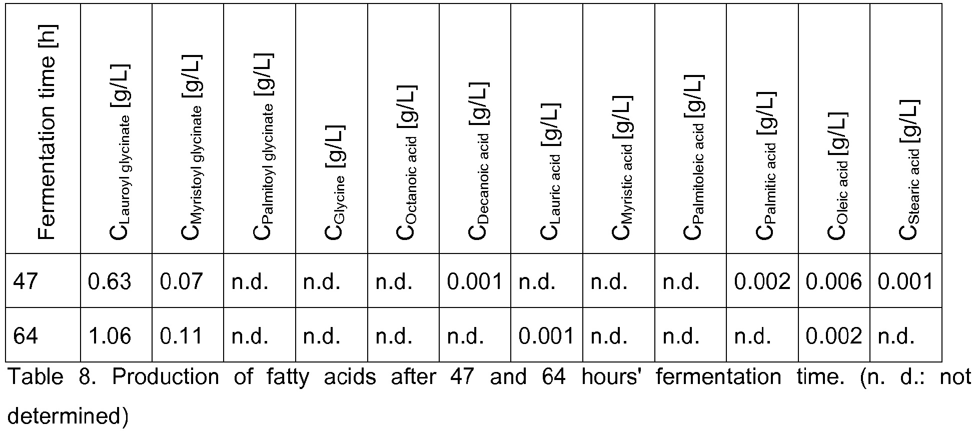

### Example 9

The strain of Example 5 was fermented in a fed-batch fermentation to study the ability of linking lauric acid and glycine to give lauroyl glycinate. This fermentation was carried out in a parallel fermentation system from DASGIP with 8 bioreactors.

The experimental setting was as described in Example 8 except that 100 g/l glycine in demineralized water and 100 g/l laurate in lauric acid methyl ester were fed rather than glucose. To quantify lauroyl, myristoyl and palmitoyl glycinate in fermentation samples, samples were taken 23 h and 42 h after the start of the fermentation. These samples were prepared for analysis, and analyzed as described in Example 7. The results are shown in Tables 9 and 10.

It has been possible to demonstrate that the strain *E.coli* W3110 ΔfadE pCDF{Ptac}[hGLYAT2(co_Ec)_fadD_Ec] {Plavuv5} [alkLmod1] is capable of linking lauric acid and glycine and of producing lauroyl glycinate.

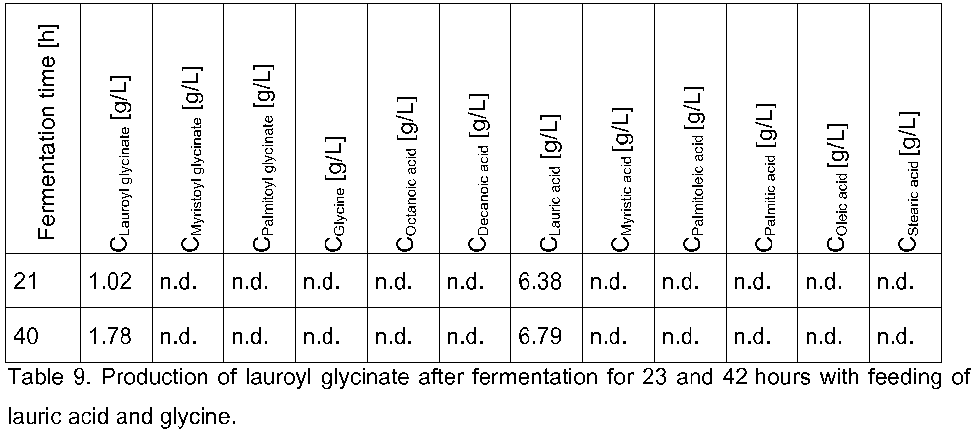

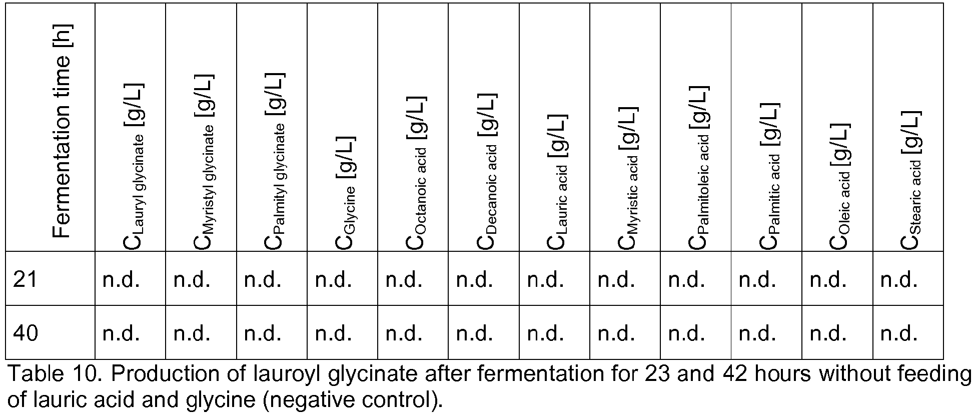

### Example 10

### Generation of vectors for expression of the Homo sapiens genes hGLYAT3 and hGLYAT2 in E.coli strains producing fatty acids via Malonyl-CoA and Acetyl-CoA

To generate vectors for the expression of the *Homo sapiens* genes hGLYAT2 (SEQ ID NO: 4) or hGYLAT3 (SEQ ID NO: 5) in the fatty acid producing strains listed in Table 13 below.(From Table 3.2 of WO2014026162A1), the genes hGLYAT2 and hGYLAT3 were first amplified. The gene hGLYAT2 was amplified from the plasmid pCDF{Ptac}[hGLYAT2(co_Ec)-fadD_Ec]{Placuv5}[alkLmod1] (SEQ ID NO: 13) and the hGYLAT3 gene was amplified from the plasmid pCDF{Ptac}[hGLYAT3(co_Ec)-fadD_Ec] (SEQ ID No: 9) by means of sequence-specific oligonucleotides. The PCR products were cleaved with the restriction endonucleases *NotI* and *SacI* and ligated in the correspondingly cleaved vector pET-28b (SEQ ID NO: 14). The correct insertion of the target genes was checked by restriction analysis and the authenticity of the introduced genes was verified by DNA sequencing. The resulting expression vectors were named pET-28b{Ptac}[hGLYAT2(co_Ec)] (SEQ ID No: 15) and pET-28b{Ptac}[hGLYAT3(co_Ec)] (SEQ ID No: 16).

### Example 11

### Production of fatty acid amino acid adducts via Malonyl-CoA and Acetyl-CoA by strains overexpressing hGLYAT2 or hGLYAT3 in a shake flask experiment

The vectors produced according to Example 10 were then used to generate a microorganism strain from Table 11 below (OPX Biotechnologies Inc., USA) using any transformation method known in the art. In particular, the methods provided in section IV of WO2014026162A1 were used.

The strains generated were used for studying their ability to produce fatty acids, in particular amino acid adducts from glucose. For this purpose, the strains were transformed with the vectors pET-28b{Ptac}[hGLYAT2(co_Ec)] (SEQ ID NO: 15) and pET-28b{Ptac}[hGLYAT3(co_Ec)] (SEQ ID NO: 16) and cultured in shake flasks (Subsection C of section IV of WO2014026162A1). Strain BXF_031 (OPX Biotechnologies Inc., USA) harbouring the empty vector pET-28b was used as a control.

Triplicate evaluations were performed. Briefly, overnight starter cultures were made in 50 mL of Terrific Broth including the appropriate antibiotics and incubated 16-24 hours at 30°C., while shaking at 225 rpm. These cultures were used to inoculate 150 mL cultures of each strain in SM11 minimal medium to an OD₆₀₀ of 0.8 and 5% TB culture carryover as starting inoculum, and antibiotics. 1 L SM11 medium consists of: 2 mL FM10 Trace Mineral Stock, 2.26 mL 1M MgSO₄, 30 g glucose, 200 mM MOPS (pH 7.4), 1 g/L yeast extract, 1.25 mL VM1 Vitamin Mix, 0.329 g K₂HPO₄, 0.173 g KH₂PO₄, 3 g (NH₄)₂SO₄, 0.15 g citric acid (anhydrous); FM10 Trace Mineral Stock consists of: 1 mL of concentrated HCI, 4.9 g CaCl₂^{∗}2H₂O,0.97 g FeCl₃^{∗}6H₂O, 0.04 g CoCl₂^{∗}6H₂O, 0.27 g CuCl₂^{∗}2H₂O, 0.02 g ZnCl₂, 0.024 g Na₂MoO₄^{∗}2H₂O, 0.007 g H₃BO₃, 0.036 g MnCl2^{∗}4H₂O, Q.S. with DI water to 100 mL; VM1 Vitamin Mix Solution consists of: 5 g Thiamine, 5.4 g Pantothenic acid, 6.0 g Niacin, 0.06 g, Q.S. with DI water to 1000 mL. All ingredients for the culture mediums used in this example are provided in (Subsection A of section IV of WO2014026162A1).

Cultures were incubated for 2 hours at 30°C., while shaking at 225 rpm. After 2 hours, the cells were washed with SM11 (SM11 medium without phosphate). Cells were twice spun down (4,000 rpm, 15 min), the supernatant decanted, the pellet re-suspended in 150 mL of SM11 (SM11 medium without phosphate). The cultures were used to inoculate 3×50 mL of each strain in SM11 (no phosphate). The cultures were grown at 30° C. for approximately 2 h to an OD₆₀₀ of 1.0-1.5 after 2 h cells and shifted to 37° C. and samples removed periodically for product measurement over the course of 72 hrs.

The quantification of N-lauroylglycine, N-myristoylglycine and N-palmitoylglycine and the detection of other acylamino acids in fermentation samples was performed by HPLC-ESI/MS as described in Example 7.

**Table 11. List of microorganism strains that were used to introduce the genes hGLYAT2 or hGLYAT3 in examples 10 and 11. The method of production and the sequences of the strains are provided in Table 3.2 of WO2014026162A1 (OPX Biotechnologies Inc., USA).**

| **Strain designation** | **Host** | **Plasmid** | **SEQ ID NOs.** |
|---|---|---|---|
| BXF_0012 | BX_864 | 1)pBMT-3_ccdAB | 17 |
| BXF_0013 | BX_864 | 1)pBMT-3_ccdAB_P_{T7}-'tesA | 18 |
| BXF_0014 | BX_864 | 1)pBMT-3_ccdAB_P_{T7}-nphT7-hbd-crt-ter | 19 |
| BXF_0015 | BX_864 | 1)pBMT-3_ccdAB_P_{T7}-'tesA_PT7-nph_{T7}-hbd-crt-ter | 20 |
| BXF_0020 | BX_860 | 1)pBMT-3_ccdAB_PT7-'tesA_PT7-nphT7-hbd-crt-ter | 20 |
| BXF_0021 | BX_876 | 1)pBMT-3_ccdAB_PT7-'tesA_PT7-nphT7-hbd-crt-ter | 20 |
| BXF_0022 | BX_874 | 1)pBMT-3_ccdAB | 17 |
| BXF_0023 | BX_874 | 1)pBMT-3_ccdAB_PT7-'tesA | 18 |
| BXF_0024 | BX_874 | 1)pBMT-3_ccdAB_PT7-'tesA_PT7-nphT7-hbd-crt-ter | 20 |
| BXF_0025 | BX_875 | 1)pBMT-3_ccdAB | 17 |
| BXF_0026 | BX_875 | 1)pBMT-3_ccdAB_PT7-'tesA | 18 |
| BXF_0027 | BX_875 | 1)pBMT-3_ccdAB_PT7-'tesA_PT7-nphT7-hbd-crt-ter | 20 |
| BXF_0028 | BX_878 | 1)pBMT-3ccdAB-T7-'tesA-PT7_nphT7_hbd_crt_ter | 20 |
| BXF_0028 | BX_878 | 1)pBMT-3_ccdAB_PT7-'tesA_PT7-nphT7-hbd-crt-ter | 20 |
| BXF_0029 | BX_879 | 1)pBMT-3_ccdAB_PT7-'tesA_PT7-nphT7-hbd-crt-ter | 20 |
| BXF_0030 | BX_881 | 1)pBMT-3_ccdAB_PT7-'tesA_PT7-nphT7-hbd-crt-ter | 20 |
| BXF_0031 | BX_864 | 1)pBMT-3_ccdAB_PT7-'tesA_PT7-nphT7-hbd-crt-ter | 20 |
| | | 2)pET-28b(empty vector) | 21 |
| BXF_0033 | BX_878 | 1)pBMT-3_ccdAB_PT7-nphT7-hbd-crt-ter | 19 |
| BXF_0034 | BX_879 | 2)pBMT-3_ccdAB_PT7-nphT7-hbd-crt-ter | 19 |

### Example 12

### Generation of a vector for deletion of the gcvTHP operon in Escherichia coli W3110 ΔfadE

To generate a vector for the deletion of the *gcvTHP* operon of *E. coli* W3110, which encodes a glycine cleavage system (*gcvT*: aminomethyltransferase,tetrahydrofolate-dependent, subunit (T protein) of glycine cleavage complex; *gcvH*: glycine cleavage complex lipoylprotein; *gcvP*: glycine decarboxylase, PLP-dependent, subunit (protein P) of glycine cleavage complex), approx. 500 bp upstream and downstream of the *gcvTHP* operon were amplified via PCR. The upstream region of gcvTHP was amplified using the oligonucleotides o-MO-40 (SEQ ID No:22). And o-MO-41 (SEQ ID No:23) The downstream region of *gcvTHP* was amplified using the oligonucleotides o-MO-42 (SEQ ID No:24) and o-MO-43 (SEQ ID No:25). The PCR procedure is described above in Example 3.

In each case PCR fragments of the expected size could be amplified (PCR 1,553 bp, (SEQ ID No:26); PCR 2,547 bp, SEQ ID No:27). The PCR samples were separated via agarose gel electrophoresis and DNA fragments were isolated with *QiaQuick Gel extraction Kit* (Qiagen, Hilden). The purified PCR fragments were cloned into the vector pKO3 (SEQ ID No:28), and cut with BamHI using the Geneart® Seamless Cloning and Assembly Kit (Life Technologies, Carlsbad, CA, USA). The assembled product was transformed into chemically competent *E. coli* DH5a cells (New England Biolabs, Frankfurt). Procedure of PCR purification, in-vitro cloning and transformation were carried out according to manufacturer's manual. The correct insertion of the target genes was checked by restriction analysis and the authenticity of the introduced DNA fragments was verified by DNA sequencing. The resulting knock-out vector was named pKO3 delta gcvTHP (SEQ ID No:29).

The construction of strain *E. coli* W3110 *ΔfadE ΔgcvTHP* was carried out with the help of pKO3 delta gcvTHP using the method described in Link et al., 1997. The DNA sequence after deletion of *gcvTHP* is SEQ ID No: 30. The *E. coli* strain W3110 *ΔfadE ΔgcvTHP* was transformed with the plasmid pCDF{Ptac}[hGLYAT2(co_Ec)-fadD_Ec]{Placuv5}[alkLmod1] (SEQ ID No: 13, Example 3) by means of electroporation and plated onto LB-agar plates supplemented with spectinomycin (100 µg/mL). Transformants were checked for the presence of the correct plasmids by plasmid preparation and analytic restriction analysis. The resulting strain was named *E. coli* W3110 Δ*fadE*Δ*gcvTHP* pCDF{Ptac}[hGLYAT2(co_Ec)-fad D _Ec]{Placuv5}[alkLmod1].

### Example 13

### Production of lauroylglycinate by E. coli strains with deletion in the fadE or fadE I gcvTHP gene, overexpressing the hGLYAT2, fadD and alkL genes

The strain generated in Example 1 was used to study its ability to produce more lauroylglycinate, in comparison to the reference strain without *gcvTHP* deletion.

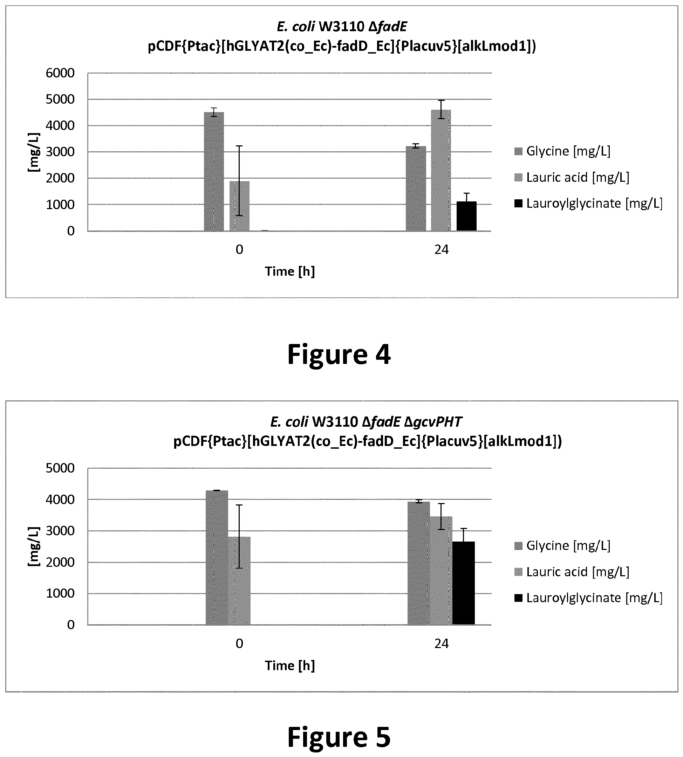

Starting from a -80 °C glycerol culture, the strains to be studied were first plated onto an LB-agar plate supplemented with 100 µg/mL spectinomycin and incubated overnight at 37 °C. Starting from a single colony in each case, the strains were then grown as a 5-mL preculture in LB-broth, Miller (Merck, Darmstadt) supplemented with 100 µg/mL spectinomycin. The further culture steps were performed in M9-FIT medium. The medium, composed of 38 mM disodium hydrogenphosphate dihydrate, 22 mM potassium dihydrogenphosphate, 8.6 mM sodium chloride, 37 mM ammonium chloride, 2 mM magnesium sulphate heptahydrate (all chemicals from Merck, Darmstadt), 5 % (w/v) maltodextrin solution (dextrose equivalent 13.0-17.0, Sigma Aldrich, Taufkirchen), 1 % (w/v) amyloglycosidase from *Aspergillus niger* (Sigma-Aldrich, Taufkirchen), 1 drop Delamex 180 (Bussetti & Co, Wien) and 0.1% (v/v) trace element solution, was brought to pH 7.4 with 25 % strength ammonium hydroxide solution. The trace element solution added, composed of 9.7 mM manganese(II) chloride tetrahydrate, 6.5 mM zinc sulphate heptahydrate, 2.5 mM sodium-EDTA (Titriplex III), 4.9 mM boric acid, 1 mM sodium molybdate dihydrate, 32 mM calcium chloride dihydrate, 64 mM iron(II) sulphate heptahydrate and 0.9 mM copper(II) chloride dihydrate, dissolved in 1 M hydrochloric acid (all chemicals from Merck, Darmstadt) was filter-sterilized before being added to the M9 medium. 20 mL of M9 medium supplemented with 100 µg/mL spectinomycin were introduced into baffled 100-mL Erlenmeyer flasks and inoculated with 0.5 mL preculture. The flasks were cultured at 37 °C and 200 rpm in a shaker-incubator. After a culture time of 8 hours, 50 mL of M9 medium supplemented with 100 µg/mL spectinomycin and were introduced into a baffled 250-mL Erlenmeyer flask and inoculated with the 10-mL culture to achieve an optical density (600 nm) of 0.1. The flasks were cultured at 37 °C and 200 rpm in a shaker-incubator. When an optical density (600 nm) of 0.6 to 0.8 was reached, gene expression was induced by addition of 1 mM IPTG. The strains were cultured for a further 48 hours at 37 °C and 200 rpm. 1-3 h after the induction, 6 g/L glycine and 6 g/L lauric acid (dissolved in lauric acid methyl ester) were added to the cultures. After 0 h and 24 h cultivation samples were taken, and lauroylglycinate, lauric acid and glycine present were analysed. The results are shown in Figures 4 and 5. It has been possible to demonstrate that both *E. coli* strains W3110 Δ*fadE* pCDF{Ptac}[hGLYAT2(co_Ec)-fadD_Ec]{Placuv5}[alkLmod1] and *E. coli* strain W3110 Δ*fadE* Δ*gcvTHP* pCDF{Ptac}[hGLYAT2(co_Ec)-fadD_Ec]{Placuv5}[alkLmod1] are capable of forming lauroylglycinate. But in the new strain background Δ*fadE* Δ*gcvTHP* higher amounts of lauroylglycinate were synthesized and higher amounts of glycine are detected. It appears that less glycine is metabolized in the Δ*gcvTHP* background and can be used for lauroylglycinate synthesis.

### Example 14

### Generation of a vector for deletion of the glyA gene in Escherichia coli W3110 ΔfadE

To generate a vector for the deletion of the *glyA*-gene encoding a component of the Glycine hydroxymethyltransferase of *E. coli* W3110 approx. 500 bp upstream and downstream of the *glyA*-gene were amplified via PCR. The upstream region of *glyA* was amplified using the oligonucleotides o-MO-44 (SEQ ID No:31) and o-MO-45 (SEQ ID No:32). The downstream region of glyA was amplified using the oligonucleotides o-MO-46 (SEQ ID No:33) and o-MO-47 (SEQ ID No:34).

In each case PCR fragments of the expected size could be amplified (PCR 1,546 bp, (SEQ ID No:35); PCR 2,520 bp, SEQ ID No:36). The PCR samples were separated via agarose gel electrophoresis and DNA fragments were isolated with *QiaQuick Gel extraction Kit* (Qiagen, Hilden). The purified PCR fragments were assembled via a crossover PCR. The generated fragment was purified and subcloned into the cloning vector pCR®-Blunt IITOPO (Life technologies) according to manufacturer's manual. To clone the fragment into the target plasmid pKO3 (SEQ ID No:28) it was amplified with flanking Ba*m*HI restriction sites, using the oligonucleotides o-MO-52 (SEQ ID No:37) and o-MO-53 (SEQ ID No:38). The purified, BamHI cleaved PCR 3 fragment (SEQ ID No:39) was ligated into the correspondingly cleaved vector pKO3 (SEQ ID No:28). The assembled product was transformed into chemically competent *E. coli* DH5α cells (New England Biolabs, Frankfurt). Procedure of PCR purification, in-vitro cloning and transformation were carried out according to manufacturer's manual. The correct insertion of the target genes was checked by restriction analysis and the authenticity of the introduced genes was verified by DNA sequencing. The resulting knock-out vector was named pKO3 delta glyA (SEQ ID No:40).

The construction of strain *E. coli* W3110 Δ*fadE* Δ*glyA* was carried out with the help of pKO3 delta glyA using the method described in Link et al., 1997. SEQ ID No:41 is the DNA sequence after deletion of *glyA.* The *E. coli* strain W3110 Δ*fadE* Δ*gcvTHP* was transformed with the plasmid pCDF{Ptac}[hGLYAT2(co_Ec)-fadD_Ec]{Placuv5}[alkLmod1] (SEQ ID No: 13 from Example 3), by means of electroporation and plated onto LB-agar plates supplemented with spectinomycin (100 µg/mL). Transformants were checked for the presence of the correct plasmids by plasmid preparation and analytic restriction analysis. The resulting strain was named *E. coli* W3110 Δ*fadE*Δ*glyA* pCDF{Ptac}[hGLYAT2(co_Ec)-fad D_Ec]{Placuv5}[alkLmod1].

### Example 15

### Generation of a vector for deletion of the ltaE gene in Escherichia coli W3110 ΔfadE

To generate a vector for the deletion of the *ltaE*-gene encoding the L-allo-threonine aldolase of *E. coli* W3110 approximately 500 bp upstream and downstream of the *ltaE* were amplified via PCR as described above. The upstream region of *ltaE* was amplified using the oligonucleotides ltaE-UP_fw (SEQ ID No:42) and ltAE-UP-Xhol_rev (SEQ ID No:43). The downstream region of *ltaE* was amplified using the oligonucleotides ItaE-DOWN_fw (SEQ ID No:44) and ltaE-DOWN_rev (SEQ ID No:45).

In each case PCR fragments of the expected size could be amplified (PCR 4,550 bp, (SEQ ID No:46); PCR 5, 536 bp, SEQ ID No:47). The PCR samples were separated via agarose gel electrophoresis and DNA fragments were isolated with *QiaQuick Gel extraction Kit* (Qiagen, Hilden). The purified PCR fragments were assembled via a crossover PCR. The generated fragment was purified and cloned into the cloning vector pCR®-Blunt IITOPO (Life technologies) according to manufacturer's manual. To clone the fragment into the target plasmid pKO3 (SEQ ID No:28) it was amplified with flanking *Bam*HI restriction sites, using the oligonucleotides o-MO-54 (SEQ ID No:48) and o-MO-55 (SEQ ID No:49). The purified, BamHI cleaved PCR 6 fragment (SEQ ID No:50) was ligated into the correspondingly cleaved vector pKO3 (SEQ ID No:28). The assembled product was transformed into chemically competent *E. coli* DH5α cells (New England Biolabs, Frankfurt). Procedure of PCR purification, in-vitro cloning and transformation were carried out according to manufacturer's manual. The correct insertion of the target genes was checked by restriction analysis and the authenticity of the introduced genes was verified by DNA sequencing. The resulting knock-out vector was named pKO3 delta ltaE (SEQ ID No:51).

The construction of strain *E. coli* W3110 Δ*fadE* Δ*ltaE* was carried out with the help of pKO3 delta ltaE using the method described in Link et al., 1997 (Link AJ, Phillips D, Church GM. J.Bacteriol. 1997. 179(20). The DNA sequence after deletion of *ltaE* is described in SEQ ID No:52). The *E. coli* strain W3110 Δ*fadE* Δ*ltaE* was transformed with the plasmid pCDF{Ptac}[hGLYAT2(co_Ec)-fadD_Ec]{Placuv5}[alkLmod1] (SEQ ID NO: 13 from Example 3), by means of electroporation and plated onto LB-agar plates supplemented with spectinomycin (100 µg/mL). Transformants were checked for the presence of the correct plasmids by plasmid preparation and analytic restriction analysis. The resulting strain was named *E. coli* W3110 Δ*fadE*Δ*ltaE* pCDF{Ptac}[hGLYAT2(co_Ec)-fadD_Ec]{Placuv5}[alkLmod1].

### Example 16

### LC-ESI/MS²-based quantification of lauric acid

Quantification of lauric acid in fermentation samples was carried out by means of LC-ESI/MS² on the basis of an external calibration for lauric acid (0.1 - 50 mg/L) and by using the internal standard d3-LS.

The following instruments were used:
- HPLC system 1260 (Agilent; Böblingen) with Autosampler (G1367E), binary pump (G1312B) and thermo-statted column (G1316A)
- Mass spectrometer TripelQuad 6410 (Agilent; Böblingen) with ESI source
- HPLC column: Kinetex C18, 100 x 2.1 mm, particle size: 2.6 µm, pore size 100 Å (Phenomenex; Aschaffenburg)
- Pre-column: KrudKatcher Ultra HPLC In-Line Filter; 0.5 µm filter depth and 0.004 mm inner diameter (Phenomenex; Aschaffenburg)
-

The samples were prepared by pipetting 1900 µL of solvent (80 % (v/v) ACN, 20% double-distilled H₂O (v/v), + 0.1 % formic acid) and 100 µL of sample into a 2 mL reaction vessel. The mixture was vortexed for approx. 10 seconds and then centrifuged at approx. 13 000 rpm for 5 min. The clear supernatant was removed using a pipette and analysed after appropriate dilution with a diluent (80 % (v/v) ACN, 20 % double-distilled H₂O (v/v), + 0.1 % formic acid). In each case, 100 µL of ISTD were added to 900 µL of sample (10 µL with a sample volume of 90 µL).

HPLC separation was carried out using the above-mentioned column and pre-column. The injection volume is 1.0 µL, the column temperature 50 °C, the flow rate is 0.6 mL/min. the mobile phase consists of eluent A (0.1 % strength (v/v) aqueous formic acid) and eluent B (acetonitrile with 0.1 % (v/v) formic acid). The gradient shown it Table 14 was utilized:

**Table 12. Concentrations of Eluent A and B used in Example 16**

| **Time [min]** | **Eluent A [%]** | **Eluent B [%]** |
|---|---|---|
| 0 | 85 | 15 |
| 1 | 85 | 15 |
| 5 | 2 | 98 |
| 8 | 2 | 98 |
| 8.1 | 85 | 15 |
| 12 | 85 | 15 |

ESI-MS² analysis was carried out in positive mode with the following parameters of the ESI source:
- Gas temperature 320°C
- Gas flow 11 L/min
- Nebulizer pressure 50 psi
- Capillary voltage 4000 V

Detection and quantification of lauric acid was carried out with the following MRM parameters.

**Table 13. MRM parameters used in detection and quantification of lauric acid**

| **Analyte** | **Precursor ion [m/z]** | **Product ion [m/z]** | **Collision energy [eV]** |
|---|---|---|---|
| LS | 201.1 | 201.1 | 110 |
| d3-LS | 204.1 | 204.1 | 110 |

### Example 17

### Detection of glycine

Detection of glycine was performed via derivatization with ortho-phthaldialdehyde (OPA) and UV/VIS detection using an Agilent 1200 HPLC system.

200 µL of a homogeneous fermentation broth simple was mixed with 1800 µL of 30% (v/v) 1-propanol, vortexed for 10 s and subsequently centrifuged at 13,000 x g for 5 min. The supernatant was removed and used for HPLC analysis using the following parameters:

| | | | | |
|---|---|---|---|---|
| **Mobile phase:** | Eluent A | | | |
| | 2.5 mL acetic acid per 1 L distilled water, pH adjustment with NaOH @ 6.0 | | | |
| | Eluent B | | | |
| | Methanol | | | |
| **Column:** | Luna 5µ C8 100A (100 x 4.6 mm); Phenomenex | | | |
| **Column oven temperature:** | 40 °C | | | |
| **Flow:** | 1.0 mL/min | | | |

| **Gradient:** | **Time** | **%B** | **Flow** | **Max. Press.** |
|---|---|---|---|---|
| | 0.0 | 30.0 | 1.0 | 400 |
| | 1.0 | 30.0 | 1.0 | 400 |
| | 17.0 | 90.0 | 1.0 | 400 |
| | 19.5 | 90.0 | 1.0 | 400 |
| | 19.6 | 30.0 | 1.0 | 400 |
| | 20.5 | 30.0 | 1.0 | 400 |
| **Run time:** | 22 min | | | |
| **Detector:** | DAD | | | |
| | 334 nm | | | |
| | Spectrum | | | |
| | Store: all | | | |
| | Range: 200-400 nm | | | |
| | step 2 nm | | | |
| | | | | |
| | FLD (excitation @ 330 nm; emission @ 450 nm, PMT gain 13) | | | |
| **Derivatization:** | | automatically with injection program: | | |

| | **Inject** | **Programm** | | |
|---|---|---|---|---|
| | # | Command | | |
| | 1 | DRAW 4.5 µL from Vial 1*, def. speed, def. offset | | |
| | 2 | DRAW 1.5 µL from sample, def. speed, def. offset | | |
| | 3 | DRAW 0.5 µL from air, def. speed | | |
| | 4 | NEEDLE wash in flush Port. 15.0 sec | | |
| | 5 | DRAW 4.5 µL from Vial 1, def. speed, def. offset | | |
| | 6 | MIX 11.0 µL in seat, def. speed, 1 times | | |
| | 7 | WAIT 1.00 min | | |
| | 8 | INJECT | | |
| | 9 | WAIT 0.50 min | | |
| | 10 | VALVE bypass | | |
| | 11 | Draw 100.0 µL from Vial 2*, def. speed, def. offset | | |
| | 12 | Eject 100.0 µL from Vial 2, def. speed, def. offset | | |
| | 13 | Valve mainpass | | |

| | | | | |
|---|---|---|---|---|
| *vial 1 contains OPA reagent (see below); vial 2 contains water | | | | |

### Preparation of OPA reagent

100 mg o-phthaldialdehyde was dissolved in 1 mL methanol and subsequently 0.4 mM borate buffer (pH 10.4) was added to give 10 mL. Subsequently, 50 µL mercaptoethanol was added and the reagent stored at 4°C. Additional 10 µL mercaptoethanol was added before use.

### Preparation of borate buffer (0.4 mM H₃BO₄):

38.1 g Na₂B₄O₇ ^{∗} 10 H₂O (0.1 mol) were dissolved in 1 L distilled water and the pH adjusted to 10.4 M NaOH auf 10,4 eingestellt. Subsequently, 1 mL 25% Brij35 (v/v) was added.
Retention time: Glycine: 7.153 min

### Example 18

***Generation of vectors for the expression of hGLYAT2-homologs***To generate vectors for the expression of *N-acyltransferases* of different organisms, variants found in the NCBI databases with homology to HGLYAT2 were synthesized and codon-optimized for *E. coli.* These were *glycine N-acyltransferase-like* protein 2 isoform 1 of Nomascus leucogenys *(NI, XP_003275392.1, SEQ ID No:53), glycine N-acyltransferase-like protein 2 of* Saimiri boliviensis *(Sb, XP_003920208.1, SEQ ID No:54), glycine-N-acyltransferase-like 2 of* Felis catus *(Fc, XP_003993512.1, SEQ ID No:55), glycine N-acyltransferase-like protein 2 of Bos* taurus *(Bt, NP_001178259.1, SEQ ID No:56), and glycine N-acyltransferase of* Mus musculus *(Mm, NP_666047.1, SEQ ID No:57).*

The hGLYAT2-gene of pCDF{Ptac}[hGLYAT2(co_Ec)-fadD_Ec]{Placuv5}[alkLmod1] (SEQ ID No: 13 of Example 3) was replaced by this variants as follows: The synthesized DNA fragments were digested with the restriction endonucleases *Bam*HI and *AsiS*I and ligated into the correspondingly cut pCDF{Ptac}[hGLYAT2(co_Ec)-fadD_Ec]{Placuv5}[alkLmod1].

The correct insertion of the target genes was checked by restriction analysis and the authenticity of the introduced genes was verified by DNA sequencing. The resulting expression vectors were named:
pCDF{Ptac}[GLYAT_NI(co_Ec)-fadD_Ec]{Placuv5}[alkLmod1]
pCDF{Ptac}[GLYAT_Sb(co_Ec)-fadD_Ec]{Placuv5}[alkLmod1]
pCDF{Ptac}[GLYAT_Fc(co_Ec)-fadD_Ec]{Placuv5}[alkLmod1]
pCDF{Ptac}[GLYAT_Bt(co_Ec)-fadD_Ec]{Placuv5}[alkLmod1]
pCDF{Ptac}[GLYAT_Mm(co_Ec)-fadD_Ec]{Placuv5}[alkLmod1]

### Example 19

### Production of lauroylglycinate by E. coli strains with deletion in the fadE gene, overexpressing the hGLYAT-variants, fadD and alkL genes

The strains generated in Example 18 were used to study their ability to produce lauroylglycinate, in comparison to the reference strain expressing hGLYAT2 harbouring the plasmid pCDF{Ptac}[hGLYAT2(co_Ec)-fadD_Ec]{Placuv5}[alkLmod1], applying the protocol described in Example 12.

1-3 h after the induction, 6 g/L glycine and 6 g/L lauric acid (dissolved in lauric acid methyl ester) were added to the cultures. After a cultivation time of 48 h the entire broth of a shake flask was extracted with Acetone (ratio 1:2). Further sample treatment is described in Example 7. Samples were taken, and lauroylglycinate, lauric acid and glycine present were analysed. The results are shown in Table 14.

All strains except the none-plasmid control produced lauroylglycinate in amounts between 0.44 and 2109.8 mg/L.

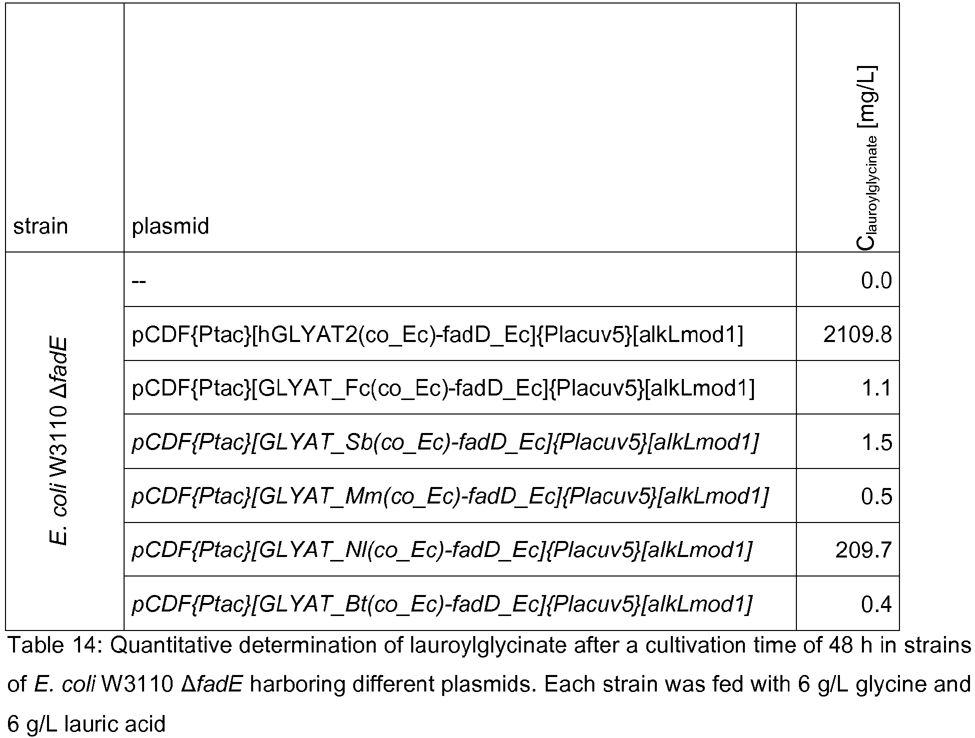

## Claims

1. A bacterial cell expressing an amino acid-N-acyl-transferase, and an acyl-CoA synthetase, wherein the cell has a deleted, or inactivated gene encoding an enzyme involved in the β-oxidation pathway that results in a reduced fatty acid degradation capacity of the cell, wherein the enzyme involved in the β-oxidation pathway is selected from the group comprising acyl-CoA dehydrogenase, 2,4-dienoyl-CoA reductase, enoyl-CoA hydratase and 3-ketoacyl-CoA thiolase, and
the amino acid-N-acyl-transferase is capable of catalysing the conversion of acyl-CoA and an amino acid to an acyl amino acid.

2. The cell according to claim 1, wherein the amino acid-N-acyl-transferase is SEQ ID NO: 4, SEQ ID NO 5: or a variant thereof and the variant comprises a sequence at least 70% identical to SEQ ID NO: 4 or SEQ ID NO 5.

3. The cell according to either claim 1 or 2, wherein the cell is an enterobacterial cell.

4. The cell according to any of claims 1 to 3, wherein the cell is capable of making proteinogenic amino acids and/or fatty acids.

5. The cell according to claim 4, wherein the cell comprises at least one enzyme selected from the group consisting of β-ketoacyl-ACP synthase I, 3-oxoacyl-ACP-synthase I, Malonyl-CoA-ACP transacylase, enoyl ACP reductase, and β-ketoacyl-ACP synthase III capable of making proteinogenic amino acids and/or fatty acids.

6. The cell according to any of the preceding claims, wherein the cell expresses an acyl-CoA thioesterase.

7. The cell according to any of the preceding claims, wherein the acyl-CoA synthetase is SEQ ID NO: 6 or a variant thereof and the variant comprises a sequence at least 70% identical to SEQ ID NO: 6.

8. The cell according to any of the preceding claims, wherein the cell is genetically modified to increase the expression of at least one transporter protein compared to the wild type cell, wherein the transporter protein is selected from the group consisting of FadL and AlkL and the increase in enzymatic activity is achieved by increasing the copy number of the gene sequence or gene sequences that code for the transporter protein.

9. A method for producing acyl amino acids, comprising the steps
a) converting a fatty acid to an acyl CoA using an acyl-CoA synthetase, and
b) contacting an amino acid and the acyl CoA in the presence of an amino acid-N-acyl-transferase, which is capable of catalysing the conversion of acyl-CoA and an amino acid to the acyl amino acid, and/or
c) hydrogenating acyl residues of the acyl amino acid from b)
wherein the acyl-CoA synthetase and the amino acid-N-acyl-transferase are provided in the form of a bacterial cell and wherein the cell has a deleted, or inactivated gene encoding an enzyme involved in the β-oxidation pathway that results in a reduced fatty acid degradation capacity of the cell,
wherein the enzyme involved in the β-oxidation pathway is selected from the group comprising acyl-CoA dehydrogenase, 2,4-dienoyl-CoA reductase, enoyl-CoA hydratase and 3-ketoacyl-CoA thiolase, and
the amino acid-N-acyl-transferase is capable of catalysing the conversion of acyl-CoA and an amino acid to an acyl amino acid;
or culturing the cell according to any of claims 1 to 8.

10. The method according to claim 9, wherein the amino acid-N-acyl-transferase is SEQ ID NO: 4, SEQ ID NO: 5 or a variant thereof and the variant comprises a sequence at least 70% identical to SEQ ID NO: 4 or SEQ ID NO 5, and the acyl-CoA synthetase is SEQ ID NO:6 or a variant thereof and the variant comprises a sequence at least 70% identical to SEQ ID NO: 6.

11. A reaction mixture comprising
an amino acid-N-acyl-transferase, which is preferably isolated and/or recombinant,
an acyl-CoA synthetase, which is preferably isolated and/or recombinant,
an amino acid and
a fatty acid,
wherein the acyl-CoA synthetase and the amino acid-N-acyl-transferase are provided in the form of a bacterial cell and wherein the cell has a deleted, or inactivated gene encoding an enzyme involved in the β-oxidation pathway that results in a reduced fatty acid degradation capacity of the cell,
wherein the enzyme involved in the β-oxidation pathway is selected from the group comprising acyl-CoA dehydrogenase, 2,4-dienoyl-CoA reductase, enoyl-CoA hydratase and 3-ketoacyl-CoA thiolase, and
the amino acid-N-acyl-transferase is capable of catalysing the conversion of acyl-CoA and an amino acid to an acyl amino acid.

12. The reaction mixture according to claim 11, wherein the cell is according to any one of claims 1 to 8.

## Patentansprüche

1. Bakterienzelle, exprimierend eine Aminosäure-N-Acyl-Transferase und eine Acyl-CoA-Synthetase, wobei die Zelle ein deletiertes oder inaktiviertes Gen, das ein am β-Oxidationsweg beteiligtes Enzym codiert, aufweist, was zu einer verminderten Fettsäureabbaukapazität der Zelle führt, wobei das am β-Oxidationsweg beteiligte Enzym aus der Gruppe umfassend Acyl-CoA-Dehydrogenase, 2,4-Dienoyl-CoA-Reduktase, Enoyl-CoA-Hydratase und 3-Ketoacyl-CoA-Thiolase ausgewählt ist, und
die Aminosäure-N-Acyl-Transferase zur Katalyse der Umwandlung von Acyl-CoA und einer Aminosäure in eine Acyl-Aminosäure fähig ist.

2. Zelle nach Anspruch 1, wobei es sich bei der Aminosäure-N-Acyl-Transferase um SEQ ID NO: 4, SEQ ID NO: 5 oder eine Variante davon handelt und die Variante eine mit SEQ ID NO: 4 oder SEQ ID NO: 5 zu wenigstens 70% identische Sequenz umfasst.

3. Zelle nach entweder Anspruch 1 oder 2, wobei es sich bei der Zelle um eine Enterobakterienzelle handelt.

4. Zelle nach einem der Ansprüche 1 bis 3, wobei die Zelle zur Erzeugung von proteinogenen Aminosäuren und/oder Fettsäuren fähig ist.

5. Zelle nach Anspruch 4, wobei die Zelle wenigstens ein aus der Gruppe bestehend aus β-Ketoacyl-ACP-Synthase I, 3-Oxoacyl-ACP-Synthase I, Malonyl-CoA-ACP-Transacylase, Enoyl-ACP-Reduktase und β-Ketoacyl-ACP-Synthase III ausgewähltes Enzym umfasst, das zur Erzeugung von proteinogenen Aminosäuren und/oder Fettsäuren fähig ist.

6. Zelle nach einem der vorhergehenden Ansprüche, wobei die Zelle eine Acyl-CoA-Thioesterase exprimiert.

7. Zelle nach einem der vorhergehenden Ansprüche, wobei es sich bei der Acyl-CoA-Synthetase um SEQ ID NO: 6 oder eine Variante davon handelt und die Variante eine mit SEQ ID NO: 6 zu wenigstens 70% identische Sequenz umfasst.

8. Zelle nach einem der vorhergehenden Ansprüche, wobei die Zelle gentechnisch verändert ist, so dass die Expression wenigstens eines Transporterproteins verglichen mit der Wildtypzelle erhöht ist, wobei das Transporterprotein aus der Gruppe bestehend aus FadL und AlkL ausgewählt ist und die Steigerung der enzymatischen Aktivität durch Erhöhen der Kopienzahl der Gensequenz oder Gensequenzen, die für das Transporterprotein codieren, erreicht wird.

9. Verfahren zur Herstellung von Acyl-Aminosäuren, umfassend die Schritte
a) Umwandeln einer Fettsäure in ein Acyl-CoA unter Verwendung einer Acyl-CoA-Synthetase und
b) Inkontaktbringen einer Aminosäure und des Acyl-CoA in Gegenwart einer Aminosäure-N-Acyl-Transferase, die zur Katalyse der Umwandlung von Acyl-CoA und einer Aminosäure in die Acyl-Aminosäure fähig ist, und/oder
c) Hydrieren von Acylresten der Acyl-Aminosäure aus b),
wobei die Acyl-CoA-Synthetase und die Aminosäure-N-Acyl-Transferase in Form einer Bakterienzelle bereitgestellt werden und wobei die Zelle ein deletiertes oder inaktiviertes Gen, das ein am β-Oxidationsweg beteiligtes Enzym codiert, aufweist, was zu einer verminderten Fettsäureabbaukapazität der Zelle führt,
wobei das am β-Oxidationsweg beteiligte Enzym aus der Gruppe umfassend Acyl-CoA-Dehydrogenase, 2,4-Dienoyl-CoA-Reduktase, Enoyl-CoA-Hydratase und 3-Ketoacyl-CoA-Thiolase ausgewählt ist, und
die Aminosäure-N-Acyl-Transferase zur Katalyse der Umwandlung von Acyl-CoA und einer Aminosäure in eine Acyl-Aminosäure fähig ist;
oder Kultivieren der Zelle nach einem der Ansprüche 1 bis 8.

10. Verfahren nach Anspruch 9, wobei es sich bei der Aminosäure-N-Acyl-Transferase um SEQ ID NO: 4, SEQ ID NO: 5 oder eine Variante davon handelt und die Variante eine mit SEQ ID NO: 4 oder SEQ ID NO: 5 zu wenigstens 70% identische Sequenz umfasst und es sich bei der Acyl-CoA-Synthetase um SEQ ID NO: 6 oder eine Variante davon handelt und die Variante eine mit SEQ ID NO: 6 zu wenigstens 70% identische Sequenz umfasst.

11. Reaktionsansatz, umfassend
eine Aminosäure-N-Acyl-Transferase, die vorzugsweise isoliert und/oder rekombinant ist,
eine Acyl-CoA-Synthetase, die vorzugsweise isoliert und/oder rekombinant ist,
eine Aminosäure und
eine Fettsäure,
wobei die Acyl-CoA-Synthetase und die Aminosäure-N-Acyl-Transferase in Form einer Bakterienzelle bereitgestellt werden und wobei die Zelle ein deletiertes oder inaktiviertes Gen, das ein am β-Oxidationsweg beteiligtes Enzym codiert, aufweist, was zu einer verminderten Fettsäureabbaukapazität der Zelle führt,
wobei das am β-Oxidationsweg beteiligte Enzym aus der Gruppe umfassend Acyl-CoA-Dehydrogenase, 2,4-Dienoyl-CoA-Reduktase, Enoyl-CoA-Hydratase und 3-Ketoacyl-CoA-Thiolase ausgewählt ist, und
die Aminosäure-N-Acyl-Transferase zur Katalyse der Umwandlung von Acyl-CoA und einer Aminosäure in eine Acyl-Aminosäure fähig ist.

12. Reaktionsansatz nach Anspruch 11, wobei es sich um die Zelle nach einem der Ansprüche 1 bis 8 handelt.

## Revendications

1. Cellule bactérienne exprimant une acide aminé N-acyl-transférase, et une acyl-CoA synthétase, la cellule comportant un gène délété ou inactivé codant pour une enzyme impliquée dans la voie de β-oxydation qui conduit à une capacité réduite de dégradation des acides gras de la cellule, dans laquelle l'enzyme impliquée dans la voie de β-oxydation est choisie dans le groupe comprenant les acyl-CoA déshydrogénase, 2,4-diénoyl-CoA réductase, énoyl-CoA hydratase et 3-cétoacyl-CoA thiolase, et l'acide aminé N-acyl-transférase est capable de catalyser la conversion d'acyl-CoA et d'un acide aminé en acyl-aminoacide.

2. Cellule selon la revendication 1, dans laquelle l'acide aminé N-acyl-transférase est SEQ ID NO: 4, SEQ ID NO: 5 ou un variant de ceux-ci et le variant comprend une séquence au moins 70 % identique à SEQ ID NO: 4 ou SEQ ID NO: 5.

3. Cellule selon la revendication 1 ou 2, la cellule étant une cellule entérobactérienne.

4. Cellule selon l'une quelconque des revendications 1 à 3, la cellule étant capable de fabriquer des acides aminés protéinogènes et/ou des acides gras.

5. Cellule selon la revendication 4, la cellule comprenant au moins une enzyme choisie dans le groupe constitué des β-cétoacyl-ACP synthase I, 3-oxoacyl-ACP-synthase I, malonyl-CoA-ACP transacylase, énoyl-ACP réductase, et β-cétoacyl-ACP synthase III capables de fabriquer des acides aminés protéinogènes et/ou des acides gras.

6. Cellule selon l'une quelconque des revendications précédentes, la cellule exprimant une acyl-CoA thioestérase.

7. Cellule selon l'une quelconque des revendications précédentes, dans laquelle l'acyl-CoA synthétase est SEQ ID NO: 6 ou un variant de celui-ci et le variant comprend une séquence au moins 70 % identique à SEQ ID NO: 6.

8. Cellule selon l'une quelconque des revendications précédentes, la cellule étant génétiquement modifiée de façon à augmenter l'expression d'au moins une protéine transporteuse par rapport à la cellule de type sauvage, dans laquelle la protéine transporteuse est choisie dans le groupe constitué de FadL et AlkL et l'augmentation d'activité enzymatique est obtenue par augmentation du nombre de copies de la séquence de gène ou des séquences de gène qui codent pour la protéine transporteuse.

9. Procédé de production d'acyl-aminoacides, comprenant les étapes de
a) conversion d'un acide gras en acyl-CoA au moyen d'une acyl-CoA synthétase, et
b) mise en contact d'un acide aminé et de l'acyl-CoA en présence d'une acide aminé N-acyl-transférase, qui est capable de catalyser la conversion d'acyl-CoA et d'un acide aminé en acyl-aminoacide, et/ou
c) hydrogénation de résidus acyle de l'acyl-aminoacide de b)
dans lequel l'acyl-CoA synthétase et l'acide aminé N-acyl-transférase sont fournies sous la forme d'une cellule bactérienne et dans lequel la cellule comporte un gène délété ou inactivé codant pour une enzyme impliquée dans la voie de β-oxydation qui conduit à une capacité réduite de dégradation des acides gras de la cellule,
dans lequel l'enzyme impliquée dans la voie de β-oxydation est choisie dans le groupe comprenant les acyl-CoA déshydrogénase, 2,4-diénoyl-CoA réductase, énoyl-CoA hydratase et 3-cétoacyl-CoA thiolase, et
l'acide aminé N-acyl-transférase est capable de catalyser la conversion d'acyl-CoA et d'un acide aminé en acyl-aminoacide ;
ou culture de la cellule selon l'une quelconque des revendications 1 à 8.

10. Procédé selon la revendication 9, dans lequel l'acide aminé N-acyl-transférase est SEQ ID NO: 4, SEQ ID NO: 5 ou un variant de ceux-ci et le variant comprend une séquence au moins 70 % identique à SEQ ID NO: 4 ou SEQ ID NO 5, et l'acyl-CoA synthétase est SEQ ID NO: 6 ou un variant de celui-ci et le variant comprend une séquence au moins 70 % identique à SEQ ID NO: 6.

11. Mélange de réaction comprenant
une acide aminé N-acyl-transférase, qui est de préférence isolée et/ou recombinante,
une acyl-CoA synthétase, qui est de préférence isolée et/ou recombinante,
un acide aminé et
un acide gras,
dans lequel l'acyl-CoA synthétase et l'acide aminé N-acyl-transférase sont fournies sous la forme d'une cellule bactérienne et dans lequel la cellule comporte un gène délété ou inactivé codant pour une enzyme impliquée dans la voie de β-oxydation qui conduit à une capacité réduite de dégradation des acides gras de la cellule,
dans lequel l'enzyme impliquée dans la voie de β-oxydation est choisie dans le groupe comprenant les acyl-CoA déshydrogénase, 2,4-diénoyl-CoA réductase, énoyl-CoA hydratase et 3-cétoacyl-CoA thiolase, et
l'acide aminé N-acyl-transférase est capable de catalyser la conversion d'acyl-CoA et d'un acide aminé en acyl-aminoacide.

12. Mélange de réaction selon la revendication 11, dans lequel la cellule est selon l'une quelconque des revendications 1 à 8.


### SEQUENCE LISTING

<110> Evonik Industries AG
<120> A method for producing acyl amino acids
<130> 201300087WO
<150> 13181841.1
   <151> 2013-08-27
<150> 14169650.0
   <151> 2014-05-23
<160> 57
<170> PatentIn version 3.5
<210> 1
   <211> 906
   <212> DNA
   <213> Umbellularia californica
<220>
   <221> misc_feature
   <222> (1)..(906)
   <223> synUcTE (an acyl-CoA thioesterase) gene (codon-optimized)
<400> 1
   atgactctag agtggaaacc gaaaccaaaa ctgcctcaac tgctggatga tcacttcggt      60
   ctgcacggtc tggtgtttcg tcgtactttc gcaattcgtt cttatgaagt gggtccagat     120
   cgttctacct ccatcctggc cgtcatgaac cacatgcagg aagccaccct gaatcacgcg     180
   aaatctgttg gtatcctggg tgatggtttc ggcactactc tggaaatgtc taaacgtgac     240
   ctgatgtggg tagtgcgtcg cacccacgta gcagtagagc gctaccctac ttggggtgac     300
   actgtggaag tcgagtgttg gattggcgcg tccggtaaca atggtatgcg tcgcgatttt     360
   ctggtccgtg actgtaaaac gggcgaaatc ctgacgcgtt gcacctccct gagcgttctg     420
   atgaacaccc gcactcgtcg cctgtctacc atcccggacg aagtgcgcgg tgagatcggt     480
   cctgctttca tcgataacgt ggcagttaaa gacgacgaaa tcaagaaact gcaaaaactg     540
   aacgactcca ccgcggacta catccagggc ggtctgactc cgcgctggaa cgacctggat     600
   gttaatcagc atgtgaacaa cctgaaatac gttgcttggg tcttcgagac tgtgccggac     660
   agcattttcg aaagccatca catttcctct tttactctgg agtaccgtcg cgaatgtact     720
   cgcgactccg ttctgcgcag cctgaccacc gtaagcggcg gttctagcga ggcaggtctg     780
   gtctgcgacc atctgctgca actggaaggc ggctccgaag tcctgcgtgc gcgtacggag     840
   tggcgtccaa agctgacgga ttctttccgc ggcatctccg taattccggc ggaacctcgt     900
   gtttaa                                                                906
<210> 2
   <211> 159
   <212> DNA
   <213> Artificial Sequence
<220>
   <223> tac promotor for synUcTE expression
<400> 2
   ggatccaatt gtgagcggat aacaattacg agcttcatgc acagtgatcg acgctgttga 60
   caattaatca tcggctcgta taatgtgtgg atgtggaatt gtgagcgctc acaattccac 120
   aacggtttcc ctctagaaat aattttgttt aacaggagg 159
<210> 3
   <211> 4977
   <212> DNA
   <213> Artificial Sequence
<220>
   <223> Vector pJ294[Ptac-synUcTE]
<400> 3
   accaatgctt aatcagtgag gcacctatct cagcgatctg tctatttcgt tcatccatag 60
   ttgcctgact ccccgtcgtg tagataacta cgatacggga gggcttacca tctggcccca 120
   gcgctgcgat gataccgcga gaaccacgct caccggctcc ggatttatca gcaataaacc 180
   agccagccgg aagggccgag cgcagaagtg gtcctgcaac tttatccgcc tccatccagt 240
   ctattaattg ttgccgggaa gctagagtaa gtagttcgcc agttaatagt ttgcgcaacg 300
   ttgttgccat cgctacaggc atcgtggtgt cacgctcgtc gtttggtatg gcttcattca 360
   gctccggttc ccaacgatca aggcgagtta catgatcccc catgttgtgc aaaaaagcgg 420
   ttagctcctt cggtcctccg atcgttgtca gaagtaagtt ggccgcagtg ttatcactca 480
   tggttatggc agcactgcat aattctctta ctgtcatgcc atccgtaaga tgcttttctg 540
   tgactggtga gtactcaacc aagtcattct gagaatagtg tatgcggcga ccgagttgct 600
   cttgcccggc gtcaatacgg gataataccg cgccacatag cagaacttta aaagtgctca 660
   tcattggaaa acgttcttcg gggcgaaaac tctcaaggat cttaccgctg ttgagatcca 720
   gttcgatgta acccactcgt gcacccaact gatcttcagc atcttttact ttcaccagcg 780
   tttctgggtg agcaaaaaca ggaaggcaaa atgccgcaaa aaagggaata agggcgacac 840
   ggaaatgttg aatactcata ttcttccttt ttcaatatta ttgaagcatt tatcagggtt 900
   attgtctcat gagcggatac atatttgaat gtatttagaa aaataaacaa ataggggtca 960
   gtgttacaac caattaacca attctgaaca ttatcgcgag cccatttata cctgaatatg 1020
   gctcataaca ccccttgttt gcctggcggc agtagcgcgg tggtcccacc tgaccccatg 1080
   ccgaactcag aagtgaaacg ccgtagcgcc gatggtagtg tggggactcc ccatgcgaga 1140
   gtagggaact gccaggcatc aaataaaacg aaaggctcag tcgaaagact gggcctttcg 1200
   cccgggctaa ttatggggtg tcgcccttat tcgactctat agtgaagttc ctattctcta 1260
   gaaagtatag gaacttctga agtgggggcg gccgcaaatt cagtaagcag aaagtcaaaa 1320
   gcctccgacc ggaggctttt gactaaagct taaacacgag gttccgccgg aattacggag 1380
   atgccgcgga aagaatccgt cagctttgga cgccactccg tacgcgcacg caggacttcg 1440
   gagccgcctt ccagttgcag cagatggtcg cagaccagac ctgcctcgct agaaccgccg 1500
   cttacggtgg tcaggctgcg cagaacggag tcgcgagtac attcgcgacg gtactccaga 1560
   gtaaaagagg aaatgtgatg gctttcgaaa atgctgtccg gcacagtctc gaagacccaa 1620
   gcaacgtatt tcaggttgtt cacatgctga ttaacatcca ggtcgttcca gcgcggagtc 1680
   agaccgccct ggatgtagtc cgcggtggag tcgttcagtt tttgcagttt cttgatttcg 1740
   tcgtctttaa ctgccacgtt atcgatgaaa gcaggaccga tctcaccgcg cacttcgtcc 1800
   gggatggtag acaggcgacg agtgcgggtg ttcatcagaa cgctcaggga ggtgcaacgc 1860
   gtcaggattt cgcccgtttt acagtcacgg accagaaaat cgcgacgcat accattgtta 1920
   ccggacgcgc caatccaaca ctcgacttcc acagtgtcac cccaagtagg gtagcgctct 1980
   actgctacgt gggtgcgacg cactacccac atcaggtcac gtttagacat ttccagagta 2040
   gtgccgaaac catcacccag gataccaaca gatttcgcgt gattcagggt ggcttcctgc 2100
   atgtggttca tgacggccag gatggaggta gaacgatctg gacccacttc ataagaacga 2160
   attgcgaaag tacgacgaaa caccagaccg tgcagaccga agtgatcatc cagcagttga 2220
   ggcagttttg gtttcggttt ccactctaga gtcattttat aatcctcctt gtaaacaaaa 2280
   ttatttctag agggaaaccg ttgtggaatt gtgagcgctc acaattccac atccacacat 2340
   tatacgagcc gatgattaat tgtcaacagc gtcgatcact gtgcatgaag ctcgtaattg 2400
   ttatccgctc acaattggat ccaaaatgaa gggaagttcc tatactttct agagaatagg 2460
   aacttctata gggagtcgaa taagggcgac acaaaaggta ttctaaatgc ataataaata 2520
   ctgataacat cttatagttt gtattatatt ttgtattatc gttgacatgt ataattttga 2580
   tatcaaaaac tgattttccc tttattattt tcgagattta ttttcttaat tctctttaac 2640
   aaactagaaa tattgtatat acaaaaaatc ataaataata gatgaatagt ttaattatag 2700
   gtgttcatca atcgaaaaag caacgtatct tatttaaagt gcgttgcttt tttctcattt 2760
   ataaggttaa ataattctca tatatcaagc aaagtgacag gcgcccttaa atattctgac 2820
   aaatgctctt tccctaaact ccccccataa aaaaacccgc cgaagcgggt ttttacgtta 2880
   tttgcggatt aacgattact cgttatcaga accgcccagg atgcctggca gttccctact 2940
   ctcgccgctg cgctcggtcg ttcggctgcg ggacctcagc gctagcggag tgtatactgg 3000
   cttactatgt tggcactgat gagggtgtca gtgaagtgct tcatgtggca ggagaaaaaa 3060
   ggctgcaccg gtgcgtcagc agaatatgtg atacaggata tattccgctt cctcgctcac 3120
   tgactcgcta cgctcggtcg ttcgactgcg gcgagcggaa atggcttacg aacggggcgg 3180
   agatttcctg gaagatgcca ggaagatact taacagggaa gtgagagggc cgcggcaaag 3240
   ccgtttttcc ataggctccg cccccctgac aagcatcacg aaatctgacg ctcaaatcag 3300
   tggtggcgaa acccgacagg actataaaga taccaggcgt ttccccctgg cggctccctc 3360
   gtgcgctctc ctgttcctgc ctttcggttt accggtgtca ttccgctgtt atggccgcgt 3420
   ttgtctcatt ccacgcctga cactcagttc cgggtaggca gttcgctcca agctggactg 3480
   tatgcacgaa ccccccgttc agtccgaccg ctgcgcctta tccggtaact atcgtcttga 3540
   gtccaacccg gaaagacatg caaaagcacc actggcagca gccactggta attgatttag 3600
   aggagttagt cttgaagtca tgcgccggtt aaggctaaac tgaaaggaca agttttggtg 3660
   actgcgctcc tccaagccag ttacctcggt tcaaagagtt ggtagctcag agaaccttcg 3720
   aaaaaccgcc ctgcaaggcg gttttttcgt tttcagagca agagattacg cgcagaccaa 3780
   aacgatctca agaagatcat cttattaatc actgcccgct ttccagtcgg gaaacctgtc 3840
   gtgccagctg cattaatgaa tcggccaacg cgcggggaga ggcggtttgc gtattgggcg 3900
   ccagggtggt ttttcttttc accagtgaga ctggcaacag ctgattgccc ttcaccgcct 3960
   ggccctgaga gagttgcagc aagcggtcca cgctggtttg ccccagcagg cgaaaatcct 4020
   gtttgatggt ggttaacggc gggatataac atgagctatc ttcggtatcg tcgtatccca 4080
   ctaccgagat atccgcacca acgcgcagcc cggactcggt aatggcgcgc attgcgccca 4140
   gcgccatctg atcgttggca accagcatcg cagtgggaac gatgccctca ttcagcattt 4200
   gcatggtttg ttgaaaaccg gacatggcac tccagtcgcc ttcccgttcc gctatcggct 4260
   gaatttgatt gcgagtgaga tatttatgcc agccagccag acgcagacgc gccgagacag 4320
   aacttaatgg gcccgctaac agcgcgattt gctggtgacc caatgcgacc agatgctcca 4380
   cgcccagtcg cgtaccgtcc tcatgggaga aaataatact gttgatgggt gtctggtcag 4440
   agacatcaag aaataacgcc ggaacattag tgcaggcagc ttccacagca atggcatcct 4500
   ggtcatccag cggatagtta atgatcagcc cactgacgcg ttgcgcgaga agattgtgca 4560
   ccgccgcttt acaggcttcg acgccgcttc gttctaccat cgacaccacc acgctggcac 4620
   ccagttgatc ggcgcgagat ttaatcgccg cgacaatttg cgacggcgcg tgcagggcca 4680
   gactggaggt ggcaacgcca atcagcaacg actgtttgcc cgccagttgt tgtgccacgc 4740
   ggttgggaat gtaattcagc tccgccatcg ccgcttccac tttttcccgc gttttcgcag 4800
   aaacgtggct ggcctggttc accacgcggg aaacggtctg ataagagaca ccggcatact 4860
   ctgcgacatc gtataacgtt actggtttca tattcaccac cctgaattga ctctcttccg 4920
   ggcgctatca tgccataccg cgaaaggttt tgcgccattc gatggcgcgc cgctttt 4977
<210> 4
   <211> 882
   <212> DNA
   <213> Homo sapiens
<220>
   <221> misc_feature
   <222> (1)..(882)
   <223> hGLYAT2 (an amino acid N-acyl transferase) codon optimised for E.coli
<400> 4
   atgctggttc tgcacaacag ccaaaaactg caaattctgt acaaatctct ggaaaaatct 60
   attcctgaaa gcattaaagt ttatggtgcg atcttcaaca tcaaggataa aaaccctttc 120
   aatatggagg tgctggtcga cgcgtggcct gattaccaaa tcgtcatcac ccgtccgcag 180
   aagcaagaaa tgaaagatga ccaggaccac tatactaaca cctaccacat cttcactaaa 240
   gcgccggaca aactggaaga agttctgagc tactccaatg ttatctcctg ggaacaaacg 300
   ctgcagattc aaggttgcca ggaaggtctg gatgaggcga ttcgtaaggt cgctacctct 360
   aagtccgttc aagtcgatta catgaaaacc atcctgttta tcccggagct gccgaagaaa 420
   cacaaaacct cctctaacga caagatggag ctgttcgaag tagacgatga caacaaagag 480
   ggtaactttt ccaatatgtt cctggacgcc tcccacgccg gtctggttaa tgagcactgg 540
   gcgttcggta aaaacgaacg ctccctgaag tacattgagc gttgtctgca ggattttctg 600
   ggttttggtg tcctgggtcc agagggtcaa ctggtctctt ggatcgttat ggaacagtct 660
   tgtgaactgc gtatgggtta tactgtgcca aaataccgtc accaaggtaa tatgctgcaa 720
   atcggttatc atctggagaa gtacctgagc cagaaggaaa tcccgttcta tttccatgtt 780
   gccgataata acgaaaagag cctgcaagcc ctgaacaatc tgggcttcaa gatctgccct 840
   tgcggttggc accagtggaa gtgcacgcct aaaaagtact gt 882
<210> 5
   <211> 864
   <212> DNA
   <213> Homo sapiens
<220>
   <221> misc_feature
   <222> (1)..(864)
   <223> hGLYAT3 (another amino acid N-acyl transferase) (codon optimised for E.coli)
<400> 5
   atgctggttc tgaattgctc tactaaactg ctgatcctgg agaaaatgct gaaatcctgt      60
   ttcccggaat ctctgaaagt ttatggtgcg gtaatgaata tcaaccgtgg taatccgttt     120
   caaaaagaag ttgttctgga ctcttggcca gatttcaagg cggttatcac ccgtcgtcag     180
   cgtgaagcgg aaaccgacaa cctggaccac tataccaacg cgtatgcggt tttctataaa     240
   gatgttcgcg cgtaccgtca gctgctggaa gaatgcgatg ttttcaactg ggaccaggtt     300
   tttcagatcc aaggtctgca atccgagctg tacgacgttt ctaaggccgt ggcgaactct     360
   aaacaactga acatcaagct gacctctttc aaagcggttc atttctctcc ggtatccagc     420
   ctgccggaca cctccttcct gaaaggtccg tctccgcgtc tgacctacct gtctgttgcg     480
   aatgcggatc tgctgaaccg tacttggtcc cgtggtggca acgaacagtg cctgcgttac     540
   attgctaacc tgatctcttg cttcccgagc gtctgcgttc gtgatgaaaa aggtaaccca     600
   gtatcttggt ctatcaccga ccagttcgcg accatgtgtc atggctacac cctgcctgaa     660
   caccgtcgta agggttactc tcgtctggtt gcgctgaccc tggcgcgtaa gctgcagtct     720
   cgtggttttc cgtcccaggg taacgtcctg gacgacaaca ctgcgtccat ctccctgctg     780
   aagtccctgc atgccgaatt cctgccgtgc cgtttccacc gtctgattct gaccccagcg     840
   accttctctg gtctgccgca cctg                                            864
<210> 6
   <211> 1686
   <212> DNA
   <213> Escherichia coli
<220>
   <221> misc_feature
   <222> (1)..(1686)
   <223> fadD (an acyl-CoA synthetase)
<400> 6
   ttgaagaagg tttggcttaa ccgttatccc gcggacgttc cgacggagat caaccctgac 60
   cgttatcaat ctctggtaga tatgtttgag cagtcggtcg cgcgctacgc cgatcaacct 120
   gcgtttgtga atatggggga ggtaatgacc ttccgcaagc tggaagaacg cagtcgcgcg 180
   tttgccgctt atttgcaaca agggttgggg ctgaagaaag gcgatcgcgt tgcgttgatg 240
   atgcctaatt tattgcaata tccggtggcg ctgtttggca ttttgcgtgc cgggatgatc 300
   gtcgtaaacg ttaacccgtt gtataccccg cgtgagcttg agcatcagct taacgatagc 360
   ggcgcatcgg cgattgttat cgtgtctaac tttgctcaca cactggaaaa agtggttgat 420
   aaaaccgccg ttcagcacgt aattctgacc cgtatgggcg atcagctatc tacggcaaaa 480
   ggcacggtag tcaatttcgt tgttaaatac atcaagcgtt tggtgccgaa ataccatctg 540
   ccagatgcca tttcatttcg tagcgcactg cataacggct accggatgca gtacgtcaaa 600
   cccgaactgg tgccggaaga tttagctttt ctgcaataca ccggcggcac cactggtgtg 660
   gcgaaaggcg cgatgctgac tcaccgcaat atgctggcga acctggaaca ggttaacgcg 720
   acctatggtc cgctgttgca tccgggcaaa gagctggtgg tgacggcgct gccgctgtat 780
   cacatttttg ccctgaccat taactgcctg ctgtttatcg aactgggtgg gcagaacctg 840
   cttatcacta acccgcgcga tattccaggg ttggtaaaag agttagcgaa atatccgttt 900
   accgctatca cgggcgttaa caccttgttc aatgcgttgc tgaacaataa agagttccag 960
   cagctggatt tctccagtct gcatctttcc gcaggcggtg ggatgccagt gcagcaagtg 1020
   gtggcagagc gttgggtgaa actgaccgga cagtatctgc tggaaggcta tggccttacc 1080
   gagtgtgcgc cgctggtcag cgttaaccca tatgatattg attatcatag tggtagcatc 1140
   ggtttgccgg tgccgtcgac ggaagccaaa ctggtggatg atgatgataa tgaagtacca 1200
   ccaggtcaac cgggtgagct ttgtgtcaaa ggaccgcagg tgatgctggg ttactggcag 1260
   cgtcccgatg ctaccgatga aatcatcaaa aatggctggt tacacaccgg cgacatcgcg 1320
   gtaatggatg aagaaggatt cctgcgcatt gtcgatcgta aaaaagacat gattctggtt 1380
   tccggtttta acgtctatcc caacgagatt gaagatgtcg tcatgcagca tcctggcgta 1440
   caggaagtcg cggctgttgg cgtaccttcc ggctccagtg gtgaagcggt gaaaatcttc 1500
   gtagtgaaaa aagatccatc gcttaccgaa gagtcactgg tgactttttg ccgccgtcag 1560
   ctcacgggat acaaagtacc gaagctggtg gagtttcgtg atgagttacc gaaatctaac 1620
   gtcggaaaaa ttttgcgacg agaattacgt gacgaagcgc gcggcaaagt ggacaataaa 1680
   gcctga 1686
<210> 7
   <211> 7138
   <212> DNA
   <213> artificial sequence
<220>
   <223> Vector pCDF[atfA1_Ab(co_Ec)-fadD_Ec]
<220>
   <221> misc_feature
   <222> (1)..(7138)
   <223> Vector pCDF[atfA1_Ab(co_Ec)-fadD_Ec]
<400> 7
   cgggatctcg acgctctccc ttatgcgact cctgcgttta gggaaagagc atttgtcaga      60
   atatttaagg gcgcctgtca ctttgcttga tatatgagaa ttatttaacc ttataaatga     120
   gaaaaaagca acgcacttta aataagatac gttgcttttt cgattgatga acacctataa     180
   ttaaactatt catctattat ttatgatttt ttgtatatac aatatttcta gtttgttaaa     240
   gagaattaag aaaataaatc tcgaaaataa taaagggaaa atcagttttt gatatcaaaa     300
   ttatacatgt caacgataat acaaaatata atacaaacta taagatgtta tcagtattta     360
   ttatgcattt agaatacctt ttgtgtcgcc cttattcgac tccctataga agttcctatt 420
   ctctagaaag tataggaact tcccttcatt ttggatccaa ttgtgagcgg ataacaatta 480
   cgagcttcat gcacagtgat cgacgctgtt gacaattaat catcggctcg tataatgtgt 540
   ggatgtggaa ttgtgagcgc tcacaattcc acaacggttt ccctctagaa ataattttgt 600
   ttaacaggag gtaaaacata tgaaagcgct gtccccggtg gatcagctgt tcctgtggct 660
   ggagaaacgt cagcaaccga tgcatgttgg tggtctgcag ctgtttagct tcccagaggg 720
   tgcgggtcct aagtacgtta gcgagttggc tcagcaaatg cgtgactact gtcacccggt 780
   ggccccgttc aatcagcgcc tgacccgtcg cctgggtcaa tactattgga cgcgcgacaa 840
   acaatttgac attgaccacc attttcgcca tgaagcgctg ccgaagccgg gtcgcattcg 900
   cgaactgctg tccttggtta gcgcggagca cagcaatctg ttggaccgcg agcgccctat 960
   gtgggaagcg cacctgattg agggcatccg tggccgccaa ttcgctttgt actataagat 1020
   ccaccatagc gtgatggatg gcatcagcgc tatgcgcatt gcgtctaaaa cgctgagcac 1080
   cgacccgtct gagcgcgaaa tggcaccagc atgggccttc aataccaaga aacgtagccg 1140
   tagcctgcct tccaatccag ttgatatggc gagcagcatg gcccgtctga cggcttctat 1200
   cagcaaacag gcggcgaccg ttccgggtct ggctcgtgaa gtgtacaagg tcacccagaa 1260
   agctaagaag gatgagaact acgtgagcat ttttcaagcg ccggatacca tcttgaacaa 1320
   caccattacc ggttcccgtc gttttgcagc acagagcttt ccgctgccgc gtctgaaagt 1380
   tatcgcgaag gcgtataact gcaccatcaa caccgtcgtc ctgtctatgt gtggccacgc 1440
   gctgcgtgaa tatctgatta gccaacacgc actgccggat gagccgctga tcgcgatggt 1500
   gccgatgagc ctgcgtcagg acgacagcac gggtggtaat cagattggca tgatcctggc 1560
   caacctgggc acccatatct gcgatccggc gaatcgtttg cgtgttattc acgactccgt 1620
   cgaggaagcg aaatcgcgtt tcagccagat gagcccggag gagatcctga actttaccgc 1680
   actgactatg gccccgacgg gcttgaatct gctgaccggc ctggcaccga aatggcgtgc 1740
   gttcaacgtc gtcattagca acattccggg tccgaaggaa ccgctgtatt ggaatggcgc 1800
   ccagctgcaa ggtgtgtacc cggtaagcat tgcgttggac cgtattgcac tgaacatcac 1860
   gctgactagc tacgtggatc agatggagtt cggtctgatc gcatgccgtc gcacgctgcc 1920
   gagcatgcaa cgtctgctgg attatctgga acaaagcatt cgtgagttgg aaatcggtgc 1980
   cggcatcaag taagcgttaa gtctgagagg tgaagaattg aagaaggttt ggcttaaccg 2040
   ttatcccgcg gacgttccga cggagatcaa ccctgaccgt tatcaatctc tggtagatat 2100
   gtttgagcag tcggtcgcgc gctacgccga tcaacctgcg tttgtgaata tgggggaggt 2160
   aatgaccttc cgcaagctgg aagaacgcag tcgcgcgttt gccgcttatt tgcaacaagg 2220
   gttggggctg aagaaaggcg atcgcgttgc gttgatgatg cctaatttat tgcaatatcc 2280
   ggtggcgctg tttggcattt tgcgtgccgg gatgatcgtc gtaaacgtta acccgttgta 2340
   taccccgcgt gagcttgagc atcagcttaa cgatagcggc gcatcggcga ttgttatcgt 2400
   gtctaacttt gctcacacac tggaaaaagt ggttgataaa accgccgttc agcacgtaat 2460
   tctgacccgt atgggcgatc agctatctac ggcaaaaggc acggtagtca atttcgttgt 2520
   taaatacatc aagcgtttgg tgccgaaata ccatctgcca gatgccattt catttcgtag 2580
   cgcactgcat aacggctacc ggatgcagta cgtcaaaccc gaactggtgc cggaagattt 2640
   agcttttctg caatacaccg gcggcaccac tggtgtggcg aaaggcgcga tgctgactca 2700
   ccgcaatatg ctggcgaacc tggaacaggt taacgcgacc tatggtccgc tgttgcatcc 2760
   gggcaaagag ctggtggtga cggcgctgcc gctgtatcac atttttgccc tgaccattaa 2820
   ctgcctgctg tttatcgaac tgggtgggca gaacctgctt atcactaacc cgcgcgatat 2880
   tccagggttg gtaaaagagt tagcgaaata tccgtttacc gctatcacgg gcgttaacac 2940
   cttgttcaat gcgttgctga acaataaaga gttccagcag ctggatttct ccagtctgca 3000
   tctttccgca ggcggtggga tgccagtgca gcaagtggtg gcagagcgtt gggtgaaact 3060
   gaccggacag tatctgctgg aaggctatgg ccttaccgag tgtgcgccgc tggtcagcgt 3120
   taacccatat gatattgatt atcatagtgg tagcatcggt ttgccggtgc cgtcgacgga 3180
   agccaaactg gtggatgatg atgataatga agtaccacca ggtcaaccgg gtgagctttg 3240
   tgtcaaagga ccgcaggtga tgctgggtta ctggcagcgt cccgatgcta ccgatgaaat 3300
   catcaaaaat ggctggttac acaccggcga catcgcggta atggatgaag aaggattcct 3360
   gcgcattgtc gatcgtaaaa aagacatgat tctggtttcc ggttttaacg tctatcccaa 3420
   cgagattgaa gatgtcgtca tgcagcatcc tggcgtacag gaagtcgcgg ctgttggcgt 3480
   accttccggc tccagtggtg aagcggtgaa aatcttcgta gtgaaaaaag atccatcgct 3540
   taccgaagag tcactggtga ctttttgccg ccgtcagctc acgggataca aagtaccgaa 3600
   gctggtggag tttcgtgatg agttaccgaa atctaacgtc ggaaaaattt tgcgacgaga 3660
   attacgtgac gaagcgcgcg gcaaagtgga caataaagcc tgagcgttaa gtcagtcgtc 3720
   agacgccggt taatccggcg ttttttttga cgcccactaa agagaaaaca atctcgagtc 3780
   tggtaaagaa accgctgctg cgaaatttga acgccagcac atggactcgt ctactagcgc 3840
   agcttaatta acctaggctg ctgccaccgc tgagcaataa ctagcataac cccttggggc 3900
   ctctaaacgg gtcttgaggg gttttttgct gaaacctcag gcatttgaga agcacacggt 3960
   cacactgctt ccggtagtca ataaaccggt aaaccagcaa tagacataag cggctattta 4020
   acgaccctgc cctgaaccga cgaccgggtc atcgtggccg gatcttgcgg cccctcggct 4080
   tgaacgaatt gttagacatt atttgccgac taccttggtg atctcgcctt tcacgtagtg 4140
   gacaaattct tccaactgat ctgcgcgcga ggccaagcga tcttcttctt gtccaagata 4200
   agcctgtcta gcttcaagta tgacgggctg atactgggcc ggcaggcgct ccattgccca 4260
   gtcggcagcg acatccttcg gcgcgatttt gccggttact gcgctgtacc aaatgcggga 4320
   caacgtaagc actacatttc gctcatcgcc agcccagtcg ggcggcgagt tccatagcgt 4380
   taaggtttca tttagcgcct caaatagatc ctgttcagga accggatcaa agagttcctc 4440
   cgccgctgga cctaccaagg caacgctatg ttctcttgct tttgtcagca agatagccag 4500
   atcaatgtcg atcgtggctg gctcgaagat acctgcaaga atgtcattgc gctgccattc 4560
   tccaaattgc agttcgcgct tagctggata acgccacgga atgatgtcgt cgtgcacaac 4620
   aatggtgact tctacagcgc ggagaatctc gctctctcca ggggaagccg aagtttccaa 4680
   aaggtcgttg atcaaagctc gccgcgttgt ttcatcaagc cttacggtca ccgtaaccag 4740
   caaatcaata tcactgtgtg gcttcaggcc gccatccact gcggagccgt acaaatgtac 4800
   ggccagcaac gtcggttcga gatggcgctc gatgacgcca actacctctg atagttgagt 4860
   cgatacttcg gcgatcaccg cttccctcat actcttcctt tttcaatatt attgaagcat 4920
   ttatcagggt tattgtctca tgagcggata catatttgaa tgtatttaga aaaataaaca 4980
   aatagctagc tcactcggtc gctacgctcc gggcgtgaga ctgcggcggg cgctgcggac 5040
   acatacaaag ttacccacag attccgtgga taagcagggg actaacatgt gaggcaaaac 5100
   agcagggccg cgccggtggc gtttttccat aggctccgcc ctcctgccag agttcacata 5160
   aacagacgct tttccggtgc atctgtggga gccgtgaggc tcaaccatga atctgacagt 5220
   acgggcgaaa cccgacagga cttaaagatc cccaccgttt ccggcgggtc gctccctctt 5280
   gcgctctcct gttccgaccc tgccgtttac cggatacctg ttccgccttt ctcccttacg 5340
   ggaagtgtgg cgctttctca tagctcacac actggtatct cggctcggtg taggtcgttc 5400
   gctccaagct gggctgtaag caagaactcc ccgttcagcc cgactgctgc gccttatccg 5460
   gtaactgttc acttgagtcc aacccggaaa agcacggtaa aacgccactg gcagcagcca 5520
   ttggtaactg ggagttcgca gaggatttgt ttagctaaac acgcggttgc tcttgaagtg 5580
   tgcgccaaag tccggctaca ctggaaggac agatttggtt gctgtgctct gcgaaagcca 5640
   gttaccacgg ttaagcagtt ccccaactga cttaaccttc gatcaaacca cctccccagg 5700
   tggttttttc gtttacaggg caaaagatta cgcgcagaaa aaaaggatct caagaagatc 5760
   ctttgatctt ttctactgaa ccgctctaga tttcagtgca atttatctct tcaaatgtag 5820
   cacctgaagt cagccccata cgatataagt tgtaattctc atgttagtca tgccccgcgc 5880
   ccaccggaag gagctgactg ggttgaaggc tctcaagggc atcggtcgag atcccggtgc 5940
   ctaatgagtg agctaactta cattaattgc gttgcgctca ctgcccgctt tccagtcggg 6000
   aaacctgtcg tgccagctgc attaatgaat cggccaacgc gcggggagag gcggtttgcg 6060
   tattgggcgc cagggtggtt tttcttttca ccagtgagac gggcaacagc tgattgccct 6120
   tcaccgcctg gccctgagag agttgcagca agcggtccac gctggtttgc cccagcaggc 6180
   gaaaatcctg tttgatggtg gttaacggcg ggatataaca tgagctgtct tcggtatcgt 6240
   cgtatcccac taccgagatg tccgcaccaa cgcgcagccc ggactcggta atggcgcgca 6300
   ttgcgcccag cgccatctga tcgttggcaa ccagcatcgc agtgggaacg atgccctcat 6360
   tcagcatttg catggtttgt tgaaaaccgg acatggcact ccagtcgcct tcccgttccg 6420
   ctatcggctg aatttgattg cgagtgagat atttatgcca gccagccaga cgcagacgcg 6480
   ccgagacaga acttaatggg cccgctaaca gcgcgatttg ctggtgaccc aatgcgacca 6540
   gatgctccac gcccagtcgc gtaccgtctt catgggagaa aataatactg ttgatgggtg 6600
   tctggtcaga gacatcaaga aataacgccg gaacattagt gcaggcagct tccacagcaa 6660
   tggcatcctg gtcatccagc ggatagttaa tgatcagccc actgacgcgt tgcgcgagaa 6720
   gattgtgcac cgccgcttta caggcttcga cgccgcttcg ttctaccatc gacaccacca 6780
   cgctggcacc cagttgatcg gcgcgagatt taatcgccgc gacaatttgc gacggcgcgt 6840
   gcagggccag actggaggtg gcaacgccaa tcagcaacga ctgtttgccc gccagttgtt 6900
   gtgccacgcg gttgggaatg taattcagct ccgccatcgc cgcttccact ttttcccgcg 6960
   ttttcgcaga aacgtggctg gcctggttca ccacgcggga aacggtctga taagagacac 7020
   cggcatactc tgcgacatcg tataacgtta ctggtttcac attcaccacc ctgaattgac 7080
   tctcttccgg gcgctatcat gccataccgc gaaaggtttt gcgccattcg atggtgtc 7138
<210> 8
   <211> 6649
   <212> DNA
   <213> artificial sequence
<220>
   <223> Vector pCDF{Ptac}[hGLYAT2(co_Ec)-fadD_Ec]
<220>
   <221> misc_feature
   <222> (1)..(6649)
   <223> Vector pCDF{Ptac}[hGLYAT2(co_Ec)-fadD_Ec]
<400> 8
   cgggatctcg acgctctccc ttatgcgact cctgcgttta gggaaagagc atttgtcaga 60
   atatttaagg gcgcctgtca ctttgcttga tatatgagaa ttatttaacc ttataaatga 120
   gaaaaaagca acgcacttta aataagatac gttgcttttt cgattgatga acacctataa 180
   ttaaactatt catctattat ttatgatttt ttgtatatac aatatttcta gtttgttaaa 240
   gagaattaag aaaataaatc tcgaaaataa taaagggaaa atcagttttt gatatcaaaa 300
   ttatacatgt caacgataat acaaaatata atacaaacta taagatgtta tcagtattta 360
   ttatgcattt agaatacctt ttgtgtcgcc cttattcgac tccctataga agttcctatt 420
   ctctagaaag tataggaact tcccttcatt ttggatccaa ttgtgagcgg ataacaatta 480
   cgagcttcat gcacagtgat cgacgctgtt gacaattaat catcggctcg tataatgtgt 540
   ggatgtggaa ttgtgagcgc tcacaattcc acaacggttt ccctctagaa ataattttgt 600
   ttaacaggag gtaaaacata tgctggttct gcacaacagc caaaaactgc aaattctgta 660
   caaatctctg gaaaaatcta ttcctgaaag cattaaagtt tatggtgcga tcttcaacat 720
   caaggataaa aaccctttca atatggaggt gctggtcgac gcgtggcctg attaccaaat 780
   cgtcatcacc cgtccgcaga agcaagaaat gaaagatgac caggaccact atactaacac 840
   ctaccacatc ttcactaaag cgccggacaa actggaagaa gttctgagct actccaatgt 900
   tatctcctgg gaacaaacgc tgcagattca aggttgccag gaaggtctgg atgaggcgat 960
   tcgtaaggtc gctacctcta agtccgttca agtcgattac atgaaaacca tcctgtttat 1020
   cccggagctg ccgaagaaac acaaaacctc ctctaacgac aagatggagc tgttcgaagt 1080
   agacgatgac aacaaagagg gtaacttttc caatatgttc ctggacgcct cccacgccgg 1140
   tctggttaat gagcactggg cgttcggtaa aaacgaacgc tccctgaagt acattgagcg 1200
   ttgtctgcag gattttctgg gttttggtgt cctgggtcca gagggtcaac tggtctcttg 1260
   gatcgttatg gaacagtctt gtgaactgcg tatgggttat actgtgccaa aataccgtca 1320
   ccaaggtaat atgctgcaaa tcggttatca tctggagaag tacctgagcc agaaggaaat 1380
   cccgttctat ttccatgttg ccgataataa cgaaaagagc ctgcaagccc tgaacaatct 1440
   gggcttcaag atctgccctt gcggttggca ccagtggaag tgcacgccta aaaagtactg 1500
   ttaagcgtta agtctgagag gtgaagaatt gaagaaggtt tggcttaacc gttatcccgc 1560
   ggacgttccg acggagatca accctgaccg ttatcaatct ctggtagata tgtttgagca 1620
   gtcggtcgcg cgctacgccg atcaacctgc gtttgtgaat atgggggagg taatgacctt 1680
   ccgcaagctg gaagaacgca gtcgcgcgtt tgccgcttat ttgcaacaag ggttggggct 1740
   gaagaaaggc gatcgcgttg cgttgatgat gcctaattta ttgcaatatc cggtggcgct 1800
   gtttggcatt ttgcgtgccg ggatgatcgt cgtaaacgtt aacccgttgt ataccccgcg 1860
   tgagcttgag catcagctta acgatagcgg cgcatcggcg attgttatcg tgtctaactt 1920
   tgctcacaca ctggaaaaag tggttgataa aaccgccgtt cagcacgtaa ttctgacccg 1980
   tatgggcgat cagctatcta cggcaaaagg cacggtagtc aatttcgttg ttaaatacat 2040
   caagcgtttg gtgccgaaat accatctgcc agatgccatt tcatttcgta gcgcactgca 2100
   taacggctac cggatgcagt acgtcaaacc cgaactggtg ccggaagatt tagcttttct 2160
   gcaatacacc ggcggcacca ctggtgtggc gaaaggcgcg atgctgactc accgcaatat 2220
   gctggcgaac ctggaacagg ttaacgcgac ctatggtccg ctgttgcatc cgggcaaaga 2280
   gctggtggtg acggcgctgc cgctgtatca catttttgcc ctgaccatta actgcctgct 2340
   gtttatcgaa ctgggtgggc agaacctgct tatcactaac ccgcgcgata ttccagggtt 2400
   ggtaaaagag ttagcgaaat atccgtttac cgctatcacg ggcgttaaca ccttgttcaa 2460
   tgcgttgctg aacaataaag agttccagca gctggatttc tccagtctgc atctttccgc 2520
   aggcggtggg atgccagtgc agcaagtggt ggcagagcgt tgggtgaaac tgaccggaca 2580
   gtatctgctg gaaggctatg gccttaccga gtgtgcgccg ctggtcagcg ttaacccata 2640
   tgatattgat tatcatagtg gtagcatcgg tttgccggtg ccgtcgacgg aagccaaact 2700
   ggtggatgat gatgataatg aagtaccacc aggtcaaccg ggtgagcttt gtgtcaaagg 2760
   accgcaggtg atgctgggtt actggcagcg tcccgatgct accgatgaaa tcatcaaaaa 2820
   tggctggtta cacaccggcg acatcgcggt aatggatgaa gaaggattcc tgcgcattgt 2880
   cgatcgtaaa aaagacatga ttctggtttc cggttttaac gtctatccca acgagattga 2940
   agatgtcgtc atgcagcatc ctggcgtaca ggaagtcgcg gctgttggcg taccttccgg 3000
   ctccagtggt gaagcggtga aaatcttcgt agtgaaaaaa gatccatcgc ttaccgaaga 3060
   gtcactggtg actttttgcc gccgtcagct cacgggatac aaagtaccga agctggtgga 3120
   gtttcgtgat gagttaccga aatctaacgt cggaaaaatt ttgcgacgag aattacgtga 3180
   cgaagcgcgc ggcaaagtgg acaataaagc ctgagcgtta agtcagtcgt cagacgccgg 3240
   ttaatccggc gttttttttg acgcccacta aagagaaaac aatctcgagt ctggtaaaga 3300
   aaccgctgct gcgaaatttg aacgccagca catggactcg tctactagcg cagcttaatt 3360
   aacctaggct gctgccaccg ctgagcaata actagcataa ccccttgggg cctctaaacg 3420
   ggtcttgagg ggttttttgc tgaaacctca ggcatttgag aagcacacgg tcacactgct 3480
   tccggtagtc aataaaccgg taaaccagca atagacataa gcggctattt aacgaccctg 3540
   ccctgaaccg acgaccgggt catcgtggcc ggatcttgcg gcccctcggc ttgaacgaat 3600
   tgttagacat tatttgccga ctaccttggt gatctcgcct ttcacgtagt ggacaaattc 3660
   ttccaactga tctgcgcgcg aggccaagcg atcttcttct tgtccaagat aagcctgtct 3720
   agcttcaagt atgacgggct gatactgggc cggcaggcgc tccattgccc agtcggcagc 3780
   gacatccttc ggcgcgattt tgccggttac tgcgctgtac caaatgcggg acaacgtaag 3840
   cactacattt cgctcatcgc cagcccagtc gggcggcgag ttccatagcg ttaaggtttc 3900
   atttagcgcc tcaaatagat cctgttcagg aaccggatca aagagttcct ccgccgctgg 3960
   acctaccaag gcaacgctat gttctcttgc ttttgtcagc aagatagcca gatcaatgtc 4020
   gatcgtggct ggctcgaaga tacctgcaag aatgtcattg cgctgccatt ctccaaattg 4080
   cagttcgcgc ttagctggat aacgccacgg aatgatgtcg tcgtgcacaa caatggtgac 4140
   ttctacagcg cggagaatct cgctctctcc aggggaagcc gaagtttcca aaaggtcgtt 4200
   gatcaaagct cgccgcgttg tttcatcaag ccttacggtc accgtaacca gcaaatcaat 4260
   atcactgtgt ggcttcaggc cgccatccac tgcggagccg tacaaatgta cggccagcaa 4320
   cgtcggttcg agatggcgct cgatgacgcc aactacctct gatagttgag tcgatacttc 4380
   ggcgatcacc gcttccctca tactcttcct ttttcaatat tattgaagca tttatcaggg 4440
   ttattgtctc atgagcggat acatatttga atgtatttag aaaaataaac aaatagctag 4500
   ctcactcggt cgctacgctc cgggcgtgag actgcggcgg gcgctgcgga cacatacaaa 4560
   gttacccaca gattccgtgg ataagcaggg gactaacatg tgaggcaaaa cagcagggcc 4620
   gcgccggtgg cgtttttcca taggctccgc cctcctgcca gagttcacat aaacagacgc 4680
   ttttccggtg catctgtggg agccgtgagg ctcaaccatg aatctgacag tacgggcgaa 4740
   acccgacagg acttaaagat ccccaccgtt tccggcgggt cgctccctct tgcgctctcc 4800
   tgttccgacc ctgccgttta ccggatacct gttccgcctt tctcccttac gggaagtgtg 4860
   gcgctttctc atagctcaca cactggtatc tcggctcggt gtaggtcgtt cgctccaagc 4920
   tgggctgtaa gcaagaactc cccgttcagc ccgactgctg cgccttatcc ggtaactgtt 4980
   cacttgagtc caacccggaa aagcacggta aaacgccact ggcagcagcc attggtaact 5040
   gggagttcgc agaggatttg tttagctaaa cacgcggttg ctcttgaagt gtgcgccaaa 5100
   gtccggctac actggaagga cagatttggt tgctgtgctc tgcgaaagcc agttaccacg 5160
   gttaagcagt tccccaactg acttaacctt cgatcaaacc acctccccag gtggtttttt 5220
   cgtttacagg gcaaaagatt acgcgcagaa aaaaaggatc tcaagaagat cctttgatct 5280
   tttctactga accgctctag atttcagtgc aatttatctc ttcaaatgta gcacctgaag 5340
   tcagccccat acgatataag ttgtaattct catgttagtc atgccccgcg cccaccggaa 5400
   ggagctgact gggttgaagg ctctcaaggg catcggtcga gatcccggtg cctaatgagt 5460
   gagctaactt acattaattg cgttgcgctc actgcccgct ttccagtcgg gaaacctgtc 5520
   gtgccagctg cattaatgaa tcggccaacg cgcggggaga ggcggtttgc gtattgggcg 5580
   ccagggtggt ttttcttttc accagtgaga cgggcaacag ctgattgccc ttcaccgcct 5640
   ggccctgaga gagttgcagc aagcggtcca cgctggtttg ccccagcagg cgaaaatcct 5700
   gtttgatggt ggttaacggc gggatataac atgagctgtc ttcggtatcg tcgtatccca 5760
   ctaccgagat gtccgcacca acgcgcagcc cggactcggt aatggcgcgc attgcgccca 5820
   gcgccatctg atcgttggca accagcatcg cagtgggaac gatgccctca ttcagcattt 5880
   gcatggtttg ttgaaaaccg gacatggcac tccagtcgcc ttcccgttcc gctatcggct 5940
   gaatttgatt gcgagtgaga tatttatgcc agccagccag acgcagacgc gccgagacag 6000
   aacttaatgg gcccgctaac agcgcgattt gctggtgacc caatgcgacc agatgctcca 6060
   cgcccagtcg cgtaccgtct tcatgggaga aaataatact gttgatgggt gtctggtcag 6120
   agacatcaag aaataacgcc ggaacattag tgcaggcagc ttccacagca atggcatcct 6180
   ggtcatccag cggatagtta atgatcagcc cactgacgcg ttgcgcgaga agattgtgca 6240
   ccgccgcttt acaggcttcg acgccgcttc gttctaccat cgacaccacc acgctggcac 6300
   ccagttgatc ggcgcgagat ttaatcgccg cgacaatttg cgacggcgcg tgcagggcca 6360
   gactggaggt ggcaacgcca atcagcaacg actgtttgcc cgccagttgt tgtgccacgc 6420
   ggttgggaat gtaattcagc tccgccatcg ccgcttccac tttttcccgc gttttcgcag 6480
   aaacgtggct ggcctggttc accacgcggg aaacggtctg ataagagaca ccggcatact 6540
   ctgcgacatc gtataacgtt actggtttca cattcaccac cctgaattga ctctcttccg 6600
   ggcgctatca tgccataccg cgaaaggttt tgcgccattc gatggtgtc 6649
<210> 9
   <211> 6631
   <212> DNA
   <213> Artificial Sequence
<220>
   <223> Vector pCDF{Ptac}[hGLYAT3(co_Ec)-fadD_Ec]
<220>
   <221> misc_feature
   <222> (1)..(6631)
<400> 9
   cgggatctcg acgctctccc ttatgcgact cctgcgttta gggaaagagc atttgtcaga 60
   atatttaagg gcgcctgtca ctttgcttga tatatgagaa ttatttaacc ttataaatga 120
   gaaaaaagca acgcacttta aataagatac gttgcttttt cgattgatga acacctataa 180
   ttaaactatt catctattat ttatgatttt ttgtatatac aatatttcta gtttgttaaa 240
   gagaattaag aaaataaatc tcgaaaataa taaagggaaa atcagttttt gatatcaaaa 300
   ttatacatgt caacgataat acaaaatata atacaaacta taagatgtta tcagtattta 360
   ttatgcattt agaatacctt ttgtgtcgcc cttattcgac tccctataga agttcctatt 420
   ctctagaaag tataggaact tcccttcatt ttggatccaa ttgtgagcgg ataacaatta 480
   cgagcttcat gcacagtgat cgacgctgtt gacaattaat catcggctcg tataatgtgt 540
   ggatgtggaa ttgtgagcgc tcacaattcc acaacggttt ccctctagaa ataattttgt 600
   ttaacaggag gtaaaacata tgctggttct gaattgctct actaaactgc tgatcctgga 660
   gaaaatgctg aaatcctgtt tcccggaatc tctgaaagtt tatggtgcgg taatgaatat 720
   caaccgtggt aatccgtttc aaaaagaagt tgttctggac tcttggccag atttcaaggc 780
   ggttatcacc cgtcgtcagc gtgaagcgga aaccgacaac ctggaccact ataccaacgc 840
   gtatgcggtt ttctataaag atgttcgcgc gtaccgtcag ctgctggaag aatgcgatgt 900
   tttcaactgg gaccaggttt ttcagatcca aggtctgcaa tccgagctgt acgacgtttc 960
   taaggccgtg gcgaactcta aacaactgaa catcaagctg acctctttca aagcggttca 1020
   tttctctccg gtatccagcc tgccggacac ctccttcctg aaaggtccgt ctccgcgtct 1080
   gacctacctg tctgttgcga atgcggatct gctgaaccgt acttggtccc gtggtggcaa 1140
   cgaacagtgc ctgcgttaca ttgctaacct gatctcttgc ttcccgagcg tctgcgttcg 1200
   tgatgaaaaa ggtaacccag tatcttggtc tatcaccgac cagttcgcga ccatgtgtca 1260
   tggctacacc ctgcctgaac accgtcgtaa gggttactct cgtctggttg cgctgaccct 1320
   ggcgcgtaag ctgcagtctc gtggttttcc gtcccagggt aacgtcctgg acgacaacac 1380
   tgcgtccatc tccctgctga agtccctgca tgccgaattc ctgccgtgcc gtttccaccg 1440
   tctgattctg accccagcga ccttctctgg tctgccgcac ctgtaagcgt taagtctgag 1500
   aggtgaagaa ttgaagaagg tttggcttaa ccgttatccc gcggacgttc cgacggagat 1560
   caaccctgac cgttatcaat ctctggtaga tatgtttgag cagtcggtcg cgcgctacgc 1620
   cgatcaacct gcgtttgtga atatggggga ggtaatgacc ttccgcaagc tggaagaacg 1680
   cagtcgcgcg tttgccgctt atttgcaaca agggttgggg ctgaagaaag gcgatcgcgt 1740
   tgcgttgatg atgcctaatt tattgcaata tccggtggcg ctgtttggca ttttgcgtgc 1800
   cgggatgatc gtcgtaaacg ttaacccgtt gtataccccg cgtgagcttg agcatcagct 1860
   taacgatagc ggcgcatcgg cgattgttat cgtgtctaac tttgctcaca cactggaaaa 1920
   agtggttgat aaaaccgccg ttcagcacgt aattctgacc cgtatgggcg atcagctatc 1980
   tacggcaaaa ggcacggtag tcaatttcgt tgttaaatac atcaagcgtt tggtgccgaa 2040
   ataccatctg ccagatgcca tttcatttcg tagcgcactg cataacggct accggatgca 2100
   gtacgtcaaa cccgaactgg tgccggaaga tttagctttt ctgcaataca ccggcggcac 2160
   cactggtgtg gcgaaaggcg cgatgctgac tcaccgcaat atgctggcga acctggaaca 2220
   ggttaacgcg acctatggtc cgctgttgca tccgggcaaa gagctggtgg tgacggcgct 2280
   gccgctgtat cacatttttg ccctgaccat taactgcctg ctgtttatcg aactgggtgg 2340
   gcagaacctg cttatcacta acccgcgcga tattccaggg ttggtaaaag agttagcgaa 2400
   atatccgttt accgctatca cgggcgttaa caccttgttc aatgcgttgc tgaacaataa 2460
   agagttccag cagctggatt tctccagtct gcatctttcc gcaggcggtg ggatgccagt 2520
   gcagcaagtg gtggcagagc gttgggtgaa actgaccgga cagtatctgc tggaaggcta 2580
   tggccttacc gagtgtgcgc cgctggtcag cgttaaccca tatgatattg attatcatag 2640
   tggtagcatc ggtttgccgg tgccgtcgac ggaagccaaa ctggtggatg atgatgataa 2700
   tgaagtacca ccaggtcaac cgggtgagct ttgtgtcaaa ggaccgcagg tgatgctggg 2760
   ttactggcag cgtcccgatg ctaccgatga aatcatcaaa aatggctggt tacacaccgg 2820
   cgacatcgcg gtaatggatg aagaaggatt cctgcgcatt gtcgatcgta aaaaagacat 2880
   gattctggtt tccggtttta acgtctatcc caacgagatt gaagatgtcg tcatgcagca 2940
   tcctggcgta caggaagtcg cggctgttgg cgtaccttcc ggctccagtg gtgaagcggt 3000
   gaaaatcttc gtagtgaaaa aagatccatc gcttaccgaa gagtcactgg tgactttttg 3060
   ccgccgtcag ctcacgggat acaaagtacc gaagctggtg gagtttcgtg atgagttacc 3120
   gaaatctaac gtcggaaaaa ttttgcgacg agaattacgt gacgaagcgc gcggcaaagt 3180
   ggacaataaa gcctgagcgt taagtcagtc gtcagacgcc ggttaatccg gcgttttttt 3240
   tgacgcccac taaagagaaa acaatctcga gtctggtaaa gaaaccgctg ctgcgaaatt 3300
   tgaacgccag cacatggact cgtctactag cgcagcttaa ttaacctagg ctgctgccac 3360
   cgctgagcaa taactagcat aaccccttgg ggcctctaaa cgggtcttga ggggtttttt 3420
   gctgaaacct caggcatttg agaagcacac ggtcacactg cttccggtag tcaataaacc 3480
   ggtaaaccag caatagacat aagcggctat ttaacgaccc tgccctgaac cgacgaccgg 3540
   gtcatcgtgg ccggatcttg cggcccctcg gcttgaacga attgttagac attatttgcc 3600
   gactaccttg gtgatctcgc ctttcacgta gtggacaaat tcttccaact gatctgcgcg 3660
   cgaggccaag cgatcttctt cttgtccaag ataagcctgt ctagcttcaa gtatgacggg 3720
   ctgatactgg gccggcaggc gctccattgc ccagtcggca gcgacatcct tcggcgcgat 3780
   tttgccggtt actgcgctgt accaaatgcg ggacaacgta agcactacat ttcgctcatc 3840
   gccagcccag tcgggcggcg agttccatag cgttaaggtt tcatttagcg cctcaaatag 3900
   atcctgttca ggaaccggat caaagagttc ctccgccgct ggacctacca aggcaacgct 3960
   atgttctctt gcttttgtca gcaagatagc cagatcaatg tcgatcgtgg ctggctcgaa 4020
   gatacctgca agaatgtcat tgcgctgcca ttctccaaat tgcagttcgc gcttagctgg 4080
   ataacgccac ggaatgatgt cgtcgtgcac aacaatggtg acttctacag cgcggagaat 4140
   ctcgctctct ccaggggaag ccgaagtttc caaaaggtcg ttgatcaaag ctcgccgcgt 4200
   tgtttcatca agccttacgg tcaccgtaac cagcaaatca atatcactgt gtggcttcag 4260
   gccgccatcc actgcggagc cgtacaaatg tacggccagc aacgtcggtt cgagatggcg 4320
   ctcgatgacg ccaactacct ctgatagttg agtcgatact tcggcgatca ccgcttccct 4380
   catactcttc ctttttcaat attattgaag catttatcag ggttattgtc tcatgagcgg 4440
   atacatattt gaatgtattt agaaaaataa acaaatagct agctcactcg gtcgctacgc 4500
   tccgggcgtg agactgcggc gggcgctgcg gacacataca aagttaccca cagattccgt 4560
   ggataagcag gggactaaca tgtgaggcaa aacagcaggg ccgcgccggt ggcgtttttc 4620
   cataggctcc gccctcctgc cagagttcac ataaacagac gcttttccgg tgcatctgtg 4680
   ggagccgtga ggctcaacca tgaatctgac agtacgggcg aaacccgaca ggacttaaag 4740
   atccccaccg tttccggcgg gtcgctccct cttgcgctct cctgttccga ccctgccgtt 4800
   taccggatac ctgttccgcc tttctccctt acgggaagtg tggcgctttc tcatagctca 4860
   cacactggta tctcggctcg gtgtaggtcg ttcgctccaa gctgggctgt aagcaagaac 4920
   tccccgttca gcccgactgc tgcgccttat ccggtaactg ttcacttgag tccaacccgg 4980
   aaaagcacgg taaaacgcca ctggcagcag ccattggtaa ctgggagttc gcagaggatt 5040
   tgtttagcta aacacgcggt tgctcttgaa gtgtgcgcca aagtccggct acactggaag 5100
   gacagatttg gttgctgtgc tctgcgaaag ccagttacca cggttaagca gttccccaac 5160
   tgacttaacc ttcgatcaaa ccacctcccc aggtggtttt ttcgtttaca gggcaaaaga 5220
   ttacgcgcag aaaaaaagga tctcaagaag atcctttgat cttttctact gaaccgctct 5280
   agatttcagt gcaatttatc tcttcaaatg tagcacctga agtcagcccc atacgatata 5340
   agttgtaatt ctcatgttag tcatgccccg cgcccaccgg aaggagctga ctgggttgaa 5400
   ggctctcaag ggcatcggtc gagatcccgg tgcctaatga gtgagctaac ttacattaat 5460
   tgcgttgcgc tcactgcccg ctttccagtc gggaaacctg tcgtgccagc tgcattaatg 5520
   aatcggccaa cgcgcgggga gaggcggttt gcgtattggg cgccagggtg gtttttcttt 5580
   tcaccagtga gacgggcaac agctgattgc ccttcaccgc ctggccctga gagagttgca 5640
   gcaagcggtc cacgctggtt tgccccagca ggcgaaaatc ctgtttgatg gtggttaacg 5700
   gcgggatata acatgagctg tcttcggtat cgtcgtatcc cactaccgag atgtccgcac 5760
   caacgcgcag cccggactcg gtaatggcgc gcattgcgcc cagcgccatc tgatcgttgg 5820
   caaccagcat cgcagtggga acgatgccct cattcagcat ttgcatggtt tgttgaaaac 5880
   cggacatggc actccagtcg ccttcccgtt ccgctatcgg ctgaatttga ttgcgagtga 5940
   gatatttatg ccagccagcc agacgcagac gcgccgagac agaacttaat gggcccgcta 6000
   acagcgcgat ttgctggtga cccaatgcga ccagatgctc cacgcccagt cgcgtaccgt 6060
   cttcatggga gaaaataata ctgttgatgg gtgtctggtc agagacatca agaaataacg 6120
   ccggaacatt agtgcaggca gcttccacag caatggcatc ctggtcatcc agcggatagt 6180
   taatgatcag cccactgacg cgttgcgcga gaagattgtg caccgccgct ttacaggctt 6240
   cgacgccgct tcgttctacc atcgacacca ccacgctggc acccagttga tcggcgcgag 6300
   atttaatcgc cgcgacaatt tgcgacggcg cgtgcagggc cagactggag gtggcaacgc 6360
   caatcagcaa cgactgtttg cccgccagtt gttgtgccac gcggttggga atgtaattca 6420
   gctccgccat cgccgcttcc actttttccc gcgttttcgc agaaacgtgg ctggcctggt 6480
   tcaccacgcg ggaaacggtc tgataagaga caccggcata ctctgcgaca tcgtataacg 6540
   ttactggttt cacattcacc accctgaatt gactctcttc cgggcgctat catgccatac 6600
   cgcgaaaggt tttgcgccat tcgatggtgt c 6631
<210> 10
   <211> 693
   <212> DNA
   <213> Pseudomonas putida
<220>
   <221> misc_feature
   <222> (1)..(693)
   <223> alkL (an importer facilitating transport of hydrophobic acyl across cell membranes) gene
<400> 10
   gtgagttttt ctaattataa agtaatcgcg atgccggtgt tggttgctaa ttttgttttg 60
   ggggcggcca ctgcatgggc gaatgaaaat tatccggcga aatctgctgg ctataatcag 120
   ggtgactggg tcgctagctt caatttttct aaggtctatg tgggtgagga gcttggcgat 180
   ctaaatgttg gagggggggc tttgccaaat gctgatgtaa gtattggtaa tgatacaaca 240
   cttacgtttg atatcgccta ttttgttagc tcaaatatag cggtggattt ttttgttggg 300
   gtgccagcta gggctaaatt tcaaggtgag aaatcaatct cctcgctggg aagagtcagt 360
   gaagttgatt acggccctgc aattctttcg cttcaatatc attacgatag ctttgagcga 420
   ctttatccat atgttggggt tggtgttggt cgggtgctat tttttgataa aaccgacggt 480
   gctttgagtt cgtttgatat taaggataaa tgggcgcctg cttttcaggt tggccttaga 540
   tatgaccttg gtaactcatg gatgctaaat tcagatgtgc gttatattcc tttcaaaacg 600
   gacgtcacag gtactcttgg cccggttcct gtttctacta aaattgaggt tgatcctttc 660
   attctcagtc ttggtgcgtc atatgttttc taa 693
<210> 11
   <211> 153
   <212> DNA
   <213> Artificial Sequence
<220>
   <223> lacuv5 promoter
<220>
   <221> misc_feature
   <222> (1)..(153)
<400> 11
   ggcagtgagc gcaacgcaat taatgtaagt tagctcactc attaggcacc ccaggcttga 60
   cactttatgc ttccggctcg tataatgtgt ggaattgtga gcggataaca ataacaattt 120
   cacacaggat ctaggaacca aggagagtgg cat 153
<210> 12
   <211> 4876
   <212> DNA
   <213> Artificial Sequence
<220>
   <223> Vector pCDF[alkLmodl]
<220>
   <221> misc_feature
   <222> (1)..(4876)
   <223> Vector pCDF[alkLmodl]
<400> 12
   ggggaattgt gagcggataa caattcccct gtagaaataa ttttgtttaa ctttaataag      60
   gagatatacc atgggcagca gccatcacca tcatcaccac agccaggatc cttcaatatt     120
   attgaagcat ttatcagggt tattgtctca tgagcggata catatttgaa tgtatttaga     180
   aaaataaaca aataggggtc agtgttacaa ccaattaacc aattctgaac attatcgcga     240
   gcccatttat acctgaatat ggctcataac accccttgtt tgcctggcgg cagtagcgcg     300
   gtggtcccac ctgaccccat gccgaactca gaagtgaaac gccgtagcgc cgatggtagt     360
   gtggggactc cccatgcgag agtagggaac tgccaggcat caaataaaac gaaaggctca     420
   gtcgaaagac tgggcctttc gcccgggcta attatggggt gtcgccctta ttcgactcta     480
   tagtgaagtt cctattctct agaaagtata ggaacttctg aagtggggat ccggccggcc     540
   tgcagccccg cagggcctgt ctcggtcgat cattcagccc ggctcataga tatgcgggca     600
   gtgagcgcaa cgcaattaat gtaagttagc tcactcatta ggcaccccag gcttgacact     660
   ttatgcttcc ggctcgtata atgtgtggaa ttgtgagcgg ataacaataa caatttcaca     720
   caggatctag gaaccaagga gagtggcatg tgagtttttc taattataaa gtaatcgcga     780
   tgccggtgtt ggttgctaat tttgttttgg gggcggccac tgcatgggcg aatgaaaatt     840
   atccggcgaa atctgctggc tataatcagg gtgactgggt cgctagcttc aatttttcta     900
   aggtctatgt gggtgaggag cttggcgatc taaatgttgg agggggggct ttgccaaatg     960
   ctgatgtaag tattggtaat gatacaacac ttacgtttga tatcgcctat tttgttagct    1020
   caaatatagc ggtggatttt tttgttgggg tgccagctag ggctaaattt caaggtgaga    1080
   aatcaatctc ctcgctggga agagtcagtg aagttgatta cggccctgca attctttcgc    1140
   ttcaatatca ttacgatagc tttgagcgac tttatccata tgttggggtt ggtgttggtc    1200
   gggtgctatt ttttgataaa accgacggtg ctttgagttc gtttgatatt aaggataaat    1260
   gggcgcctgc ttttcaggtt ggccttagat atgaccttgg taactcatgg atgctaaatt    1320
   cagatgtgcg ttatattcct ttcaaaacgg acgtcacagg tactcttggc ccggttcctg 1380
   tttctactaa aattgaggtt gatcctttca ttctcagtct tggtgcgtca tatgttttct 1440
   aaggtaccct cgagtctggt aaagaaaccg ctgctgcgaa atttgaacgc cagcacatgg 1500
   actcgtctac tagcgcagct taattaacct aggctgctgc caccgctgag caataactag 1560
   cataacccct tggggcctct aaacgggtct tgaggggttt tttgctgaaa cctcaggcat 1620
   ttgagaagca cacggtcaca ctgcttccgg tagtcaataa accggtaaac cagcaataga 1680
   cataagcggc tatttaacga ccctgccctg aaccgacgac cgggtcatcg tggccggatc 1740
   ttgcggcccc tcggcttgaa cgaattgtta gacattattt gccgactacc ttggtgatct 1800
   cgcctttcac gtagtggaca aattcttcca actgatctgc gcgcgaggcc aagcgatctt 1860
   cttcttgtcc aagataagcc tgtctagctt caagtatgac gggctgatac tgggccggca 1920
   ggcgctccat tgcccagtcg gcagcgacat ccttcggcgc gattttgccg gttactgcgc 1980
   tgtaccaaat gcgggacaac gtaagcacta catttcgctc atcgccagcc cagtcgggcg 2040
   gcgagttcca tagcgttaag gtttcattta gcgcctcaaa tagatcctgt tcaggaaccg 2100
   gatcaaagag ttcctccgcc gctggaccta ccaaggcaac gctatgttct cttgcttttg 2160
   tcagcaagat agccagatca atgtcgatcg tggctggctc gaagatacct gcaagaatgt 2220
   cattgcgctg ccattctcca aattgcagtt cgcgcttagc tggataacgc cacggaatga 2280
   tgtcgtcgtg cacaacaatg gtgacttcta cagcgcggag aatctcgctc tctccagggg 2340
   aagccgaagt ttccaaaagg tcgttgatca aagctcgccg cgttgtttca tcaagcctta 2400
   cggtcaccgt aaccagcaaa tcaatatcac tgtgtggctt caggccgcca tccactgcgg 2460
   agccgtacaa atgtacggcc agcaacgtcg gttcgagatg gcgctcgatg acgccaacta 2520
   cctctgatag ttgagtcgat acttcggcga tcaccgcttc cctcatactc ttcctttttc 2580
   aatattattg aagcatttat cagggttatt gtctcatgag cggatacata tttgaatgta 2640
   tttagaaaaa taaacaaata gctagctcac tcggtcgcta cgctccgggc gtgagactgc 2700
   ggcgggcgct gcggacacat acaaagttac ccacagattc cgtggataag caggggacta 2760
   acatgtgagg caaaacagca gggccgcgcc ggtggcgttt ttccataggc tccgccctcc 2820
   tgccagagtt cacataaaca gacgcttttc cggtgcatct gtgggagccg tgaggctcaa 2880
   ccatgaatct gacagtacgg gcgaaacccg acaggactta aagatcccca ccgtttccgg 2940
   cgggtcgctc cctcttgcgc tctcctgttc cgaccctgcc gtttaccgga tacctgttcc 3000
   gcctttctcc cttacgggaa gtgtggcgct ttctcatagc tcacacactg gtatctcggc 3060
   tcggtgtagg tcgttcgctc caagctgggc tgtaagcaag aactccccgt tcagcccgac 3120
   tgctgcgcct tatccggtaa ctgttcactt gagtccaacc cggaaaagca cggtaaaacg 3180
   ccactggcag cagccattgg taactgggag ttcgcagagg atttgtttag ctaaacacgc 3240
   ggttgctctt gaagtgtgcg ccaaagtccg gctacactgg aaggacagat ttggttgctg 3300
   tgctctgcga aagccagtta ccacggttaa gcagttcccc aactgactta accttcgatc 3360
   aaaccacctc cccaggtggt tttttcgttt acagggcaaa agattacgcg cagaaaaaaa 3420
   ggatctcaag aagatccttt gatcttttct actgaaccgc tctagatttc agtgcaattt 3480
   atctcttcaa atgtagcacc tgaagtcagc cccatacgat ataagttgta attctcatgt 3540
   tagtcatgcc ccgcgcccac cggaaggagc tgactgggtt gaaggctctc aagggcatcg 3600
   gtcgagatcc cggtgcctaa tgagtgagct aacttacatt aattgcgttg cgctcactgc 3660
   ccgctttcca gtcgggaaac ctgtcgtgcc agctgcatta atgaatcggc caacgcgcgg 3720
   ggagaggcgg tttgcgtatt gggcgccagg gtggtttttc ttttcaccag tgagacgggc 3780
   aacagctgat tgcccttcac cgcctggccc tgagagagtt gcagcaagcg gtccacgctg 3840
   gtttgcccca gcaggcgaaa atcctgtttg atggtggtta acggcgggat ataacatgag 3900
   ctgtcttcgg tatcgtcgta tcccactacc gagatgtccg caccaacgcg cagcccggac 3960
   tcggtaatgg cgcgcattgc gcccagcgcc atctgatcgt tggcaaccag catcgcagtg 4020
   ggaacgatgc cctcattcag catttgcatg gtttgttgaa aaccggacat ggcactccag 4080
   tcgccttccc gttccgctat cggctgaatt tgattgcgag tgagatattt atgccagcca 4140
   gccagacgca gacgcgccga gacagaactt aatgggcccg ctaacagcgc gatttgctgg 4200
   tgacccaatg cgaccagatg ctccacgccc agtcgcgtac cgtcttcatg ggagaaaata 4260
   atactgttga tgggtgtctg gtcagagaca tcaagaaata acgccggaac attagtgcag 4320
   gcagcttcca cagcaatggc atcctggtca tccagcggat agttaatgat cagcccactg 4380
   acgcgttgcg cgagaagatt gtgcaccgcc gctttacagg cttcgacgcc gcttcgttct 4440
   accatcgaca ccaccacgct ggcacccagt tgatcggcgc gagatttaat cgccgcgaca 4500
   atttgcgacg gcgcgtgcag ggccagactg gaggtggcaa cgccaatcag caacgactgt 4560
   ttgcccgcca gttgttgtgc cacgcggttg ggaatgtaat tcagctccgc catcgccgct 4620
   tccacttttt cccgcgtttt cgcagaaacg tggctggcct ggttcaccac gcgggaaacg 4680
   gtctgataag agacaccggc atactctgcg acatcgtata acgttactgg tttcacattc 4740
   accaccctga attgactctc ttccgggcgc tatcatgcca taccgcgaaa ggttttgcgc 4800
   cattcgatgg tgtccgggat ctcgacgctc tcccttatgc gactcctgca ttaggaaatt 4860
   aatacgactc actata 4876
<210> 13
   <211> 7468
   <212> DNA
   <213> Artificial Sequence
<220>
   <223> Vector pCDF{Ptac}[hGLYAT2(co_Ec)-fadD_Ec]{Placuv5}[alkLmod1]
<220>
   <221> misc_feature
   <222> (1)..(7468)
   <223> Vector pCDF{Ptac}[hGLYAT2(co_Ec)-fadD_Ec]{Placuv5}[alkLmod1]
<400> 13
   cgggatctcg acgctctccc ttatgcgact cctgcgttta gggaaagagc atttgtcaga      60
   atatttaagg gcgcctgtca ctttgcttga tatatgagaa ttatttaacc ttataaatga     120
   gaaaaaagca acgcacttta aataagatac gttgcttttt cgattgatga acacctataa     180
   ttaaactatt catctattat ttatgatttt ttgtatatac aatatttcta gtttgttaaa     240
   gagaattaag aaaataaatc tcgaaaataa taaagggaaa atcagttttt gatatcaaaa     300
   ttatacatgt caacgataat acaaaatata atacaaacta taagatgtta tcagtattta     360
   ttatgcattg tctcggtcga tcattcagcc cggctcatag atatgcgggc agtgagcgca     420
   acgcaattaa tgtaagttag ctcactcatt aggcacccca ggcttgacac tttatgcttc     480
   cggctcgtat aatgtgtgga attgtgagcg gataacaata acaatttcac acaggatcta     540
   ggaaccaagg agagtggcat gtgagttttt ctaattataa agtaatcgcg atgccggtgt     600
   tggttgctaa ttttgttttg ggggcggcca ctgcatgggc gaatgaaaat tatccggcga     660
   aatctgctgg ctataatcag ggtgactggg tcgctagctt caatttttct aaggtctatg     720
   tgggtgagga gcttggcgat ctaaatgttg gagggggggc tttgccaaat gctgatgtaa     780
   gtattggtaa tgatacaaca cttacgtttg atatcgccta ttttgttagc tcaaatatag     840
   cggtggattt ttttgttggg gtgccagcta gggctaaatt tcaaggtgag aaatcaatct     900
   cctcgctggg aagagtcagt gaagttgatt acggccctgc aattctttcg cttcaatatc     960
   attacgatag ctttgagcga ctttatccat atgttggggt tggtgttggt cgggtgctat    1020
   tttttgataa aaccgacggt gctttgagtt cgtttgatat taaggataaa tgggcgcctg    1080
   cttttcaggt tggccttaga tatgaccttg gtaactcatg gatgctaaat tcagatgtgc    1140
   gttatattcc tttcaaaacg gacgtcacag gtactcttgg cccggttcct gtttctacta    1200
   aaattgaggt tgatcctttc attctcagtc ttggtgcgtc atatgttttc taaggtaccc    1260
   tcgagtctgg tggatccaat tgtgagcgga taacaattac gagcttcatg cacagtgatc    1320
   gacgctgttg acaattaatc atcggctcgt ataatgtgtg gatgtggaat tgtgagcgct    1380
   cacaattcca caacggtttc cctctagaaa taattttgtt taacaggagg taaaacatat    1440
   gctggttctg cacaacagcc aaaaactgca aattctgtac aaatctctgg aaaaatctat    1500
   tcctgaaagc attaaagttt atggtgcgat cttcaacatc aaggataaaa accctttcaa    1560
   tatggaggtg ctggtcgacg cgtggcctga ttaccaaatc gtcatcaccc gtccgcagaa 1620
   gcaagaaatg aaagatgacc aggaccacta tactaacacc taccacatct tcactaaagc 1680
   gccggacaaa ctggaagaag ttctgagcta ctccaatgtt atctcctggg aacaaacgct 1740
   gcagattcaa ggttgccagg aaggtctgga tgaggcgatt cgtaaggtcg ctacctctaa 1800
   gtccgttcaa gtcgattaca tgaaaaccat cctgtttatc ccggagctgc cgaagaaaca 1860
   caaaacctcc tctaacgaca agatggagct gttcgaagta gacgatgaca acaaagaggg 1920
   taacttttcc aatatgttcc tggacgcctc ccacgccggt ctggttaatg agcactgggc 1980
   gttcggtaaa aacgaacgct ccctgaagta cattgagcgt tgtctgcagg attttctggg 2040
   ttttggtgtc ctgggtccag agggtcaact ggtctcttgg atcgttatgg aacagtcttg 2100
   tgaactgcgt atgggttata ctgtgccaaa ataccgtcac caaggtaata tgctgcaaat 2160
   cggttatcat ctggagaagt acctgagcca gaaggaaatc ccgttctatt tccatgttgc 2220
   cgataataac gaaaagagcc tgcaagccct gaacaatctg ggcttcaaga tctgcccttg 2280
   cggttggcac cagtggaagt gcacgcctaa aaagtactgt taagcgttaa gtctgagagg 2340
   tgaagaattg aagaaggttt ggcttaaccg ttatcccgcg gacgttccga cggagatcaa 2400
   ccctgaccgt tatcaatctc tggtagatat gtttgagcag tcggtcgcgc gctacgccga 2460
   tcaacctgcg tttgtgaata tgggggaggt aatgaccttc cgcaagctgg aagaacgcag 2520
   tcgcgcgttt gccgcttatt tgcaacaagg gttggggctg aagaaaggcg atcgcgttgc 2580
   gttgatgatg cctaatttat tgcaatatcc ggtggcgctg tttggcattt tgcgtgccgg 2640
   gatgatcgtc gtaaacgtta acccgttgta taccccgcgt gagcttgagc atcagcttaa 2700
   cgatagcggc gcatcggcga ttgttatcgt gtctaacttt gctcacacac tggaaaaagt 2760
   ggttgataaa accgccgttc agcacgtaat tctgacccgt atgggcgatc agctatctac 2820
   ggcaaaaggc acggtagtca atttcgttgt taaatacatc aagcgtttgg tgccgaaata 2880
   ccatctgcca gatgccattt catttcgtag cgcactgcat aacggctacc ggatgcagta 2940
   cgtcaaaccc gaactggtgc cggaagattt agcttttctg caatacaccg gcggcaccac 3000
   tggtgtggcg aaaggcgcga tgctgactca ccgcaatatg ctggcgaacc tggaacaggt 3060
   taacgcgacc tatggtccgc tgttgcatcc gggcaaagag ctggtggtga cggcgctgcc 3120
   gctgtatcac atttttgccc tgaccattaa ctgcctgctg tttatcgaac tgggtgggca 3180
   gaacctgctt atcactaacc cgcgcgatat tccagggttg gtaaaagagt tagcgaaata 3240
   tccgtttacc gctatcacgg gcgttaacac cttgttcaat gcgttgctga acaataaaga 3300
   gttccagcag ctggatttct ccagtctgca tctttccgca ggcggtggga tgccagtgca 3360
   gcaagtggtg gcagagcgtt gggtgaaact gaccggacag tatctgctgg aaggctatgg 3420
   ccttaccgag tgtgcgccgc tggtcagcgt taacccatat gatattgatt atcatagtgg 3480
   tagcatcggt ttgccggtgc cgtcgacgga agccaaactg gtggatgatg atgataatga 3540
   agtaccacca ggtcaaccgg gtgagctttg tgtcaaagga ccgcaggtga tgctgggtta 3600
   ctggcagcgt cccgatgcta ccgatgaaat catcaaaaat ggctggttac acaccggcga 3660
   catcgcggta atggatgaag aaggattcct gcgcattgtc gatcgtaaaa aagacatgat 3720
   tctggtttcc ggttttaacg tctatcccaa cgagattgaa gatgtcgtca tgcagcatcc 3780
   tggcgtacag gaagtcgcgg ctgttggcgt accttccggc tccagtggtg aagcggtgaa 3840
   aatcttcgta gtgaaaaaag atccatcgct taccgaagag tcactggtga ctttttgccg 3900
   ccgtcagctc acgggataca aagtaccgaa gctggtggag tttcgtgatg agttaccgaa 3960
   atctaacgtc ggaaaaattt tgcgacgaga attacgtgac gaagcgcgcg gcaaagtgga 4020
   caataaagcc tgagcgttaa gtcagtcgtc agacgccggt taatccggcg ttttttttga 4080
   cgcccactaa agagaaaaca atctcgagtc tggtaaagaa accgctgctg cgaaatttga 4140
   acgccagcac atggactcgt ctactagcgc agcttaatta acctaggctg ctgccaccgc 4200
   tgagcaataa ctagcataac cccttggggc ctctaaacgg gtcttgaggg gttttttgct 4260
   gaaacctcag gcatttgaga agcacacggt cacactgctt ccggtagtca ataaaccggt 4320
   aaaccagcaa tagacataag cggctattta acgaccctgc cctgaaccga cgaccgggtc 4380
   atcgtggccg gatcttgcgg cccctcggct tgaacgaatt gttagacatt atttgccgac 4440
   taccttggtg atctcgcctt tcacgtagtg gacaaattct tccaactgat ctgcgcgcga 4500
   ggccaagcga tcttcttctt gtccaagata agcctgtcta gcttcaagta tgacgggctg 4560
   atactgggcc ggcaggcgct ccattgccca gtcggcagcg acatccttcg gcgcgatttt 4620
   gccggttact gcgctgtacc aaatgcggga caacgtaagc actacatttc gctcatcgcc 4680
   agcccagtcg ggcggcgagt tccatagcgt taaggtttca tttagcgcct caaatagatc 4740
   ctgttcagga accggatcaa agagttcctc cgccgctgga cctaccaagg caacgctatg 4800
   ttctcttgct tttgtcagca agatagccag atcaatgtcg atcgtggctg gctcgaagat 4860
   acctgcaaga atgtcattgc gctgccattc tccaaattgc agttcgcgct tagctggata 4920
   acgccacgga atgatgtcgt cgtgcacaac aatggtgact tctacagcgc ggagaatctc 4980
   gctctctcca ggggaagccg aagtttccaa aaggtcgttg atcaaagctc gccgcgttgt 5040
   ttcatcaagc cttacggtca ccgtaaccag caaatcaata tcactgtgtg gcttcaggcc 5100
   gccatccact gcggagccgt acaaatgtac ggccagcaac gtcggttcga gatggcgctc 5160
   gatgacgcca actacctctg atagttgagt cgatacttcg gcgatcaccg cttccctcat 5220
   actcttcctt tttcaatatt attgaagcat ttatcagggt tattgtctca tgagcggata 5280
   catatttgaa tgtatttaga aaaataaaca aatagctagc tcactcggtc gctacgctcc 5340
   gggcgtgaga ctgcggcggg cgctgcggac acatacaaag ttacccacag attccgtgga 5400
   taagcagggg actaacatgt gaggcaaaac agcagggccg cgccggtggc gtttttccat 5460
   aggctccgcc ctcctgccag agttcacata aacagacgct tttccggtgc atctgtggga 5520
   gccgtgaggc tcaaccatga atctgacagt acgggcgaaa cccgacagga cttaaagatc 5580
   cccaccgttt ccggcgggtc gctccctctt gcgctctcct gttccgaccc tgccgtttac 5640
   cggatacctg ttccgccttt ctcccttacg ggaagtgtgg cgctttctca tagctcacac 5700
   actggtatct cggctcggtg taggtcgttc gctccaagct gggctgtaag caagaactcc 5760
   ccgttcagcc cgactgctgc gccttatccg gtaactgttc acttgagtcc aacccggaaa 5820
   agcacggtaa aacgccactg gcagcagcca ttggtaactg ggagttcgca gaggatttgt 5880
   ttagctaaac acgcggttgc tcttgaagtg tgcgccaaag tccggctaca ctggaaggac 5940
   agatttggtt gctgtgctct gcgaaagcca gttaccacgg ttaagcagtt ccccaactga 6000
   cttaaccttc gatcaaacca cctccccagg tggttttttc gtttacaggg caaaagatta 6060
   cgcgcagaaa aaaaggatct caagaagatc ctttgatctt ttctactgaa ccgctctaga 6120
   tttcagtgca atttatctct tcaaatgtag cacctgaagt cagccccata cgatataagt 6180
   tgtaattctc atgttagtca tgccccgcgc ccaccggaag gagctgactg ggttgaaggc 6240
   tctcaagggc atcggtcgag atcccggtgc ctaatgagtg agctaactta cattaattgc 6300
   gttgcgctca ctgcccgctt tccagtcggg aaacctgtcg tgccagctgc attaatgaat 6360
   cggccaacgc gcggggagag gcggtttgcg tattgggcgc cagggtggtt tttcttttca 6420
   ccagtgagac gggcaacagc tgattgccct tcaccgcctg gccctgagag agttgcagca 6480
   agcggtccac gctggtttgc cccagcaggc gaaaatcctg tttgatggtg gttaacggcg 6540
   ggatataaca tgagctgtct tcggtatcgt cgtatcccac taccgagatg tccgcaccaa 6600
   cgcgcagccc ggactcggta atggcgcgca ttgcgcccag cgccatctga tcgttggcaa 6660
   ccagcatcgc agtgggaacg atgccctcat tcagcatttg catggtttgt tgaaaaccgg 6720
   acatggcact ccagtcgcct tcccgttccg ctatcggctg aatttgattg cgagtgagat 6780
   atttatgcca gccagccaga cgcagacgcg ccgagacaga acttaatggg cccgctaaca 6840
   gcgcgatttg ctggtgaccc aatgcgacca gatgctccac gcccagtcgc gtaccgtctt 6900
   catgggagaa aataatactg ttgatgggtg tctggtcaga gacatcaaga aataacgccg 6960
   gaacattagt gcaggcagct tccacagcaa tggcatcctg gtcatccagc ggatagttaa 7020
   tgatcagccc actgacgcgt tgcgcgagaa gattgtgcac cgccgcttta caggcttcga 7080
   cgccgcttcg ttctaccatc gacaccacca cgctggcacc cagttgatcg gcgcgagatt 7140
   taatcgccgc gacaatttgc gacggcgcgt gcagggccag actggaggtg gcaacgccaa 7200
   tcagcaacga ctgtttgccc gccagttgtt gtgccacgcg gttgggaatg taattcagct 7260
   ccgccatcgc cgcttccact ttttcccgcg ttttcgcaga aacgtggctg gcctggttca 7320
   ccacgcggga aacggtctga taagagacac cggcatactc tgcgacatcg tataacgtta 7380
   ctggtttcac attcaccacc ctgaattgac tctcttccgg gcgctatcat gccataccgc 7440
   gaaaggtttt gcgccattcg atggtgtc 7468
<210> 14
   <211> 5367
   <212> DNA
   <213> Artificial Sequence
<220>
   <223> Vector pET-28b
<220>
   <221> misc_feature
   <222> (1)..(5367)
   <223> Vector pET-28b
<400> 14
   ggcgaatggg acgcgccctg tagcggcgca ttaagcgcgg cgggtgtggt ggttacgcgc 60
   agcgtgaccg ctacacttgc cagcgcccta gcgcccgctc ctttcgcttt cttcccttcc 120
   tttctcgcca cgttcgccgg ctttccccgt caagctctaa atcgggggct ccctttaggg 180
   ttccgattta gtgctttacg gcacctcgac cccaaaaaac ttgattaggg tgatggttca 240
   cgtagtgggc catcgccctg atagacggtt tttcgccctt tgacgttgga gtccacgttc 300
   tttaatagtg gactcttgtt ccaaactgga acaacactca accctatctc ggtctattct 360
   tttgatttat aagggatttt gccgatttcg gcctattggt taaaaaatga gctgatttaa 420
   caaaaattta acgcgaattt taacaaaata ttaacgttta caatttcagg tggcactttt 480
   cggggaaatg tgcgcggaac ccctatttgt ttatttttct aaatacattc aaatatgtat 540
   ccgctcatga attaattctt agaaaaactc atcgagcatc aaatgaaact gcaatttatt 600
   catatcagga ttatcaatac catatttttg aaaaagccgt ttctgtaatg aaggagaaaa 660
   ctcaccgagg cagttccata ggatggcaag atcctggtat cggtctgcga ttccgactcg 720
   tccaacatca atacaaccta ttaatttccc ctcgtcaaaa ataaggttat caagtgagaa 780
   atcaccatga gtgacgactg aatccggtga gaatggcaaa agtttatgca tttctttcca 840
   gacttgttca acaggccagc cattacgctc gtcatcaaaa tcactcgcat caaccaaacc 900
   gttattcatt cgtgattgcg cctgagcgag acgaaatacg cgatcgctgt taaaaggaca 960
   attacaaaca ggaatcgaat gcaaccggcg caggaacact gccagcgcat caacaatatt 1020
   ttcacctgaa tcaggatatt cttctaatac ctggaatgct gttttcccgg ggatcgcagt 1080
   ggtgagtaac catgcatcat caggagtacg gataaaatgc ttgatggtcg gaagaggcat 1140
   aaattccgtc agccagttta gtctgaccat ctcatctgta acatcattgg caacgctacc 1200
   tttgccatgt ttcagaaaca actctggcgc atcgggcttc ccatacaatc gatagattgt 1260
   cgcacctgat tgcccgacat tatcgcgagc ccatttatac ccatataaat cagcatccat 1320
   gttggaattt aatcgcggcc tagagcaaga cgtttcccgt tgaatatggc tcataacacc 1380
   ccttgtatta ctgtttatgt aagcagacag ttttattgtt catgaccaaa atcccttaac 1440
   gtgagttttc gttccactga gcgtcagacc ccgtagaaaa gatcaaagga tcttcttgag 1500
   atcctttttt tctgcgcgta atctgctgct tgcaaacaaa aaaaccaccg ctaccagcgg 1560
   tggtttgttt gccggatcaa gagctaccaa ctctttttcc gaaggtaact ggcttcagca 1620
   gagcgcagat accaaatact gtccttctag tgtagccgta gttaggccac cacttcaaga 1680
   actctgtagc accgcctaca tacctcgctc tgctaatcct gttaccagtg gctgctgcca 1740
   gtggcgataa gtcgtgtctt accgggttgg actcaagacg atagttaccg gataaggcgc 1800
   agcggtcggg ctgaacgggg ggttcgtgca cacagcccag cttggagcga acgacctaca 1860
   ccgaactgag atacctacag cgtgagctat gagaaagcgc cacgcttccc gaagggagaa 1920
   aggcggacag gtatccggta agcggcaggg tcggaacagg agagcgcacg agggagcttc 1980
   cagggggaaa cgcctggtat ctttatagtc ctgtcgggtt tcgccacctc tgacttgagc 2040
   gtcgattttt gtgatgctcg tcaggggggc ggagcctatg gaaaaacgcc agcaacgcgg 2100
   cctttttacg gttcctggcc ttttgctggc cttttgctca catgttcttt cctgcgttat 2160
   cccctgattc tgtggataac cgtattaccg cctttgagtg agctgatacc gctcgccgca 2220
   gccgaacgac cgagcgcagc gagtcagtga gcgaggaagc ggaagagcgc ctgatgcggt 2280
   attttctcct tacgcatctg tgcggtattt cacaccgcat atatggtgca ctctcagtac 2340
   aatctgctct gatgccgcat agttaagcca gtatacactc cgctatcgct acgtgactgg 2400
   gtcatggctg cgccccgaca cccgccaaca cccgctgacg cgccctgacg ggcttgtctg 2460
   ctcccggcat ccgcttacag acaagctgtg accgtctccg ggagctgcat gtgtcagagg 2520
   ttttcaccgt catcaccgaa acgcgcgagg cagctgcggt aaagctcatc agcgtggtcg 2580
   tgaagcgatt cacagatgtc tgcctgttca tccgcgtcca gctcgttgag tttctccaga 2640
   agcgttaatg tctggcttct gataaagcgg gccatgttaa gggcggtttt ttcctgtttg 2700
   gtcactgatg cctccgtgta agggggattt ctgttcatgg gggtaatgat accgatgaaa 2760
   cgagagagga tgctcacgat acgggttact gatgatgaac atgcccggtt actggaacgt 2820
   tgtgagggta aacaactggc ggtatggatg cggcgggacc agagaaaaat cactcagggt 2880
   caatgccagc gcttcgttaa tacagatgta ggtgttccac agggtagcca gcagcatcct 2940
   gcgatgcaga tccggaacat aatggtgcag ggcgctgact tccgcgtttc cagactttac 3000
   gaaacacgga aaccgaagac cattcatgtt gttgctcagg tcgcagacgt tttgcagcag 3060
   cagtcgcttc acgttcgctc gcgtatcggt gattcattct gctaaccagt aaggcaaccc 3120
   cgccagccta gccgggtcct caacgacagg agcacgatca tgcgcacccg tggggccgcc 3180
   atgccggcga taatggcctg cttctcgccg aaacgtttgg tggcgggacc agtgacgaag 3240
   gcttgagcga gggcgtgcaa gattccgaat accgcaagcg acaggccgat catcgtcgcg 3300
   ctccagcgaa agcggtcctc gccgaaaatg acccagagcg ctgccggcac ctgtcctacg 3360
   agttgcatga taaagaagac agtcataagt gcggcgacga tagtcatgcc ccgcgcccac 3420
   cggaaggagc tgactgggtt gaaggctctc aagggcatcg gtcgagatcc cggtgcctaa 3480
   tgagtgagct aacttacatt aattgcgttg cgctcactgc ccgctttcca gtcgggaaac 3540
   ctgtcgtgcc agctgcatta atgaatcggc caacgcgcgg ggagaggcgg tttgcgtatt 3600
   gggcgccagg gtggtttttc ttttcaccag tgagacgggc aacagctgat tgcccttcac 3660
   cgcctggccc tgagagagtt gcagcaagcg gtccacgctg gtttgcccca gcaggcgaaa 3720
   atcctgtttg atggtggtta acggcgggat ataacatgag ctgtcttcgg tatcgtcgta 3780
   tcccactacc gagatatccg caccaacgcg cagcccggac tcggtaatgg cgcgcattgc 3840
   gcccagcgcc atctgatcgt tggcaaccag catcgcagtg ggaacgatgc cctcattcag 3900
   catttgcatg gtttgttgaa aaccggacat ggcactccag tcgccttccc gttccgctat 3960
   cggctgaatt tgattgcgag tgagatattt atgccagcca gccagacgca gacgcgccga 4020
   gacagaactt aatgggcccg ctaacagcgc gatttgctgg tgacccaatg cgaccagatg 4080
   ctccacgccc agtcgcgtac cgtcttcatg ggagaaaata atactgttga tgggtgtctg 4140
   gtcagagaca tcaagaaata acgccggaac attagtgcag gcagcttcca cagcaatggc 4200
   atcctggtca tccagcggat agttaatgat cagcccactg acgcgttgcg cgagaagatt 4260
   gtgcaccgcc gctttacagg cttcgacgcc gcttcgttct accatcgaca ccaccacgct 4320
   ggcacccagt tgatcggcgc gagatttaat cgccgcgaca atttgcgacg gcgcgtgcag 4380
   ggccagactg gaggtggcaa cgccaatcag caacgactgt ttgcccgcca gttgttgtgc 4440
   cacgcggttg ggaatgtaat tcagctccgc catcgccgct tccacttttt cccgcgtttt 4500
   cgcagaaacg tggctggcct ggttcaccac gcgggaaacg gtctgataag agacaccggc 4560
   atactctgcg acatcgtata acgttactgg tttcacattc accaccctga attgactctc 4620
   ttccgggcgc tatcatgcca taccgcgaaa ggttttgcgc cattcgatgg tgtccgggat 4680
   ctcgacgctc tcccttatgc gactcctgca ttaggaagca gcccagtagt aggttgaggc 4740
   cgttgagcac cgccgccgca aggaatggtg catgcaagga gatggcgccc aacagtcccc 4800
   cggccacggg gcctgccacc atacccacgc cgaaacaagc gctcatgagc ccgaagtggc 4860
   gagcccgatc ttccccatcg gtgatgtcgg cgatataggc gccagcaacc gcacctgtgg 4920
   cgccggtgat gccggccacg atgcgtccgg cgtagaggat cgagatctcg atcccgcgaa      4980
   attaatacga ctcactatag gggaattgtg agcggataac aattcccctc tagaaataat      5040
   tttgtttaac tttaagaagg agatatacca tgggcagcag ccatcatcat catcatcaca      5100
   gcagcggcct ggtgccgcgc ggcagccata tggctagcat gactggtgga cagcaaatgg      5160
   gtcgggatcc gaattcgagc tccgtcgaca agcttgcggc cgcactcgag caccaccacc      5220
   accaccactg agatccggct gctaacaaag cccgaaagga agctgagttg gctgctgcca      5280
   ccgctgagca ataactagca taaccccttg gggcctctaa acgggtcttg aggggttttt      5340
   tgctgaaagg aggaactata tccggat                                          5367
<210> 15
   <211> 6432
   <212> DNA
   <213> Artificial Sequence
<220>
   <223> Vector pET-28b{Ptac}[hGLYAT2(co_Ec)]
<220>
   <221> misc_feature
   <222> (1)..(6432)
   <223> Vector pET-28b{Ptac}[hGLYAT2(co_Ec)]
<400> 15
   ggccgctctg gtggatccaa ttgtgagcgg ataacaatta cgagcttcat gcacagtgat 60
   cgacgctgtt gacaattaat catcggctcg tataatgtgt ggatgtggaa ttgtgagcgc 120
   tcacaattcc acaacggttt ccctctagaa ataattttgt ttaacaggag gtaaaacata 180
   tgctggttct gcacaacagc caaaaactgc aaattctgta caaatctctg gaaaaatcta 240
   ttcctgaaag cattaaagtt tatggtgcga tcttcaacat caaggataaa aaccctttca 300
   atatggaggt gctggtcgac gcgtggcctg attaccaaat cgtcatcacc cgtccgcaga 360
   agcaagaaat gaaagatgac caggaccact atactaacac ctaccacatc ttcactaaag 420
   cgccggacaa actggaagaa gttctgagct actccaatgt tatctcctgg gaacaaacgc 480
   tgcagattca aggttgccag gaaggtctgg atgaggcgat tcgtaaggtc gctacctcta 540
   agtccgttca agtcgattac atgaaaacca tcctgtttat cccggagctg ccgaagaaac 600
   acaaaacctc ctctaacgac aagatggagc tgttcgaagt agacgatgac aacaaagagg 660
   gtaacttttc caatatgttc ctggacgcct cccacgccgg tctggttaat gagcactggg 720
   cgttcggtaa aaacgaacgc tccctgaagt acattgagcg ttgtctgcag gattttctgg 780
   gttttggtgt cctgggtcca gagggtcaac tggtctcttg gatcgttatg gaacagtctt 840
   gtgaactgcg tatgggttat actgtgccaa aataccgtca ccaaggtaat atgctgcaaa 900
   tcggttatca tctggagaag tacctgagcc agaaggaaat cccgttctat ttccatgttg 960
   ccgataataa cgaaaagagc ctgcaagccc tgaacaatct gggcttcaag atctgccctt 1020
   gcggttggca ccagtggaag tgcacgccta aaaagtactg ttaagcgtta agtctgagag 1080
   gtgagagctc gaattcggat cccgacccat ttgctgtcca ccagtcatgc tagccatatg 1140
   gctgccgcgc ggcaccaggc cgctgctgtg atgatgatga tgatggctgc tgcccatggt 1200
   atatctcctt cttaaagtta aacaaaatta tttctagagg ggaattgtta tccgctcaca 1260
   attcccctat agtgagtcgt attaatttcg cgggatcgag atctcgatcc tctacgccgg 1320
   acgcatcgtg gccggcatca ccggcgccac aggtgcggtt gctggcgcct atatcgccga 1380
   catcaccgat ggggaagatc gggctcgcca cttcgggctc atgagcgctt gtttcggcgt 1440
   gggtatggtg gcaggccccg tggccggggg actgttgggc gccatctcct tgcatgcacc 1500
   attccttgcg gcggcggtgc tcaacggcct caacctacta ctgggctgct tcctaatgca 1560
   ggagtcgcat aagggagagc gtcgagatcc cggacaccat cgaatggcgc aaaacctttc 1620
   gcggtatggc atgatagcgc ccggaagaga gtcaattcag ggtggtgaat gtgaaaccag 1680
   taacgttata cgatgtcgca gagtatgccg gtgtctctta tcagaccgtt tcccgcgtgg 1740
   tgaaccaggc cagccacgtt tctgcgaaaa cgcgggaaaa agtggaagcg gcgatggcgg 1800
   agctgaatta cattcccaac cgcgtggcac aacaactggc gggcaaacag tcgttgctga 1860
   ttggcgttgc cacctccagt ctggccctgc acgcgccgtc gcaaattgtc gcggcgatta 1920
   aatctcgcgc cgatcaactg ggtgccagcg tggtggtgtc gatggtagaa cgaagcggcg 1980
   tcgaagcctg taaagcggcg gtgcacaatc ttctcgcgca acgcgtcagt gggctgatca 2040
   ttaactatcc gctggatgac caggatgcca ttgctgtgga agctgcctgc actaatgttc 2100
   cggcgttatt tcttgatgtc tctgaccaga cacccatcaa cagtattatt ttctcccatg 2160
   aagacggtac gcgactgggc gtggagcatc tggtcgcatt gggtcaccag caaatcgcgc 2220
   tgttagcggg cccattaagt tctgtctcgg cgcgtctgcg tctggctggc tggcataaat 2280
   atctcactcg caatcaaatt cagccgatag cggaacggga aggcgactgg agtgccatgt 2340
   ccggttttca acaaaccatg caaatgctga atgagggcat cgttcccact gcgatgctgg 2400
   ttgccaacga tcagatggcg ctgggcgcaa tgcgcgccat taccgagtcc gggctgcgcg 2460
   ttggtgcgga tatctcggta gtgggatacg acgataccga agacagctca tgttatatcc 2520
   cgccgttaac caccatcaaa caggattttc gcctgctggg gcaaaccagc gtggaccgct 2580
   tgctgcaact ctctcagggc caggcggtga agggcaatca gctgttgccc gtctcactgg 2640
   tgaaaagaaa aaccaccctg gcgcccaata cgcaaaccgc ctctccccgc gcgttggccg 2700
   attcattaat gcagctggca cgacaggttt cccgactgga aagcgggcag tgagcgcaac 2760
   gcaattaatg taagttagct cactcattag gcaccgggat ctcgaccgat gcccttgaga 2820
   gccttcaacc cagtcagctc cttccggtgg gcgcggggca tgactatcgt cgccgcactt 2880
   atgactgtct tctttatcat gcaactcgta ggacaggtgc cggcagcgct ctgggtcatt 2940
   ttcggcgagg accgctttcg ctggagcgcg acgatgatcg gcctgtcgct tgcggtattc 3000
   ggaatcttgc acgccctcgc tcaagccttc gtcactggtc ccgccaccaa acgtttcggc 3060
   gagaagcagg ccattatcgc cggcatggcg gccccacggg tgcgcatgat cgtgctcctg 3120
   tcgttgagga cccggctagg ctggcggggt tgccttactg gttagcagaa tgaatcaccg 3180
   atacgcgagc gaacgtgaag cgactgctgc tgcaaaacgt ctgcgacctg agcaacaaca 3240
   tgaatggtct tcggtttccg tgtttcgtaa agtctggaaa cgcggaagtc agcgccctgc 3300
   accattatgt tccggatctg catcgcagga tgctgctggc taccctgtgg aacacctaca 3360
   tctgtattaa cgaagcgctg gcattgaccc tgagtgattt ttctctggtc ccgccgcatc 3420
   cataccgcca gttgtttacc ctcacaacgt tccagtaacc gggcatgttc atcatcagta 3480
   acccgtatcg tgagcatcct ctctcgtttc atcggtatca ttacccccat gaacagaaat 3540
   cccccttaca cggaggcatc agtgaccaaa caggaaaaaa ccgcccttaa catggcccgc 3600
   tttatcagaa gccagacatt aacgcttctg gagaaactca acgagctgga cgcggatgaa 3660
   caggcagaca tctgtgaatc gcttcacgac cacgctgatg agctttaccg cagctgcctc 3720
   gcgcgtttcg gtgatgacgg tgaaaacctc tgacacatgc agctcccgga gacggtcaca 3780
   gcttgtctgt aagcggatgc cgggagcaga caagcccgtc agggcgcgtc agcgggtgtt 3840
   ggcgggtgtc ggggcgcagc catgacccag tcacgtagcg atagcggagt gtatactggc 3900
   ttaactatgc ggcatcagag cagattgtac tgagagtgca ccatatatgc ggtgtgaaat 3960
   accgcacaga tgcgtaagga gaaaataccg catcaggcgc tcttccgctt cctcgctcac 4020
   tgactcgctg cgctcggtcg ttcggctgcg gcgagcggta tcagctcact caaaggcggt 4080
   aatacggtta tccacagaat caggggataa cgcaggaaag aacatgtgag caaaaggcca 4140
   gcaaaaggcc aggaaccgta aaaaggccgc gttgctggcg tttttccata ggctccgccc 4200
   ccctgacgag catcacaaaa atcgacgctc aagtcagagg tggcgaaacc cgacaggact 4260
   ataaagatac caggcgtttc cccctggaag ctccctcgtg cgctctcctg ttccgaccct 4320
   gccgcttacc ggatacctgt ccgcctttct cccttcggga agcgtggcgc tttctcatag 4380
   ctcacgctgt aggtatctca gttcggtgta ggtcgttcgc tccaagctgg gctgtgtgca 4440
   cgaacccccc gttcagcccg accgctgcgc cttatccggt aactatcgtc ttgagtccaa 4500
   cccggtaaga cacgacttat cgccactggc agcagccact ggtaacagga ttagcagagc 4560
   gaggtatgta ggcggtgcta cagagttctt gaagtggtgg cctaactacg gctacactag 4620
   aaggacagta tttggtatct gcgctctgct gaagccagtt accttcggaa aaagagttgg 4680
   tagctcttga tccggcaaac aaaccaccgc tggtagcggt ggtttttttg tttgcaagca 4740
   gcagattacg cgcagaaaaa aaggatctca agaagatcct ttgatctttt ctacggggtc 4800
   tgacgctcag tggaacgaaa actcacgtta agggattttg gtcatgaaca ataaaactgt 4860
   ctgcttacat aaacagtaat acaaggggtg ttatgagcca tattcaacgg gaaacgtctt 4920
   gctctaggcc gcgattaaat tccaacatgg atgctgattt atatgggtat aaatgggctc 4980
   gcgataatgt cgggcaatca ggtgcgacaa tctatcgatt gtatgggaag cccgatgcgc 5040
   cagagttgtt tctgaaacat ggcaaaggta gcgttgccaa tgatgttaca gatgagatgg 5100
   tcagactaaa ctggctgacg gaatttatgc ctcttccgac catcaagcat tttatccgta 5160
   ctcctgatga tgcatggtta ctcaccactg cgatccccgg gaaaacagca ttccaggtat 5220
   tagaagaata tcctgattca ggtgaaaata ttgttgatgc gctggcagtg ttcctgcgcc 5280
   ggttgcattc gattcctgtt tgtaattgtc cttttaacag cgatcgcgta tttcgtctcg 5340
   ctcaggcgca atcacgaatg aataacggtt tggttgatgc gagtgatttt gatgacgagc 5400
   gtaatggctg gcctgttgaa caagtctgga aagaaatgca taaacttttg ccattctcac 5460
   cggattcagt cgtcactcat ggtgatttct cacttgataa ccttattttt gacgagggga 5520
   aattaatagg ttgtattgat gttggacgag tcggaatcgc agaccgatac caggatcttg 5580
   ccatcctatg gaactgcctc ggtgagtttt ctccttcatt acagaaacgg ctttttcaaa 5640
   aatatggtat tgataatcct gatatgaata aattgcagtt tcatttgatg ctcgatgagt 5700
   ttttctaaga attaattcat gagcggatac atatttgaat gtatttagaa aaataaacaa 5760
   ataggggttc cgcgcacatt tccccgaaaa gtgccacctg aaattgtaaa cgttaatatt 5820
   ttgttaaaat tcgcgttaaa tttttgttaa atcagctcat tttttaacca ataggccgaa 5880
   atcggcaaaa tcccttataa atcaaaagaa tagaccgaga tagggttgag tgttgttcca 5940
   gtttggaaca agagtccact attaaagaac gtggactcca acgtcaaagg gcgaaaaacc 6000
   gtctatcagg gcgatggccc actacgtgaa ccatcaccct aatcaagttt tttggggtcg 6060
   aggtgccgta aagcactaaa tcggaaccct aaagggagcc cccgatttag agcttgacgg 6120
   ggaaagccgg cgaacgtggc gagaaaggaa gggaagaaag cgaaaggagc gggcgctagg 6180
   gcgctggcaa gtgtagcggt cacgctgcgc gtaaccacca cacccgccgc gcttaatgcg 6240
   ccgctacagg gcgcgtccca ttcgccatcc ggatatagtt cctcctttca gcaaaaaacc 6300
   cctcaagacc cgtttagagg ccccaagggg ttatgctagt tattgctcag cggtggcagc 6360
   agccaactca gcttcctttc gggctttgtt agcagccgga tctcagtggt ggtggtggtg 6420
   gtgctcgagt gc 6432
<210> 16
   <211> 6418
   <212> DNA
   <213> Artificial Sequence
<220>
   <223> pET-28b{Ptac}[hGLYAT3(co_Ec)]
<220>
   <221> misc_feature
   <222> (1)..(6418)
   <223> pET-28b{Ptac}[hGLYAT3(co_Ec)]
<400> 16
   ggccgccctt cattttggat ccaattgtga gcggataaca attacgagct tcatgcacag      60
   tgatcgacgc tgttgacaat taatcatcgg ctcgtataat gtgtggatgt ggaattgtga     120
   gcgctcacaa ttccacaacg gtttccctct agaaataatt ttgtttaaca ggaggtaaaa     180
   catatgctgg ttctgaattg ctctactaaa ctgctgatcc tggagaaaat gctgaaatcc     240
   tgtttcccgg aatctctgaa agtttatggt gcggtaatga atatcaaccg tggtaatccg     300
   tttcaaaaag aagttgttct ggactcttgg ccagatttca aggcggttat cacccgtcgt     360
   cagcgtgaag cggaaaccga caacctggac cactatacca acgcgtatgc ggttttctat     420
   aaagatgttc gcgcgtaccg tcagctgctg gaagaatgcg atgttttcaa ctgggaccag     480
   gtttttcaga tccaaggtct gcaatccgag ctgtacgacg tttctaaggc cgtggcgaac     540
   tctaaacaac tgaacatcaa gctgacctct ttcaaagcgg ttcatttctc tccggtatcc     600
   agcctgccgg acacctcctt cctgaaaggt ccgtctccgc gtctgaccta cctgtctgtt     660
   gcgaatgcgg atctgctgaa ccgtacttgg tcccgtggtg gcaacgaaca gtgcctgcgt     720
   tacattgcta acctgatctc ttgcttcccg agcgtctgcg ttcgtgatga aaaaggtaac     780
   ccagtatctt ggtctatcac cgaccagttc gcgaccatgt gtcatggcta caccctgcct     840
   gaacaccgtc gtaagggtta ctctcgtctg gttgcgctga ccctggcgcg taagctgcag     900
   tctcgtggtt ttccgtccca gggtaacgtc ctggacgaca acactgcgtc catctccctg     960
   ctgaagtccc tgcatgccga attcctgccg tgccgtttcc accgtctgat tctgacccca    1020
   gcgaccttct ctggtctgcc gcacctgtaa gcgttaagtc tgagaggtga gagctcgaat    1080
   tcggatcccg acccatttgc tgtccaccag tcatgctagc catatggctg ccgcgcggca    1140
   ccaggccgct gctgtgatga tgatgatgat ggctgctgcc catggtatat ctccttctta    1200
   aagttaaaca aaattatttc tagaggggaa ttgttatccg ctcacaattc ccctatagtg    1260
   agtcgtatta atttcgcggg atcgagatct cgatcctcta cgccggacgc atcgtggccg    1320
   gcatcaccgg cgccacaggt gcggttgctg gcgcctatat cgccgacatc accgatgggg    1380
   aagatcgggc tcgccacttc gggctcatga gcgcttgttt cggcgtgggt atggtggcag    1440
   gccccgtggc cgggggactg ttgggcgcca tctccttgca tgcaccattc cttgcggcgg    1500
   cggtgctcaa cggcctcaac ctactactgg gctgcttcct aatgcaggag tcgcataagg    1560
   gagagcgtcg agatcccgga caccatcgaa tggcgcaaaa cctttcgcgg tatggcatga 1620
   tagcgcccgg aagagagtca attcagggtg gtgaatgtga aaccagtaac gttatacgat 1680
   gtcgcagagt atgccggtgt ctcttatcag accgtttccc gcgtggtgaa ccaggccagc 1740
   cacgtttctg cgaaaacgcg ggaaaaagtg gaagcggcga tggcggagct gaattacatt 1800
   cccaaccgcg tggcacaaca actggcgggc aaacagtcgt tgctgattgg cgttgccacc 1860
   tccagtctgg ccctgcacgc gccgtcgcaa attgtcgcgg cgattaaatc tcgcgccgat 1920
   caactgggtg ccagcgtggt ggtgtcgatg gtagaacgaa gcggcgtcga agcctgtaaa 1980
   gcggcggtgc acaatcttct cgcgcaacgc gtcagtgggc tgatcattaa ctatccgctg 2040
   gatgaccagg atgccattgc tgtggaagct gcctgcacta atgttccggc gttatttctt 2100
   gatgtctctg accagacacc catcaacagt attattttct cccatgaaga cggtacgcga 2160
   ctgggcgtgg agcatctggt cgcattgggt caccagcaaa tcgcgctgtt agcgggccca 2220
   ttaagttctg tctcggcgcg tctgcgtctg gctggctggc ataaatatct cactcgcaat 2280
   caaattcagc cgatagcgga acgggaaggc gactggagtg ccatgtccgg ttttcaacaa 2340
   accatgcaaa tgctgaatga gggcatcgtt cccactgcga tgctggttgc caacgatcag 2400
   atggcgctgg gcgcaatgcg cgccattacc gagtccgggc tgcgcgttgg tgcggatatc 2460
   tcggtagtgg gatacgacga taccgaagac agctcatgtt atatcccgcc gttaaccacc 2520
   atcaaacagg attttcgcct gctggggcaa accagcgtgg accgcttgct gcaactctct 2580
   cagggccagg cggtgaaggg caatcagctg ttgcccgtct cactggtgaa aagaaaaacc 2640
   accctggcgc ccaatacgca aaccgcctct ccccgcgcgt tggccgattc attaatgcag 2700
   ctggcacgac aggtttcccg actggaaagc gggcagtgag cgcaacgcaa ttaatgtaag 2760
   ttagctcact cattaggcac cgggatctcg accgatgccc ttgagagcct tcaacccagt 2820
   cagctccttc cggtgggcgc ggggcatgac tatcgtcgcc gcacttatga ctgtcttctt 2880
   tatcatgcaa ctcgtaggac aggtgccggc agcgctctgg gtcattttcg gcgaggaccg 2940
   ctttcgctgg agcgcgacga tgatcggcct gtcgcttgcg gtattcggaa tcttgcacgc 3000
   cctcgctcaa gccttcgtca ctggtcccgc caccaaacgt ttcggcgaga agcaggccat 3060
   tatcgccggc atggcggccc cacgggtgcg catgatcgtg ctcctgtcgt tgaggacccg 3120
   gctaggctgg cggggttgcc ttactggtta gcagaatgaa tcaccgatac gcgagcgaac 3180
   gtgaagcgac tgctgctgca aaacgtctgc gacctgagca acaacatgaa tggtcttcgg 3240
   tttccgtgtt tcgtaaagtc tggaaacgcg gaagtcagcg ccctgcacca ttatgttccg 3300
   gatctgcatc gcaggatgct gctggctacc ctgtggaaca cctacatctg tattaacgaa 3360
   gcgctggcat tgaccctgag tgatttttct ctggtcccgc cgcatccata ccgccagttg 3420
   tttaccctca caacgttcca gtaaccgggc atgttcatca tcagtaaccc gtatcgtgag 3480
   catcctctct cgtttcatcg gtatcattac ccccatgaac agaaatcccc cttacacgga 3540
   ggcatcagtg accaaacagg aaaaaaccgc ccttaacatg gcccgcttta tcagaagcca 3600
   gacattaacg cttctggaga aactcaacga gctggacgcg gatgaacagg cagacatctg 3660
   tgaatcgctt cacgaccacg ctgatgagct ttaccgcagc tgcctcgcgc gtttcggtga 3720
   tgacggtgaa aacctctgac acatgcagct cccggagacg gtcacagctt gtctgtaagc 3780
   ggatgccggg agcagacaag cccgtcaggg cgcgtcagcg ggtgttggcg ggtgtcgggg 3840
   cgcagccatg acccagtcac gtagcgatag cggagtgtat actggcttaa ctatgcggca 3900
   tcagagcaga ttgtactgag agtgcaccat atatgcggtg tgaaataccg cacagatgcg 3960
   taaggagaaa ataccgcatc aggcgctctt ccgcttcctc gctcactgac tcgctgcgct 4020
   cggtcgttcg gctgcggcga gcggtatcag ctcactcaaa ggcggtaata cggttatcca 4080
   cagaatcagg ggataacgca ggaaagaaca tgtgagcaaa aggccagcaa aaggccagga 4140
   accgtaaaaa ggccgcgttg ctggcgtttt tccataggct ccgcccccct gacgagcatc 4200
   acaaaaatcg acgctcaagt cagaggtggc gaaacccgac aggactataa agataccagg 4260
   cgtttccccc tggaagctcc ctcgtgcgct ctcctgttcc gaccctgccg cttaccggat 4320
   acctgtccgc ctttctccct tcgggaagcg tggcgctttc tcatagctca cgctgtaggt 4380
   atctcagttc ggtgtaggtc gttcgctcca agctgggctg tgtgcacgaa ccccccgttc 4440
   agcccgaccg ctgcgcctta tccggtaact atcgtcttga gtccaacccg gtaagacacg 4500
   acttatcgcc actggcagca gccactggta acaggattag cagagcgagg tatgtaggcg 4560
   gtgctacaga gttcttgaag tggtggccta actacggcta cactagaagg acagtatttg 4620
   gtatctgcgc tctgctgaag ccagttacct tcggaaaaag agttggtagc tcttgatccg 4680
   gcaaacaaac caccgctggt agcggtggtt tttttgtttg caagcagcag attacgcgca 4740
   gaaaaaaagg atctcaagaa gatcctttga tcttttctac ggggtctgac gctcagtgga 4800
   acgaaaactc acgttaaggg attttggtca tgaacaataa aactgtctgc ttacataaac 4860
   agtaatacaa ggggtgttat gagccatatt caacgggaaa cgtcttgctc taggccgcga 4920
   ttaaattcca acatggatgc tgatttatat gggtataaat gggctcgcga taatgtcggg 4980
   caatcaggtg cgacaatcta tcgattgtat gggaagcccg atgcgccaga gttgtttctg 5040
   aaacatggca aaggtagcgt tgccaatgat gttacagatg agatggtcag actaaactgg 5100
   ctgacggaat ttatgcctct tccgaccatc aagcatttta tccgtactcc tgatgatgca 5160
   tggttactca ccactgcgat ccccgggaaa acagcattcc aggtattaga agaatatcct 5220
   gattcaggtg aaaatattgt tgatgcgctg gcagtgttcc tgcgccggtt gcattcgatt 5280
   cctgtttgta attgtccttt taacagcgat cgcgtatttc gtctcgctca ggcgcaatca 5340
   cgaatgaata acggtttggt tgatgcgagt gattttgatg acgagcgtaa tggctggcct 5400
   gttgaacaag tctggaaaga aatgcataaa cttttgccat tctcaccgga ttcagtcgtc 5460
   actcatggtg atttctcact tgataacctt atttttgacg aggggaaatt aataggttgt 5520
   attgatgttg gacgagtcgg aatcgcagac cgataccagg atcttgccat cctatggaac 5580
   tgcctcggtg agttttctcc ttcattacag aaacggcttt ttcaaaaata tggtattgat 5640
   aatcctgata tgaataaatt gcagtttcat ttgatgctcg atgagttttt ctaagaatta 5700
   attcatgagc ggatacatat ttgaatgtat ttagaaaaat aaacaaatag gggttccgcg 5760
   cacatttccc cgaaaagtgc cacctgaaat tgtaaacgtt aatattttgt taaaattcgc 5820
   gttaaatttt tgttaaatca gctcattttt taaccaatag gccgaaatcg gcaaaatccc 5880
   ttataaatca aaagaataga ccgagatagg gttgagtgtt gttccagttt ggaacaagag 5940
   tccactatta aagaacgtgg actccaacgt caaagggcga aaaaccgtct atcagggcga 6000
   tggcccacta cgtgaaccat caccctaatc aagttttttg gggtcgaggt gccgtaaagc 6060
   actaaatcgg aaccctaaag ggagcccccg atttagagct tgacggggaa agccggcgaa 6120
   cgtggcgaga aaggaaggga agaaagcgaa aggagcgggc gctagggcgc tggcaagtgt 6180
   agcggtcacg ctgcgcgtaa ccaccacacc cgccgcgctt aatgcgccgc tacagggcgc 6240
   gtcccattcg ccatccggat atagttcctc ctttcagcaa aaaacccctc aagacccgtt 6300
   tagaggcccc aaggggttat gctagttatt gctcagcggt ggcagcagcc aactcagctt 6360
   cctttcgggc tttgttagca gccggatctc agtggtggtg gtggtggtgc tcgagtgc 6418
<210> 17
   <211> 4334
   <212> DNA
   <213> Escherichia coli
<220>
   <221> misc_feature
   <222> (1)..(4334)
   <223> pBMT-3_ccdAB
<400> 17
   ctgatggaca ggctgcgcct gcccacgagc ttgaccacag ggattgccca ccggctaccc      60
   agccttcgac cacataccca ccggctccaa ctgcgcggcc tgcggccttg ccccatcaat     120
   ttttttaatt ttctctgggg aaaagcctcc ggcctgcggc ctgcgcgctt cgcttgccgg     180
   ttggacacca agtggaaggc gggtcaaggc tcgcgcagcg accgcgcagc ggcttggcct     240
   tgacgcgcct ggaacgaccc aagcctatgc gagtgggggc agtcgaaggc gaagcccgcc     300
   cgcctgcccc ccgagcctca cggcggcgag tgcgggggtt ccaagggggc agcgccacct      360
   tgggcaaggc cgaaggccgc gcagtcgatc aacaagcccc ggaggggcca ctttttgccg     420
   gagggggagc cgcgccgaag gcgtggggga accccgcagg ggtgcccttc tttgggcacc 480
   aaagaactag atatagggcg aaatgcgaaa gacttaaaaa tcaacaactt aaaaaagggg 540
   ggtacgcaac agctcattgc ggcacccccc gcaatagctc attgcgtagg ttaaagaaaa 600
   tctgtaattg actgccactt ttacgcaacg cataattgtt gtcgcgctgc cgaaaagttg 660
   cagctgattg cgcatggtgc cgcaaccgtg cggcacccta ccgcatggag ataagcatgg 720
   ccacgcagtc cagagaaatc ggcattcaag ccaagaacaa gcccggtcac tgggtgcaaa 780
   cggaacgcaa agcgcatgag gcgtgggccg ggcttattgc gaggaaaccc acggcggcaa 840
   tgctgctgca tcacctcgtg gcgcagatgg gccaccagaa cgccgtggtg gtcagccaga 900
   agacactttc caagctcatc ggacgttctt tgcggacggt ccaatacgca gtcaaggact 960
   tggtggccga gcgctggatc tccgtcgtga agctcaacgg ccccggcacc gtgtcggcct 1020
   acgtggtcaa tgaccgcgtg gcgtggggcc agccccgcga ccagttgcgc ctgtcggtgt 1080
   tcagtgccgc cgtggtggtt gatcacgacg accaggacga atcgctgttg gggcatggcg 1140
   acctgcgccg catcccgacc ctgtatccgg gcgagcagca actaccgacc ggccccggcg 1200
   aggagccgcc cagccagccc ggcattccgg gcatggaacc agacctgcca gccttgaccg 1260
   aaacggagga atgggaacgg cgcgggcagc agcgcctgcc gatgcccgat gagccgtgtt 1320
   ttctggacga tggcgagccg ttggagccgc cgacacgggt cacgctgccg cgccggtagt 1380
   acgtacccgg aattgccagc tggggcgccc tctggtaagg ttgggaagcc ctgcaaagta 1440
   aactggatgg ctttcttgcc gccaaggatc tgatggcgca ggggatcaag ctctgatcaa 1500
   gagacaggat gaggatcgtt tcgatgaagc agcgtattac agtgacagtt gacagcgaca 1560
   gctatcagtt gctcaaggca tatgatgtca atatctccgg tctggtaagc acaaccatgc 1620
   agaatgaagc ccgtcgtctg cgtgccgaac gctggaaagc ggaaaatcag gaagggatgg 1680
   ctgaggtcgc ccggtttatt gaaatgaacg gctcttttgc tgacgagaac agggactggt 1740
   gaaatgcagt ttaaggttta cacctataaa agagagagcc gttatcgtct gtttgtggat 1800
   gtacagagcg atattattga cacgcccggg cgacggatgg tgatccccct ggccagtgca 1860
   cgtctgctgt cagataaagt ctcccgtgaa ctttacccgg tggtgcatat cggggatgaa 1920
   agctggcgca tgatgaccac cgatatggcc agtgtgccgg tctccgttat cggggaagaa 1980
   gtggctgatc tcagccaccg cgaaaatgac atcaaaaacg ccattaacct gatgttctgg 2040
   ggaatataaa tcctagacga attctctagt agaggttcca actttcacca taatgaaata 2100
   agatcactac cgggcgtatt ttttgagtta tcgagatttt caggagctaa ggaagctaaa 2160
   atggagaaaa aaatcactgg atataccacc gttgatatat cccaatggca tcgtaaagaa 2220
   cattttgagg catttcagtc agttgctcaa tgtacctata accagaccgt tcagctggat 2280
   attacggcct ttttaaagac cgtaaagaaa aataagcaca agttttatcc ggcctttatt 2340
   cacattcttg cccgcctgat gaatgctcat ccggaattcc gtatggcaat gaaagacggt 2400
   gagctggtga tatgggatag tgttcaccct tgttacaccg ttttccatga gcaaactgaa 2460
   acgttttcat cgctctggag tgaataccac gacgatttcc ggcagtttct acacatatat 2520
   tcgcaagatg tggcgtgtta cggtgaaaac ctggcctatt tccctaaagg gtttattgag 2580
   aatatgtttt tcgtctcagc caatccctgg gtgagtttca ccagttttga tttaaacgtg 2640
   gccaatatgg acaacttctt cgcccccgtt ttcaccatgg gcaaatatta tacgcaaggc 2700
   gacaaggtgc tgatgccgct ggcgattcag gttcatcatg ccgtttgtga tggcttccat 2760
   gtcggcagaa tgcttaatga attacaacag tactgcgatg agtggcaggg cggggcgtaa 2820
   agatctggat ccccctcaag tcaaaagcct ccggtcggag gcttttgact ttctgctatg 2880
   gaggtcaggt atgatttaaa tggtcagtat tgagcgatat ctagagaatt cgtcaacgaa 2940
   ttcaagcttg atatcattca ggacgagcct cagactccag cgtaactgga ctgaaaacaa 3000
   actaaagcgc ccttgtggcg ctttagtttt gttccgctca tgataataat ggtttcttag 3060
   acgtcaggtg gcacttttcg gggaaatgtg cgcgcccgcg ttcctgctgg cgctgggcct 3120
   gtttctggcg ctggacttcc cgctgttccg tcagcagctt ttcgcccacg gccttgatga 3180
   tcgcggcggc cttggcctgc atatcccgat tcaacggccc cagggcgtcc agaacgggct 3240
   tcaggcgctc ccgaaggtct cgggccgtct cttgggcttg atcggccttc ttgcgcatct 3300
   cacgcgctcc tgcggcggcc tgtagggcag gctcataccc ctgccgaacc gcttttgtca 3360
   gccggtcggc cacggcttcc ggcgtctcaa cgcgctttga gattcccagc ttttcggcca 3420
   atccctgcgg tgcataggcg cgtggctcga ccgcttgcgg gctgatggtg acgtggccca 3480
   ctggtggccg ctccagggcc tcgtagaacg cctgaatgcg cgtgtgacgt gccttgctgc 3540
   cctcgatgcc ccgttgcagc cctagatcgg ccacagcggc cgcaaacgtg gtctggtcgc 3600
   gggtcatctg cgctttgttg ccgatgaact ccttggccga cagcctgccg tcctgcgtca 3660
   gcggcaccac gaacgcggtc atgtgcgggc tggtttcgtc acggtggatg ctggccgtca 3720
   cgatgcgatc cgccccgtac ttgtccgcca gccacttgtg cgccttctcg aagaacgccg 3780
   cctgctgttc ttggctggcc gacttccacc attccgggct ggccgtcatg acgtactcga 3840
   ccgccaacac agcgtccttg cgccgcttct ctggcagcaa ctcgcgcagt cggcccatcg 3900
   cttcatcggt gctgctggcc gcccagtgct cgttctctgg cgtcctgctg gcgtcagcgt 3960
   tgggcgtctc gcgctcgcgg taggcgtgct tgagactggc cgccacgttg cccattttcg 4020
   ccagcttctt gcatcgcatg atcgcgtatg ccgccatgcc tgcccctccc ttttggtgtc 4080
   caaccggctc gacgggggca gcgcaaggcg gtgcctccgg cgggccactc aatgcttgag 4140
   tatactcact agactttgct tcgcaaagtc gtgaccgcct acggcggctg cggcgcccta 4200
   cgggcttgct ctccgggctt cgccctgcgc ggtcgctgcg ctcccttgcc agcccgtgga 4260
   tatgtggacg atggccgcga gcggccaccg gctggctcgc ttcgctcggc ccgtggacaa 4320
   ccctgctgga caag 4334
<210> 18
   <211> 5024
   <212> DNA
   <213> Escherichia coli
<220>
   <221> misc_feature
   <222> (1)..(5024)
   <223> pBMT-3_ccdAB_PT7-'tesA
<400> 18
   caaaaaaccc ctcaagaccc gtttagaggc cccaaggggt tatgctagtt attgctcagc      60
   ggtggcagca gcctaggtta attaagctgc gctagtagac gagtccatgt gctggcgttc     120
   aaatttcgca gcagcggttt ctttaccaga ctcgaaaatt caggagtcgt ggttgaccag     180
   cggctgcagt tgcttggcca tccagtcggc gatgaacggc tgcgcgtcgc ggttcgggtg     240
   gatgccgtcg tcctgcatcc actgcggctt caggtagact tcctccatga agaacggcag     300
   cagcggcacg tcgaattcct tggccagctt cgggtagatc gcggagaagg cctcgttgta     360
   gcgacggccg tagttcgccg gcaggcggat ctgcatcagc agcggctcgg cgttcgcggc     420
   cttcacgtcc tgcaggatct ggcgcagcgt ctgttcggtc tgctgcggct ggaagccgcg     480
   caggccgtcg ttgccgccca gctcgaccag cacccagcgc ggctggtgct gcttcagcag     540
   cgccggcagg cgggccaggc cctgctggct cgtgtcgccg ctgatcgagg cgttgaccac     600
   cgaggtcttg gactgccact tgtcgttcag cagcgccggc cacgccgccg acgccgacat     660
   gcggtagccc gccgacaggg agtcgcccag gatcagcagg gtgtccgccg ccatggtata     720
   tctccttatt aaagttaaac aaaattattt ctacagggga attgttatcc gctcacaatt     780
   cccctatagt gagtcgtatt aatttcctaa tgcaggagtc gatcattcag gacgagcctc     840
   agactccagc gtaactggac tgaaaacaaa ctaaagcgcc cttgtggcgc tttagttttg     900
   ttccgctcat gataataatg gtttcttaga cgtcaggtgg cacttttcgg ggaaatgtgc     960
   gcgcccgcgt tcctgctggc gctgggcctg tttctggcgc tggacttccc gctgttccgt    1020
   cagcagcttt tcgcccacgg ccttgatgat cgcggcggcc ttggcctgca tatcccgatt    1080
   caacggcccc agggcgtcca gaacgggctt caggcgctcc cgaaggtctc gggccgtctc    1140
   ttgggcttga tcggccttct tgcgcatctc acgcgctcct gcggcggcct gtagggcagg    1200
   ctcatacccc tgccgaaccg cttttgtcag ccggtcggcc acggcttccg gcgtctcaac    1260
   gcgctttgag attcccagct tttcggccaa tccctgcggt gcataggcgc gtggctcgac    1320
   cgcttgcggg ctgatggtga cgtggcccac tggtggccgc tccagggcct cgtagaacgc 1380
   ctgaatgcgc gtgtgacgtg ccttgctgcc ctcgatgccc cgttgcagcc ctagatcggc 1440
   cacagcggcc gcaaacgtgg tctggtcgcg ggtcatctgc gctttgttgc cgatgaactc 1500
   cttggccgac agcctgccgt cctgcgtcag cggcaccacg aacgcggtca tgtgcgggct 1560
   ggtttcgtca cggtggatgc tggccgtcac gatgcgatcc gccccgtact tgtccgccag 1620
   ccacttgtgc gccttctcga agaacgccgc ctgctgttct tggctggccg acttccacca 1680
   ttccgggctg gccgtcatga cgtactcgac cgccaacaca gcgtccttgc gccgcttctc 1740
   tggcagcaac tcgcgcagtc ggcccatcgc ttcatcggtg ctgctggccg cccagtgctc 1800
   gttctctggc gtcctgctgg cgtcagcgtt gggcgtctcg cgctcgcggt aggcgtgctt 1860
   gagactggcc gccacgttgc ccattttcgc cagcttcttg catcgcatga tcgcgtatgc 1920
   cgccatgcct gcccctccct tttggtgtcc aaccggctcg acgggggcag cgcaaggcgg 1980
   tgcctccggc gggccactca atgcttgagt atactcacta gactttgctt cgcaaagtcg 2040
   tgaccgccta cggcggctgc ggcgccctac gggcttgctc tccgggcttc gccctgcgcg 2100
   gtcgctgcgc tcccttgcca gcccgtggat atgtggacga tggccgcgag cggccaccgg 2160
   ctggctcgct tcgctcggcc cgtggacaac cctgctggac aagctgatgg acaggctgcg 2220
   cctgcccacg agcttgacca cagggattgc ccaccggcta cccagccttc gaccacatac 2280
   ccaccggctc caactgcgcg gcctgcggcc ttgccccatc aattttttta attttctctg 2340
   gggaaaagcc tccggcctgc ggcctgcgcg cttcgcttgc cggttggaca ccaagtggaa 2400
   ggcgggtcaa ggctcgcgca gcgaccgcgc agcggcttgg ccttgacgcg cctggaacga 2460
   cccaagccta tgcgagtggg ggcagtcgaa ggcgaagccc gcccgcctgc cccccgagcc 2520
   tcacggcggc gagtgcgggg gttccaaggg ggcagcgcca ccttgggcaa ggccgaaggc 2580
   cgcgcagtcg atcaacaagc cccggagggg ccactttttg ccggaggggg agccgcgccg 2640
   aaggcgtggg ggaaccccgc aggggtgccc ttctttgggc accaaagaac tagatatagg 2700
   gcgaaatgcg aaagacttaa aaatcaacaa cttaaaaaag gggggtacgc aacagctcat 2760
   tgcggcaccc cccgcaatag ctcattgcgt aggttaaaga aaatctgtaa ttgactgcca 2820
   cttttacgca acgcataatt gttgtcgcgc tgccgaaaag ttgcagctga ttgcgcatgg 2880
   tgccgcaacc gtgcggcacc ctaccgcatg gagataagca tggccacgca gtccagagaa 2940
   atcggcattc aagccaagaa caagcccggt cactgggtgc aaacggaacg caaagcgcat 3000
   gaggcgtggg ccgggcttat tgcgaggaaa cccacggcgg caatgctgct gcatcacctc 3060
   gtggcgcaga tgggccacca gaacgccgtg gtggtcagcc agaagacact ttccaagctc 3120
   atcggacgtt ctttgcggac ggtccaatac gcagtcaagg acttggtggc cgagcgctgg 3180
   atctccgtcg tgaagctcaa cggccccggc accgtgtcgg cctacgtggt caatgaccgc 3240
   gtggcgtggg gccagccccg cgaccagttg cgcctgtcgg tgttcagtgc cgccgtggtg 3300
   gttgatcacg acgaccagga cgaatcgctg ttggggcatg gcgacctgcg ccgcatcccg 3360
   accctgtatc cgggcgagca gcaactaccg accggccccg gcgaggagcc gcccagccag 3420
   cccggcattc cgggcatgga accagacctg ccagccttga ccgaaacgga ggaatgggaa 3480
   cggcgcgggc agcagcgcct gccgatgccc gatgagccgt gttttctgga cgatggcgag 3540
   ccgttggagc cgccgacacg ggtcacgctg ccgcgccggt agtacgtacc cggaattgcc 3600
   agctggggcg ccctctggta aggttgggaa gccctgcaaa gtaaactgga tggctttctt 3660
   gccgccaagg atctgatggc gcaggggatc aagctctgat caagagacag gatgaggatc 3720
   gtttcgatga agcagcgtat tacagtgaca gttgacagcg acagctatca gttgctcaag 3780
   gcatatgatg tcaatatctc cggtctggta agcacaacca tgcagaatga agcccgtcgt 3840
   ctgcgtgccg aacgctggaa agcggaaaat caggaaggga tggctgaggt cgcccggttt 3900
   attgaaatga acggctcttt tgctgacgag aacagggact ggtgaaatgc agtttaaggt 3960
   ttacacctat aaaagagaga gccgttatcg tctgtttgtg gatgtacaga gcgatattat 4020
   tgacacgccc gggcgacgga tggtgatccc cctggccagt gcacgtctgc tgtcagataa 4080
   agtctcccgt gaactttacc cggtggtgca tatcggggat gaaagctggc gcatgatgac 4140
   caccgatatg gccagtgtgc cggtctccgt tatcggggaa gaagtggctg atctcagcca 4200
   ccgcgaaaat gacatcaaaa acgccattaa cctgatgttc tggggaatat aaatcctaga 4260
   cgaattctct agtagaggtt ccaactttca ccataatgaa ataagatcac taccgggcgt 4320
   attttttgag ttatcgagat tttcaggagc taaggaagct aaaatggaga aaaaaatcac 4380
   tggatatacc accgttgata tatcccaatg gcatcgtaaa gaacattttg aggcatttca 4440
   gtcagttgct caatgtacct ataaccagac cgttcagctg gatattacgg cctttttaaa 4500
   gaccgtaaag aaaaataagc acaagtttta tccggccttt attcacattc ttgcccgcct 4560
   gatgaatgct catccggaat tccgtatggc aatgaaagac ggtgagctgg tgatatggga 4620
   tagtgttcac ccttgttaca ccgttttcca tgagcaaact gaaacgtttt catcgctctg 4680
   gagtgaatac cacgacgatt tccggcagtt tctacacata tattcgcaag atgtggcgtg 4740
   ttacggtgaa aacctggcct atttccctaa agggtttatt gagaatatgt ttttcgtctc 4800
   agccaatccc tgggtgagtt tcaccagttt tgatttaaac gtggccaata tggacaactt 4860
   cttcgccccc gttttcacca tgggcaaata ttatacgcaa ggcgacaagg tgctgatgcc 4920
   gctggcgatt caggttcatc atgccgtttg tgatggcttc catgtcggca gaatgcttaa 4980
   tgaattacaa cagtactgcg atgagtggca gggcggggcg taaa 5024
<210> 19
   <211> 8268
   <212> DNA
   <213> Escherichia coli
<220>
   <221> misc_feature
   <222> (1)..(8268)
   <223> pBMT-3_ccdAB_PT7-nphT7-hbd-crt-ter
<400> 19
   ccttgccagc ccgtggatat gtggacgatg gccgcgagcg gccaccggct ggctcgcttc 60
   gctcggcccg tggacaaccc tgctggacaa gctgatggac aggctgcgcc tgcccacgag 120
   cttgaccaca gggattgccc accggctacc cagccttcga ccacataccc accggctcca 180
   actgcgcggc ctgcggcctt gccccatcaa tttttttaat tttctctggg gaaaagcctc 240
   cggcctgcgg cctgcgcgct tcgcttgccg gttggacacc aagtggaagg cgggtcaagg 300
   ctcgcgcagc gaccgcgcag cggcttggcc ttgacgcgcc tggaacgacc caagcctatg 360
   cgagtggggg cagtcgaagg cgaagcccgc ccgcctgccc cccgagcctc acggcggcga 420
   gtgcgggggt tccaaggggg cagcgccacc ttgggcaagg ccgaaggccg cgcagtcgat 480
   caacaagccc cggaggggcc actttttgcc ggagggggag ccgcgccgaa ggcgtggggg 540
   aaccccgcag gggtgccctt ctttgggcac caaagaacta gatatagggc gaaatgcgaa 600
   agacttaaaa atcaacaact taaaaaaggg gggtacgcaa cagctcattg cggcaccccc 660
   cgcaatagct cattgcgtag gttaaagaaa atctgtaatt gactgccact tttacgcaac 720
   gcataattgt tgtcgcgctg ccgaaaagtt gcagctgatt gcgcatggtg ccgcaaccgt 780
   gcggcaccct accgcatgga gataagcatg gccacgcagt ccagagaaat cggcattcaa 840
   gccaagaaca agcccggtca ctgggtgcaa acggaacgca aagcgcatga ggcgtgggcc 900
   gggcttattg cgaggaaacc cacggcggca atgctgctgc atcacctcgt ggcgcagatg 960
   ggccaccaga acgccgtggt ggtcagccag aagacacttt ccaagctcat cggacgttct 1020
   ttgcggacgg tccaatacgc agtcaaggac ttggtggccg agcgctggat ctccgtcgtg 1080
   aagctcaacg gccccggcac cgtgtcggcc tacgtggtca atgaccgcgt ggcgtggggc 1140
   cagccccgcg accagttgcg cctgtcggtg ttcagtgccg ccgtggtggt tgatcacgac 1200
   gaccaggacg aatcgctgtt ggggcatggc gacctgcgcc gcatcccgac cctgtatccg 1260
   ggcgagcagc aactaccgac cggccccggc gaggagccgc ccagccagcc cggcattccg 1320
   ggcatggaac cagacctgcc agccttgacc gaaacggagg aatgggaacg gcgcgggcag 1380
   cagcgcctgc cgatgcccga tgagccgtgt tttctggacg atggcgagcc gttggagccg 1440
   ccgacacggg tcacgctgcc gcgccggtag tacgtacccg gaattgccag ctggggcgcc 1500
   ctctggtaag gttgggaagc cctgcaaagt aaactggatg gctttcttgc cgccaaggat 1560
   ctgatggcgc aggggatcaa gctctgatca agagacagga tgaggatcgt ttcgatgaag 1620
   cagcgtatta cagtgacagt tgacagcgac agctatcagt tgctcaaggc atatgatgtc 1680
   aatatctccg gtctggtaag cacaaccatg cagaatgaag cccgtcgtct gcgtgccgaa 1740
   cgctggaaag cggaaaatca ggaagggatg gctgaggtcg cccggtttat tgaaatgaac 1800
   ggctcttttg ctgacgagaa cagggactgg tgaaatgcag tttaaggttt acacctataa 1860
   aagagagagc cgttatcgtc tgtttgtgga tgtacagagc gatattattg acacgcccgg 1920
   gcgacggatg gtgatccccc tggccagtgc acgtctgctg tcagataaag tctcccgtga 1980
   actttacccg gtggtgcata tcggggatga aagctggcgc atgatgacca ccgatatggc 2040
   cagtgtgccg gtctccgtta tcggggaaga agtggctgat ctcagccacc gcgaaaatga 2100
   catcaaaaac gccattaacc tgatgttctg gggaatataa atcctagacg aattctctag 2160
   tagaggttcc aactttcacc ataatgaaat aagatcacta ccgggcgtat tttttgagtt 2220
   atcgagattt tcaggagcta aggaagctaa aatggagaaa aaaatcactg gatataccac 2280
   cgttgatata tcccaatggc atcgtaaaga acattttgag gcatttcagt cagttgctca 2340
   atgtacctat aaccagaccg ttcagctgga tattacggcc tttttaaaga ccgtaaagaa 2400
   aaataagcac aagttttatc cggcctttat tcacattctt gcccgcctga tgaatgctca 2460
   tccggaattc cgtatggcaa tgaaagacgg tgagctggtg atatgggata gtgttcaccc 2520
   ttgttacacc gttttccatg agcaaactga aacgttttca tcgctctgga gtgaatacca 2580
   cgacgatttc cggcagtttc tacacatata ttcgcaagat gtggcgtgtt acggtgaaaa 2640
   cctggcctat ttccctaaag ggtttattga gaatatgttt ttcgtctcag ccaatccctg 2700
   ggtgagtttc accagttttg atttaaacgt ggccaatatg gacaacttct tcgcccccgt 2760
   tttcaccatg ggcaaatatt atacgcaagg cgacaaggtg ctgatgccgc tggcgattca 2820
   ggttcatcat gccgtttgtg atggcttcca tgtcggcaga atgcttaatg aattacaaca 2880
   gtactgcgat gagtggcagg gcggggcgta aaaaatccct tatgcgactc ctgcattagg 2940
   aaattaatac gactcactat aggggaattg tgagcggata acaattcccc tgtagaaata 3000
   attttgttta actttaataa ggagatatac catgacggat gtccgctttc gtatcattgg 3060
   cacgggcgct tacgtcccgg aacgcattgt cagcaacgac gaagtcggcg caccggctgg 3120
   tgtggatgac gattggatta cgcgtaaaac cggtatccgc cagcgtcgct gggcggccga 3180
   cgatcaagca acgagcgatc tggctaccgc agctggtcgt gcagcactga aagcagctgg 3240
   cattacgccg gaacagctga ccgtcatcgc agtggctacc tctacgccgg accgtccgca 3300
   gccgccgacc gcggcctatg ttcaacatca cctgggtgca accggcacgg cagcttttga 3360
   tgttaacgct gtgtgcagcg gtacggtttt cgcgctgagc tctgttgccg gcaccctggt 3420
   ctatcgtggc ggttacgcac tggtcattgg tgctgatctg tactcacgta tcctgaatcc 3480
   ggcggaccgc aaaaccgtgg ttctgttcgg cgatggtgcg ggcgcgatgg tgctgggccc 3540
   gaccagcacc ggcaccggtc cgattgtgcg tcgcgttgca ctgcatacgt ttggcggtct 3600
   gaccgatctg atccgtgttc cggccggcgg ttcccgccag ccgctggaca ccgatggtct 3660
   ggacgcaggc ctgcaatatt ttgcgatgga tggccgcgaa gtccgtcgct tcgtgaccga 3720
   acatctgccg cagctgatta aaggttttct gcacgaagcg ggcgtggatg cggcggatat 3780
   tagccatttc gtgccgcacc aagccaacgg tgtgatgctg gacgaagttt ttggcgaact 3840
   gcatctgccg cgtgcaacca tgcaccgtac ggtggaaacc tacggtaata cgggcgcagc 3900
   tagtattccg atcacgatgg atgcggccgt tcgtgcaggt tccttccgtc cgggcgaact 3960
   ggttctgctg gcgggctttg gcggcggtat ggcggcttcg tttgctctga ttgaatggtg 4020
   acctaatgca ggctgcaggc ggatacgagg aggaataaac catgaaaaag gtatgtgtta 4080
   taggtgcagg tactatgggt tcaggaattg ctcaggcatt tgcagctaaa ggatttgaag 4140
   tagtattaag agatattaaa gatgaatttg ttgatagagg attagatttt atcaataaaa 4200
   atctttctaa attagttaaa aaaggaaaga tagaagaagc tactaaagtt gaaatcttaa 4260
   ctagaatttc cggaacagtt gaccttaata tggcagctga ttgcgattta gttatagaag 4320
   cagctgttga aagaatggat attaaaaagc agatttttgc tgacttagac aatatatgca 4380
   agccagaaac aattcttgca tcaaatacat catcactttc aataacagaa gtggcatcag 4440
   caactaaaag acctgataag gttataggta tgcatttctt taatccagct cctgttatga 4500
   agcttgtaga ggtaataaga ggaatagcta catcacaaga aacttttgat gcagttaaag 4560
   agacatctat agcaatagga aaagatcctg tagaagtagc agaagcacca ggatttgttg 4620
   taaatagaat attaatacca atgattaatg aagcagttgg tatattagca gaaggaatag 4680
   cttcagtaga agacatagat aaagctatga aacttggagc taatcaccca atgggaccat 4740
   tagaattagg tgattttata ggtcttgata tatgtcttgc tataatggat gttttatact 4800
   cagaaactgg agattctaag tatagaccac atacattact taagaagtat gtaagagcag 4860
   gatggcttgg aagaaaatca ggaaaaggtt tctacgatta ttcaaaataa gtttacagga 4920
   tctgcaggga ggaggaaatc atggagttga acaacgttat tctggagaaa gaaggcaagg 4980
   tggcggttgt caccattaac cgtccaaagg ccctgaacgc tctgaactcg gataccctga 5040
   aagagatgga ttacgttatt ggcgagattg agaatgacag cgaagtgctg gctgtgattc 5100
   tgaccggtgc gggtgagaag agctttgtcg cgggtgcgga catcagcgag atgaaagaaa 5160
   tgaacaccat cgaaggccgt aagttcggta ttctgggcaa caaggtgttt cgtcgtctgg 5220
   aactgctgga gaaacctgtc attgctgccg tgaacggttt cgcgctgggc ggtggttgcg 5280
   agatcgctat gagctgcgat attcgtatcg catcgtccaa cgcacgcttt ggtcaaccgg 5340
   aggtcggtct gggtatcact ccgggtttcg gcggtacgca acgtctgagc cgcctggttg 5400
   gcatgggcat ggcgaaacag ttgattttca cggcacagaa cattaaggcg gatgaggcgc 5460
   tgcgtattgg tctggtgaat aaggtcgttg agccaagcga actgatgaat accgcgaaag 5520
   aaattgcgaa caagatcgtt agcaatgccc cggtggccgt taagctgtcg aaacaggcaa 5580
   tcaaccgtgg catgcagtgt gacatcgaca ccgccctggc gtttgagagc gaggcgtttg 5640
   gtgagtgctt ctccaccgag gaccaaaagg atgcgatgac cgcgttcatt gagaaacgca 5700
   agatcgaggg tttcaagaat cgttaataga ggaggatagg aggttttcat atgattgtga 5760
   aaccgatggt ccgtaataat atctgtctga atgctcaccc gcagggctgt aaaaaaggcg 5820
   tggaagatca aattgaatat accaaaaaac gtattacggc agaagtgaaa gccggcgcaa 5880
   aagctccgaa aaacgtgctg gttctgggtt gcagcaatgg ctatggtctg gcttctcgca 5940
   ttaccgcggc ctttggctac ggtgcagcta cgatcggcgt tagtttcgaa aaagcaggtt 6000
   ccgaaaccaa atatggcacg ccgggttggt acaacaatct ggcttttgat gaagcggcca 6060
   aacgtgaagg cctgtatagt gtcaccattg atggtgacgc gttctccgat gaaattaaag 6120
   cacaggtgat cgaagaagcg aagaaaaaag gcattaaatt tgacctgatc gtttacagcc 6180
   tggcatctcc ggtccgtacc gatccggaca cgggtatcat gcataaatct gtgctgaaac 6240
   cgtttggcaa aaccttcacg ggtaaaaccg ttgatccgtt cacgggcgaa ctgaaagaaa 6300
   ttagcgcgga accggccaac gatgaagaag cagctgcgac cgtcaaagtg atgggcggtg 6360
   aagactggga acgttggatc aaacagctga gtaaagaagg cctgctggaa gaaggttgca 6420
   ttaccctggc gtattcctac atcggcccgg aagcaaccca agctctgtat cgcaaaggca 6480
   cgattggtaa agcgaaagaa catctggaag cgaccgccca ccgtctgaac aaagaaaatc 6540
   cgtcaatccg cgccttcgtt tcggtcaata aaggtctggt tacccgtgca tcagctgtga 6600
   ttccggttat cccgctgtac ctggcatcgc tgtttaaagt catgaaagaa aaaggcaacc 6660
   atgaaggttg tattgaacag atcacccgcc tgtatgccga acgtctgtac cgcaaagatg 6720
   gtacgattcc ggtggacgaa gaaaatcgta ttcgcatcga tgactgggaa ctggaagaag 6780
   atgtccaaaa agccgtgagc gccctgatgg aaaaagttac cggcgaaaac gcggaatctc 6840
   tgacggatct ggccggttat cgtcacgact ttctggcgag taatggtttt gatgttgaag 6900
   gcattaacta cgaagctgaa gtggaacgct ttgatcgcat ttgatctaga gaattcgtca 6960
   acgaattcaa gcttgatatc attcaggacg agcctcagac tccagcgtaa ctggactgaa 7020
   aacaaactaa agcgcccttg tggcgcttta gttttgttcc gctcatgata ataatggttt 7080
   cttagacgtc aggtggcact tttcggggaa atgtgcgcgc ccgcgttcct gctggcgctg 7140
   ggcctgtttc tggcgctgga cttcccgctg ttccgtcagc agcttttcgc ccacggcctt 7200
   gatgatcgcg gcggccttgg cctgcatatc ccgattcaac ggccccaggg cgtccagaac 7260
   gggcttcagg cgctcccgaa ggtctcgggc cgtctcttgg gcttgatcgg ccttcttgcg 7320
   catctcacgc gctcctgcgg cggcctgtag ggcaggctca tacccctgcc gaaccgcttt 7380
   tgtcagccgg tcggccacgg cttccggcgt ctcaacgcgc tttgagattc ccagcttttc 7440
   ggccaatccc tgcggtgcat aggcgcgtgg ctcgaccgct tgcgggctga tggtgacgtg 7500
   gcccactggt ggccgctcca gggcctcgta gaacgcctga atgcgcgtgt gacgtgcctt 7560
   gctgccctcg atgccccgtt gcagccctag atcggccaca gcggccgcaa acgtggtctg 7620
   gtcgcgggtc atctgcgctt tgttgccgat gaactccttg gccgacagcc tgccgtcctg 7680
   cgtcagcggc accacgaacg cggtcatgtg cgggctggtt tcgtcacggt ggatgctggc 7740
   cgtcacgatg cgatccgccc cgtacttgtc cgccagccac ttgtgcgcct tctcgaagaa 7800
   cgccgcctgc tgttcttggc tggccgactt ccaccattcc gggctggccg tcatgacgta 7860
   ctcgaccgcc aacacagcgt ccttgcgccg cttctctggc agcaactcgc gcagtcggcc 7920
   catcgcttca tcggtgctgc tggccgccca gtgctcgttc tctggcgtcc tgctggcgtc 7980
   agcgttgggc gtctcgcgct cgcggtaggc gtgcttgaga ctggccgcca cgttgcccat 8040
   tttcgccagc ttcttgcatc gcatgatcgc gtatgccgcc atgcctgccc ctcccttttg 8100
   gtgtccaacc ggctcgacgg gggcagcgca aggcggtgcc tccggcgggc cactcaatgc 8160
   ttgagtatac tcactagact ttgcttcgca aagtcgtgac cgcctacggc ggctgcggcg 8220
   ccctacgggc ttgctctccg ggcttcgccc tgcgcggtcg ctgcgctc 8268
<210> 20
   <211> 8959
   <212> DNA
   <213> Escherichia coli
<220>
   <221> misc_feature
   <222> (1)..(8959)
   <223> pBMT-3_ccdAB_PT7-?tesA_PT7-nphT7-hbd-crt-ter
<400> 20
   ccttgccagc ccgtggatat gtggacgatg gccgcgagcg gccaccggct ggctcgcttc        60
   gctcggcccg tggacaaccc tgctggacaa gctgatggac aggctgcgcc tgcccacgag       120
   cttgaccaca gggattgccc accggctacc cagccttcga ccacataccc accggctcca       180
   actgcgcggc ctgcggcctt gccccatcaa tttttttaat tttctctggg gaaaagcctc       240
   cggcctgcgg cctgcgcgct tcgcttgccg gttggacacc aagtggaagg cgggtcaagg       300
   ctcgcgcagc gaccgcgcag cggcttggcc ttgacgcgcc tggaacgacc caagcctatg       360
   cgagtggggg cagtcgaagg cgaagcccgc ccgcctgccc cccgagcctc acggcggcga       420
   gtgcgggggt tccaaggggg cagcgccacc ttgggcaagg ccgaaggccg cgcagtcgat 480
   caacaagccc cggaggggcc actttttgcc ggagggggag ccgcgccgaa ggcgtggggg 540
   aaccccgcag gggtgccctt ctttgggcac caaagaacta gatatagggc gaaatgcgaa 600
   agacttaaaa atcaacaact taaaaaaggg gggtacgcaa cagctcattg cggcaccccc 660
   cgcaatagct cattgcgtag gttaaagaaa atctgtaatt gactgccact tttacgcaac 720
   gcataattgt tgtcgcgctg ccgaaaagtt gcagctgatt gcgcatggtg ccgcaaccgt 780
   gcggcaccct accgcatgga gataagcatg gccacgcagt ccagagaaat cggcattcaa 840
   gccaagaaca agcccggtca ctgggtgcaa acggaacgca aagcgcatga ggcgtgggcc 900
   gggcttattg cgaggaaacc cacggcggca atgctgctgc atcacctcgt ggcgcagatg 960
   ggccaccaga acgccgtggt ggtcagccag aagacacttt ccaagctcat cggacgttct 1020
   ttgcggacgg tccaatacgc agtcaaggac ttggtggccg agcgctggat ctccgtcgtg 1080
   aagctcaacg gccccggcac cgtgtcggcc tacgtggtca atgaccgcgt ggcgtggggc 1140
   cagccccgcg accagttgcg cctgtcggtg ttcagtgccg ccgtggtggt tgatcacgac 1200
   gaccaggacg aatcgctgtt ggggcatggc gacctgcgcc gcatcccgac cctgtatccg 1260
   ggcgagcagc aactaccgac cggccccggc gaggagccgc ccagccagcc cggcattccg 1320
   ggcatggaac cagacctgcc agccttgacc gaaacggagg aatgggaacg gcgcgggcag 1380
   cagcgcctgc cgatgcccga tgagccgtgt tttctggacg atggcgagcc gttggagccg 1440
   ccgacacggg tcacgctgcc gcgccggtag tacgtacccg gaattgccag ctggggcgcc 1500
   ctctggtaag gttgggaagc cctgcaaagt aaactggatg gctttcttgc cgccaaggat 1560
   ctgatggcgc aggggatcaa gctctgatca agagacagga tgaggatcgt ttcgatgaag 1620
   cagcgtatta cagtgacagt tgacagcgac agctatcagt tgctcaaggc atatgatgtc 1680
   aatatctccg gtctggtaag cacaaccatg cagaatgaag cccgtcgtct gcgtgccgaa 1740
   cgctggaaag cggaaaatca ggaagggatg gctgaggtcg cccggtttat tgaaatgaac 1800
   ggctcttttg ctgacgagaa cagggactgg tgaaatgcag tttaaggttt acacctataa 1860
   aagagagagc cgttatcgtc tgtttgtgga tgtacagagc gatattattg acacgcccgg 1920
   gcgacggatg gtgatccccc tggccagtgc acgtctgctg tcagataaag tctcccgtga 1980
   actttacccg gtggtgcata tcggggatga aagctggcgc atgatgacca ccgatatggc 2040
   cagtgtgccg gtctccgtta tcggggaaga agtggctgat ctcagccacc gcgaaaatga 2100
   catcaaaaac gccattaacc tgatgttctg gggaatataa atcctagacg aattctctag 2160
   tagaggttcc aactttcacc ataatgaaat aagatcacta ccgggcgtat tttttgagtt 2220
   atcgagattt tcaggagcta aggaagctaa aatggagaaa aaaatcactg gatataccac 2280
   cgttgatata tcccaatggc atcgtaaaga acattttgag gcatttcagt cagttgctca 2340
   atgtacctat aaccagaccg ttcagctgga tattacggcc tttttaaaga ccgtaaagaa 2400
   aaataagcac aagttttatc cggcctttat tcacattctt gcccgcctga tgaatgctca 2460
   tccggaattc cgtatggcaa tgaaagacgg tgagctggtg atatgggata gtgttcaccc 2520
   ttgttacacc gttttccatg agcaaactga aacgttttca tcgctctgga gtgaatacca 2580
   cgacgatttc cggcagtttc tacacatata ttcgcaagat gtggcgtgtt acggtgaaaa 2640
   cctggcctat ttccctaaag ggtttattga gaatatgttt ttcgtctcag ccaatccctg 2700
   ggtgagtttc accagttttg atttaaacgt ggccaatatg gacaacttct tcgcccccgt 2760
   tttcaccatg ggcaaatatt atacgcaagg cgacaaggtg ctgatgccgc tggcgattca 2820
   ggttcatcat gccgtttgtg atggcttcca tgtcggcaga atgcttaatg aattacaaca 2880
   gtactgcgat gagtggcagg gcggggcgta aagatcttct cgacgctctc ccttatgcga 2940
   ctcctgcatt aggaaattaa tacgactcac tataggggaa ttgtgagcgg ataacaattc 3000
   ccctgtagaa ataattttgt ttaactttaa taaggagata taccatggcg gcggacaccc 3060
   tgctgatcct gggcgactcc ctgtcggcgg gctaccgcat gtcggcgtcg gcggcgtggc 3120
   cggcgctgct gaacgacaag tggcagtcca agacctcggt ggtcaacgcc tcgatcagcg 3180
   gcgacacgag ccagcagggc ctggcccgcc tgccggcgct gctgaagcag caccagccgc 3240
   gctgggtgct ggtcgagctg ggcggcaacg acggcctgcg cggcttccag ccgcagcaga 3300
   ccgaacagac gctgcgccag atcctgcagg acgtgaaggc cgcgaacgcc gagccgctgc 3360
   tgatgcagat ccgcctgccg gcgaactacg gccgtcgcta caacgaggcc ttctccgcga 3420
   tctacccgaa gctggccaag gaattcgacg tgccgctgct gccgttcttc atggaggaag 3480
   tctacctgaa gccgcagtgg atgcaggacg acggcatcca cccgaaccgc gacgcgcagc 3540
   cgttcatcgc cgactggatg gccaagcaac tgcagccgct ggtcaaccac gactcctgaa 3600
   tttaaatccc ttatgcgact cctgcattag gaaattaata cgactcacta taggggaatt 3660
   gtgagcggat aacaattccc ctgtagaaat aattttgttt aactttaata aggagatata 3720
   ccatgacgga tgtccgcttt cgtatcattg gcacgggcgc ttacgtcccg gaacgcattg 3780
   tcagcaacga cgaagtcggc gcaccggctg gtgtggatga cgattggatt acgcgtaaaa 3840
   ccggtatccg ccagcgtcgc tgggcggccg acgatcaagc aacgagcgat ctggctaccg 3900
   cagctggtcg tgcagcactg aaagcagctg gcattacgcc ggaacagctg accgtcatcg 3960
   cagtggctac ctctacgccg gaccgtccgc agccgccgac cgcggcctat gttcaacatc 4020
   acctgggtgc aaccggcacg gcagcttttg atgttaacgc tgtgtgcagc ggtacggttt 4080
   tcgcgctgag ctctgttgcc ggcaccctgg tctatcgtgg cggttacgca ctggtcattg 4140
   gtgctgatct gtactcacgt atcctgaatc cggcggaccg caaaaccgtg gttctgttcg 4200
   gcgatggtgc gggcgcgatg gtgctgggcc cgaccagcac cggcaccggt ccgattgtgc 4260
   gtcgcgttgc actgcatacg tttggcggtc tgaccgatct gatccgtgtt ccggccggcg 4320
   gttcccgcca gccgctggac accgatggtc tggacgcagg cctgcaatat tttgcgatgg 4380
   atggccgcga agtccgtcgc ttcgtgaccg aacatctgcc gcagctgatt aaaggttttc 4440
   tgcacgaagc gggcgtggat gcggcggata ttagccattt cgtgccgcac caagccaacg 4500
   gtgtgatgct ggacgaagtt tttggcgaac tgcatctgcc gcgtgcaacc atgcaccgta 4560
   cggtggaaac ctacggtaat acgggcgcag ctagtattcc gatcacgatg gatgcggccg 4620
   ttcgtgcagg ttccttccgt ccgggcgaac tggttctgct ggcgggcttt ggcggcggta 4680
   tggcggcttc gtttgctctg attgaatggt gacctaatgc aggctgcagg cggatacgag 4740
   gaggaataaa ccatgaaaaa ggtatgtgtt ataggtgcag gtactatggg ttcaggaatt 4800
   gctcaggcat ttgcagctaa aggatttgaa gtagtattaa gagatattaa agatgaattt 4860
   gttgatagag gattagattt tatcaataaa aatctttcta aattagttaa aaaaggaaag 4920
   atagaagaag ctactaaagt tgaaatctta actagaattt ccggaacagt tgaccttaat 4980
   atggcagctg attgcgattt agttatagaa gcagctgttg aaagaatgga tattaaaaag 5040
   cagatttttg ctgacttaga caatatatgc aagccagaaa caattcttgc atcaaataca 5100
   tcatcacttt caataacaga agtggcatca gcaactaaaa gacctgataa ggttataggt 5160
   atgcatttct ttaatccagc tcctgttatg aagcttgtag aggtaataag aggaatagct 5220
   acatcacaag aaacttttga tgcagttaaa gagacatcta tagcaatagg aaaagatcct 5280
   gtagaagtag cagaagcacc aggatttgtt gtaaatagaa tattaatacc aatgattaat 5340
   gaagcagttg gtatattagc agaaggaata gcttcagtag aagacataga taaagctatg 5400
   aaacttggag ctaatcaccc aatgggacca ttagaattag gtgattttat aggtcttgat 5460
   atatgtcttg ctataatgga tgttttatac tcagaaactg gagattctaa gtatagacca 5520
   catacattac ttaagaagta tgtaagagca ggatggcttg gaagaaaatc aggaaaaggt 5580
   ttctacgatt attcaaaata agtttacagg atctgcaggg aggaggaaat catggagttg 5640
   aacaacgtta ttctggagaa agaaggcaag gtggcggttg tcaccattaa ccgtccaaag 5700
   gccctgaacg ctctgaactc ggataccctg aaagagatgg attacgttat tggcgagatt 5760
   gagaatgaca gcgaagtgct ggctgtgatt ctgaccggtg cgggtgagaa gagctttgtc 5820
   gcgggtgcgg acatcagcga gatgaaagaa atgaacacca tcgaaggccg taagttcggt 5880
   attctgggca acaaggtgtt tcgtcgtctg gaactgctgg agaaacctgt cattgctgcc 5940
   gtgaacggtt tcgcgctggg cggtggttgc gagatcgcta tgagctgcga tattcgtatc 6000
   gcatcgtcca acgcacgctt tggtcaaccg gaggtcggtc tgggtatcac tccgggtttc 6060
   ggcggtacgc aacgtctgag ccgcctggtt ggcatgggca tggcgaaaca gttgattttc 6120
   acggcacaga acattaaggc ggatgaggcg ctgcgtattg gtctggtgaa taaggtcgtt 6180
   gagccaagcg aactgatgaa taccgcgaaa gaaattgcga acaagatcgt tagcaatgcc 6240
   ccggtggccg ttaagctgtc gaaacaggca atcaaccgtg gcatgcagtg tgacatcgac 6300
   accgccctgg cgtttgagag cgaggcgttt ggtgagtgct tctccaccga ggaccaaaag 6360
   gatgcgatga ccgcgttcat tgagaaacgc aagatcgagg gtttcaagaa tcgttaatag 6420
   aggaggatag gaggttttca tatgattgtg aaaccgatgg tccgtaataa tatctgtctg 6480
   aatgctcacc cgcagggctg taaaaaaggc gtggaagatc aaattgaata taccaaaaaa 6540
   cgtattacgg cagaagtgaa agccggcgca aaagctccga aaaacgtgct ggttctgggt 6600
   tgcagcaatg gctatggtct ggcttctcgc attaccgcgg cctttggcta cggtgcagct 6660
   acgatcggcg ttagtttcga aaaagcaggt tccgaaacca aatatggcac gccgggttgg 6720
   tacaacaatc tggcttttga tgaagcggcc aaacgtgaag gcctgtatag tgtcaccatt 6780
   gatggtgacg cgttctccga tgaaattaaa gcacaggtga tcgaagaagc gaagaaaaaa 6840
   ggcattaaat ttgacctgat cgtttacagc ctggcatctc cggtccgtac cgatccggac 6900
   acgggtatca tgcataaatc tgtgctgaaa ccgtttggca aaaccttcac gggtaaaacc 6960
   gttgatccgt tcacgggcga actgaaagaa attagcgcgg aaccggccaa cgatgaagaa 7020
   gcagctgcga ccgtcaaagt gatgggcggt gaagactggg aacgttggat caaacagctg 7080
   agtaaagaag gcctgctgga agaaggttgc attaccctgg cgtattccta catcggcccg 7140
   gaagcaaccc aagctctgta tcgcaaaggc acgattggta aagcgaaaga acatctggaa 7200
   gcgaccgccc accgtctgaa caaagaaaat ccgtcaatcc gcgccttcgt ttcggtcaat 7260
   aaaggtctgg ttacccgtgc atcagctgtg attccggtta tcccgctgta cctggcatcg 7320
   ctgtttaaag tcatgaaaga aaaaggcaac catgaaggtt gtattgaaca gatcacccgc 7380
   ctgtatgccg aacgtctgta ccgcaaagat ggtacgattc cggtggacga agaaaatcgt 7440
   attcgcatcg atgactggga actggaagaa gatgtccaaa aagccgtgag cgccctgatg 7500
   gaaaaagtta ccggcgaaaa cgcggaatct ctgacggatc tggccggtta tcgtcacgac 7560
   tttctggcga gtaatggttt tgatgttgaa ggcattaact acgaagctga agtggaacgc 7620
   tttgatcgca tttgatctag agaattcgtc aacgaattca agcttgatat cattcaggac 7680
   gagcctcaga ctccagcgta actggactga aaacaaacta aagcgccctt gtggcgcttt 7740
   agttttgttc cgctcatgat aataatggtt tcttagacgt caggtggcac ttttcgggga 7800
   aatgtgcgcg cccgcgttcc tgctggcgct gggcctgttt ctggcgctgg acttcccgct 7860
   gttccgtcag cagcttttcg cccacggcct tgatgatcgc ggcggccttg gcctgcatat 7920
   cccgattcaa cggccccagg gcgtccagaa cgggcttcag gcgctcccga aggtctcggg 7980
   ccgtctcttg ggcttgatcg gccttcttgc gcatctcacg cgctcctgcg gcggcctgta 8040
   gggcaggctc atacccctgc cgaaccgctt ttgtcagccg gtcggccacg gcttccggcg 8100
   tctcaacgcg ctttgagatt cccagctttt cggccaatcc ctgcggtgca taggcgcgtg 8160
   gctcgaccgc ttgcgggctg atggtgacgt ggcccactgg tggccgctcc agggcctcgt 8220
   agaacgcctg aatgcgcgtg tgacgtgcct tgctgccctc gatgccccgt tgcagcccta 8280
   gatcggccac agcggccgca aacgtggtct ggtcgcgggt catctgcgct ttgttgccga 8340
   tgaactcctt ggccgacagc ctgccgtcct gcgtcagcgg caccacgaac gcggtcatgt 8400
   gcgggctggt ttcgtcacgg tggatgctgg ccgtcacgat gcgatccgcc ccgtacttgt 8460
   ccgccagcca cttgtgcgcc ttctcgaaga acgccgcctg ctgttcttgg ctggccgact 8520
   tccaccattc cgggctggcc gtcatgacgt actcgaccgc caacacagcg tccttgcgcc 8580
   gcttctctgg cagcaactcg cgcagtcggc ccatcgcttc atcggtgctg ctggccgccc 8640
   agtgctcgtt ctctggcgtc ctgctggcgt cagcgttggg cgtctcgcgc tcgcggtagg 8700
   cgtgcttgag actggccgcc acgttgccca ttttcgccag cttcttgcat cgcatgatcg 8760
   cgtatgccgc catgcctgcc cctccctttt ggtgtccaac cggctcgacg ggggcagcgc 8820
   aaggcggtgc ctccggcggg ccactcaatg cttgagtata ctcactagac tttgcttcgc 8880
   aaagtcgtga ccgcctacgg cggctgcggc gccctacggg cttgctctcc gggcttcgcc 8940
   ctgcgcggtc gctgcgctc 8959
<210> 21
   <211> 5367
   <212> DNA
   <213> Escherichia coli
<220>
   <221> misc_feature
   <222> (1)..(5367)
   <223> pET-28b(empty vector)
<400> 21
   ggcgaatggg acgcgccctg tagcggcgca ttaagcgcgg cgggtgtggt ggttacgcgc      60
   agcgtgaccg ctacacttgc cagcgcccta gcgcccgctc ctttcgcttt cttcccttcc     120
   tttctcgcca cgttcgccgg ctttccccgt caagctctaa atcgggggct ccctttaggg     180
   ttccgattta gtgctttacg gcacctcgac cccaaaaaac ttgattaggg tgatggttca     240
   cgtagtgggc catcgccctg atagacggtt tttcgccctt tgacgttgga gtccacgttc     300
   tttaatagtg gactcttgtt ccaaactgga acaacactca accctatctc ggtctattct     360
   tttgatttat aagggatttt gccgatttcg gcctattggt taaaaaatga gctgatttaa     420
   caaaaattta acgcgaattt taacaaaata ttaacgttta caatttcagg tggcactttt     480
   cggggaaatg tgcgcggaac ccctatttgt ttatttttct aaatacattc aaatatgtat 540
   ccgctcatga attaattctt agaaaaactc atcgagcatc aaatgaaact gcaatttatt 600
   catatcagga ttatcaatac catatttttg aaaaagccgt ttctgtaatg aaggagaaaa 660
   ctcaccgagg cagttccata ggatggcaag atcctggtat cggtctgcga ttccgactcg 720
   tccaacatca atacaaccta ttaatttccc ctcgtcaaaa ataaggttat caagtgagaa 780
   atcaccatga gtgacgactg aatccggtga gaatggcaaa agtttatgca tttctttcca 840
   gacttgttca acaggccagc cattacgctc gtcatcaaaa tcactcgcat caaccaaacc 900
   gttattcatt cgtgattgcg cctgagcgag acgaaatacg cgatcgctgt taaaaggaca 960
   attacaaaca ggaatcgaat gcaaccggcg caggaacact gccagcgcat caacaatatt 1020
   ttcacctgaa tcaggatatt cttctaatac ctggaatgct gttttcccgg ggatcgcagt 1080
   ggtgagtaac catgcatcat caggagtacg gataaaatgc ttgatggtcg gaagaggcat 1140
   aaattccgtc agccagttta gtctgaccat ctcatctgta acatcattgg caacgctacc 1200
   tttgccatgt ttcagaaaca actctggcgc atcgggcttc ccatacaatc gatagattgt 1260
   cgcacctgat tgcccgacat tatcgcgagc ccatttatac ccatataaat cagcatccat 1320
   gttggaattt aatcgcggcc tagagcaaga cgtttcccgt tgaatatggc tcataacacc 1380
   ccttgtatta ctgtttatgt aagcagacag ttttattgtt catgaccaaa atcccttaac 1440
   gtgagttttc gttccactga gcgtcagacc ccgtagaaaa gatcaaagga tcttcttgag 1500
   atcctttttt tctgcgcgta atctgctgct tgcaaacaaa aaaaccaccg ctaccagcgg 1560
   tggtttgttt gccggatcaa gagctaccaa ctctttttcc gaaggtaact ggcttcagca 1620
   gagcgcagat accaaatact gtccttctag tgtagccgta gttaggccac cacttcaaga 1680
   actctgtagc accgcctaca tacctcgctc tgctaatcct gttaccagtg gctgctgcca 1740
   gtggcgataa gtcgtgtctt accgggttgg actcaagacg atagttaccg gataaggcgc 1800
   agcggtcggg ctgaacgggg ggttcgtgca cacagcccag cttggagcga acgacctaca 1860
   ccgaactgag atacctacag cgtgagctat gagaaagcgc cacgcttccc gaagggagaa 1920
   aggcggacag gtatccggta agcggcaggg tcggaacagg agagcgcacg agggagcttc 1980
   cagggggaaa cgcctggtat ctttatagtc ctgtcgggtt tcgccacctc tgacttgagc 2040
   gtcgattttt gtgatgctcg tcaggggggc ggagcctatg gaaaaacgcc agcaacgcgg 2100
   cctttttacg gttcctggcc ttttgctggc cttttgctca catgttcttt cctgcgttat 2160
   cccctgattc tgtggataac cgtattaccg cctttgagtg agctgatacc gctcgccgca 2220
   gccgaacgac cgagcgcagc gagtcagtga gcgaggaagc ggaagagcgc ctgatgcggt 2280
   attttctcct tacgcatctg tgcggtattt cacaccgcat atatggtgca ctctcagtac 2340
   aatctgctct gatgccgcat agttaagcca gtatacactc cgctatcgct acgtgactgg 2400
   gtcatggctg cgccccgaca cccgccaaca cccgctgacg cgccctgacg ggcttgtctg 2460
   ctcccggcat ccgcttacag acaagctgtg accgtctccg ggagctgcat gtgtcagagg 2520
   ttttcaccgt catcaccgaa acgcgcgagg cagctgcggt aaagctcatc agcgtggtcg 2580
   tgaagcgatt cacagatgtc tgcctgttca tccgcgtcca gctcgttgag tttctccaga 2640
   agcgttaatg tctggcttct gataaagcgg gccatgttaa gggcggtttt ttcctgtttg 2700
   gtcactgatg cctccgtgta agggggattt ctgttcatgg gggtaatgat accgatgaaa 2760
   cgagagagga tgctcacgat acgggttact gatgatgaac atgcccggtt actggaacgt 2820
   tgtgagggta aacaactggc ggtatggatg cggcgggacc agagaaaaat cactcagggt 2880
   caatgccagc gcttcgttaa tacagatgta ggtgttccac agggtagcca gcagcatcct 2940
   gcgatgcaga tccggaacat aatggtgcag ggcgctgact tccgcgtttc cagactttac 3000
   gaaacacgga aaccgaagac cattcatgtt gttgctcagg tcgcagacgt tttgcagcag 3060
   cagtcgcttc acgttcgctc gcgtatcggt gattcattct gctaaccagt aaggcaaccc 3120
   cgccagccta gccgggtcct caacgacagg agcacgatca tgcgcacccg tggggccgcc 3180
   atgccggcga taatggcctg cttctcgccg aaacgtttgg tggcgggacc agtgacgaag 3240
   gcttgagcga gggcgtgcaa gattccgaat accgcaagcg acaggccgat catcgtcgcg 3300
   ctccagcgaa agcggtcctc gccgaaaatg acccagagcg ctgccggcac ctgtcctacg 3360
   agttgcatga taaagaagac agtcataagt gcggcgacga tagtcatgcc ccgcgcccac 3420
   cggaaggagc tgactgggtt gaaggctctc aagggcatcg gtcgagatcc cggtgcctaa 3480
   tgagtgagct aacttacatt aattgcgttg cgctcactgc ccgctttcca gtcgggaaac 3540
   ctgtcgtgcc agctgcatta atgaatcggc caacgcgcgg ggagaggcgg tttgcgtatt 3600
   gggcgccagg gtggtttttc ttttcaccag tgagacgggc aacagctgat tgcccttcac 3660
   cgcctggccc tgagagagtt gcagcaagcg gtccacgctg gtttgcccca gcaggcgaaa 3720
   atcctgtttg atggtggtta acggcgggat ataacatgag ctgtcttcgg tatcgtcgta 3780
   tcccactacc gagatatccg caccaacgcg cagcccggac tcggtaatgg cgcgcattgc 3840
   gcccagcgcc atctgatcgt tggcaaccag catcgcagtg ggaacgatgc cctcattcag 3900
   catttgcatg gtttgttgaa aaccggacat ggcactccag tcgccttccc gttccgctat 3960
   cggctgaatt tgattgcgag tgagatattt atgccagcca gccagacgca gacgcgccga 4020
   gacagaactt aatgggcccg ctaacagcgc gatttgctgg tgacccaatg cgaccagatg 4080
   ctccacgccc agtcgcgtac cgtcttcatg ggagaaaata atactgttga tgggtgtctg 4140
   gtcagagaca tcaagaaata acgccggaac attagtgcag gcagcttcca cagcaatggc 4200
   atcctggtca tccagcggat agttaatgat cagcccactg acgcgttgcg cgagaagatt 4260
   gtgcaccgcc gctttacagg cttcgacgcc gcttcgttct accatcgaca ccaccacgct 4320
   ggcacccagt tgatcggcgc gagatttaat cgccgcgaca atttgcgacg gcgcgtgcag 4380
   ggccagactg gaggtggcaa cgccaatcag caacgactgt ttgcccgcca gttgttgtgc 4440
   cacgcggttg ggaatgtaat tcagctccgc catcgccgct tccacttttt cccgcgtttt 4500
   cgcagaaacg tggctggcct ggttcaccac gcgggaaacg gtctgataag agacaccggc 4560
   atactctgcg acatcgtata acgttactgg tttcacattc accaccctga attgactctc 4620
   ttccgggcgc tatcatgcca taccgcgaaa ggttttgcgc cattcgatgg tgtccgggat 4680
   ctcgacgctc tcccttatgc gactcctgca ttaggaagca gcccagtagt aggttgaggc 4740
   cgttgagcac cgccgccgca aggaatggtg catgcaagga gatggcgccc aacagtcccc 4800
   cggccacggg gcctgccacc atacccacgc cgaaacaagc gctcatgagc ccgaagtggc 4860
   gagcccgatc ttccccatcg gtgatgtcgg cgatataggc gccagcaacc gcacctgtgg 4920
   cgccggtgat gccggccacg atgcgtccgg cgtagaggat cgagatctcg atcccgcgaa 4980
   attaatacga ctcactatag gggaattgtg agcggataac aattcccctc tagaaataat 5040
   tttgtttaac tttaagaagg agatatacca tgggcagcag ccatcatcat catcatcaca 5100
   gcagcggcct ggtgccgcgc ggcagccata tggctagcat gactggtgga cagcaaatgg 5160
   gtcgggatcc gaattcgagc tccgtcgaca agcttgcggc cgcactcgag caccaccacc 5220
   accaccactg agatccggct gctaacaaag cccgaaagga agctgagttg gctgctgcca 5280
   ccgctgagca ataactagca taaccccttg gggcctctaa acgggtcttg aggggttttt 5340
   tgctgaaagg aggaactata tccggat 5367
<210> 22
   <211> 43
   <212> DNA
   <213> Artificial Sequence
<220>
   <223> oligonucleotide o-MO-40 for gcvTHP amplification
<400> 22
   ccggggatcg cggccgcgga ccgaacttat ccgccaggca atg 43
<210> 23
   <211> 51
   <212> DNA
   <213> Artificial Sequence
<220>
   <223> oligonucleotide o-MO-41 for gcvTHP amplification
<400> 23
   ttactggtat tcgctaatcg gctcgagcat cttgtcctca ttgaataagc g 51
<210> 24
   <211> 30
   <212> DNA
   <213> Artificial Sequence
<220>
   <223> oligonucleotide o-MO-42 for gcvTHP amplification
<400> 24
   ctcgagccga ttagcgaata ccagtaattc 30
<210> 25
   <211> 41
   <212> DNA
   <213> Artificial Sequence
<220>
   <223> oligonucleotide o-MO-43 for gcvTHP amplification
<400> 25
   ggatcgcggc cgctctagag ttcatcgtgc tgatcgattg c 41
<210> 26
   <211> 553
   <212> DNA
   <213> Artificial Sequence
<220>
   <223> PCR product 1 from example 12, 1553bp
<400> 26
   ccggggatcg cggccgcgga ccgaacttat ccgccaggca atgggattaa acgatttgcc      60
   tgaatggctg cgttaaaaat ttctcctctg ttgtttattt gatacccatc acactttcat     120
   ctcccggttt tttcgccggg agattttcct catttgaaat aaactaattt cacctccgtt     180
   ttcgcattat attttctaat gccattattt tttgatttag tgttttttga cattttttta     240
   gctcttaata ttgtcttatt caaattgact ttctcatcac atcatctttg tatagaaact     300
   ggtgtatttt ttggtttttt attctgtcgc gatttttgca ttttttaacc ataagctaat     360
   gtgatgatca attttacctt atggttaaca gtctgtttcg gtggtaagtt caggcaaaag     420
   agaacgattg cgttggggac cgggagtggc tccgatgctg ggtttcgtgg tgataatttc     480
   accatgaaaa agttgtcagc cccgcttatt caatgaggac aagatgctcg agccgattag     540
   cgaataccag taa                                                        553
<210> 27
   <211> 547
   <212> DNA
   <213> Artificial Sequence
<220>
   <223> PCR product 2 from example 12, 1553bp
<400> 27
   ctcgagccga ttagcgaata ccagtaattc actgattcga ctattttcta aaggcgcttc        60
   ggcgcctttt tagtcagatg acaaagtaca aaagtgctca gacagtcccc tcgccccttt       120
   ggggagaggg ttagggtgag gggaacaggc cggcactggc gcgaatattt accctcaccc       180
   cggccctctc cctgaaaggg cgagggggaa aagcgtgcca acattgaaga ttgagccagt       240
   ttgttagcaa tctcaaagat acgtcaacga attaattttt ctcggaaaaa caaatggcta       300
   tagcacttgt gactggtggc agtcgcggca tcgggcgggc aactgcatta ctgttggcgc       360
   aagaagggta tacggtggcg gttaattatc agcaaaacct ccacgcggcg caggaagtga       420
   tgaacttaat aacgcaagcc ggtggcaaag cattcgtgct ccaggcggat atcagcgacg       480
   aaaaccaggt cgttgcgatg tttacagcaa tcgatcagca cgatgaactc tagagcggcc       540
   gcgatcc                                                                 547
<210> 28
   <211> 5667
   <212> DNA
   <213> Artificial Sequence
<220>
   <223> vector pKO3
<400> 28
   cctttcgtct tcgaataaat acctgtgacg gaagatcact tcgcagaata aataaatcct      60
   ggtgtccctg ttgataccgg gaagccctgg gccaactttt ggcgaaaatg agacgttgat     120
   cggcacgtaa gaggttccaa ctttcaccat aatgaaataa gatcactacc gggcgtattt     180
   tttgagttat cgagattttc aggagctaag gaagctaaaa tggagaaaaa aatcactgga     240
   tataccaccg ttgatatatc ccaatggcat cgtaaagaac attttgaggc atttcagtca     300
   gttgctcaat gtacctataa ccagaccgtt cagctggata ttacggcctt tttaaagacc     360
   gtaaagaaaa ataagcacaa gttttatccg gcctttattc acattcttgc ccgcctgatg     420
   aatgctcatc cggaattccg tatggcaatg aaagacggtg agctggtgat atgggatagt     480
   gttcaccctt gttacaccgt tttccatgag caaactgaaa cgttttcatc gctctggagt     540
   gaataccacg acgatttccg gcagtttcta cacatatatt cgcaagatgt ggcgtgttac     600
   ggtgaaaacc tggcctattt ccctaaaggg tttattgaga atatgttttt cgtctcagcc     660
   aatccctggg tgagtttcac cagttttgat ttaaacgtgg ccaatatgga caacttcttc     720
   gcccccgttt tcaccatggg caaatattat acgcaaggcg acaaggtgct gatgccgctg     780
   gcgattcagg ttcatcatgc cgtttgtgat ggcttccatg tcggcagaat gcttaatgaa     840
   ttacaacagt actgcgatga gtggcagggc ggggcgtaat ttttttaagg cagttattgg     900
   tgcccttaaa cgcctggttg ctacgcctga ataagtgata ataagcggat gaatggcaga     960
   aattcgaaag caaattcgac ccggtcgtcg gttcagggca gggtcgttaa atagccgctt    1020
   atgtctattg ctggtctcgg tacccgggga tcgcggccgc ggaccggatc ctctagagcg 1080
   gccgcgatcc tctagagtcg accggtggcg aatgggacgc gccctgtagc ggcgcattaa 1140
   gcgcggcggg tgtggtggtt acgcgcagcg tgaccgctac acttgccagc gccctagcgc 1200
   ccgctccttt cgctttcttc ccttcctttc tcgccacgtt cgccggcttt ccccgtcaag 1260
   ctctaaatcg ggggctccct ttagggttcc gatttagtgc tttacggcac ctcgacccca 1320
   aaaaacttga ttagggtgat ggttcacgta gtgggccatc gccctgatag acggtttttc 1380
   gccctttgac gttggagtcc acgttcttta atagtggact cttgttccaa actggaacaa 1440
   cactcaaccc tatctcggtc tattcttttg atttataagg gattttgccg atttcggcct 1500
   attggttaaa aaatgagctg atttaacaaa aatttaacgc gaattttaac aaaatattaa 1560
   cgcttacaat ttaggtggca cttttcgggg aaatgtgcgc ggaaccccta tttgtttatt 1620
   tttctaaata cattcaaata tgtatccgct caccgcgatc ctttttaacc catcacatat 1680
   acctgccgtt cactattatt tagtgaaatg agatattatg atattttctg aattgtgatt 1740
   aaaaaggcaa ctttatgccc atgcaacaga aactataaaa aatacagaga atgaaaagaa 1800
   acagatagat tttttagttc tttaggcccg tagtctgcaa atccttttat gattttctat 1860
   caaacaaaag aggaaaatag accagttgca atccaaacga gagtctaata gaatgaggtc 1920
   gaaaagtaaa tcgcgcgggt ttgttactga taaagcaggc aagacctaaa atgtgtaaag 1980
   ggcaaagtgt atactttggc gtcacccctt acatatttta ggtctttttt tattgtgcgt 2040
   aactaacttg ccatcttcaa acaggagggc tggaagaagc agaccgctaa cacagtacat 2100
   aaaaaaggag acatgaacga tgaacatcaa aaagtttgca aaacaagcaa cagtattaac 2160
   ctttactacc gcactgctgg caggaggcgc aactcaagcg tttgcgaaag aaacgaacca 2220
   aaagccatat aaggaaacat acggcatttc ccatattaca cgccatgata tgctgcaaat 2280
   ccctgaacag caaaaaaatg aaaaatatca agttcctgag ttcgattcgt ccacaattaa 2340
   aaatatctct tctgcaaaag gcctggacgt ttgggacagc tggccattac aaaacgctga 2400
   cggcactgtc gcaaactatc acggctacca catcgtcttt gcattagccg gagatcctaa 2460
   aaatgcggat gacacatcga tttacatgtt ctatcaaaaa gtcggcgaaa cttctattga 2520
   cagctggaaa aacgctggcc gcgtctttaa agacagcgac aaattcgatg caaatgattc 2580
   tatcctaaaa gaccaaacac aagaatggtc aggttcagcc acatttacat ctgacggaaa 2640
   aatccgttta ttctacactg atttctccgg taaacattac ggcaaacaaa cactgacaac 2700
   tgcacaagtt aacgtatcag catcagacag ctctttgaac atcaacggtg tagaggatta 2760
   taaatcaatc tttgacggtg acggaaaaac gtatcaaaat gtacagcagt tcatcgatga 2820
   aggcaactac agctcaggcg acaaccatac gctgagagat cctcactacg tagaagataa 2880
   aggccacaaa tacttagtat ttgaagcaaa cactggaact gaagatggct accaaggcga 2940
   agaatcttta tttaacaaag catactatgg caaaagcaca tcattcttcc gtcaagaaag 3000
   tcaaaaactt ctgcaaagcg ataaaaaacg cacggctgag ttagcaaacg gcgctctcgg 3060
   tatgattgag ctaaacgatg attacacact gaaaaaagtg atgaaaccgc tgattgcatc 3120
   taacacagta acagatgaaa ttgaacgcgc gaacgtcttt aaaatgaacg gcaaatggta 3180
   cctgttcact gactcccgcg gatcaaaaat gacgattgac ggcattacgt ctaacgatat 3240
   ttacatgctt ggttatgttt ctaattcttt aactggccca tacaagccgc tgaacaaaac 3300
   tggccttgtg ttaaaaatgg atcttgatcc taacgatgta acctttactt actcacactt 3360
   cgctgtacct caagcgaaag gaaacaatgt cgtgattaca agctatatga caaacagagg 3420
   attctacgca gacaaacaat caacgtttgc gccaagcttc ctgctgaaca tcaaaggcaa 3480
   gaaaacatct gttgtcaaag acagcatcct tgaacaagga caattaacag ttaacaaata 3540
   aaaacgcaaa agaaaatgcc gatattgact accggaagca gtgtgaccgt gtgcttctca 3600
   aatgcctgat tcaggctgtc tatgtgtgac tgttgagctg taacaagttg tctcaggtgt 3660
   tcaatttcat gttctagttg ctttgtttta ctggtttcac ctgttctatt aggtgttaca 3720
   tgctgttcat ctgttacatt gtcgatctgt tcatggtgaa cagctttaaa tgcaccaaaa 3780
   actcgtaaaa gctctgatgt atctatcttt tttacaccgt tttcatctgt gcatatggac 3840
   agttttccct ttgatatgta acggtgaaca gttgttctac ttttgtttgt tagtcttgat 3900
   gcttcactga tagatacaag agccataaga acctcagatc cttccgtatt tagccagtat 3960
   gttctctagt gtggttcgtt gtttttgcgt gagccatgag aacgaaccat tgagatcata 4020
   cttactttgc atgtcactca aaaattttgc ctcaaaactg gtgagctgaa tttttgcagt 4080
   taaagcatcg tgtagtgttt ttcttagtcc gttatgtagg taggaatctg atgtaatggt 4140
   tgttggtatt ttgtcaccat tcatttttat ctggttgttc tcaagttcgg ttacgagatc 4200
   catttgtcta tctagttcaa cttggaaaat caacgtatca gtcgggcggc ctcgcttatc 4260
   aaccaccaat ttcatattgc tgtaagtgtt taaatcttta cttattggtt tcaaaaccca 4320
   ttggttaagc cttttaaact catggtagtt attttcaagc attaacatga acttaaattc 4380
   atcaaggcta atctctatat ttgccttgtg agttttcttt tgtgttagtt cttttaataa 4440
   ccactcataa atcctcatag agtatttgtt ttcaaaagac ttaacatgtt ccagattata 4500
   ttttatgaat ttttttaact ggaaaagata aggcaatatc tcttcactaa aaactaattc 4560
   taatttttcg cttgagaact tggcatagtt tgtccactgg aaaatctcaa agcctttaac 4620
   caaaggattc ctgatttcca cagttctcgt catcagctct ctggttgctt tagctaatac 4680
   accataagca ttttccctac tgatgttcat catctgaacg tattggttat aagtgaacga 4740
   taccgtccgt tctttccttg tagggttttc aatcgtgggg ttgagtagtg ccacacagca 4800
   taaaattagc ttggtttcat gctccgttaa gtcatagcga ctaatcgcta gttcatttgc 4860
   tttgaaaaca actaattcag acatacatct caattggtct aggtgatttt aatcactata 4920
   ccaattgaga tgggctagtc aatgataatt actagtcctt ttcctttgag ttgtgggtat 4980
   ctgtaaattc tgctagacct ttgctggaaa acttgtaaat tctgctagac cctctgtaaa 5040
   ttccgctaga cctttgtgtg ttttttttgt ttatattcaa gtggttataa tttatagaat 5100
   aaagaaagaa taaaaaaaga taaaaagaat agatcccagc cctgtgtata actcactact 5160
   ttagtcagtt ccgcagtatt acaaaaggat gtcgcaaacg ctgtttgctc ctctacaaaa 5220
   cagaccttaa aaccctaaag gcttaagtag caccctcgca agctcgggca aatcgctgaa 5280
   tattcctttt gtctccgacc atcaggcacc tgagtcgctg tctttttcgt gacattcagt 5340
   tcgctgcgct cacggctctg gcagtgaatg ggggtaaatg gcactacagg cgccttttat 5400
   ggattcatgc aaggaaacta cccataatac aagaaaagcc cgtcacgggc ttctcagggc 5460
   gttttatggc gggtctgcta tgtggtgcta tctgactttt tgctgttcag cagttcctgc 5520
   cctctgattt tccagtctga ccacttcgga ttatcccgtg acaggtcatt cagactggct 5580
   aatgcaccca gtaaggcagc ggtatcatca acaggcttac ccgtcttact gtcggggatc 5640
   gacgctctcc cttatgcgac tcctgca 5667
<210> 29
   <211> 6693
   <212> DNA
   <213> Artificial Sequence
<220>
   <223> knock-out vector pKO3 delta gcvTHP
<400> 29
   cctttcgtct tcgaataaat acctgtgacg gaagatcact tcgcagaata aataaatcct 60
   ggtgtccctg ttgataccgg gaagccctgg gccaactttt ggcgaaaatg agacgttgat 120
   cggcacgtaa gaggttccaa ctttcaccat aatgaaataa gatcactacc gggcgtattt 180
   tttgagttat cgagattttc aggagctaag gaagctaaaa tggagaaaaa aatcactgga 240
   tataccaccg ttgatatatc ccaatggcat cgtaaagaac attttgaggc atttcagtca 300
   gttgctcaat gtacctataa ccagaccgtt cagctggata ttacggcctt tttaaagacc 360
   gtaaagaaaa ataagcacaa gttttatccg gcctttattc acattcttgc ccgcctgatg 420
   aatgctcatc cggaattccg tatggcaatg aaagacggtg agctggtgat atgggatagt 480
   gttcaccctt gttacaccgt tttccatgag caaactgaaa cgttttcatc gctctggagt 540
   gaataccacg acgatttccg gcagtttcta cacatatatt cgcaagatgt ggcgtgttac 600
   ggtgaaaacc tggcctattt ccctaaaggg tttattgaga atatgttttt cgtctcagcc 660
   aatccctggg tgagtttcac cagttttgat ttaaacgtgg ccaatatgga caacttcttc      720
   gcccccgttt tcaccatggg caaatattat acgcaaggcg acaaggtgct gatgccgctg      780
   gcgattcagg ttcatcatgc cgtttgtgat ggcttccatg tcggcagaat gcttaatgaa 840
   ttacaacagt actgcgatga gtggcagggc ggggcgtaat ttttttaagg cagttattgg 900
   tgcccttaaa cgcctggttg ctacgcctga ataagtgata ataagcggat gaatggcaga 960
   aattcgaaag caaattcgac ccggtcgtcg gttcagggca gggtcgttaa atagccgctt 1020
   atgtctattg ctggtctcgg tacccgggga tcgcggccgc ggaccgaact tatccgccag 1080
   gcaatgggat taaacgattt gcctgaatgg ctgcgttaaa aatttctcct ctgttgttta 1140
   tttgataccc atcacacttt catctcccgg ttttttcgcc gggagatttt cctcatttga 1200
   aataaactaa tttcacctcc gttttcgcat tatattttct aatgccatta ttttttgatt 1260
   tagtgttttt tgacattttt ttagctctta atattgtctt attcaaattg actttctcat 1320
   cacatcatct ttgtatagaa actggtgtat tttttggttt tttattctgt cgcgattttt 1380
   gcatttttta accataagct aatgtgatga tcaattttac cttatggtta acagtctgtt 1440
   tcggtggtaa gttcaggcaa aagagaacga ttgcgttggg gaccgggagt ggctccgatg 1500
   ctgggtttcg tggtgataat ttcaccatga aaaagttgtc agccccgctt attcaatgag 1560
   gacaagatgc tcgagccgat tagcgaatac cagtaattca ctgattcgac tattttctaa 1620
   aggcgcttcg gcgccttttt agtcagatga caaagtacaa aagtgctcag acagtcccct 1680
   cgcccctttg gggagagggt tagggtgagg ggaacaggcc ggcactggcg cgaatattta 1740
   ccctcacccc ggccctctcc ctgaaagggc gagggggaaa agcgtgccaa cattgaagat 1800
   tgagccagtt tgttagcaat ctcaaagata cgtcaacgaa ttaatttttc tcggaaaaac 1860
   aaatggctat agcacttgtg actggtggca gtcgcggcat cgggcgggca actgcattac 1920
   tgttggcgca agaagggtat acggtggcgg ttaattatca gcaaaacctc cacgcggcgc 1980
   aggaagtgat gaacttaata acgcaagccg gtggcaaagc attcgtgctc caggcggata 2040
   tcagcgacga aaaccaggtc gttgcgatgt ttacagcaat cgatcagcac gatgaactct 2100
   agagcggccg cgatcctcta gagtcgaccg gtggcgaatg ggacgcgccc tgtagcggcg 2160
   cattaagcgc ggcgggtgtg gtggttacgc gcagcgtgac cgctacactt gccagcgccc 2220
   tagcgcccgc tcctttcgct ttcttccctt cctttctcgc cacgttcgcc ggctttcccc 2280
   gtcaagctct aaatcggggg ctccctttag ggttccgatt tagtgcttta cggcacctcg 2340
   accccaaaaa acttgattag ggtgatggtt cacgtagtgg gccatcgccc tgatagacgg 2400
   tttttcgccc tttgacgttg gagtccacgt tctttaatag tggactcttg ttccaaactg 2460
   gaacaacact caaccctatc tcggtctatt cttttgattt ataagggatt ttgccgattt 2520
   cggcctattg gttaaaaaat gagctgattt aacaaaaatt taacgcgaat tttaacaaaa 2580
   tattaacgct tacaatttag gtggcacttt tcggggaaat gtgcgcggaa cccctatttg 2640
   tttatttttc taaatacatt caaatatgta tccgctcacc gcgatccttt ttaacccatc 2700
   acatatacct gccgttcact attatttagt gaaatgagat attatgatat tttctgaatt 2760
   gtgattaaaa aggcaacttt atgcccatgc aacagaaact ataaaaaata cagagaatga 2820
   aaagaaacag atagattttt tagttcttta ggcccgtagt ctgcaaatcc ttttatgatt 2880
   ttctatcaaa caaaagagga aaatagacca gttgcaatcc aaacgagagt ctaatagaat 2940
   gaggtcgaaa agtaaatcgc gcgggtttgt tactgataaa gcaggcaaga cctaaaatgt 3000
   gtaaagggca aagtgtatac tttggcgtca ccccttacat attttaggtc tttttttatt 3060
   gtgcgtaact aacttgccat cttcaaacag gagggctgga agaagcagac cgctaacaca 3120
   gtacataaaa aaggagacat gaacgatgaa catcaaaaag tttgcaaaac aagcaacagt 3180
   attaaccttt actaccgcac tgctggcagg aggcgcaact caagcgtttg cgaaagaaac 3240
   gaaccaaaag ccatataagg aaacatacgg catttcccat attacacgcc atgatatgct 3300
   gcaaatccct gaacagcaaa aaaatgaaaa atatcaagtt cctgagttcg attcgtccac 3360
   aattaaaaat atctcttctg caaaaggcct ggacgtttgg gacagctggc cattacaaaa 3420
   cgctgacggc actgtcgcaa actatcacgg ctaccacatc gtctttgcat tagccggaga 3480
   tcctaaaaat gcggatgaca catcgattta catgttctat caaaaagtcg gcgaaacttc 3540
   tattgacagc tggaaaaacg ctggccgcgt ctttaaagac agcgacaaat tcgatgcaaa 3600
   tgattctatc ctaaaagacc aaacacaaga atggtcaggt tcagccacat ttacatctga 3660
   cggaaaaatc cgtttattct acactgattt ctccggtaaa cattacggca aacaaacact 3720
   gacaactgca caagttaacg tatcagcatc agacagctct ttgaacatca acggtgtaga 3780
   ggattataaa tcaatctttg acggtgacgg aaaaacgtat caaaatgtac agcagttcat 3840
   cgatgaaggc aactacagct caggcgacaa ccatacgctg agagatcctc actacgtaga 3900
   agataaaggc cacaaatact tagtatttga agcaaacact ggaactgaag atggctacca 3960
   aggcgaagaa tctttattta acaaagcata ctatggcaaa agcacatcat tcttccgtca 4020
   agaaagtcaa aaacttctgc aaagcgataa aaaacgcacg gctgagttag caaacggcgc 4080
   tctcggtatg attgagctaa acgatgatta cacactgaaa aaagtgatga aaccgctgat 4140
   tgcatctaac acagtaacag atgaaattga acgcgcgaac gtctttaaaa tgaacggcaa 4200
   atggtacctg ttcactgact cccgcggatc aaaaatgacg attgacggca ttacgtctaa 4260
   cgatatttac atgcttggtt atgtttctaa ttctttaact ggcccataca agccgctgaa 4320
   caaaactggc cttgtgttaa aaatggatct tgatcctaac gatgtaacct ttacttactc 4380
   acacttcgct gtacctcaag cgaaaggaaa caatgtcgtg attacaagct atatgacaaa 4440
   cagaggattc tacgcagaca aacaatcaac gtttgcgcca agcttcctgc tgaacatcaa 4500
   aggcaagaaa acatctgttg tcaaagacag catccttgaa caaggacaat taacagttaa 4560
   caaataaaaa cgcaaaagaa aatgccgata ttgactaccg gaagcagtgt gaccgtgtgc 4620
   ttctcaaatg cctgattcag gctgtctatg tgtgactgtt gagctgtaac aagttgtctc 4680
   aggtgttcaa tttcatgttc tagttgcttt gttttactgg tttcacctgt tctattaggt 4740
   gttacatgct gttcatctgt tacattgtcg atctgttcat ggtgaacagc tttaaatgca 4800
   ccaaaaactc gtaaaagctc tgatgtatct atctttttta caccgttttc atctgtgcat 4860
   atggacagtt ttccctttga tatgtaacgg tgaacagttg ttctactttt gtttgttagt 4920
   cttgatgctt cactgataga tacaagagcc ataagaacct cagatccttc cgtatttagc 4980
   cagtatgttc tctagtgtgg ttcgttgttt ttgcgtgagc catgagaacg aaccattgag 5040
   atcatactta ctttgcatgt cactcaaaaa ttttgcctca aaactggtga gctgaatttt 5100
   tgcagttaaa gcatcgtgta gtgtttttct tagtccgtta tgtaggtagg aatctgatgt 5160
   aatggttgtt ggtattttgt caccattcat ttttatctgg ttgttctcaa gttcggttac 5220
   gagatccatt tgtctatcta gttcaacttg gaaaatcaac gtatcagtcg ggcggcctcg 5280
   cttatcaacc accaatttca tattgctgta agtgtttaaa tctttactta ttggtttcaa 5340
   aacccattgg ttaagccttt taaactcatg gtagttattt tcaagcatta acatgaactt 5400
   aaattcatca aggctaatct ctatatttgc cttgtgagtt ttcttttgtg ttagttcttt 5460
   taataaccac tcataaatcc tcatagagta tttgttttca aaagacttaa catgttccag 5520
   attatatttt atgaattttt ttaactggaa aagataaggc aatatctctt cactaaaaac 5580
   taattctaat ttttcgcttg agaacttggc atagtttgtc cactggaaaa tctcaaagcc 5640
   tttaaccaaa ggattcctga tttccacagt tctcgtcatc agctctctgg ttgctttagc 5700
   taatacacca taagcatttt ccctactgat gttcatcatc tgaacgtatt ggttataagt 5760
   gaacgatacc gtccgttctt tccttgtagg gttttcaatc gtggggttga gtagtgccac 5820
   acagcataaa attagcttgg tttcatgctc cgttaagtca tagcgactaa tcgctagttc 5880
   atttgctttg aaaacaacta attcagacat acatctcaat tggtctaggt gattttaatc 5940
   actataccaa ttgagatggg ctagtcaatg ataattacta gtccttttcc tttgagttgt 6000
   gggtatctgt aaattctgct agacctttgc tggaaaactt gtaaattctg ctagaccctc 6060
   tgtaaattcc gctagacctt tgtgtgtttt ttttgtttat attcaagtgg ttataattta 6120
   tagaataaag aaagaataaa aaaagataaa aagaatagat cccagccctg tgtataactc 6180
   actactttag tcagttccgc agtattacaa aaggatgtcg caaacgctgt ttgctcctct 6240
   acaaaacaga ccttaaaacc ctaaaggctt aagtagcacc ctcgcaagct cgggcaaatc 6300
   gctgaatatt ccttttgtct ccgaccatca ggcacctgag tcgctgtctt tttcgtgaca 6360
   ttcagttcgc tgcgctcacg gctctggcag tgaatggggg taaatggcac tacaggcgcc 6420
   ttttatggat tcatgcaagg aaactaccca taatacaaga aaagcccgtc acgggcttct 6480
   cagggcgttt tatggcgggt ctgctatgtg gtgctatctg actttttgct gttcagcagt 6540
   tcctgccctc tgattttcca gtctgaccac ttcggattat cccgtgacag gtcattcaga 6600
   ctggctaatg cacccagtaa ggcagcggta tcatcaacag gcttacccgt cttactgtcg 6660
   gggatcgacg ctctccctta tgcgactcct gca 6693
<210> 30
   <211> 30
   <212> DNA
   <213> Artificial Sequence
<220>
   <223> DNA sequence after deletion of gcvTHP
<400> 30
   atgctcgagc cgattagcga ataccagtaa 30
<210> 31
   <211> 38
   <212> DNA
   <213> Artificial Sequence
<220>
   <223> oligonucleotide o-MO-44 for amplifying the upstream region of glyA
<400> 31
   gggatcgcgg ccgcggaccg agggcttcac gttgatcg 38
<210> 32
   <211> 45
   <212> DNA
   <213> Artificial Sequence
<220>
   <223> oligonucleotide o-MO-45 for amplifying the upstream region of glyA
<400> 32
   tatgcgtaaa ccgggtaacg ctcgagcatc cgcatctcct gactc 45
<210> 33
   <211> 22
   <212> DNA
   <213> Artificial Sequence
<220>
   <223> oligonucleotide o-MO-46 for amplifying the downstream region of glyA
<400> 33
   cgttacccgg tttacgcata ag 22
<210> 34
   <211> 39
   <212> DNA
   <213> Artificial Sequence
<220>
   <223> oligonucleotide o-MO-47 for amplifying the downstream region of glyA
<400> 34
   ggatcgcggc cgctctagag atcaccagct gggttgatc 39
<210> 35
   <211> 546
   <212> DNA
   <213> Artificial Sequence
<220>
   <223> PCR product 1 from Example 14
<400> 35
   gggatcgcgg ccgcggaccg agggcttcac gttgatcgcc attacgctgg ttactcatgt     60
   taaaaatttc tttgagttct gggttatgag taaacatacg gtcgtagaaa tgggcggtta    120
   actttggccc cgtttccacc agtaaaggga tggtggcttt tactgtagcg atggtttgag    180
   cgtcaagcat atggtcttcc tttttttgca tcttaattga tgtatctcaa atgcatctta    240
   taaaaaatag ccctgcaatg taaatggttc tttggtgttt ttcagaaaga atgtgatgaa    300
   gtgaaaaatt tgcatcacaa acctgaaaag aaatccgttt ccggttgcaa gctctttatt    360
   ctccaaagcc ttgcgtagcc tgaaggtaat cgtttgcgta aattcctttg tcaagacctg    420
   ttatcgcaca atgattcggt tatactgttc gccgttgtcc aacaggaccg cctataaagg    480
   ccaaaaattt tattgttagc tgagtcagga gatgcggatg ctcgagcgtt acccggttta    540
   cgcata                                                               546
<210> 36
   <211> 520
   <212> DNA
   <213> Artificial Sequence
<220>
   <223> PCR product 2 from Example 14
<400> 36
   cgttacccgg tttacgcata agcgaaacgg tgatttgctg tcaatgtgct cgttgttcat     60
   gccggatgcg gcgtgaacgc cttatccggc ctacaaaact ttgcaaattc aatatattgc    120
   aatctccgtg taggcctgat aagcgtagcg catcaggcaa tttttcgttt atgatcatca    180
   aggcttcctt cgggaagcct ttctacgtta tcgcgccatc aaatctgtcg taactgcgcc    240
   tcaacataca aatagccaat tcccagcacc tgttgtgcgc ggcttaattg cccaaagcca    300
   atttgcgtcg ctttcacgct cggtccttcg tcgcggtcga acgggttaaa tcctgtctgg 360
   cgaataatag tattaagcca gttctcacca aaggcacaac tgcgaccgta caaccaaatc 420
   tgattaatat tgaggatgtt gaggaagtta tacagactca aaccaatggc attggcagag 480
   cgatcaaccc agctggtgat ctctagagcg gccgcgatcc 520
<210> 37
   <211> 26
   <212> DNA
   <213> Artificial Sequence
<220>
   <223> oligonucleotide o-MO-52
<400> 37
   actgaggatc cgggatcgcg gccgcg 26
<210> 38
   <211> 31
   <212> DNA
   <213> Artificial Sequence
<220>
   <223> oligonucleotide o-MO-53
<400> 38
   actgaggatc cggatcgcgg ccgctctaga g 31
<210> 39
   <211> 1052
   <212> DNA
   <213> Artificial Sequence
<220>
   <223> PCR product 3 from Example 14
<400> 39
   gatccgggat cgcggccgcg gaccgagggc ttcacgttga tcgccattac gctggttact 60
   catgttaaaa atttctttga gttctgggtt atgagtaaac atacggtcgt agaaatgggc 120
   ggttaacttt ggccccgttt ccaccagtaa agggatggtg gcttttactg tagcgatggt 180
   ttgagcgtca agcatatggt cttccttttt ttgcatctta attgatgtat ctcaaatgca 240
   tcttataaaa aatagccctg caatgtaaat ggttctttgg tgtttttcag aaagaatgtg 300
   atgaagtgaa aaatttgcat cacaaacctg aaaagaaatc cgtttccggt tgcaagctct 360
   ttattctcca aagccttgcg tagcctgaag gtaatcgttt gcgtaaattc ctttgtcaag 420
   acctgttatc gcacaatgat tcggttatac tgttcgccgt tgtccaacag gaccgcctat 480
   aaaggccaaa aattttattg ttagctgagt caggagatgc ggatgctcga gcgttacccg 540
   gtttacgcat aagcgaaacg gtgatttgct gtcaatgtgc tcgttgttca tgccggatgc 600
   ggcgtgaacg ccttatccgg cctacaaaac tttgcaaatt caatatattg caatctccgt 660
   gtaggcctga taagcgtagc gcatcaggca atttttcgtt tatgatcatc aaggcttcct 720
   tcgggaagcc tttctacgtt atcgcgccat caaatctgtc gtaactgcgc ctcaacatac 780
   aaatagccaa ttcccagcac ctgttgtgcg cggcttaatt gcccaaagcc aatttgcgtc 840
   gctttcacgc tcggtccttc gtcgcggtcg aacgggttaa atcctgtctg gcgaataata 900
   gtattaagcc agttctcacc aaaggcacaa ctgcgaccgt acaaccaaat ctgattaata 960
   ttgaggatgt tgaggaagtt atacagactc aaaccaatgg cattggcaga gcgatcaacc 1020
   cagctggtga tctctagagc ggccgcgatc cg 1052
<210> 40
   <211> 6719
   <212> DNA
   <213> Artificial Sequence
<220>
   <223> pKO3 delta glyA
<400> 40
   cctttcgtct tcgaataaat acctgtgacg gaagatcact tcgcagaata aataaatcct      60
   ggtgtccctg ttgataccgg gaagccctgg gccaactttt ggcgaaaatg agacgttgat     120
   cggcacgtaa gaggttccaa ctttcaccat aatgaaataa gatcactacc gggcgtattt     180
   tttgagttat cgagattttc aggagctaag gaagctaaaa tggagaaaaa aatcactgga     240
   tataccaccg ttgatatatc ccaatggcat cgtaaagaac attttgaggc atttcagtca     300
   gttgctcaat gtacctataa ccagaccgtt cagctggata ttacggcctt tttaaagacc     360
   gtaaagaaaa ataagcacaa gttttatccg gcctttattc acattcttgc ccgcctgatg     420
   aatgctcatc cggaattccg tatggcaatg aaagacggtg agctggtgat atgggatagt     480
   gttcaccctt gttacaccgt tttccatgag caaactgaaa cgttttcatc gctctggagt     540
   gaataccacg acgatttccg gcagtttcta cacatatatt cgcaagatgt ggcgtgttac     600
   ggtgaaaacc tggcctattt ccctaaaggg tttattgaga atatgttttt cgtctcagcc     660
   aatccctggg tgagtttcac cagttttgat ttaaacgtgg ccaatatgga caacttcttc     720
   gcccccgttt tcaccatggg caaatattat acgcaaggcg acaaggtgct gatgccgctg     780
   gcgattcagg ttcatcatgc cgtttgtgat ggcttccatg tcggcagaat gcttaatgaa     840
   ttacaacagt actgcgatga gtggcagggc ggggcgtaat ttttttaagg cagttattgg     900
   tgcccttaaa cgcctggttg ctacgcctga ataagtgata ataagcggat gaatggcaga     960
   aattcgaaag caaattcgac ccggtcgtcg gttcagggca gggtcgttaa atagccgctt    1020
   atgtctattg ctggtctcgg tacccgggga tcgcggccgc ggaccggatc cgggatcgcg    1080
   gccgcggacc gagggcttca cgttgatcgc cattacgctg gttactcatg ttaaaaattt    1140
   ctttgagttc tgggttatga gtaaacatac ggtcgtagaa atgggcggtt aactttggcc 1200
   ccgtttccac cagtaaaggg atggtggctt ttactgtagc gatggtttga gcgtcaagca 1260
   tatggtcttc ctttttttgc atcttaattg atgtatctca aatgcatctt ataaaaaata 1320
   gccctgcaat gtaaatggtt ctttggtgtt tttcagaaag aatgtgatga agtgaaaaat 1380
   ttgcatcaca aacctgaaaa gaaatccgtt tccggttgca agctctttat tctccaaagc 1440
   cttgcgtagc ctgaaggtaa tcgtttgcgt aaattccttt gtcaagacct gttatcgcac 1500
   aatgattcgg ttatactgtt cgccgttgtc caacaggacc gcctataaag gccaaaaatt 1560
   ttattgttag ctgagtcagg agatgcggat gctcgagcgt tacccggttt acgcataagc 1620
   gaaacggtga tttgctgtca atgtgctcgt tgttcatgcc ggatgcggcg tgaacgcctt 1680
   atccggccta caaaactttg caaattcaat atattgcaat ctccgtgtag gcctgataag 1740
   cgtagcgcat caggcaattt ttcgtttatg atcatcaagg cttccttcgg gaagcctttc 1800
   tacgttatcg cgccatcaaa tctgtcgtaa ctgcgcctca acatacaaat agccaattcc 1860
   cagcacctgt tgtgcgcggc ttaattgccc aaagccaatt tgcgtcgctt tcacgctcgg 1920
   tccttcgtcg cggtcgaacg ggttaaatcc tgtctggcga ataatagtat taagccagtt 1980
   ctcaccaaag gcacaactgc gaccgtacaa ccaaatctga ttaatattga ggatgttgag 2040
   gaagttatac agactcaaac caatggcatt ggcagagcga tcaacccagc tggtgatctc 2100
   tagagcggcc gcgatccgga tcctctagag cggccgcgat cctctagagt cgaccggtgg 2160
   cgaatgggac gcgccctgta gcggcgcatt aagcgcggcg ggtgtggtgg ttacgcgcag 2220
   cgtgaccgct acacttgcca gcgccctagc gcccgctcct ttcgctttct tcccttcctt 2280
   tctcgccacg ttcgccggct ttccccgtca agctctaaat cgggggctcc ctttagggtt 2340
   ccgatttagt gctttacggc acctcgaccc caaaaaactt gattagggtg atggttcacg 2400
   tagtgggcca tcgccctgat agacggtttt tcgccctttg acgttggagt ccacgttctt 2460
   taatagtgga ctcttgttcc aaactggaac aacactcaac cctatctcgg tctattcttt 2520
   tgatttataa gggattttgc cgatttcggc ctattggtta aaaaatgagc tgatttaaca 2580
   aaaatttaac gcgaatttta acaaaatatt aacgcttaca atttaggtgg cacttttcgg 2640
   ggaaatgtgc gcggaacccc tatttgttta tttttctaaa tacattcaaa tatgtatccg 2700
   ctcaccgcga tcctttttaa cccatcacat atacctgccg ttcactatta tttagtgaaa 2760
   tgagatatta tgatattttc tgaattgtga ttaaaaaggc aactttatgc ccatgcaaca 2820
   gaaactataa aaaatacaga gaatgaaaag aaacagatag attttttagt tctttaggcc 2880
   cgtagtctgc aaatcctttt atgattttct atcaaacaaa agaggaaaat agaccagttg 2940
   caatccaaac gagagtctaa tagaatgagg tcgaaaagta aatcgcgcgg gtttgttact 3000
   gataaagcag gcaagaccta aaatgtgtaa agggcaaagt gtatactttg gcgtcacccc 3060
   ttacatattt taggtctttt tttattgtgc gtaactaact tgccatcttc aaacaggagg 3120
   gctggaagaa gcagaccgct aacacagtac ataaaaaagg agacatgaac gatgaacatc 3180
   aaaaagtttg caaaacaagc aacagtatta acctttacta ccgcactgct ggcaggaggc 3240
   gcaactcaag cgtttgcgaa agaaacgaac caaaagccat ataaggaaac atacggcatt 3300
   tcccatatta cacgccatga tatgctgcaa atccctgaac agcaaaaaaa tgaaaaatat 3360
   caagttcctg agttcgattc gtccacaatt aaaaatatct cttctgcaaa aggcctggac 3420
   gtttgggaca gctggccatt acaaaacgct gacggcactg tcgcaaacta tcacggctac 3480
   cacatcgtct ttgcattagc cggagatcct aaaaatgcgg atgacacatc gatttacatg 3540
   ttctatcaaa aagtcggcga aacttctatt gacagctgga aaaacgctgg ccgcgtcttt 3600
   aaagacagcg acaaattcga tgcaaatgat tctatcctaa aagaccaaac acaagaatgg 3660
   tcaggttcag ccacatttac atctgacgga aaaatccgtt tattctacac tgatttctcc 3720
   ggtaaacatt acggcaaaca aacactgaca actgcacaag ttaacgtatc agcatcagac 3780
   agctctttga acatcaacgg tgtagaggat tataaatcaa tctttgacgg tgacggaaaa 3840
   acgtatcaaa atgtacagca gttcatcgat gaaggcaact acagctcagg cgacaaccat 3900
   acgctgagag atcctcacta cgtagaagat aaaggccaca aatacttagt atttgaagca 3960
   aacactggaa ctgaagatgg ctaccaaggc gaagaatctt tatttaacaa agcatactat 4020
   ggcaaaagca catcattctt ccgtcaagaa agtcaaaaac ttctgcaaag cgataaaaaa 4080
   cgcacggctg agttagcaaa cggcgctctc ggtatgattg agctaaacga tgattacaca 4140
   ctgaaaaaag tgatgaaacc gctgattgca tctaacacag taacagatga aattgaacgc 4200
   gcgaacgtct ttaaaatgaa cggcaaatgg tacctgttca ctgactcccg cggatcaaaa 4260
   atgacgattg acggcattac gtctaacgat atttacatgc ttggttatgt ttctaattct 4320
   ttaactggcc catacaagcc gctgaacaaa actggccttg tgttaaaaat ggatcttgat 4380
   cctaacgatg taacctttac ttactcacac ttcgctgtac ctcaagcgaa aggaaacaat 4440
   gtcgtgatta caagctatat gacaaacaga ggattctacg cagacaaaca atcaacgttt 4500
   gcgccaagct tcctgctgaa catcaaaggc aagaaaacat ctgttgtcaa agacagcatc 4560
   cttgaacaag gacaattaac agttaacaaa taaaaacgca aaagaaaatg ccgatattga 4620
   ctaccggaag cagtgtgacc gtgtgcttct caaatgcctg attcaggctg tctatgtgtg 4680
   actgttgagc tgtaacaagt tgtctcaggt gttcaatttc atgttctagt tgctttgttt 4740
   tactggtttc acctgttcta ttaggtgtta catgctgttc atctgttaca ttgtcgatct 4800
   gttcatggtg aacagcttta aatgcaccaa aaactcgtaa aagctctgat gtatctatct 4860
   tttttacacc gttttcatct gtgcatatgg acagttttcc ctttgatatg taacggtgaa 4920
   cagttgttct acttttgttt gttagtcttg atgcttcact gatagataca agagccataa 4980
   gaacctcaga tccttccgta tttagccagt atgttctcta gtgtggttcg ttgtttttgc 5040
   gtgagccatg agaacgaacc attgagatca tacttacttt gcatgtcact caaaaatttt 5100
   gcctcaaaac tggtgagctg aatttttgca gttaaagcat cgtgtagtgt ttttcttagt 5160
   ccgttatgta ggtaggaatc tgatgtaatg gttgttggta ttttgtcacc attcattttt 5220
   atctggttgt tctcaagttc ggttacgaga tccatttgtc tatctagttc aacttggaaa 5280
   atcaacgtat cagtcgggcg gcctcgctta tcaaccacca atttcatatt gctgtaagtg 5340
   tttaaatctt tacttattgg tttcaaaacc cattggttaa gccttttaaa ctcatggtag 5400
   ttattttcaa gcattaacat gaacttaaat tcatcaaggc taatctctat atttgccttg 5460
   tgagttttct tttgtgttag ttcttttaat aaccactcat aaatcctcat agagtatttg 5520
   ttttcaaaag acttaacatg ttccagatta tattttatga atttttttaa ctggaaaaga 5580
   taaggcaata tctcttcact aaaaactaat tctaattttt cgcttgagaa cttggcatag 5640
   tttgtccact ggaaaatctc aaagccttta accaaaggat tcctgatttc cacagttctc 5700
   gtcatcagct ctctggttgc tttagctaat acaccataag cattttccct actgatgttc 5760
   atcatctgaa cgtattggtt ataagtgaac gataccgtcc gttctttcct tgtagggttt 5820
   tcaatcgtgg ggttgagtag tgccacacag cataaaatta gcttggtttc atgctccgtt 5880
   aagtcatagc gactaatcgc tagttcattt gctttgaaaa caactaattc agacatacat 5940
   ctcaattggt ctaggtgatt ttaatcacta taccaattga gatgggctag tcaatgataa 6000
   ttactagtcc ttttcctttg agttgtgggt atctgtaaat tctgctagac ctttgctgga 6060
   aaacttgtaa attctgctag accctctgta aattccgcta gacctttgtg tgtttttttt 6120
   gtttatattc aagtggttat aatttataga ataaagaaag aataaaaaaa gataaaaaga 6180
   atagatccca gccctgtgta taactcacta ctttagtcag ttccgcagta ttacaaaagg 6240
   atgtcgcaaa cgctgtttgc tcctctacaa aacagacctt aaaaccctaa aggcttaagt 6300
   agcaccctcg caagctcggg caaatcgctg aatattcctt ttgtctccga ccatcaggca 6360
   cctgagtcgc tgtctttttc gtgacattca gttcgctgcg ctcacggctc tggcagtgaa 6420
   tgggggtaaa tggcactaca ggcgcctttt atggattcat gcaaggaaac tacccataat 6480
   acaagaaaag cccgtcacgg gcttctcagg gcgttttatg gcgggtctgc tatgtggtgc 6540
   tatctgactt tttgctgttc agcagttcct gccctctgat tttccagtct gaccacttcg 6600
   gattatcccg tgacaggtca ttcagactgg ctaatgcacc cagtaaggca gcggtatcat 6660
   caacaggctt acccgtctta ctgtcgggga tcgacgctct cccttatgcg actcctgca 6719
<210> 41
   <211> 30
   <212> DNA
   <213> Artificial Sequence
<220>
   <223> DNA sequence after deletion of glyA
<400> 41
   atgctcgagc gttacccggt ttacgcataa 30
<210> 42
   <211> 39
   <212> DNA
   <213> Artificial Sequence
<220>
   <223> ltaE-UP_fw
<400> 42
   ggatcgcggc cgcggaccgc aggcgacaga gccagaacg 39
<210> 43
   <211> 47
   <212> DNA
   <213> Artificial Sequence
<220>
   <223> ltAE-UP-XhoI_rev
<400> 43
   ctctccttaa cgcgccagga actcgagcat ggcatgtcct tattatg 47
<210> 44
   <211> 21
   <212> DNA
   <213> Artificial Sequence
<220>
   <223> ltaE-DOWN_fw
<400> 44
   ttcctggcgc gttaaggaga g 21
<210> 45
   <211> 40
   <212> DNA
   <213> Artificial Sequence
<220>
   <223> ltaE-DOWN_rev
<400> 45
   ggatcgcggc cgctctagag tagaccatat cgcgcatgac 40
<210> 46
   <211> 540
   <212> DNA
   <213> Artificial Sequence
<220>
   <223> PCR product 4 from Example 15
<400> 46
   ggatcgcggc cgcggaccgc aggcgacaga gccagaacgt caggtggtcg ccatgtgcgg      60
   cgatggcggt tttagcatgt tgatgggcga tttcctctca gtagtgcaga tgaaactgcc     120
   agtgaaaatt gtcgtcttta acaacagcgt gctgggcttt gtggcgatgg agatgaaagc     180
   tggtggctat ttgactgacg gcaccgaact acacgacaca aactttgccc gcattgccga     240
   agcgtgcggc attacgggta tccgtgtaga aaaagcgtct gaagttgatg aagccctgca     300
   acgcgccttc tccatcgacg gtccggtgtt ggtggatgtg gtggtcgcca aagaagagtt     360
   agccattcca ccgcagatca aactcgaaca ggccaaaggt ttcagcctgt atatgctgcg     420
   cgcaatcatc agcggacgcg gtgatgaagt gatcgaactg gcgaaaacaa actggctaag     480
   gtaaaaaggg tggcatttcc cgtcataata aggacatgcc atgctcgagt tcctggcgcg     540
<210> 47
   <211> 536
   <212> DNA
   <213> Artificial Sequence
<220>
   <223> PCR product 5 from Example 15
<400> 47
   ttcctggcgc gttaaggaga gagacgtgcc gcaacgcatt ttagttctcg gtgccagtgg      60
   ctacattggt cagcatctgg tgcgcacact cagccagcaa gggcatcaga tcctggcggc     120
   ggcacgtcat gtcgacaggc ttgcaaagct gcaactggca aacgtcagtt gccataaagt     180
   cgatctcagc tggccggata acctaccggc cctgttgcag gatatcgata cggtctattt     240
   tctggtgcac agcatgggcg aaggcggcga ttttatcgct caggagcgcc aggtggctct     300
   caacgtccgc gatgcgctac gtgaagtacc agttaagcaa ttaatctttc ttagttcgtt     360
   gcaggccccg ccacatgagc agtcggatca tctgcgtgct cgtcaggcta cggcggacat     420
   tctgcgtgaa gcgaatgtac ccgtgaccga actgcgggcc ggaattatcg ttggcgcagg     480
   ttccgcggcg ttcgaagtca tgcgcgatat ggtctactct agagcggccg cgatcc         536
<210> 48
   <211> 26
   <212> DNA
   <213> Artificial Sequence
<220>
   <223> oligonucleotide o-MO-54 from Example 15
<400> 48
   actgaggatc cggatcgcgg ccgcgg 26
<210> 49
   <211> 29
   <212> DNA
   <213> Artificial Sequence
<220>
   <223> oligoneucleotide o-MO-55 from Example 15
<400> 49
   actgaggatc cggatcgcgg ccgctctag 29
<210> 50
   <211> 1071
   <212> DNA
   <213> Artificial Sequence
<220>
   <223> PCR product 6 from Example 15
<400> 50
   gatccggatc gcggccgcgg accgcaggcg acagagccag aacgtcaggt ggtcgccatg 60
   tgcggcgatg gcggttttag catgttgatg ggcgatttcc tctcagtagt gcagatgaaa 120
   ctgccagtga aaattgtcgt ctttaacaac agcgtgctgg gctttgtggc gatggagatg 180
   aaagctggtg gctatttgac tgacggcacc gaactacacg acacaaactt tgcccgcatt 240
   gccgaagcgt gcggcattac gggtatccgt gtagaaaaag cgtctgaagt tgatgaagcc 300
   ctgcaacgcg ccttctccat cgacggtccg gtgttggtgg atgtggtggt cgccaaagaa 360
   gagttagcca ttccaccgca gatcaaactc gaacaggcca aaggtttcag cctgtatatg 420
   ctgcgcgcaa tcatcagcgg acgcggtgat gaagtgatcg aactggcgaa aacaaactgg 480
   ctaaggtaaa aagggtggca tttcccgtca taataaggac atgccatgct cgagttcctg 540
   gcgcgttaag gagagagacg tgccgcaacg cattttagtt ctcggtgcca gtggctacat 600
   tggtcagcat ctggtgcgca cactcagcca gcaagggcat cagatcctgg cggcggcacg 660
   tcatgtcgac aggcttgcaa agctgcaact ggcaaacgtc agttgccata aagtcgatct 720
   cagctggccg gataacctac cggccctgtt gcaggatatc gatacggtct attttctggt 780
   gcacagcatg ggcgaaggcg gcgattttat cgctcaggag cgccaggtgg ctctcaacgt 840
   ccgcgatgcg ctacgtgaag taccagttaa gcaattaatc tttcttagtt cgttgcaggc 900
   cccgccacat gagcagtcgg atcatctgcg tgctcgtcag gctacggcgg acattctgcg 960
   tgaagcgaat gtacccgtga ccgaactgcg ggccggaatt atcgttggcg caggttccgc 1020
   ggcgttcgaa gtcatgcgcg atatggtcta ctctagagcg gccgcgatcc g 1071
<210> 51
   <211> 6738
   <212> DNA
   <213> Artificial Sequence
<220>
   <223> pKO3 delta ltaE
<400> 51
   cctttcgtct tcgaataaat acctgtgacg gaagatcact tcgcagaata aataaatcct 60
   ggtgtccctg ttgataccgg gaagccctgg gccaactttt ggcgaaaatg agacgttgat 120
   cggcacgtaa gaggttccaa ctttcaccat aatgaaataa gatcactacc gggcgtattt 180
   tttgagttat cgagattttc aggagctaag gaagctaaaa tggagaaaaa aatcactgga 240
   tataccaccg ttgatatatc ccaatggcat cgtaaagaac attttgaggc atttcagtca 300
   gttgctcaat gtacctataa ccagaccgtt cagctggata ttacggcctt tttaaagacc 360
   gtaaagaaaa ataagcacaa gttttatccg gcctttattc acattcttgc ccgcctgatg 420
   aatgctcatc cggaattccg tatggcaatg aaagacggtg agctggtgat atgggatagt 480
   gttcaccctt gttacaccgt tttccatgag caaactgaaa cgttttcatc gctctggagt 540
   gaataccacg acgatttccg gcagtttcta cacatatatt cgcaagatgt ggcgtgttac 600
   ggtgaaaacc tggcctattt ccctaaaggg tttattgaga atatgttttt cgtctcagcc 660
   aatccctggg tgagtttcac cagttttgat ttaaacgtgg ccaatatgga caacttcttc 720
   gcccccgttt tcaccatggg caaatattat acgcaaggcg acaaggtgct gatgccgctg 780
   gcgattcagg ttcatcatgc cgtttgtgat ggcttccatg tcggcagaat gcttaatgaa 840
   ttacaacagt actgcgatga gtggcagggc ggggcgtaat ttttttaagg cagttattgg 900
   tgcccttaaa cgcctggttg ctacgcctga ataagtgata ataagcggat gaatggcaga 960
   aattcgaaag caaattcgac ccggtcgtcg gttcagggca gggtcgttaa atagccgctt 1020
   atgtctattg ctggtctcgg tacccgggga tcgcggccgc ggaccggatc cggatcgcgg 1080
   ccgcggaccg caggcgacag agccagaacg tcaggtggtc gccatgtgcg gcgatggcgg 1140
   ttttagcatg ttgatgggcg atttcctctc agtagtgcag atgaaactgc cagtgaaaat 1200
   tgtcgtcttt aacaacagcg tgctgggctt tgtggcgatg gagatgaaag ctggtggcta 1260
   tttgactgac ggcaccgaac tacacgacac aaactttgcc cgcattgccg aagcgtgcgg 1320
   cattacgggt atccgtgtag aaaaagcgtc tgaagttgat gaagccctgc aacgcgcctt 1380
   ctccatcgac ggtccggtgt tggtggatgt ggtggtcgcc aaagaagagt tagccattcc 1440
   accgcagatc aaactcgaac aggccaaagg tttcagcctg tatatgctgc gcgcaatcat 1500
   cagcggacgc ggtgatgaag tgatcgaact ggcgaaaaca aactggctaa ggtaaaaagg 1560
   gtggcatttc ccgtcataat aaggacatgc catgctcgag ttcctggcgc gttaaggaga 1620
   gagacgtgcc gcaacgcatt ttagttctcg gtgccagtgg ctacattggt cagcatctgg 1680
   tgcgcacact cagccagcaa gggcatcaga tcctggcggc ggcacgtcat gtcgacaggc 1740
   ttgcaaagct gcaactggca aacgtcagtt gccataaagt cgatctcagc tggccggata 1800
   acctaccggc cctgttgcag gatatcgata cggtctattt tctggtgcac agcatgggcg 1860
   aaggcggcga ttttatcgct caggagcgcc aggtggctct caacgtccgc gatgcgctac 1920
   gtgaagtacc agttaagcaa ttaatctttc ttagttcgtt gcaggccccg ccacatgagc 1980
   agtcggatca tctgcgtgct cgtcaggcta cggcggacat tctgcgtgaa gcgaatgtac 2040
   ccgtgaccga actgcgggcc ggaattatcg ttggcgcagg ttccgcggcg ttcgaagtca 2100
   tgcgcgatat ggtctactct agagcggccg cgatccggat cctctagagc ggccgcgatc 2160
   ctctagagtc gaccggtggc gaatgggacg cgccctgtag cggcgcatta agcgcggcgg 2220
   gtgtggtggt tacgcgcagc gtgaccgcta cacttgccag cgccctagcg cccgctcctt 2280
   tcgctttctt cccttccttt ctcgccacgt tcgccggctt tccccgtcaa gctctaaatc 2340
   gggggctccc tttagggttc cgatttagtg ctttacggca cctcgacccc aaaaaacttg 2400
   attagggtga tggttcacgt agtgggccat cgccctgata gacggttttt cgccctttga 2460
   cgttggagtc cacgttcttt aatagtggac tcttgttcca aactggaaca acactcaacc 2520
   ctatctcggt ctattctttt gatttataag ggattttgcc gatttcggcc tattggttaa 2580
   aaaatgagct gatttaacaa aaatttaacg cgaattttaa caaaatatta acgcttacaa 2640
   tttaggtggc acttttcggg gaaatgtgcg cggaacccct atttgtttat ttttctaaat 2700
   acattcaaat atgtatccgc tcaccgcgat cctttttaac ccatcacata tacctgccgt 2760
   tcactattat ttagtgaaat gagatattat gatattttct gaattgtgat taaaaaggca 2820
   actttatgcc catgcaacag aaactataaa aaatacagag aatgaaaaga aacagataga 2880
   ttttttagtt ctttaggccc gtagtctgca aatcctttta tgattttcta tcaaacaaaa 2940
   gaggaaaata gaccagttgc aatccaaacg agagtctaat agaatgaggt cgaaaagtaa 3000
   atcgcgcggg tttgttactg ataaagcagg caagacctaa aatgtgtaaa gggcaaagtg 3060
   tatactttgg cgtcacccct tacatatttt aggtcttttt ttattgtgcg taactaactt 3120
   gccatcttca aacaggaggg ctggaagaag cagaccgcta acacagtaca taaaaaagga 3180
   gacatgaacg atgaacatca aaaagtttgc aaaacaagca acagtattaa cctttactac 3240
   cgcactgctg gcaggaggcg caactcaagc gtttgcgaaa gaaacgaacc aaaagccata 3300
   taaggaaaca tacggcattt cccatattac acgccatgat atgctgcaaa tccctgaaca 3360
   gcaaaaaaat gaaaaatatc aagttcctga gttcgattcg tccacaatta aaaatatctc 3420
   ttctgcaaaa ggcctggacg tttgggacag ctggccatta caaaacgctg acggcactgt 3480
   cgcaaactat cacggctacc acatcgtctt tgcattagcc ggagatccta aaaatgcgga 3540
   tgacacatcg atttacatgt tctatcaaaa agtcggcgaa acttctattg acagctggaa 3600
   aaacgctggc cgcgtcttta aagacagcga caaattcgat gcaaatgatt ctatcctaaa 3660
   agaccaaaca caagaatggt caggttcagc cacatttaca tctgacggaa aaatccgttt 3720
   attctacact gatttctccg gtaaacatta cggcaaacaa acactgacaa ctgcacaagt 3780
   taacgtatca gcatcagaca gctctttgaa catcaacggt gtagaggatt ataaatcaat 3840
   ctttgacggt gacggaaaaa cgtatcaaaa tgtacagcag ttcatcgatg aaggcaacta 3900
   cagctcaggc gacaaccata cgctgagaga tcctcactac gtagaagata aaggccacaa 3960
   atacttagta tttgaagcaa acactggaac tgaagatggc taccaaggcg aagaatcttt 4020
   atttaacaaa gcatactatg gcaaaagcac atcattcttc cgtcaagaaa gtcaaaaact 4080
   tctgcaaagc gataaaaaac gcacggctga gttagcaaac ggcgctctcg gtatgattga 4140
   gctaaacgat gattacacac tgaaaaaagt gatgaaaccg ctgattgcat ctaacacagt 4200
   aacagatgaa attgaacgcg cgaacgtctt taaaatgaac ggcaaatggt acctgttcac 4260
   tgactcccgc ggatcaaaaa tgacgattga cggcattacg tctaacgata tttacatgct 4320
   tggttatgtt tctaattctt taactggccc atacaagccg ctgaacaaaa ctggccttgt 4380
   gttaaaaatg gatcttgatc ctaacgatgt aacctttact tactcacact tcgctgtacc 4440
   tcaagcgaaa ggaaacaatg tcgtgattac aagctatatg acaaacagag gattctacgc 4500
   agacaaacaa tcaacgtttg cgccaagctt cctgctgaac atcaaaggca agaaaacatc 4560
   tgttgtcaaa gacagcatcc ttgaacaagg acaattaaca gttaacaaat aaaaacgcaa 4620
   aagaaaatgc cgatattgac taccggaagc agtgtgaccg tgtgcttctc aaatgcctga 4680
   ttcaggctgt ctatgtgtga ctgttgagct gtaacaagtt gtctcaggtg ttcaatttca 4740
   tgttctagtt gctttgtttt actggtttca cctgttctat taggtgttac atgctgttca 4800
   tctgttacat tgtcgatctg ttcatggtga acagctttaa atgcaccaaa aactcgtaaa 4860
   agctctgatg tatctatctt ttttacaccg ttttcatctg tgcatatgga cagttttccc 4920
   tttgatatgt aacggtgaac agttgttcta cttttgtttg ttagtcttga tgcttcactg 4980
   atagatacaa gagccataag aacctcagat ccttccgtat ttagccagta tgttctctag 5040
   tgtggttcgt tgtttttgcg tgagccatga gaacgaacca ttgagatcat acttactttg 5100
   catgtcactc aaaaattttg cctcaaaact ggtgagctga atttttgcag ttaaagcatc 5160
   gtgtagtgtt tttcttagtc cgttatgtag gtaggaatct gatgtaatgg ttgttggtat 5220
   tttgtcacca ttcattttta tctggttgtt ctcaagttcg gttacgagat ccatttgtct 5280
   atctagttca acttggaaaa tcaacgtatc agtcgggcgg cctcgcttat caaccaccaa 5340
   tttcatattg ctgtaagtgt ttaaatcttt acttattggt ttcaaaaccc attggttaag 5400
   ccttttaaac tcatggtagt tattttcaag cattaacatg aacttaaatt catcaaggct 5460
   aatctctata tttgccttgt gagttttctt ttgtgttagt tcttttaata accactcata 5520
   aatcctcata gagtatttgt tttcaaaaga cttaacatgt tccagattat attttatgaa 5580
   tttttttaac tggaaaagat aaggcaatat ctcttcacta aaaactaatt ctaatttttc 5640
   gcttgagaac ttggcatagt ttgtccactg gaaaatctca aagcctttaa ccaaaggatt 5700
   cctgatttcc acagttctcg tcatcagctc tctggttgct ttagctaata caccataagc 5760
   attttcccta ctgatgttca tcatctgaac gtattggtta taagtgaacg ataccgtccg 5820
   ttctttcctt gtagggtttt caatcgtggg gttgagtagt gccacacagc ataaaattag 5880
   cttggtttca tgctccgtta agtcatagcg actaatcgct agttcatttg ctttgaaaac 5940
   aactaattca gacatacatc tcaattggtc taggtgattt taatcactat accaattgag 6000
   atgggctagt caatgataat tactagtcct tttcctttga gttgtgggta tctgtaaatt 6060
   ctgctagacc tttgctggaa aacttgtaaa ttctgctaga ccctctgtaa attccgctag 6120
   acctttgtgt gttttttttg tttatattca agtggttata atttatagaa taaagaaaga 6180
   ataaaaaaag ataaaaagaa tagatcccag ccctgtgtat aactcactac tttagtcagt 6240
   tccgcagtat tacaaaagga tgtcgcaaac gctgtttgct cctctacaaa acagacctta 6300
   aaaccctaaa ggcttaagta gcaccctcgc aagctcgggc aaatcgctga atattccttt 6360
   tgtctccgac catcaggcac ctgagtcgct gtctttttcg tgacattcag ttcgctgcgc 6420
   tcacggctct ggcagtgaat gggggtaaat ggcactacag gcgcctttta tggattcatg 6480
   caaggaaact acccataata caagaaaagc ccgtcacggg cttctcaggg cgttttatgg 6540
   cgggtctgct atgtggtgct atctgacttt ttgctgttca gcagttcctg ccctctgatt 6600
   ttccagtctg accacttcgg attatcccgt gacaggtcat tcagactggc taatgcaccc 6660
   agtaaggcag cggtatcatc aacaggctta cccgtcttac tgtcggggat cgacgctctc 6720
   ccttatgcga ctcctgca 6738
<210> 52
   <211> 24
   <212> DNA
   <213> Artificial Sequence
<220>
   <223> DNA sequence after deletion of ltaE
<400> 52
   atgctcgagt tcctggcgcg ttaa 24
<210> 53
   <211> 294
   <212> PRT
   <213> Nomocharis pardanthina
<400> 53
   Met Leu Val Leu His Asn Ala Gln Lys Leu Gln Ile Leu Tyr Lys Ser
   1               5                   10                  15
   Leu Glu Lys Ser Ile Pro Glu Ser Ile Lys Val Tyr Gly Ala Ile Phe
               20                  25                  30
   Asn Ile Lys Asp Lys Asn Pro Phe Asn Met Glu Val Leu Val Asp Ala
           35                  40                  45
   Trp Pro Asp Tyr Gln Ile Val Ile Thr Arg Pro Gln Lys Gln Glu Met
       50                  55                  60
   Lys Asp Asp Gln Asp His Tyr Thr Asn Thr Tyr His Ile Phe Thr Lys
   65                  70                  75                  80
   Ala Pro Asp Lys Leu Glu Glu Val Leu Ser Tyr Ser Ser Val Ile Asn
                   85                  90                  95
   Trp Glu Gln Thr Leu Gln Ile Gln Gly Cys Gln Glu Gly Leu Asp Glu
               100                 105                 110
   Ala Ile Arg Lys Ala Ala Thr Ser Lys Ser Val Gln Val Asp Tyr Met
           115                 120                 125
   Lys Thr Met Leu Phe Ile Pro Glu Leu Pro Lys Lys His Lys Thr Ser
       130                 135                 140
   Ser Asn Glu Lys Met Glu Leu Phe Lys Val Asp Asp Asp Asn Lys Glu
   145                 150                 155                 160
   Gly Asn Phe Ser Asn Met Phe Leu Asp Ala Ser His Ala Gly Leu Val
                   165                 170                 175
   Asn Glu His Trp Ala Phe Gly Lys Asn Glu Arg Ser Leu Lys Tyr Ile
               180                 185                 190
   Glu Arg Cys Leu Gln Asp Phe Leu Gly Phe Gly Val Leu Gly Pro Glu
           195                 200                 205
   Gly Gln Leu Val Ser Trp Ile Val Met Glu Gln Ser Cys Glu Leu Arg
       210                 215                 220
   Met Gly Tyr Thr Val Pro Lys Tyr Arg His Gln Gly Asn Met Leu Gln
   225                 230                 235                 240
   Ile Gly Tyr His Phe Glu Lys Tyr Leu Ser Gln Lys Glu Ile Pro Phe
                   245                 250                 255
   Tyr Phe His Val Ala Asp Asn Asn Glu Lys Ser Leu Gln Ala Leu Lys
               260                 265                 270
   Asn Leu Gly Phe Lys Ile Cys Pro Cys Gly Trp His Gln Trp Lys Cys
           275                 280                 285
   Thr Pro Lys Lys Tyr Cys
       290
<210> 54
   <211> 297
   <212> PRT
   <213> Saimiri boliviensis
<400> 54
   Met Phe Val Leu His Asp Thr Gln Ile Leu Gln Thr Leu Tyr Lys Ser
   1               5                   10                  15
   Leu Glu Lys Arg Asn Pro Glu Ser Ile Lys Val Tyr Gly Ala Ile Phe
               20                  25                  30
   Asn Ile Lys Asn Lys Asn Pro Phe Asn Met Glu Val Leu Val Asp Ala
           35                  40                  45
   Trp Pro Asp Tyr Gln Thr Val Ile Ile Arg Pro Lys Lys Gln Glu Met
       50                  55                  60
   Lys Asp Asp Gln Asp His Tyr Thr Asn Thr Tyr His Ile Phe Thr Lys
   65                  70                  75                  80
   Ala Pro Asp Lys Leu Glu Glu Ile Leu Ser Cys Ser Glu Val Ile Asn
                   85                  90                  95
   Trp Glu Gln Thr Phe Gln Ile Gln Gly Cys Gln Glu Gly Leu Asn Glu
               100                 105                 110
   Ala Ile Arg Lys Val Ala Ala Ser Lys Thr Val Gln Val Asp Tyr Val
           115                 120                 125
   Lys Thr Val Leu Phe Lys Pro Glu Leu Leu Lys Glu His Lys Thr Ser
       130                 135                 140
   Ser Asn Asp Lys Met Glu Leu Phe Glu Met His Lys Val Asp Asp Asn
   145                 150                 155                 160
   Asn Lys Lys Arg Asn Phe Ser Ser Met Phe Leu Asp Ala Ser His Ala
                   165                 170                 175
   Gly Leu Ile Asn Glu His Trp Ala Phe Gly Lys Asn Glu Arg Ser Leu
   180 185 190
   Lys Tyr Thr Glu Arg Cys Leu Gln Asp Phe Leu Gly Phe Gly Val Leu
   195 200 205
   Gly Pro Glu Gly Glu Leu Ile Ser Trp Val Val Met Glu Gln Ser Cys
   210 215 220
   Glu Leu Arg Met Gly Tyr Thr Ile Pro Lys Tyr Arg Asn Gln Arg His
   225 230 235 240
   Met Ser Gln Ile Leu Cys Tyr Leu Glu Lys Tyr Leu Ser Gln Lys Glu
   245 250 255
   Ile Pro Phe Tyr Ile His Val Ala Asp Tyr Asn Val Ala Gly Leu Gln
   260 265 270
   Ile Met Lys Asn Met Gly Tyr Met Ile Cys Pro Cys Gly Trp His Gln
   275 280 285
   Trp Lys Cys Thr Pro Lys Lys Tyr Cys
   290 295
<210> 55
   <211> 298
   <212> PRT
   <213> Felis catus
<400> 55
   Met Phe Val Leu His Glu Ser Gln Lys Leu His Ile Leu Tyr Glu Ser
   1 5 10 15
   Leu Glu Lys Ser Ile Pro Glu Ser Leu Lys Val Tyr Gly Ala Val Phe
   20 25 30
   Asn Ile Lys Asn Lys Asn Pro Phe Asn Met Glu Val Leu Val Asp Ala
   35 40 45
   Trp Pro Asp Tyr Gln Ile Val Ile Thr Arg Pro Gln Arg Glu Glu Met
   50 55 60
   Lys Asp Asp Leu Asp His Tyr Thr Asn Thr Tyr Gln Ile Phe Thr Lys
   65 70 75 80
   Ala Pro Asp Lys Leu Glu Glu Val Leu Ala Gln Pro Gln Leu Ile Asn
   85 90 95
   Trp Asp Gln Val Phe Gln Ile Gln Gly Cys Gln Glu Ser Leu Gln Glu
   100 105 110
   Ala Ile Arg Lys Ile Ser Ala Ser Lys Ser Val Arg Val Asp Tyr Val
   115 120 125
   Lys Thr Ile Arg Ile Met Thr Glu Met Pro Val Lys Pro Asn Asp Asp
   130 135 140
   Gln Val Asp Val Met Glu Ser Phe Lys Met Pro Lys Val Asp Asp Asp
   145 150 155 160
   Thr Lys Lys Gly Ser Phe Gln Asn Ile Ser Leu Asp Pro Ser His Ala
   165 170 175
   Gly Leu Val Asn Glu Gln Trp Ala Phe Gly Lys Asn Asp Arg Ser Leu
   180 185 190
   Lys Tyr Ile Glu Arg Cys Leu Gln Asn Phe Pro Gly Phe Gly Val Leu
   195 200 205
   Gly Pro Glu Gly Arg Leu Ile Ser Trp Ile Val Met Glu Gln Ser Cys
   210 215 220
   Glu Met Arg Met Gly Tyr Thr Val Pro Glu Tyr Arg Lys His Gly Tyr
   225 230 235 240
   Met Lys Lys Thr Ser Val His Leu Met Asn Tyr Val Ile Gln Lys Lys
   245 250 255
   Val Pro Cys Tyr Phe His Val Ser Glu His Lys Glu Glu Tyr Asn Gln
   260 265 270
   Ala Leu Arg Asp Leu Lys Phe Lys Ala Ala Ser Cys Gly Trp His Gln
   275 280 285
   Trp Lys Cys Ser Pro Glu Lys Tyr Val Asp
   290 295
<210> 56
   <211> 299
   <212> PRT
   <213> Bos taurus
<400> 56
   Met Phe Val Leu Arg Glu Pro Gln Gln Leu Gln Ile Leu Tyr Glu Ser
   1 5 10 15
   Leu Glu Glu Ser Ile Pro Glu Ser Leu Lys Val Tyr Gly Thr Leu Phe
               20                  25                  30
   His Ile Arg Asn Lys Asn Pro Phe Asn Leu Glu Val Leu Val Asp Ala
           35                  40                  45
   Trp Pro Glu Tyr Gln Thr Val Val Ile Arg Pro Gln Lys Glu Glu Met
       50                  55                  60
   Lys Asp Asp Leu Asp Tyr Tyr Thr Asn Thr Tyr His Ile Phe Thr Lys
   65                  70                  75                  80
   Ala Pro Asp Lys Leu Glu Glu Val Leu Ala Cys Pro Gln Val Ile Asn
                   85                  90                  95
   Trp Glu Gln Ala Phe Gln Ile Gln Gly Cys Gln Glu Ser Leu Gly Glu
               100                 105                 110
   Ala Ile Gln Lys Val Ala Ala Ser Lys Ser Val Gln Val Asp Tyr Leu
           115                 120                 125
   Arg Thr Val Leu Phe Val Phe Glu Lys Pro Thr Leu Glu Ala Ser Ser
       130                 135                 140
   Gly Asp Lys Met Asp Leu Met Lys Leu Leu Lys Ile Pro Lys Val Leu
   145                 150                 155                 160
   Glu Asp Lys Arg Lys Glu Thr Phe Gln Asn Ile Phe Leu Asp Val Ser
                   165                 170                 175
   His Ala Arg Leu Val Asn Glu His Trp Glu Leu Gly Lys Asn Glu Lys
               180                 185                 190
   Ser Leu Lys Tyr Val Glu Arg Cys Leu Gln Asn Phe Ala Gly Phe Gly
           195                 200                 205
   Val Leu Ser Ser Glu Gly Lys Pro Ile Ser Trp Phe Leu Thr Glu Gln
       210                 215                 220
   Ser Cys Glu Ile Arg Met Gly Tyr Thr Phe Pro Lys Tyr Arg Gly Gln
   225                 230                 235                 240
   Gly His Met Trp Gln Met Gly Cys His Ser Ile Ala Tyr Phe Phe Ser
                   245                 250                 255
   Lys Lys Ile Pro Phe Tyr Ile His Ala Ala Glu Asp Lys Glu Lys Thr
               260                 265                 270
   Gln Gln Thr Leu Leu Ser Phe Gly Phe Lys Asn Val Leu Cys Gly Trp
   275 280 285
   His Gln Trp Lys Cys Thr Pro Lys Lys Tyr Cys
   290 295
<210> 57
   <211> 296
   <212> PRT
   <213> Mus musculus
<400> 57
   Met Ile Val Pro Leu Gln Gly Ala Gln Met Leu Gln Met Leu Glu Lys
   1 5 10 15
   Ser Leu Arg Lys Tyr Leu Pro Glu Ser Leu Lys Val Tyr Gly Thr Val
   20 25 30
   Tyr His Met Ile His Gly Asn Pro Phe Asn Leu Lys Ala Leu Val Asp
   35 40 45
   Lys Trp Pro Asp Phe Asn Thr Val Val Val Arg Pro Gln Glu Gln Glu
   50 55 60
   Met Thr Asp Asp Leu Asp Phe Tyr Ile Asn Thr Tyr Gln Val Tyr Ser
   65 70 75 80
   Lys Asp Pro Gln Asn Cys Gln Glu Phe Leu Glu Ser Ser Glu Val Ile
   85 90 95
   Asn Trp Lys Gln His Leu Gln Ile Gln Ser Ser Gln Ser His Leu Asn
   100 105 110
   Lys Thr Ile Gln Asn Leu Ala Ser Ile Gln Ser Phe Gln Ile Lys His
   115 120 125
   Ser Glu Asn Ile Leu Tyr Val Ser Ser Glu Thr Ile Lys Lys Leu Phe
   130 135 140
   Pro Ser Leu Leu Asp Thr Lys Asn Leu Ser Thr Gly Ser Gly Lys Pro
   145 150 155 160
   Lys Ala Ile Asp Gln Asp Lys Phe Lys Leu Ser Ser Leu Asp Val Val
   165 170 175
   His Ala Ala Leu Val Asn Lys Phe Trp Leu Phe Gly Gly Asn Glu Arg
   180 185 190
   Ser Gln Arg Phe Ile Glu Arg Cys Ile Lys Asn Phe Pro Ser Ser Cys
           195                 200                 205
   Val Leu Gly Pro Glu Gly Thr Pro Ala Ser Trp Thr Leu Met Asp Gln
       210                 215                 220
   Thr Gly Glu Met Arg Met Gly Gly Thr Met Pro Glu Tyr Arg Leu Gln
   225                 230                 235                 240
   Gly Leu Val Ser Phe Val Val His Ser Gln Asp Gln Ile Met Thr Lys
                   245                 250                 255
   Arg Gly Tyr Pro Val Tyr Ser His Thr Glu Lys Ser Asn Ile Ala Met
               260                 265                 270
   Gln Lys Met Ser Tyr Thr Leu Gln His Leu Pro Met Pro Cys Ala Trp
           275                 280                 285
   Asn Gln Trp Lys Cys Met Pro Met
       290                 295